# EUROPEAN PATENT APPLICATION

(11) **EP 3 763 826 A1**
(43) Date of publication of application: **13.01.2021**
(21) Application number: 19757489.0
(22) Date of filing: 21.02.2019
(51) Int. Cl.: C12Q 1/6869, C12M 1/00, C12Q 1/6806, C12Q 1/6872, C12Q 1/6888, G01N 33/50, C12N 15/09, C12N 15/10

(54) **ANALYSIS/DIAGNOSIS METHOD UTILIZING RNA MODIFICATION**

(30) Priority: 22.02.2018 JP 2018030099
(71) Applicant: Osaka University, Suita-shi, Osaka 565-0871 (JP)
(72) Inventor: ISHII, Hideshi, Suita-shi, Osaka 565-0871 (JP); KONNO, Masamitsu, Suita-shi, Osaka 565-0871 (JP); MORI, Masaki, Suita-shi, Osaka 565-0871 (JP)
(74) Representative: Uexküll & Stolberg
(86) International application number: PCT/JP2019/006588
(87) International publication number: WO 2019/163900

(57) **Abstract**

The present application provides a novel method for analyzing a biological condition or a medical condition. It is now found that information about the modification of RNA can be used for the analysis of a biological condition or a medical condition. On the basis of this finding, the present invention provides a novel method for analyzing a biological condition or a medical condition. According to the present invention, it becomes possible to analyze various biological conditions or medical conditions including diseases, and it also becomes possible to satisfactorily predict cancer (e.g., pancreatic cancer, colorectal cancer, stomach cancer) in an early stage.

SEQUENCE LISTING

<110>  Method of analysis and diagnose using RNA modification

<120>  OSAKA UNIVERSITY

<130>  F518PCT157

<150>  JP 2018-30099
<151>  2018-02-22

<160>  66

<170>  PatentIn version 3.5

<210>  1
<211>  23
<212>  RNA
<213>  Homo sapiens

<400>  1
caaagugcuu acagugcagg uag                                              23


<210>  2
<211>  22
<212>  RNA
<213>  Homo sapiens

<400>  2
uagcuuauca gacugauguu ga                                               22


<210>  3
<211>  22
<212>  RNA
<213>  Homo sapiens

<400>  3
cgucuuaccc agcaguguuu gg                                               22


<210>  4
<211>  23
<212>  RNA
<213>  Homo sapiens

<400>  4
uaauacugcc ggguaaugau gga                                              23


<210>  5
<211>  22
<212>  RNA
<213>  Homo sapiens

<400>  5
ugagguagua gguuguauag uu                                               22


<210>  6
<211>  23
<212>  DNA

<213>  artificial sequence

<220>
<223>  Capture 17-5p

<400>  6
ctacctgcac tgtaagcact ttg                                              23


<210>  7
<211>  22
<212>  DNA
<213>  artificial sequence

<220>
<223>  Capture 21-5p

<400>  7
tcaacatcag tctgataagc ta                                               22


<210>  8
<211>  22
<212>  DNA
<213>  artificial sequence

<220>
<223>  Capture 200c-5p

<400>  8
ccaaacactg ctgggtaaga cg                                               22


<210>  9
<211>  23
<212>  DNA
<213>  artificial sequence

<220>
<223>  Capture 200c-3p

<400>  9
tccatcatta cccggcagta tta                                              23


<210>  10
<211>  22
<212>  DNA
<213>  artificial sequence

<220>
<223>  Capture let7a-5p

<400>  10
aactatacaa cctactacct ca                                               22


<210>  11
<211>  22
<212>  RNA
<213>  artificial sequence

<220>

<223>  Synthetic miR-200c-5p

<220>
<221>  modified_base
<222>  (7)..(7)
<223>  methylated

<220>
<221>  modified_base
<222>  (13)..(13)
<223>  methylated

<400>  11
cgucuuaccc agcaguguuu gg                                                22

<210>  12
<211>  21
<212>  DNA
<213>  artificial sequence

<220>
<223>  Complementary DNA 369-3p

<400>  12
aataatacat ggttgatctt t                                                 21

<210>  13
<211>  21
<212>  DNA
<213>  artificial sequence

<220>
<223>  Complementary antisense DNA 369-3p

<400>  13
aaagatcaac catgtattat t                                                 21

<210>  14
<211>  21
<212>  RNA
<213>  artificial sequence

<220>
<223>  Synthetic miR-369-3p

<400>  14
aauaauacau gguugaucuu u                                                 21

<210>  15
<211>  22
<212>  RNA
<213>  Homo sapiens

<220>
<221>  modified_base
<222>  (9)..(9)
<223>  methylated

<400>  15
uagcuuauca gacugauguu ga                                                22

<210>  16
<211>  23
<212>  RNA
<213>  Homo sapiens

<220>
<221>  modified_base
<222>  (13)..(13)
<223>  methylated

<400>  16
caaagugcuu acagugcagg uag                                               23

<210>  17
<211>  22
<212>  RNA
<213>  Homo sapiens

<220>
<221>  modified_base
<222>  (19)..(19)
<223>  methylated

<400>  17
ugagguagua gguuguauag uu                                                22

<210>  18
<211>  23
<212>  RNA
<213>  Homo sapiens

<220>
<221>  modified_base
<222>  (9)..(9)
<223>  methylated

<400>  18
uaauacugcc ggguaaugau gga                                               23

<210>  19
<211>  22
<212>  RNA
<213>  Homo sapiens

<220>
<221>  modified_base
<222>  (13)..(13)
<223>  methylated

<400>  19
cgucuuaccc agcaguguuu gg                                                22

<210>  20
<211>  22
<212>  RNA
<213>  Homo sapiens

<400>  20
acuggacuug gagucagaag gc                                                22


<210>  21
<211>  20
<212>  RNA
<213>  Homo sapiens

<400>  21
acuggacuug gagucagaaa                                                   20


<210>  22
<211>  19
<212>  RNA
<213>  Homo sapiens

<400>  22
acuggacuug gagucagga                                                    19


<210>  23
<211>  20
<212>  RNA
<213>  Homo sapiens

<400>  23
acuggacuug gagccagaag                                                   20


<210>  24
<211>  21
<212>  RNA
<213>  Homo sapiens

<400>  24
acuggacuug gugucagaug g                                                 21


<210>  25
<211>  21
<212>  RNA
<213>  Homo sapiens

<400>  25
acuggacuag gagucagaag g                                                 21


<210>  26
<211>  23
<212>  RNA
<213>  Homo sapiens

<400>  26
aggaccugcg ggacaagauu cuu                                               23

<210>  27
<211>  23
<212>  RNA
<213>  Homo sapiens

<400>  27
accuggaccc agcguagaca aag                                                23


<210>  28
<211>  23
<212>  RNA
<213>  Homo sapiens

<400>  28
augcggaccu ggguuagcgg agu                                                23


<210>  29
<211>  22
<212>  RNA
<213>  Homo sapiens

<400>  29
acuggguagg uggggcucca gg                                                 22


<210>  30
<211>  22
<212>  RNA
<213>  Homo sapiens

<400>  30
guugggacaa gaggacgguc uu                                                 22


<210>  31
<211>  23
<212>  RNA
<213>  Homo sapiens

<400>  31
ucgaggacug guggaagggc cuu                                                23


<210>  32
<211>  22
<212>  RNA
<213>  Homo sapiens

<400>  32
ggcucaugug ucuguccucu uc                                                 22


<210>  33
<211>  21
<212>  RNA
<213>  Homo sapiens

<400>  33
uccccuccac uuuccuccua g                                                  21

<210>  34
<211>  23
<212>  RNA
<213>  Homo sapiens

<400>  34
   uggggggaca gauggagagg aca                                                23


<210>  35
<211>  22
<212>  RNA
<213>  Homo sapiens

<400>  35
ccaguauuaa cugugcugcu ga                                                22


<210>  36
<211>  23
<212>  RNA
<213>  Homo sapiens

<400>  36
uaggcagugu cauuagcuga uug                                               23


<210>  37
<211>  22
<212>  RNA
<213>  Homo sapiens

<400>  37
agaucgaccg uguuauauuc gc                                                22


<210>  38
<211>  22
<212>  RNA
<213>  Homo sapiens

<400>  38
ugucuugcag gccgucaugc ag                                                22


<210>  39
<211>  22
<212>  RNA
<213>  Homo sapiens

<400>  39
ugaaacauac acgggaaacc uc                                                22


<210>  40
<211>  25
<212>  RNA
<213>  Homo sapiens

<400>  40
ccuccaaagg gaagcgcuuu cuguu                                             25

<210>  41
<211>  19
<212>  RNA
<213>  Homo sapiens

<400>  41
aucgggcccu cggcgccgg                                                    19


<210>  42
<211>  22
<212>  RNA
<213>  Homo sapiens

<400>  42
auggccagag cucacacaga gg                                                22


<210>  43
<211>  22
<212>  RNA
<213>  Homo sapiens

<400>  43
uggcggcggu aguuaugggc uu                                                22


<210>  44
<211>  22
<212>  RNA
<213>  Homo sapiens

<400>  44
agccuuccag gagaaaugga ga                                                22


<210>  45
<211>  18
<212>  RNA
<213>  Homo sapiens

<400>  45
auggucaccu ccgggacu                                                     18


<210>  46
<211>  22
<212>  RNA
<213>  Homo sapiens

<400>  46
guucucccaa cguaagccca gc                                                22


<210>  47
<211>  22
<212>  RNA
<213>  Homo sapiens

<400>  47
uagcagcacg uaaauauugg cg                                                22

<210>  48
<211>  22
<212>  RNA
<213>  Homo sapiens

<400>  48
gggacccagg gagagacgua ag                                                22


<210>  49
<211>  23
<212>  RNA
<213>  Homo sapiens

<400>  49
gugcuucauc guaauuaacc uua                                               23


<210>  50
<211>  10
<212>  RNA
<213>  Homo sapiens

<400>  50
gggaggugug                                                              10


<210>  51
<211>  20
<212>  RNA
<213>  Homo sapiens

<400>  51
ggggaggugu gcagggcugg                                                   20


<210>  52
<211>  22
<212>  RNA
<213>  Homo sapiens

<400>  52
gggaggugug aucucacacu cg                                                22


<210>  53
<211>  22
<212>  RNA
<213>  Homo sapiens

<400>  53
cugggaggug ugauaucgug gu                                                22


<210>  54
<211>  22
<212>  RNA
<213>  Homo sapiens

<400>  54
cugggaggug ugauauugug gu                                                22

<210>  55
<211>  22
<212>  RNA
<213>  Homo sapiens

<400>  55
uggggaggug uggagucagc au                                                22


<210>  56
<211>  10
<212>  RNA
<213>  Homo sapiens

<400>  56
cucccugccc                                                              10


<210>  57
<211>  20
<212>  RNA
<213>  Homo sapiens

<400>  57
uccccuuccu cccugcccag                                                   20


<210>  58
<211>  23
<212>  RNA
<213>  Homo sapiens

<400>  58
ccucccugcc cgccucucug cag                                               23


<210>  59
<211>  23
<212>  RNA
<213>  Homo sapiens

<400>  59
agccgcucuu cucccugccc aca                                               23


<210>  60
<211>  12
<212>  RNA
<213>  Homo sapiens

<400>  60
gacuuggagu ca                                                           12


<210>  61
<211>  25
<212>  RNA
<213>  Homo sapiens

<400>  61
acuggacuug gagucagaag agugg                                             25

<210>  62
<211>  13
<212>  RNA
<213>  Homo sapiens

<400>  62
cccuuuagag ugu                                                          13


<210>  63
<211>  22
<212>  RNA
<213>  Homo sapiens

<400>  63
aacgcacuuc ccuuuagagu gu                                                22


<210>  64
<211>  22
<212>  RNA
<213>  Homo sapiens

<400>  64
aucgugcauc ccuuuagagu gu                                                22


<210>  65
<211>  22
<212>  RNA
<213>  Homo sapiens

<400>  65
aaaaugguuc ccuuuagagu gu                                                22


<210>  66
<211>  24
<212>  RNA
<213>  Homo sapiens

<400>  66
acaaagugcu ucccuuuaga gugu                                              24

## Description

### [Technical Field]

The present invention relates to a characteristic analysis method and classification of biological subjects. More specifically, the present invention relates to classification and characteristic analysis methodology of biological subjects based on modification information on an RNA.

### [Background Art]

A ribonucleic acid (RNA) is a molecule bearing information of an organism in the same manner as deoxyribonucleic acid (DNA). While it is known that the function of a DNA is regulated by a modification such as methylation, a case of such a modification was also reported recently for RNAs.

To analyze various biological conditions including diseases, attempts have been made to associate information such as DNA mutation, mRNA expression level, or protein expression level with a biological condition, but early stage cancer and the like often cannot be sufficiently predicted by using such information.

### [Citation List]

### [Non Patent Literature]

[NPL 1] Pagliarini DJ, Cell Metab. 2016 Jul 12; 24(1): 13-4. doi: 10.1016/j.cmet.2016.06.018.

### [Summary of Invention]

### [Solution to Problem]

The present invention provides a novel method for analyzing a biological condition or medical condition based on the findings that RNA modification information can be used to analyze a biological condition or medical condition.

Therefore, the present invention provides the following.

### (Diagnostic method)

### (Item A1)

A method of analyzing a condition of a subject, comprising: obtaining modification information on at least one type of RNA <RNA mod> in a subject; and analyzing a condition of the subject based on the modification information.

### (Item A2)

The method of any one of the preceding items, wherein the RNA comprises a microRNA.

### (Item A3)

The method of any one of the preceding items, wherein the modification comprises methylation.

### (Item A3-1)

The method of any one of the preceding items, wherein the modification comprises methylation on a nucleoside.

### (Item A3-2)

The method of any one of the preceding items, wherein the modification comprises methylation on a nucleobase.

### (Item A4)

The method of any one of the preceding items, wherein a modification on the RNA comprises methylation of a microRNA.

### (Item A4-1)

The method of any one of the preceding items, wherein the modification information on the RNA comprises methylation on a nucleoside of a microRNA.

### (Item A4-2)

The method of any one of the preceding items, wherein the modification information on the RNA comprises methylation of a nucleobase of a microRNA.

### (Item A5)

The method of any one of the preceding items, wherein the modification is m⁶A.

### (Item A6)

The method of any one of the preceding items, wherein the modification information comprises modified location information.

### (Item A7)

The method of any one of the preceding items, wherein the modification information comprises information on an amount of RNA.

### (Condition)

### (Item A8)

The method of any one of the preceding items, wherein the condition is a medical condition or a biological condition.

### (Item A9)

The method of any one of the preceding items, wherein the condition is a condition of a microorganism (for example, enterobacteria or epidermal bacteria) in the subject.

### (Item A10)

The method of any one of the preceding items, wherein the biological condition is senescence or a differentiation status of a cell.

### (Item A11)

The method of any one of the preceding items, wherein the medical condition comprises cancer, inflammatory bowel disease, or intestinal tract immunity.

### (Item A12)

The method of any one of the preceding items, wherein the cancer comprises at least one of pancreatic cancer (e.g., early stage pancreatic cancer), liver cancer, gallbladder cancer, bile duct cancer, gastric cancer, and colon cancer.

### (Item A13)

The method of any one of the preceding items, wherein the condition is responsiveness of the subject to an agent or a treatment.

### (Agent and treatment)

### (Item A14)

The method of any one of the preceding items, wherein the treatment is a radiation treatment or a surgery.

### (Item A15)

The method of any one of the preceding items, wherein the treatment is a treatment using a heavy particle beam (e.g., Carbon/HIMAC) or an X-ray.

### (Item A16)

The method of any one of the preceding items, wherein the agent is an anticancer agent, a molecularly targeted drug, an antibody drug, a biological formulation (e.g., nucleic acid or protein), or an antibiotic.

### (Item A17)

The method of any one of the preceding items, wherein the agent is Lonsurf (TAS 102), gemcitabine, CDDP, 5-FU, cetuximab, a nucleic acid drug, or a histone demethylase inhibitor.

### (Item A18)

The method of any one of the preceding items, wherein the agent is an anticancer agent, and the responsiveness comprises responsiveness as to whether the subject is resistant to the anticancer agent.

### (Item A19)

The method of any one of the preceding items, wherein an agent for treating the subject and/or a treatment for the subject is indicated based on the responsiveness.

### (Item A20)

The method of any one of the preceding items, wherein an agent for treating the condition is indicated from a plurality of agents based on the responsiveness to the plurality of agents.

### (Item A21)

The method of any one of the preceding items, wherein the analysis is based on a comparison of the modification information in the subject before and after administration of the agent or the treatment.

### (Peripheral information)

### (Item A22)

The method of any one of the preceding items, comprising analyzing the condition of the subject based further on at least one piece of information selected from the group consisting of age, sex, race, familial information, medical history, treatment history, status of smoking, status of drinking, occupational information, information on living environment, disease marker information, nucleic acid information (including nucleic acid information on bacteria in the subject), metabolite information, protein information, enterobacterial information, epidermal bacterial information, and a combination thereof of the subject.

### (Item A23)

The method of any one of the preceding items, wherein the nucleic acid information is selected from the group consisting of genomic information, epigenomic information, transcriptome expression level information, RIP sequencing information, microRNA expression level information, and a combination thereof.

### (Item A24)

The method of any one of the preceding items, wherein the RIP sequencing information comprises RIP sequencing information on an agent resistant pump P-glycoprotein.

### (Item A25)

The method of any one of the preceding items, wherein RIP sequencing information comprises RIP sequencing information on a stool of the subject.

### (Item A26)

The method of any one of the preceding items, wherein the RIP sequencing information comprises RIP sequencing information on E. coli in a stool of the subject.

### (Additional information for modification information)

### (Item A27-1)

The method of any one of the preceding items, comprising analyzing the condition of the subject based further on modification information on the RNA in an agent and treatment resistant strain, or a combination of the resistant strain and a cell strain from which the resistant strain is derived.

### (Item A27-2)

The method of any one of the preceding items, wherein the agent or treatment comprises Lonsurf (TAS 102), gemcitabine, CDDP, 5-FU, cetuximab, a nucleic acid drug, a histone demethylase inhibitor, or a treatment using a heavy particle beam (e.g., Carbon/HIMAC) or an X-ray.

### (Item A28)

The method of any one of the preceding items, which analyzes modification information on at least 2000 types of RNAs.

### (Item A29)

The method of any one of the preceding items, comprising calculating a probability of the condition based on a plurality of pieces of the modification information.

### (Item A30)

The method of any one of the preceding items, wherein the modification information comprises a plurality of pieces of modification information on RNAs comprising the same sequence.

### (Item A31)

The method of any one of the preceding items, comprising analyzing the condition of the subject based further on structural information of an RNA.

### (Item A32-1)

The method of any one of the preceding items, comprising analyzing the condition of the subject based further on modification information on an RNA in an organism with a knockdown of at least one of a methylase (e.g., Mettl3, Mett114, or Wtap), a demethylase (e.g., FTO or AlkBH5), and a methylation recognizing enzyme (e.g., family molecule with a YTH domain such as YTHDF1, YTHDF2, or YTHDF3).

### (Item A32-2)

The method of any one of the preceding items, comprising analyzing the condition of the subject based further on recognition motif information on at least one of a methylase (e.g., Mettl3, Mett114, or Wtap), a demethylase (e.g., FTO or AlkBH5), and methylation recognizing enzyme (e.g., family molecule with a YTH domain such as YTHDF1, YTHDF2, or YTHDF3).

### (Item A33)

The method of any one of the preceding items, comprising analyzing a primary component.

### (Sample)

### (Item A34) <business of analyzing a sample obtained with a commercially available kit>

The method of any one of the preceding items, wherein the modification information is obtained from a sent sample derived from the subject.

### (Item A35)

The method of any one of the preceding items, wherein the sample is cryo-transported.

### (Item A36)

The method of any one of the preceding items, wherein a sample obtained from the subject onsite is analyzed onsite.

### (Item A37-1)

The method of any one of the preceding items, wherein the sample comprises at least one of blood, a biopsy sample (e.g., liquid biopsy sample), an oral mucous membrane, saliva, sweat, tear, urine, stool, and skin epidermis.

### (Item A37-2)

The method of any one of the preceding items, wherein the RNA comprises an RNA derived from a microorganism in the subject.

### (Purification)

### (Item A38)

The method of any one of the preceding items, comprising purifying the RNA by purification means.

### (Item A39)

The method of any one of the preceding items, comprising purifying a modified RNA.

### (Item A40)

The method of any one of the preceding items, wherein the purification means comprises a nucleic acid that is at least partially complementary to the RNA.

### (Item A41)

The method of any one of the preceding items, wherein the purification means comprises an antibody.

### (Item A42)

The method of any one of the preceding items, wherein the antibody is specific to the modification.

### (Item A43)

The method of any one of the preceding items, wherein the antibody is specific to the RNA.

### (Item A44)

The method of any one of the preceding items, wherein the antibody is specific to the RNA that has been modified.

### (Item A45)

The method of any one of the preceding items, wherein the purification means is placed at a predetermined location of a plate.

### (Item A46)

The method of any one of the preceding items, wherein a predetermined location of the plate is determined by a Monte Carlo method.

### (Measurement means)

### (Item A47)

The method of any one of the preceding items, wherein the modification information is obtained by PCR.

### (Item A48)

The method of any one of the preceding items, wherein the modification information is obtained by mass spectrometry.

### (Item A49)

The method of any one of the preceding items, wherein the modification information is obtained by MALDI-MS.

### (Pretreatment for measurement)

### (Item A50)

The method of any one of the preceding items, comprising treating the RNA with bromoacetaldehyde or chloroacetaldehyde.

### (Item A51)

The method of any one of the preceding items, using a coating agent comprising 3-hydroxypicolinic acid.

### (Food testing method)

### (Item B1)

The method of any one of the preceding items, wherein the subject is food.

### (Item B2)

The method of any one of the preceding items, wherein the food is meat.

### (Item B3)

The method of any one of the preceding items, wherein a condition of the subject is quality of food.

### (Item B4)

The method of any one of the preceding items, wherein the quality of food is a production region, age, time since processing, denaturation after processing, quality of taste, status of active oxygen, status of fatty acid, or degree of maturation of the food.

### (Item B5)

The method of any one of the preceding items, comprising analyzing the condition of the subject based further on information on a metabolite.

### (Method of classifying species)

### (Item C1)

The method of any one of the preceding items, wherein the condition is classification of species.

### (Item C2)

The method of any one of the preceding items, wherein the condition is classification of species of at least one type of microorganism in a microorganism population.

### (Item C3)

The method of any one of the preceding items, wherein the condition is classification of specifies of koji yeast.

### (System)

### (Item D1)

A system for determining a condition of a subject based on RNA modification information, comprising:
a measurement unit for measuring a modification condition of an RNA;
a calculation unit for calculating a modification condition on an RNA based on a result of the measurement; and
an analysis/determination unit for analyzing/determining the condition of the subject based on the modification condition.

### (Item D2)

The system of any one of the preceding items, wherein the measurement unit is a mass spectrometer.

### (Item D3)

The system of any one of the preceding items, wherein the measurement unit is MALDI-MS.

### (Item D4)

The system of any one of the preceding items, comprising a feature of any one of items A2 to A12.

### (Measurement device (plate))

### (Item E1)

A device for determining a condition of a subject based on RNA modification information, wherein
the device comprises a placement unit for placing at least one type of RNA purified from a sample derived from the subject,
the placement unit is configured to be read by a detector and to provide modification information on the at least one type of RNA, and
the condition of the subject is determined based on the modification information.

### (Item E2)

The device of any one of the preceding items for mass spectrometry.

### (Item E3)

The device of any one of the preceding items for MALDI-MS.

### (Beads for concentration)

### (Item F1)

A composition for purifying an RNA for determining a condition of a subject based on RNA modification information, wherein
the composition comprises means for capturing at least one type of RNA in the subject,
the RNA that has been captured is read out by a detector and provides modification information on the RNA, and
the condition of the subject is determined based on the modification information.

### (Item F2)

The composition of any one of the preceding items, wherein the means comprises a nucleic acid that is at least partially complementary to the RNA.

### (Item F3)

The composition of any one of the preceding items, wherein the means is means for capturing a modified RNA.

### (Item F4)

The composition of any one of the preceding items, wherein the means comprises a molecule that is specific to a modified RNA.

### (Item F5)

The composition of any one of the preceding items, wherein the means comprises a molecule that is specific to the RNA that has been modified.

### (Item F6)

The composition of any one of the preceding items, wherein the molecule comprises an antibody.

### (Item F7)

The composition of any one of the preceding items, wherein the means comprises a magnetic carrier.

### (Kit)

### (Item H1)

A kit for determining a condition of a subject based on RNA modification information,
wherein the kit comprises at least one of the composition of any one of the preceding items and instrument for obtaining a sample from the subject, and descriptions for using at least one of the composition and the instrument.

### (Item H2)

The kit of any one of the preceding items, further comprising the device of any one of the preceding items.

### (Item H3)

The kit of any one of the preceding items, further comprising a coating agent for application on an RNA placed on the device.

### (Item H4)

The kit of any one of the preceding items, wherein the coating agent comprises 3-hydroxypicolic acid.

### (Item H5)

The kit of any one of the preceding items, wherein a destination to which the sample is to be sent is indicated.

### (Item H6)

The kit of any one of the preceding items, wherein the sample comprises at least one of blood, a biopsy sample such as a liquid biopsy sample, an oral mucous membrane, saliva, sweat, tear, urine, stool, and skin epidermis.

### (Program)

### (Item I1)

A program for determining a condition of a subject based on RNA modification information, wherein the program is configured to execute the steps of: comparing modification information on at least one type of RNA in the subject with reference modification information of the RNA; and determining the condition of the subject based on a result of an output of the comparison step.

### (Item I2)

The program of any one of the preceding items, wherein the reference modification information is composed based on modification information on the RNA in a subject that is different from the subject.

### (Item I3)

The program of any one of the preceding items, wherein the reference modification information is modification information on the RNA in the subject obtained at a time that is different from that for the modification information.

### (Method of utilizing resistant strain)

### (Item J1)

A method for determining RNA modification information associated with resistance to an agent, the method comprising obtaining modification information on at least one type of RNA derived from a cell strain with resistance to the agent, wherein modification information on the RNA is compared between the cell strain with resistance and a cell strain derived from the cell strain with resistance, and the modification information on the RNA is determined to be associated with resistance to the agent if a difference is observed.

### (Item J2)

The method of any one of the preceding items, wherein a combination of differences in a plurality of pieces of modification information on a plurality of RNAs is determined to be associated with resistance to the agent.

The present invention is intended so that one or more of the features described above can be provided not only as the explicitly disclosed combinations, but also as other combinations thereof. Additional embodiments and advantages of the invention are recognized by those skilled in the art by reading and understanding the following detailed description as needed.

### [Advantageous Effects of Invention]

The present invention can analyze and predict a condition of a subject (biological condition or medical condition) based on RNA modification information with a method that is different from conventional methods.

### [Brief Description of Drawings]

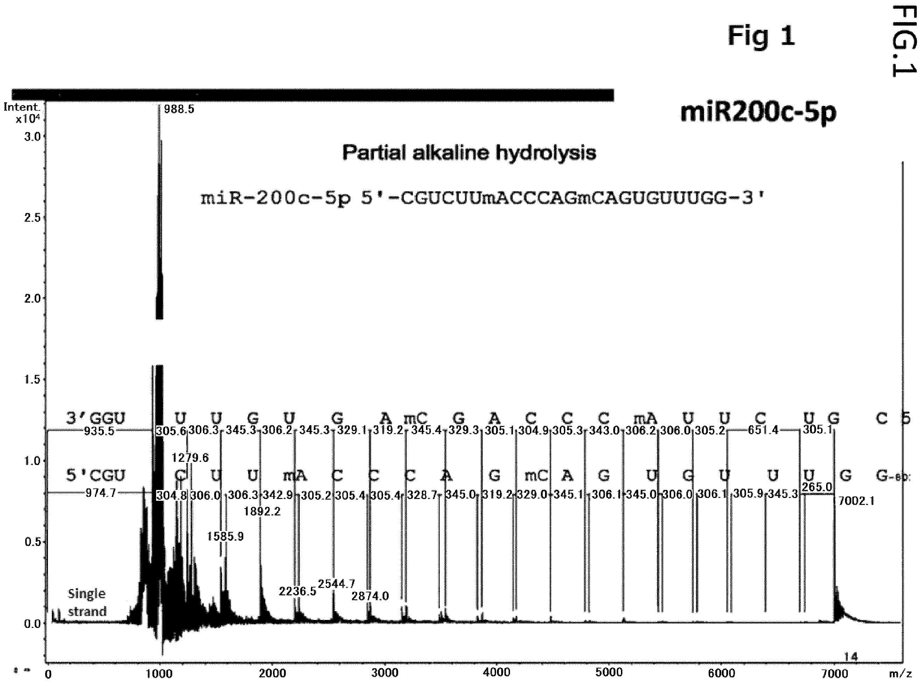

[Figure 1] Figure **1** is a diagram showing a result of MALDI-TOF/TOF measurement on synthetic microRNA 200-c-5p. Series for both the 3' end fragment and the 5' end fragment were observed.

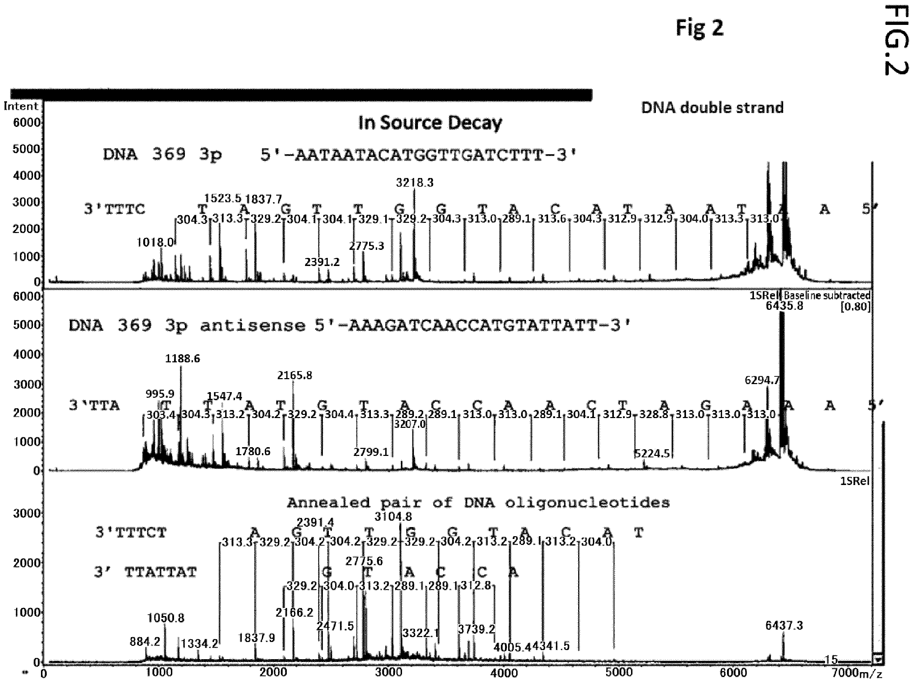

[Figure 2] Figure **2** is a diagram showing a result of MALDI-TOF/TOF measurement on a DNA double stand of a synthetic oligo DNA with a sequence complementary to microRNA-369-3p and an antisense synthetic DNA thereof.

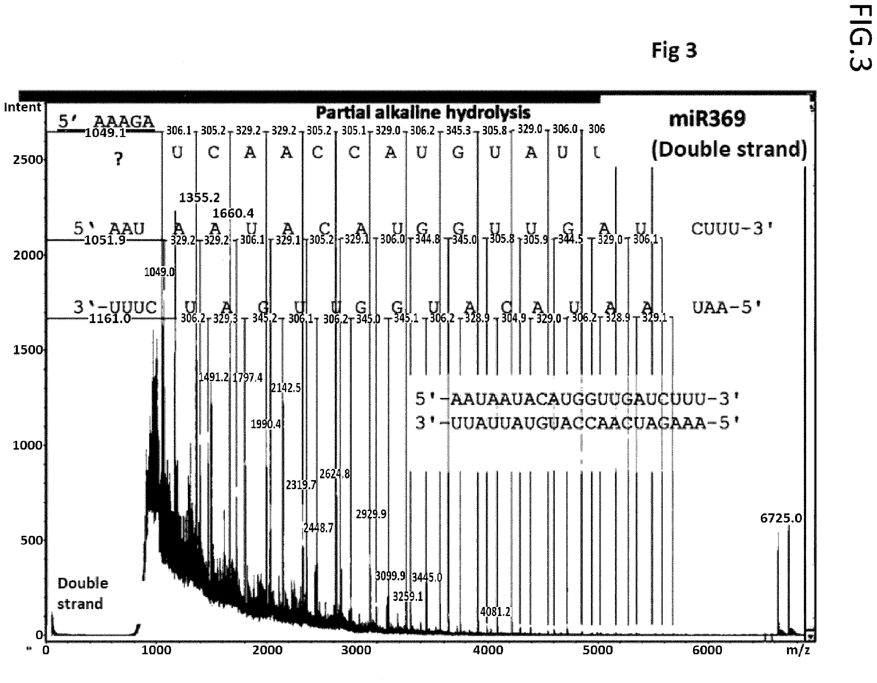

[Figure 3] Figure **3** is a diagram showing a result of MALDI-TOF/TOF measurement on double stranded synthetic miR-369.

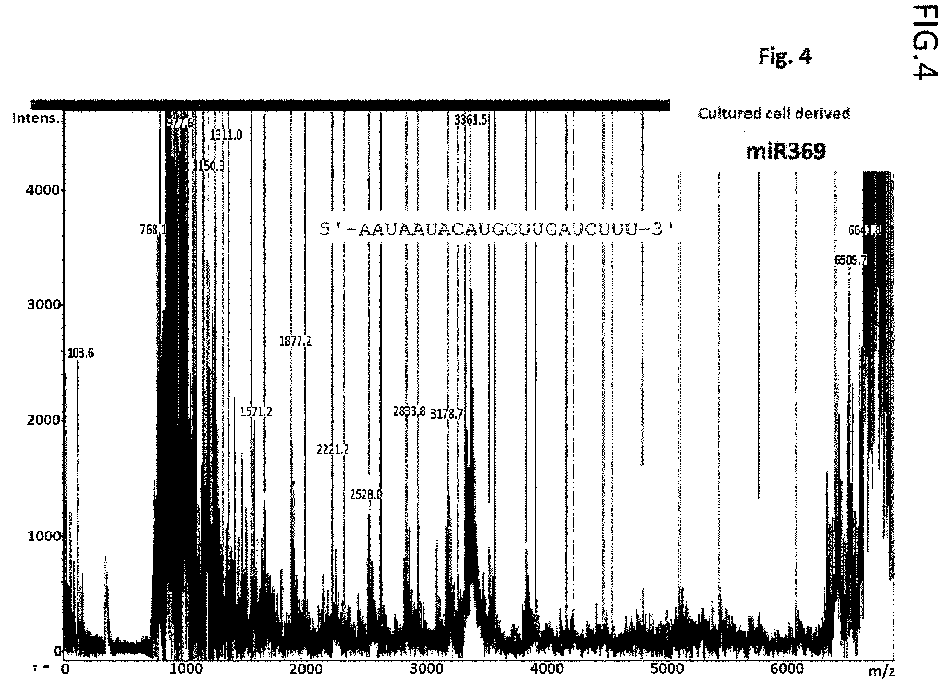

[Figure 4] Figure **4** is a diagram showing a result of MALDI-TOF/TOF measurement after a precursor corresponding to miR-369 in the RNA of a cultured cell is selected and fragmented by ISD.

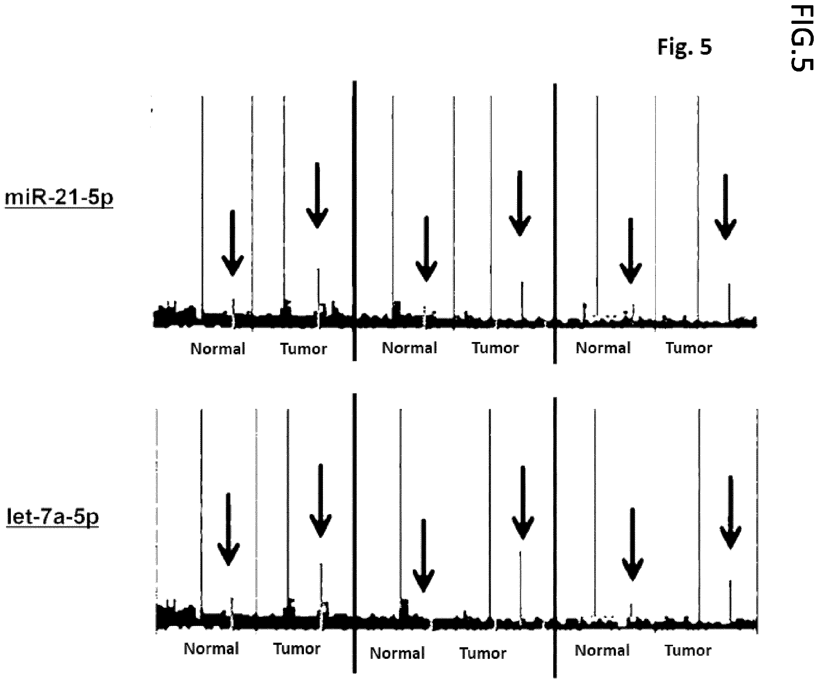

[Figure 5] Figure **5** is a diagram showing the intensity of MS signals indicating methylated miR-21-5p (top) and let-7a-5p (bottom) for normal tissue and tumor tissue. The m/z at the arrows is a signal of a fragment from a methylated RNA. The vertical axis indicates the signal intensity measured by a mass spectrometer.

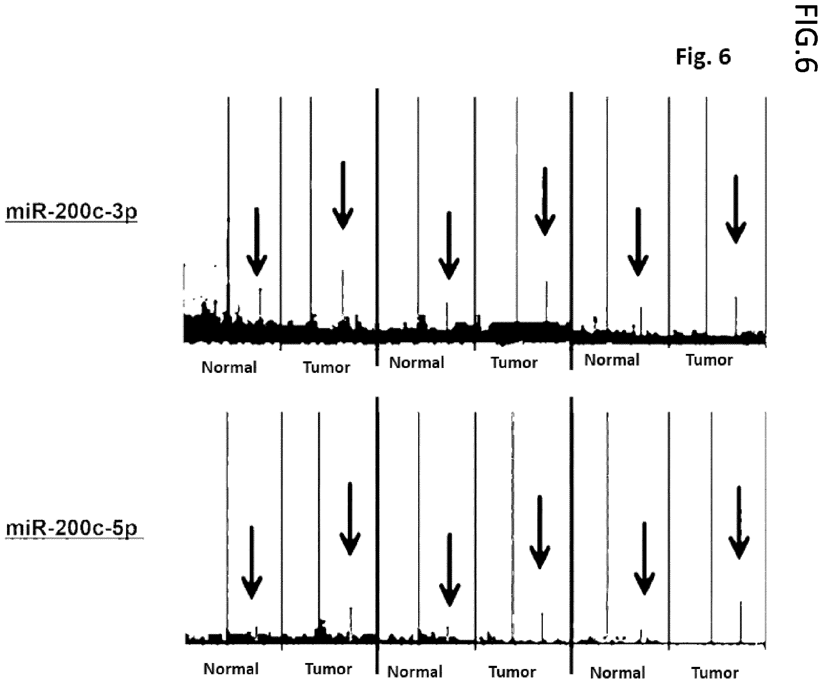

[Figure 6] Figure **6** is a diagram showing the intensity of MS signals indicating methylated miR-200c-3p (top) and miR-200c-5p (bottom) for normal tissue and tumor tissue. The m/z at the arrows is a signal of a fragment from a methylated RNA. The vertical axis indicates the signal intensity measured by a mass spectrometer.

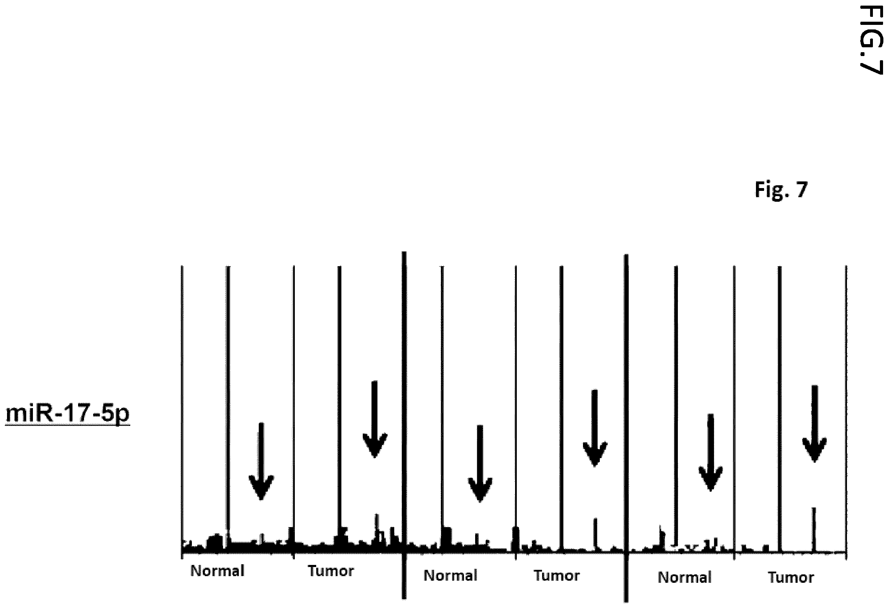

[Figure 7] Figure **7** is a diagram showing the intensity of MS signals indicating methylated miR-17c-5p for normal tissue and tumor tissue. The m/z at the arrows is a signal of a fragment from a methylated RNA. The vertical axis indicates the signal intensity measured by a mass spectrometer.

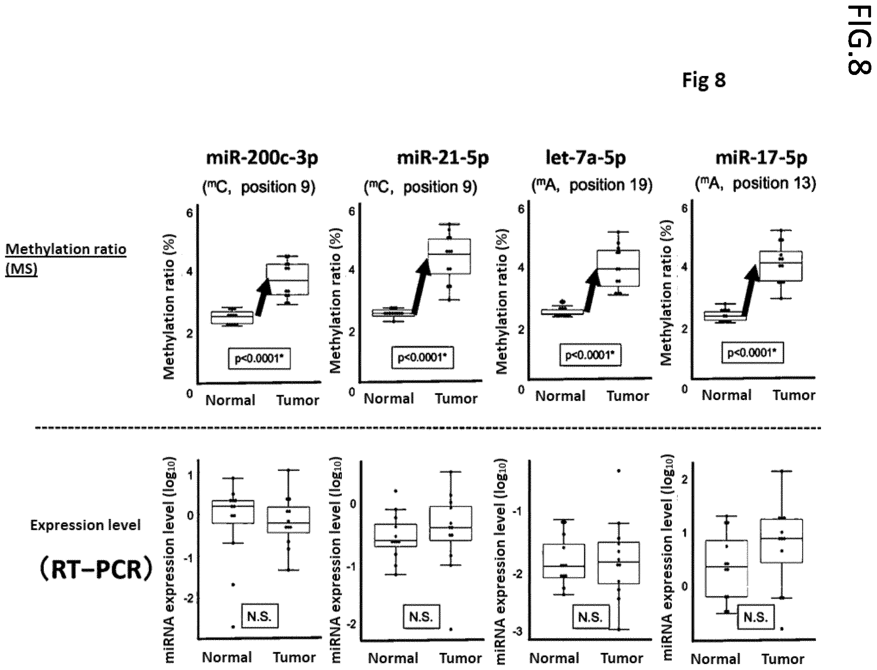

[Figure 8] Figure **8** is a diagram comparing the ratio (%) of methylated RNA (top) and RNA expression level (bottom) in an RNA of a sequence of a subject between normal tissue and tumor tissue of the large intestine for miR-200c-3p, miR-21-5p, miR-let7a-5p, and miR-17-5p. N. S. indicates no significant difference.

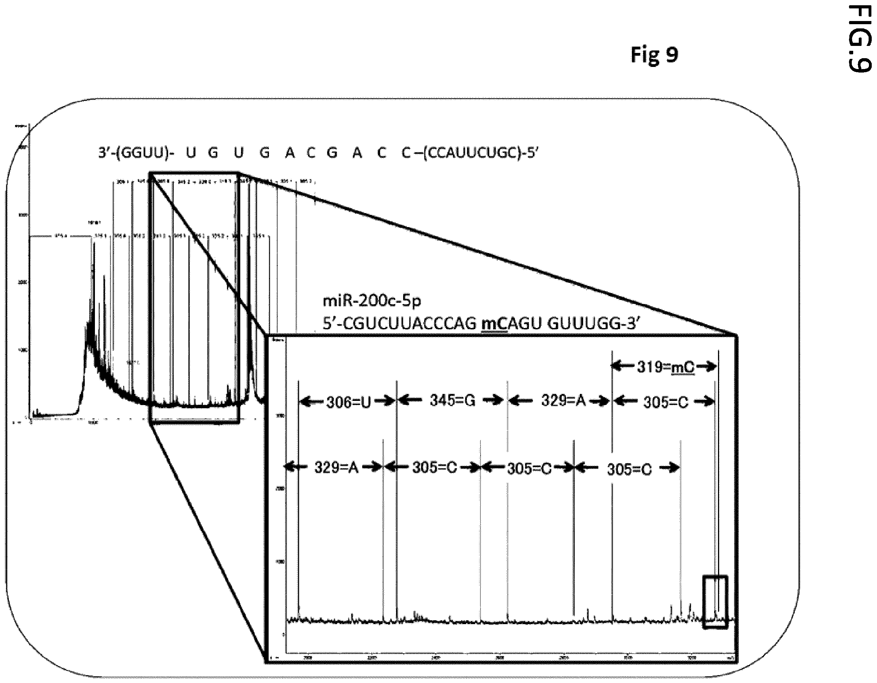

[Figure 9] Figure **9** is a diagram showing MS signals that indicate the presence of methylated miR-200c-5p. Ammonium treatment is applied to examine the internal sequence. The figure shows that the width indicated by two arrows was detected as a difference in mass of the corresponding nucleotide.

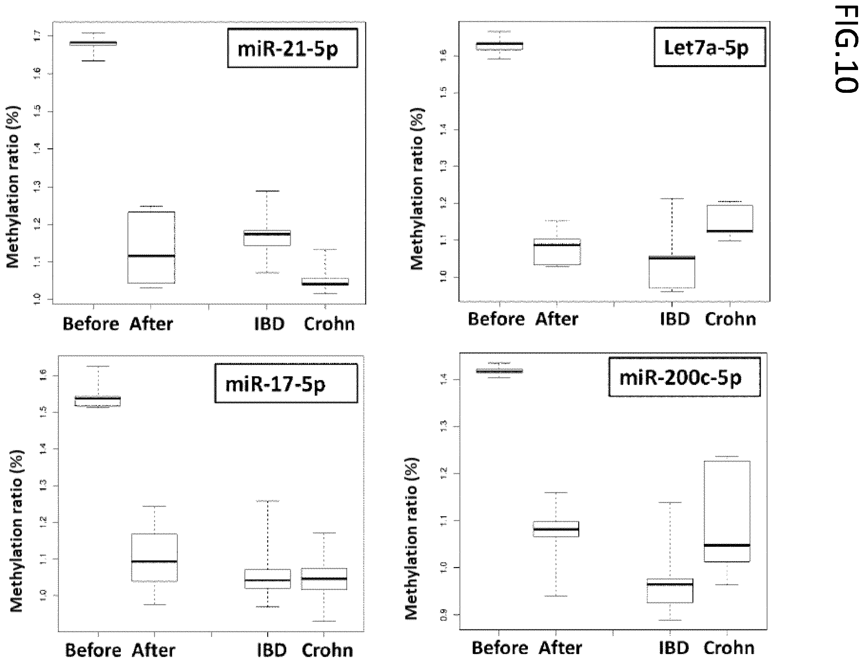

[Figure 10] Figure **10** shows a comparison of the methylation ratio of miR-21-5p, miR-17-5p, let7a-5p, and miR-200c-5p in a sample before resection of primary tumor from a human colon cancer patient found to have metastasis from 1 year to 2 years after the resection of primary tumor (Before), a sample obtained when the metastasis was found (After), and samples of serum from patients with inflammatory bowel disease (IBD) and Crohn's disease (Crohn). The vertical axis indicates the methylation ratio of the target miRNA measured by mass spectrometry.

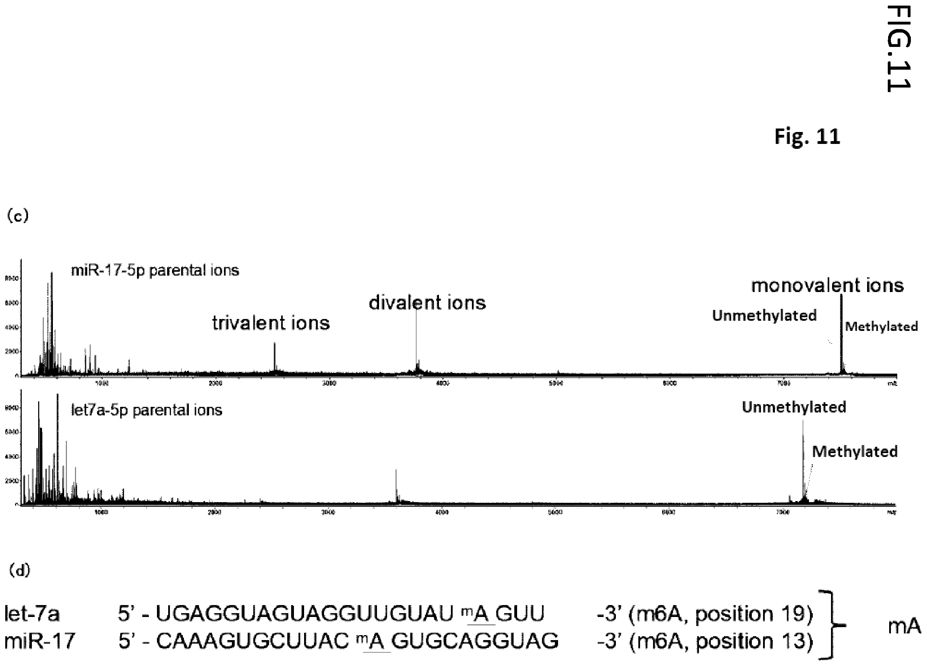

[Figure 11] Figure **11** shows an example of detection of a methylated base in a mature miRNA. (c) shows mass spectrum of miR-17 and let-7a obtained from pancreatic cancer patient derived tissue and shows that peaks of both methylated and unmethylated parts were detected. (d) shows the location of a modified nucleoside in each miRNA.

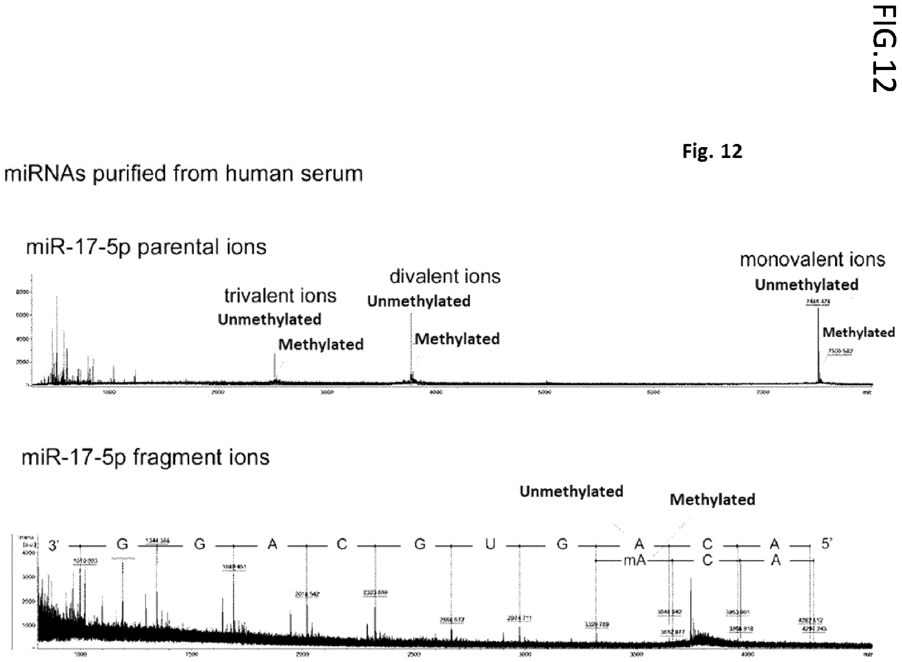

[Figure 12] Figure **12** is methylation analysis on let-17a concentrated from a serum sample of a pancreatic cancer patient by MALDI-TOF-MS/MS. The top row shows results of analyzing parental ions (MS analysis) and shows that peaks of both methylated and unmethylated parts were detected. The bottom row shows results of analyzing fragment ions (bases: 12 to 19) (MS/MS analysis) and shows that adenine at position 19 is methylated.

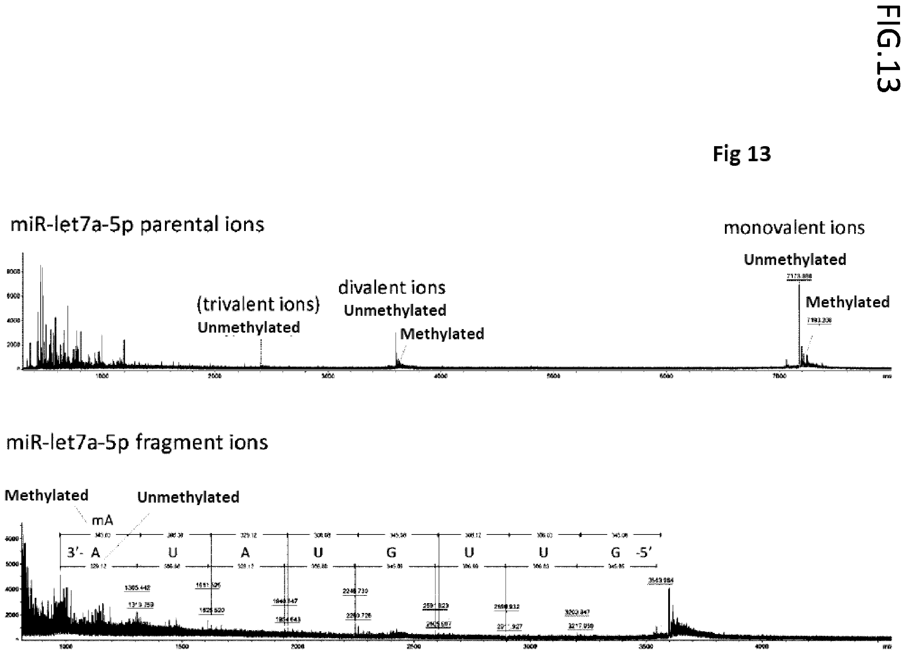

[Figure 13] Figure **13** is methylation analysis on miR-17 concentrated from a serum sample of a pancreatic cancer patient by MALDI-TOF-MS/MS. The top row shows results of analyzing parental ions (MS analysis) and shows that peaks of both methylated and unmethylated parts were detected. The bottom row shows results of analyzing fragment ions (bases: 11 to 20) (MS/MS analysis) and shows that adenine at position 13 is methylated.

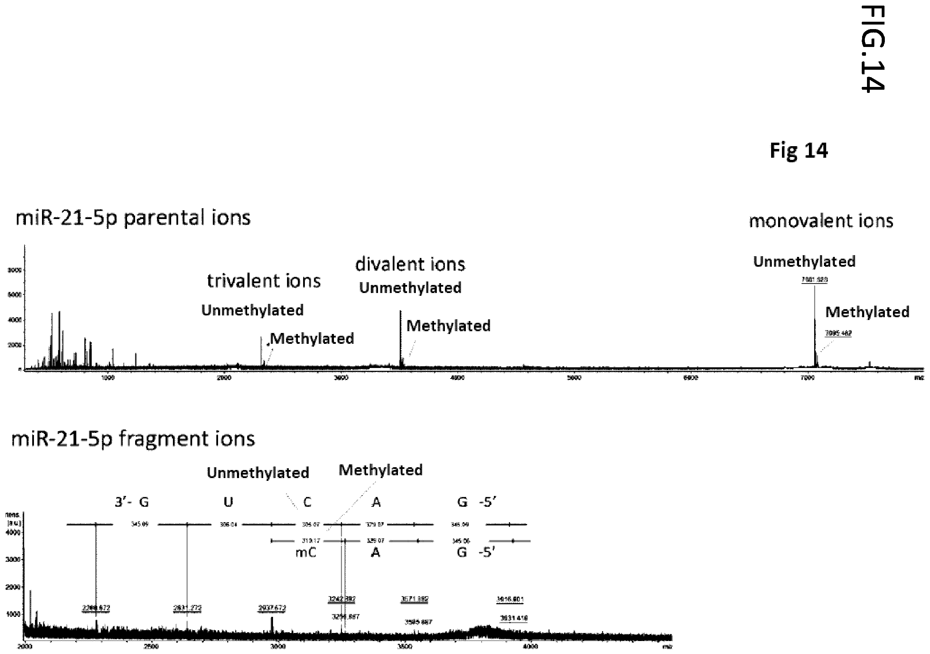

[Figure 14] Figure **14** is methylation analysis on miR-21 concentrated from a serum sample of a pancreatic cancer patient by MALDI-TOF-MS/MS. The top row shows results of analyzing parental ions (MS analysis) and shows that peaks of both methylated and unmethylated parts were detected. The bottom row shows results of analyzing fragment ions (bases: 5 to 11) (MS/MS analysis) and shows that cytosine at position 9 is methylated.

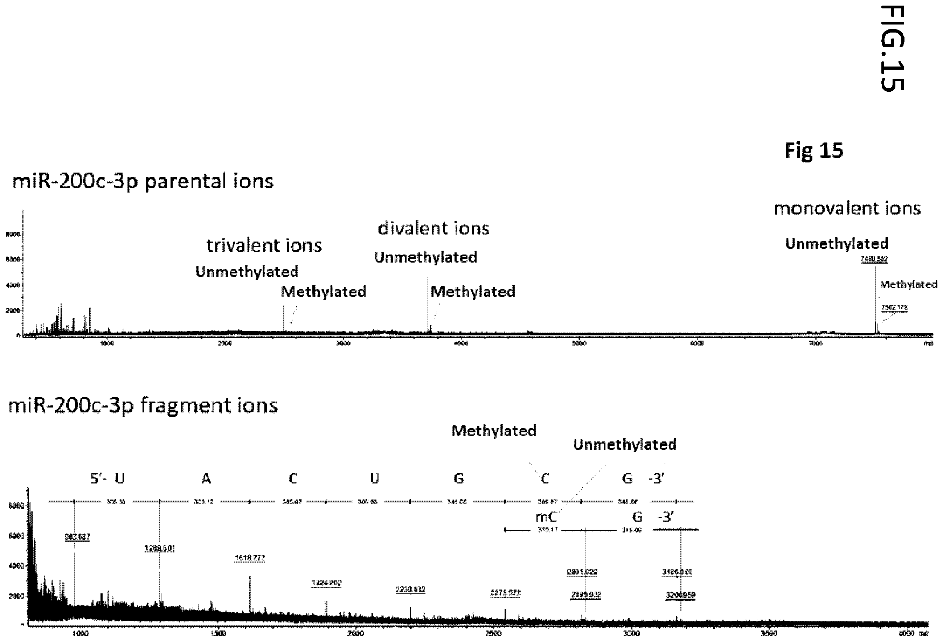

[Figure 15] Figure **15** is methylation analysis on miR-200c concentrated from a serum sample of a pancreatic cancer patient by MALDI-TOF-MS/MS. The top row shows results of analyzing parental ions (MS analysis) and shows that peaks of both methylated and unmethylated parts were detected. The bottom row shows results of analyzing fragment ions (bases: 4 to 10) (MS/MS analysis) and shows that cytosine at position 9 is methylated.

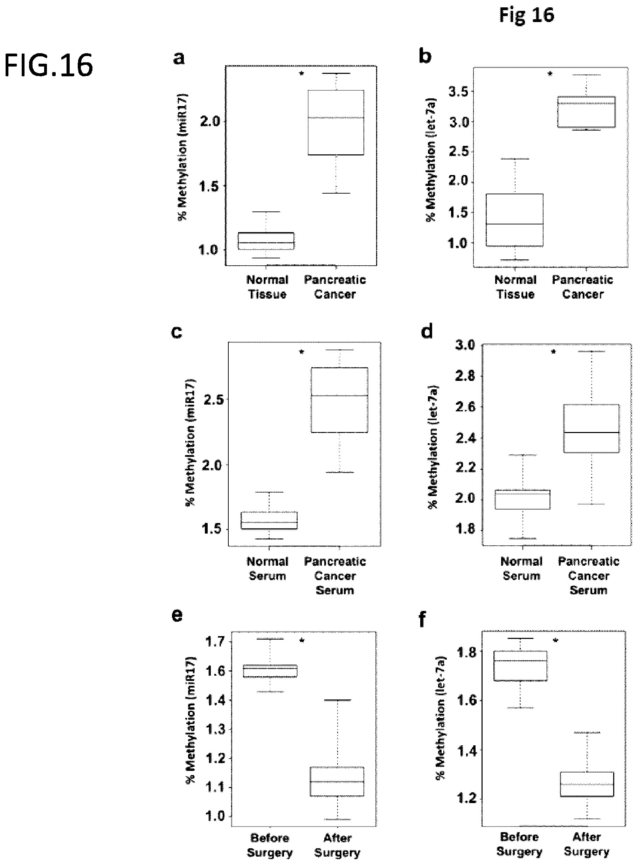

[Figure 16] Figure **16** shows that the miRNA methylation level is elevated in pancreatic cancer tissue. (a, b) shows a comparison of methylation levels of miR-17 (a) and let-7a (b) in normal tissue (n = 12, left) and pancreatic cancer tissue (n = 12, right) in pancreatic cancer patients. *P < 0.01 (t-test). (c, d) shows a comparison of methylation levels of miR-17 (c) and let-7a (d) in serum obtained from healthy controls (n = 5, left) and pancreatic cancer patients (n = 5, right). The serum of the heathy controls was obtained from a liver transplant donor confirmed to be free of cancer by endoscopic examination, CT, and detection of several tumor markers. *P < 0.01 (t-test). (e, f) shows a comparison of methylation levels of miR-17 (e) and let-7a (f) in serum before surgery (n = 21, left) and after surgery (n = 21, right) in pancreatic cancer patients. The serum of the healthy controls was obtained from a liver transplant donor confirmed to be free of cancer by endoscopic examination, CT, and detection of several tumor markers. *P < 0.01 (t-test).

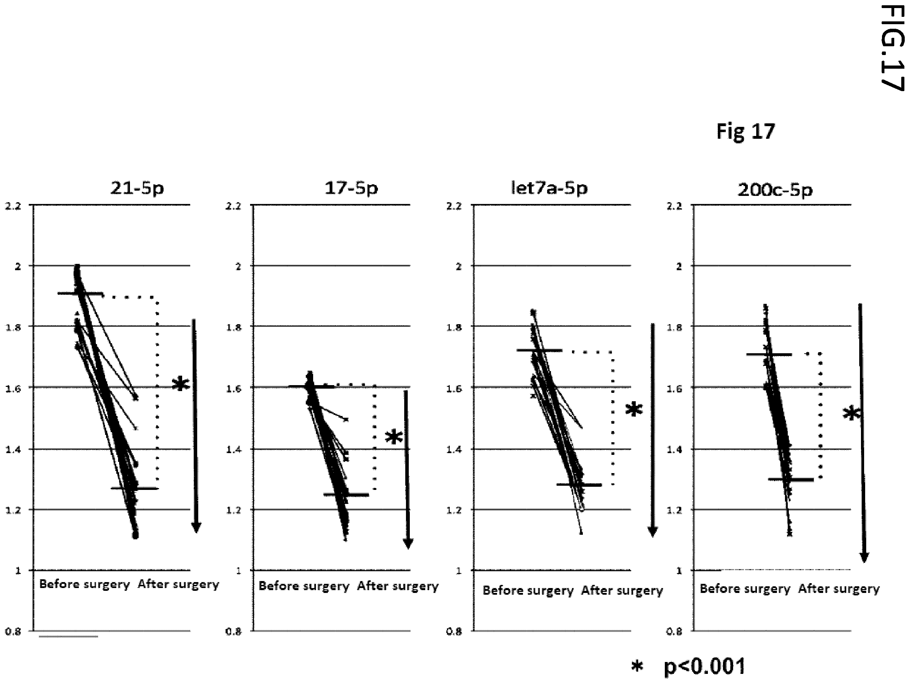

[Figure 17] Figure **17** shows a comparison of methylation levels of miRNA in serum before and after surgery of a pancreatic cancer patient for, from the left, miR-21, miR-17, let-17a, and miR-200c. The levels for before and after the surgery are matched for the same patient.

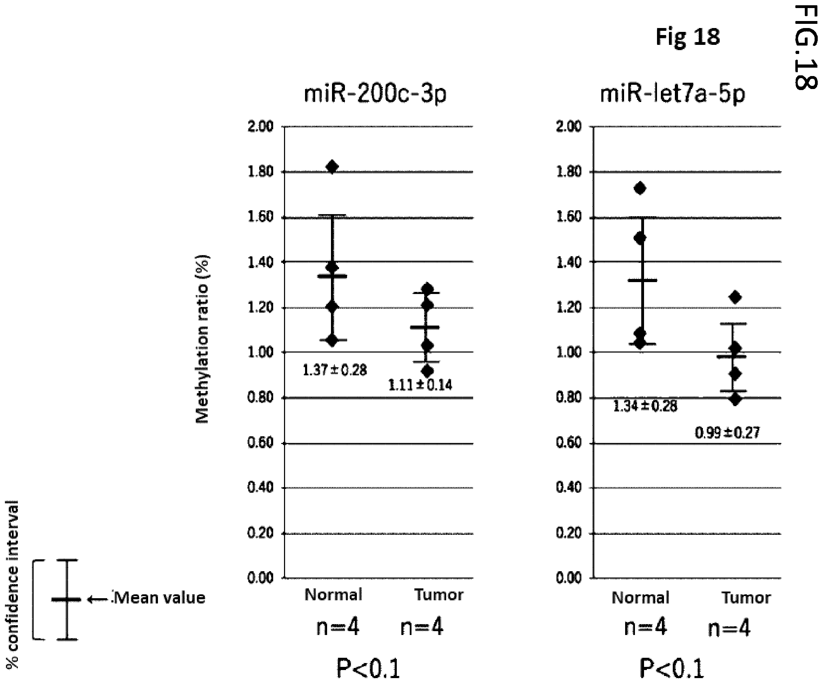

[Figure 18] Figure **18** is a diagram comparing the ratios (%) of methylated RNAs in RNAs of a sequence of a subject between normal tissue and tumor tissue in the stomach for miR-200c-3p and miR-let7a-5p.

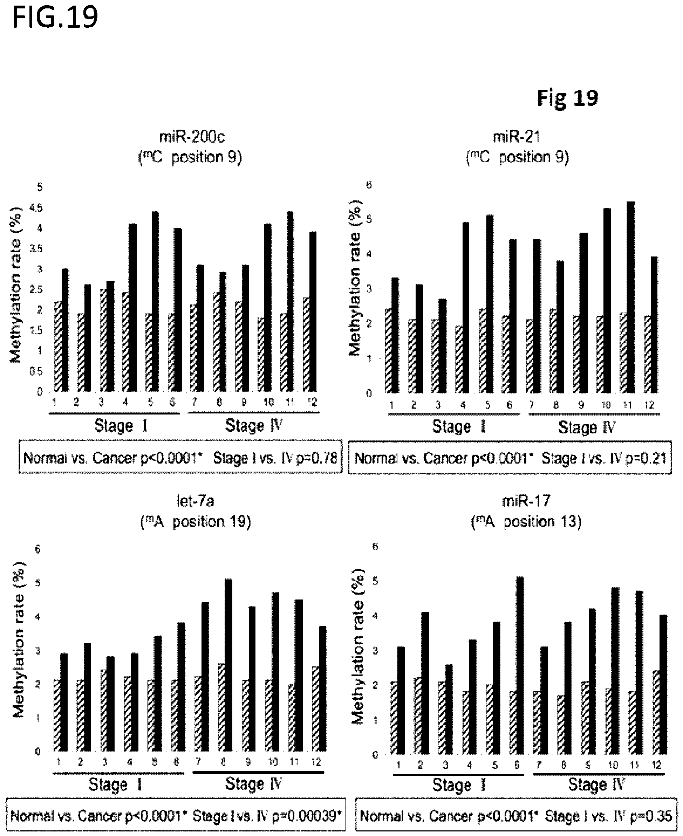

[Figure 19] Figure **19** shows the methylation ratio at a specific location of each miRNA in normal tissue (shaded bars) and colorectal cancer tissue (black bars) in a colorectal cancer patient. The numbers at the bottom of the graph indicate the patient numbers. Patients 1 to 6 are stage I colorectal cancer patients, and patients 7 to 12 are stage IV colorectal cancer patients. *P < 0.05 (t-test).

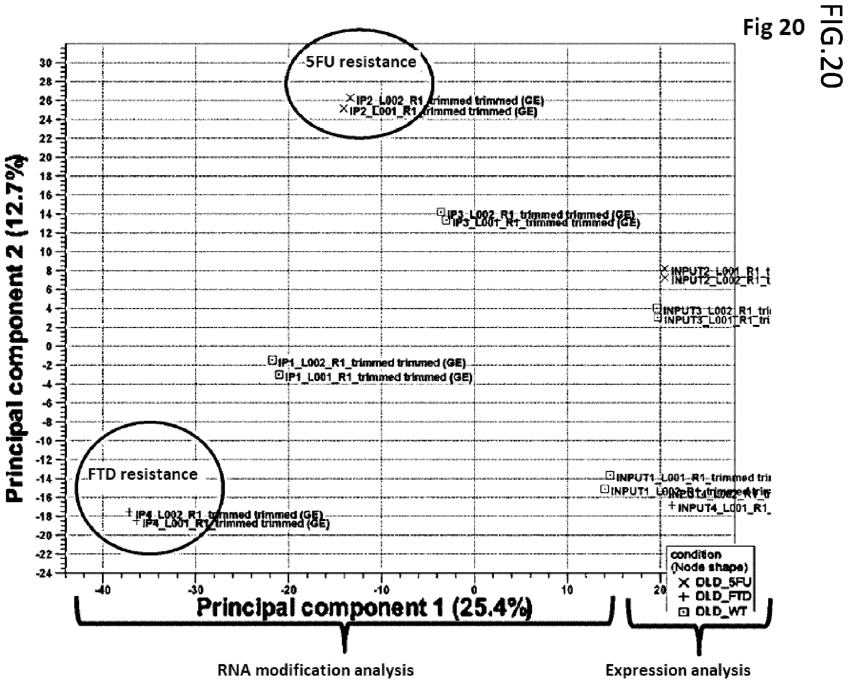

[Figure 20] Figure **20** shows results of analyzing two dimensional primary component analysis based on RIP sequencing information and RNA expression information on a 5-FU resistant strain and FTD resistant strain and the parent strain from which they originated.

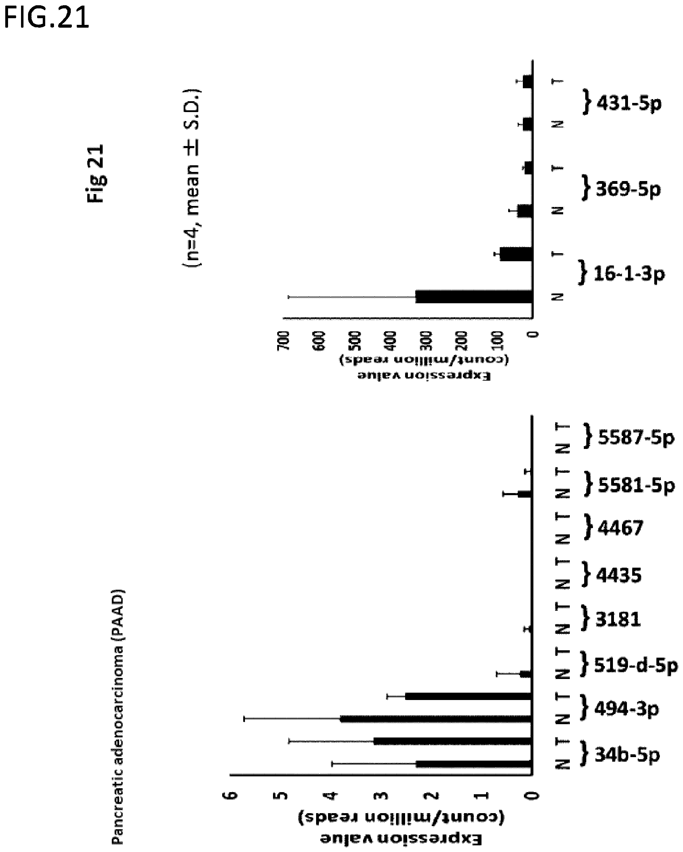

[Figure 21] Figure **21** is a diagram showing the expression level of each miRNA in normal tissue (N) and tumor tissue (T) in pancreatic adenocarcinoma (PAAD, n = 4) patients obtained from The Cancer Genome Atlas (TCGA). The vertical axis indicates the ratio of the miRNA count in a subject among all reads in sequence data (count/1 million reads).

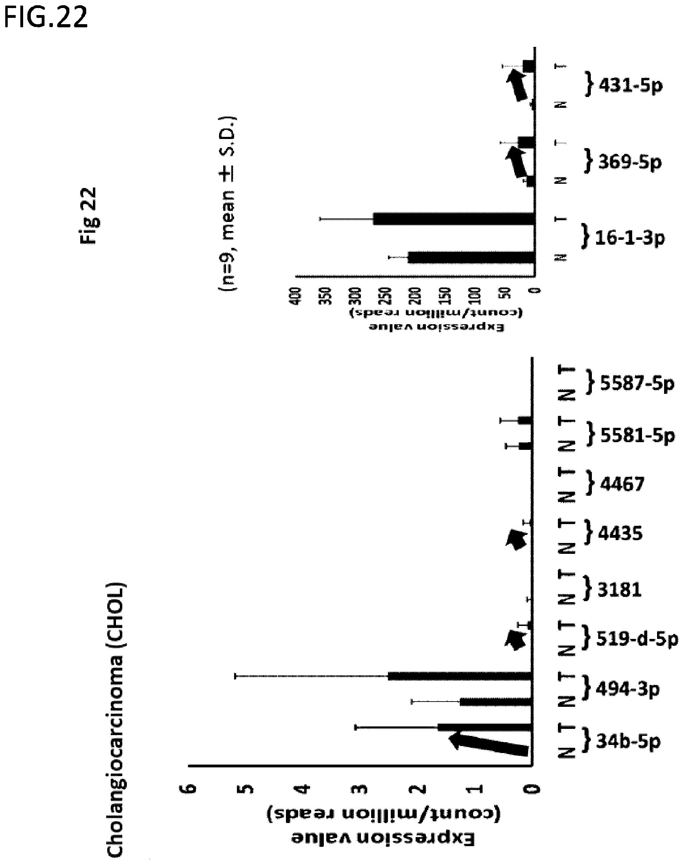

[Figure 22] Figure **22** is a diagram showing the expression level of each miRNA in normal tissue (N) and tumor tissue (T) in rectal adenocarcinoma (READ, n = 3) patients obtained from The Cancer Genome Atlas (TCGA). The vertical axis indicates the ratio of the miRNA count in a subject among all reads in sequence data (count/1 million reads).

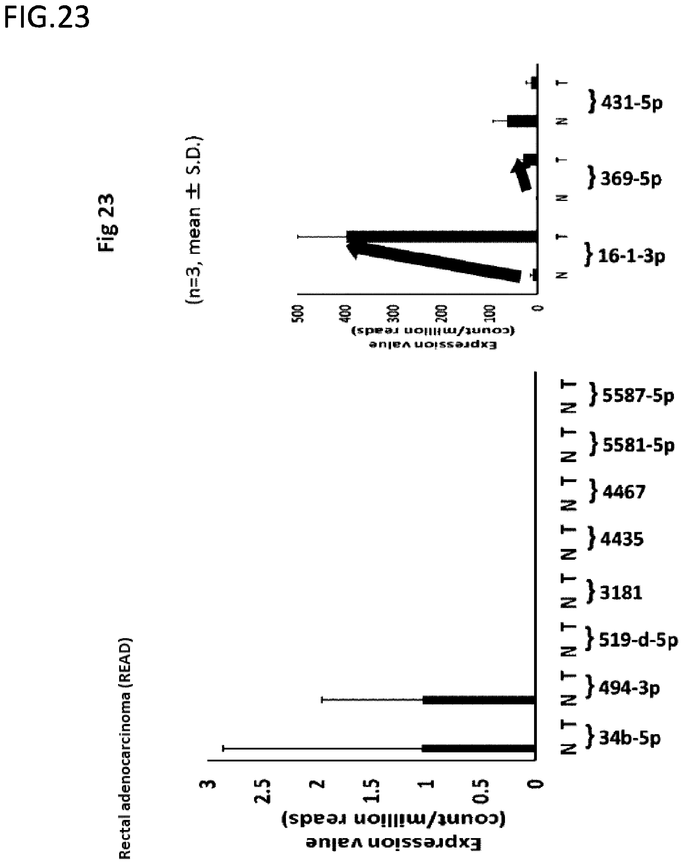

[Figure 23] Figure **23** is a diagram showing the expression level of each miRNA in normal tissue (N) and tumor tissue (T) in cholangiocarcinoma (CHOL, n = 9) patients obtained from The Cancer Genome Atlas (TCGA). The vertical axis indicates the ratio of the miRNA count in a subject among all reads in sequence data (count/1 million reads).

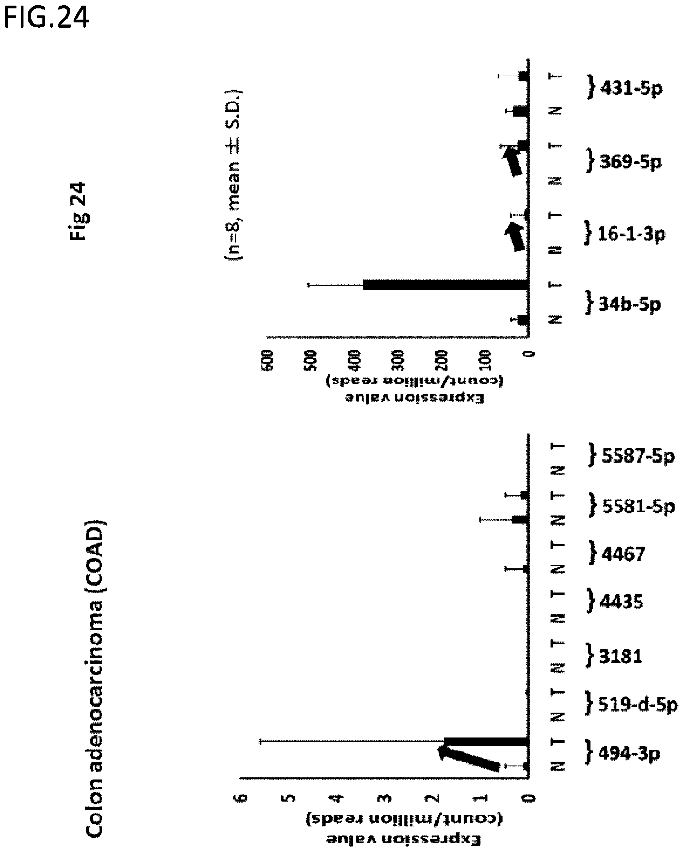

[Figure 24] Figure **24** is a diagram showing the expression level of each miRNA in normal tissue (N) and tumor tissue (T) in colon adenocarcinoma (COAD, n = 8) patients obtained from The Cancer Genome Atlas (TCGA). The vertical axis indicates the ratio of the miRNA count in a subject among all reads in sequence data (count/1 million reads).

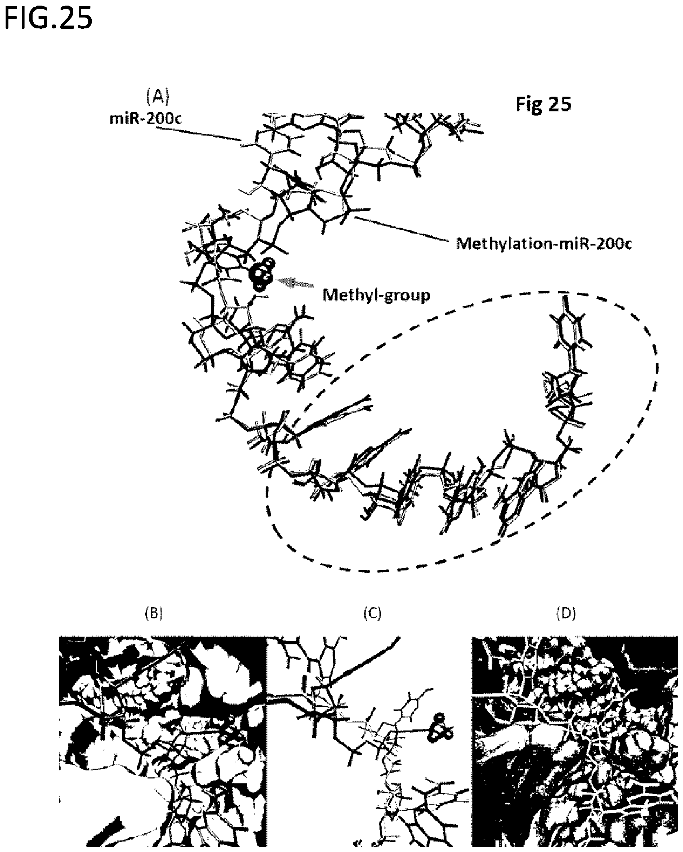

[Figure 25] Figure **25** is a prediction for miR-200c binding to an AGO2 protein by molecular dynamic analysis. (a) shows superimposition of the stable conformation of a complex of each of unmethylated miR-200c (orange) and methylated miR-200c (blue) estimated by energy minimization. The first 6 bases mostly overlapped. Meanwhile, a change in binding interaction was found near a methylated site. (b) shows that the space surrounding a methyl group is reduced due to enhanced van der Waals interaction between the methyl group and the AGO2 protein. (c) shows that the orientation changes due to the presence of a methyl group. (d) shows that a free space is greater in a complex of unmethylated miR-200c than in a complex of methylated miR-200c.

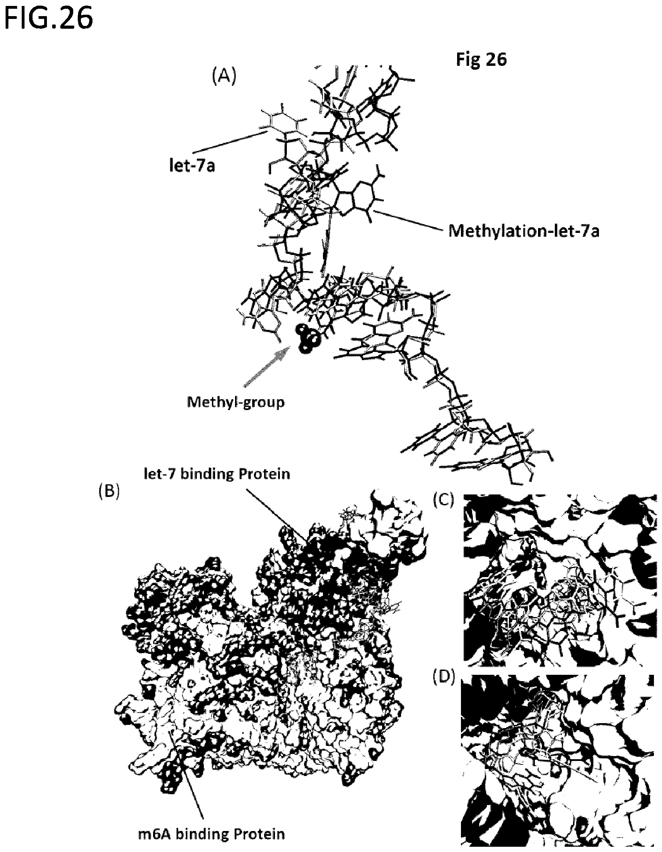

[Figure 26] Figure **26** is a prediction for let-7a binding to an AGO2 protein by molecular dynamic analysis. (a) shows superimposition of the stable conformation of a complex of each of unmethylated let-7a (orange) and methylated let-7a (blue) estimated by energy minimization. While the backbone arrangement was similar, the orientation of each base was significantly different between unmethylated let-7a and methylated let-7a. Adenine methylation of let-7a results in a structural change of the entire complex (b) and a change in the size of space of an RNA recognition site (c, d).

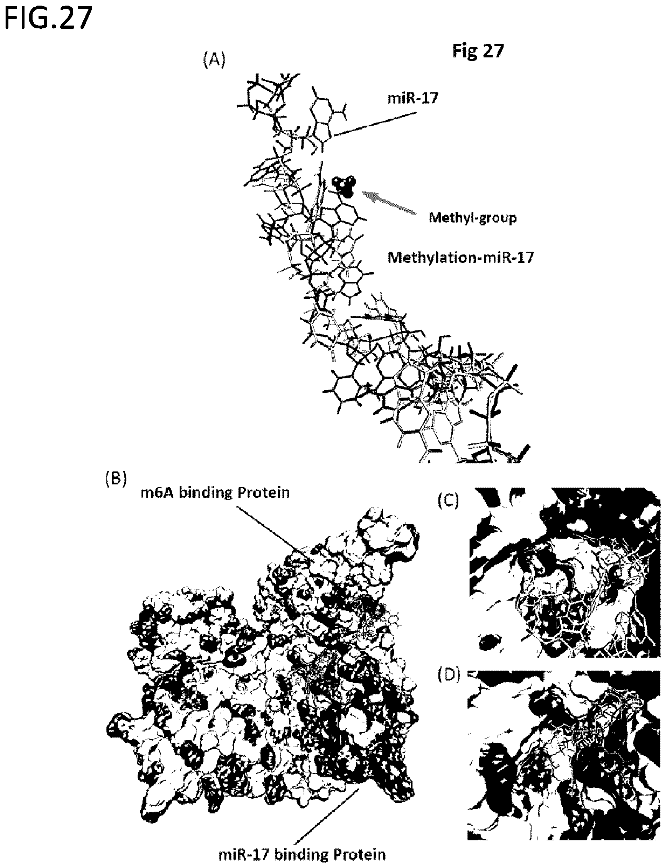

[Figure 27] Figure **27** is a prediction for miR-17 binding to an AGO2 protein by molecular dynamic analysis. (a) shows superimposition of the stable conformation of a complex of each of unmethylated miR-17 (orange) and methylated miR-17 (blue) estimated by energy minimization. The orientation of the backbone and base side chain was significantly different between unmethylated miR-17 and methylated miR-17. Adenine methylation of miR-17 results in a structural change of the entire complex (b) and a change in the size of space of an RNA recognition site (c, d).

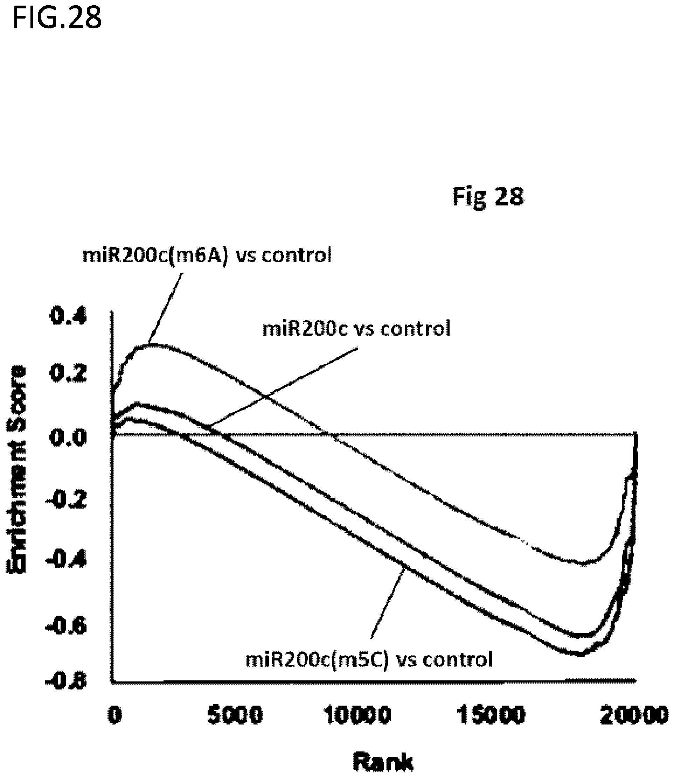

[Figure 28] Figure **28** shows results of determining the gene suppression effect due to unmodified miR-200c (blue), m5C modified miR-200c (red), and m6A modified miR-200c (green) by gene concentration analysis. Unmodified miR-200c and m5C modified miR-200c exhibited a potent gene suppression effect (P < 0.005 and P < 0.001, respectively; t-test), but the gene expression suppression effect of m6A modified miR-200c was weak.

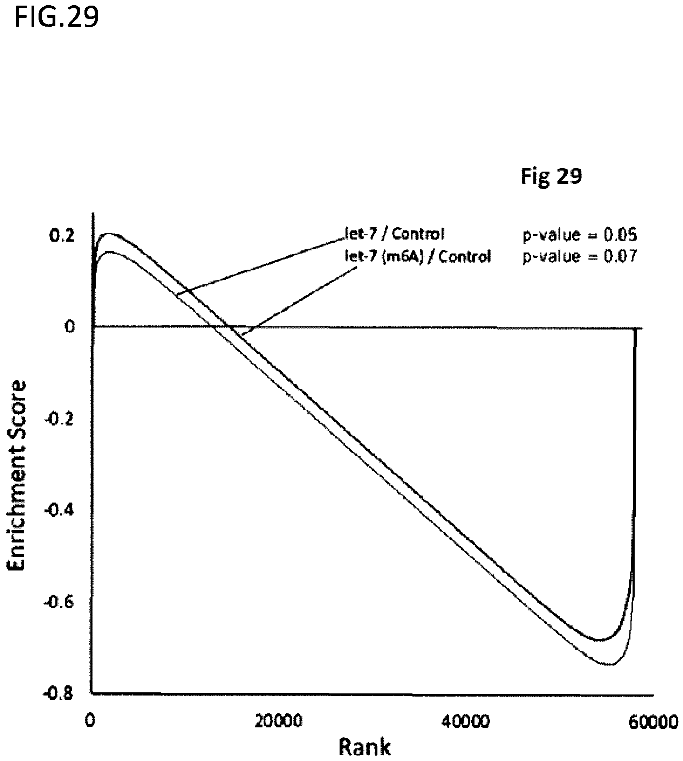

[Figure 29] Figure **29** shows results of determining the gene suppression effect due to unmodified let-7 (light blue) and m6A modified let-7 (red) by gene concentration analysis. Unmodified let-7a suppressed the expression of a target gene (P = 0.05; t-test), but methylated let-7a did not (P = 0.07; t-test).

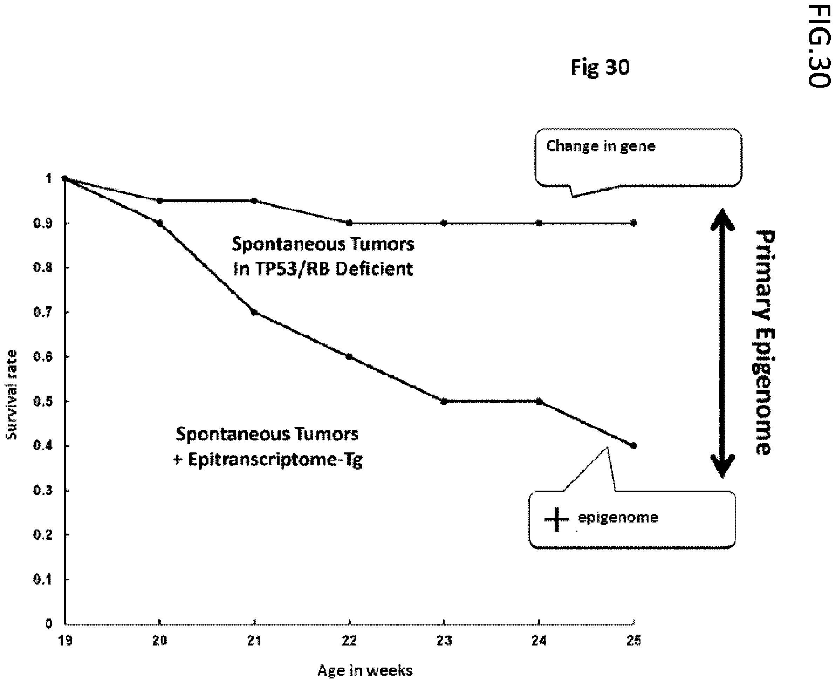

[Figure 30] Figure **30** is a diagram comparing the survival rate between EL1-SV40 mice with inactivated P53 and RB in the pancreas (top line) and double transgenic mice prepared from these mice and Mettl3 gene overexpressing mice (bottom line). The vertical axis indicates the survival rate, and the horizontal axis indicates the age in weeks.

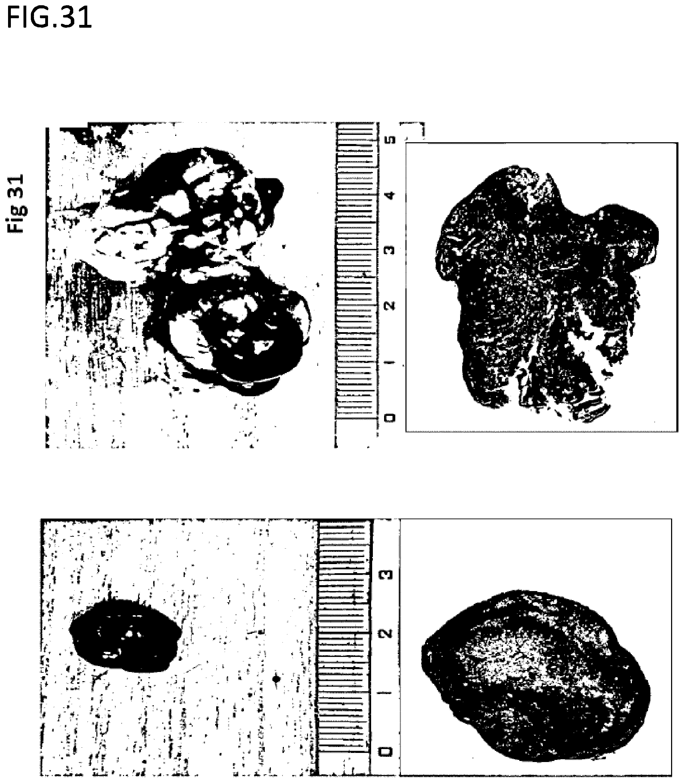

[Figure 31] Figure **31** is a diagram comparing the tumor extracted at 20-week-old between EL1-SV40 mice with inactivated P53 and RB in the pancreas (left) and double transgenic mice prepared from these mice and Mettl3 gene overexpressing mice (right). The vertical axis indicates the survival rate, and the horizontal axis indicates the age in weeks. The top row is a picture of tumor, and the bottom row is hematoxylin and eosin staining of a tissue section.

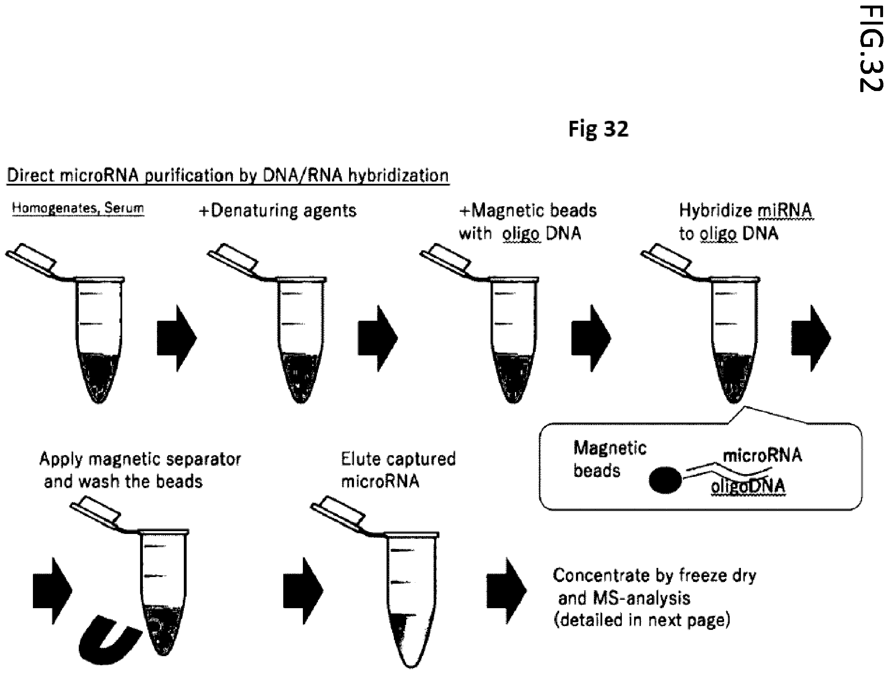

[Figure 32] Figure **32** is a schematic diagram of a method of purifying an RNA of interest using magnetic beads.

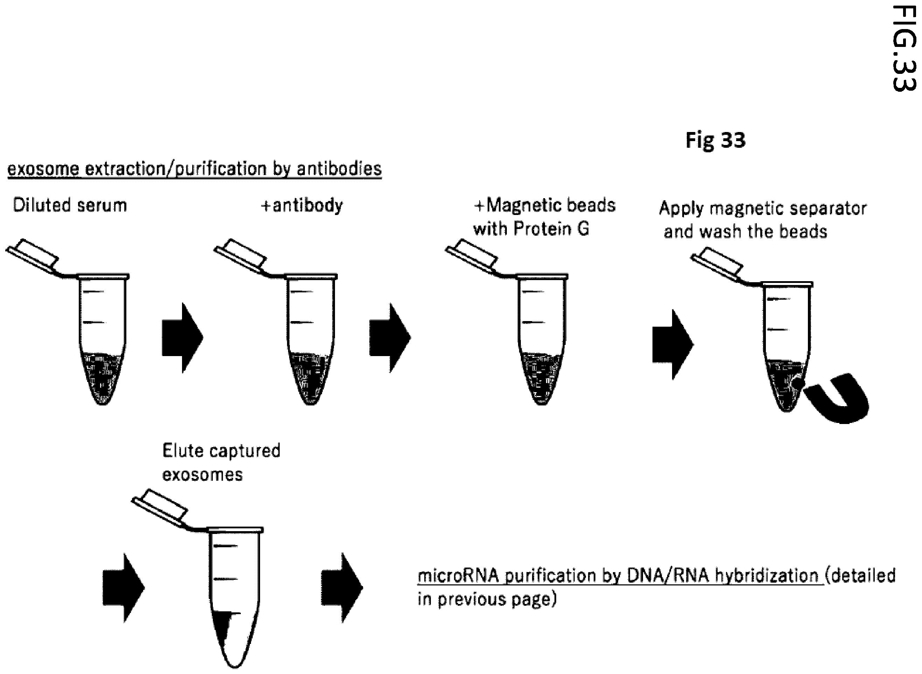

[Figure 33] Figure **33** is a schematic diagram of an RNA purification method using exosome concentration.

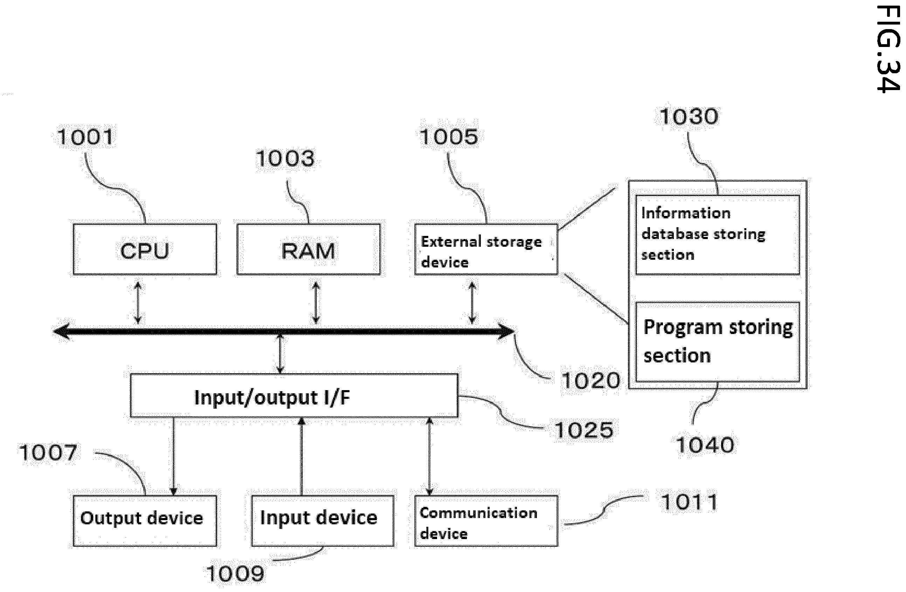

[Figure 34] Figure **34** is a schematic diagram of a configuration of a system.

### [Description of Embodiments]

The present invention is described hereinafter while showing the best mode of the invention. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence.

The definitions of the terms and/or basic technical matters especially used herein are described hereinafter when appropriate.

### (Definitions, etc.)

As used herein, "ribonucleic acid (RNA)" refers to a molecule comprising at least one ribonucleotide residue. "Ribonucleotide" refers to a nucleotide with a hydroxyl group at position 2' of β-D-ribofuranose moiety. Examples of RNA include mRNA, tRNA, rRNA, lncRNA, and miRNA.

As used herein, "messenger RNA (mRNA)" refers to an RNA prepared by using a DNA template and is associated with a transcript encoding a peptide or polypeptide. Typically, an mRNA comprises 5'-UTR, protein coding region, and 3'-UTR. Specific information (sequence and the like) of mRNAs is available from, for example, NCBI (https://www.ncbi.nlm.nih.gov/). For example, mature microRNAs in humans include those in the following table.

As used herein, "microRNA (miRNA)" refers to a functional nucleic acid, which is encoded on the genome and ultimately becomes a very small RNA with a base length of 20 to 25 after undergoing a multi-stage production process. Specific information (sequence and the like) of miRNAs is available from, for example, mirbase (http://mirbase.org). For example, mature microRNAs in humans include those in the following table.

**[Table 1-1]**

| List of human mature microRNAs | | | |
|---|---|---|---|
| Accession | ID | Accession | ID |
| MIMAT0000062 | hsa-let-7a-5p | MIMAT0018981 | hsa-miR-4459 |
| MIMAT0000063 | hsa-let-7b-5p | MIMAT0018982 | hsa-miR-4460 |
| MIMAT0000065 | hsa-let-7d-5p | MIMAT0018983 | hsa-miR-4461 |
| MIMAT0000066 | hsa-let-7e-5p | MIMAT0018984 | hsa-miR-378h |
| MIMAT0000067 | hsa-let-7f-5p | MIMAT0018985 | hsa-miR-3135b |
| MIMAT0000068 | hsa-miR-15a-5p | MIMAT0018986 | hsa-miR-4462 |
| MIMAT0000069 | hsa-miR-16-5p | MIMAT0018987 | hsa-miR-4463 |
| MIMAT0000070 | hsa-miR-17-5p | MIMAT0018988 | hsa-miR-4464 |
| MIMAT0000071 | hsa-miR-17-3p | MIMAT0018989 | hsa-miR-548ai |
| MIMAT0000072 | hsa-miR-18a-5p | MIMAT0018992 | hsa-miR-4465 |
| MIMAT0000073 | hsa-miR-19a-3p | MIMAT0018993 | hsa-miR-4466 |
| MIMAT0000074 | hsa-miR-19b-3p | MIMAT0018994 | hsa-miR-4467 |
| MIMAT0000075 | hsa-miR-20a-5p | MIMAT0018995 | hsa-miR-4468 |
| MIMAT0000076 | hsa-miR-21-5p | MIMAT0018996 | hsa-miR-4469 |
| MIMAT0000077 | hsa-miR-22-3p | MIMAT0018997 | hsa-miR-4470 |
| MIMAT0000078 | hsa-miR-23a-3p | MIMAT0018998 | hsa-miR-4471 |
| MIMAT0000079 | hsa-miR-24-1-5p | MIMAT0018999 | hsa-miR-4472 |
| MIMAT0000080 | hsa-miR-24-3p | MIMAT0019000 | hsa-miR-4473 |
| MIMAT0000081 | hsa-miR-25-3p | MIMAT0019001 | hsa-miR-4474-3p |
| MIMAT0000082 | hsa-miR-26a-5p | MIMAT0019002 | hsa-miR-4475 |
| MIMAT0000083 | hsa-miR-26b-5p | MIMAT0019003 | hsa-miR-4476 |
| MIMAT0000084 | hsa-miR-27a-3p | MIMAT0019004 | hsa-miR-4477a |
| MIMAT0000085 | hsa-miR-28-5p | MIMAT0019005 | hsa-miR-4477b |
| MIMAT0000086 | hsa-miR-29a-3p | MIMAT0019006 | hsa-miR-4478 |
| MIMAT0000087 | hsa-miR-30a-5p | MIMAT0019007 | hsa-miR-3689c |
| MIMAT0000088 | hsa-miR-30a-3p | MIMAT0019008 | hsa-miR-3689d |
| MIMAT0000089 | hsa-miR-31-5p | MIMAT0019009 | hsa-miR-3689e |
| MIMAT0000090 | hsa-miR-32-5p | MIMAT0019010 | hsa-miR-3689f |
| MIMAT0000091 | hsa-miR-33a-5p | MIMAT0019011 | hsa-miR-4479 |
| MIMAT0000092 | hsa-miR-92a-3p | MIMAT0019012 | hsa-miR-3155b |
| MIMAT0000093 | hsa-miR-93-5p | MIMAT0019013 | hsa-miR-548ak |
| MIMAT0000095 | hsa-miR-96-5p | MIMAT0019014 | hsa-miR-4480 |

**[Table 1-2]**

| | | | |
|---|---|---|---|
| MIMAT0000096 | hsa-miR-98-5p | MIMAT0019015 | hsa-miR-4481 |
| MIMAT0000097 | hsa-miR-99a-5p | MIMAT0019017 | hsa-miR-4483 |
| MIMAT0000098 | hsa-miR-100-5p | MIMAT0019018 | hsa-miR-4484 |
| MIMAT0000099 | hsa-miR-101-3p | MIMAT0019020 | hsa-miR-4486 |
| MIMAT0000100 | hsa-miR-29b-3p | MIMAT0019021 | hsa-miR-4487 |
| MIMAT0000101 | hsa-miR-103a-3p | MIMAT0019022 | hsa-miR-4488 |
| MIMAT0000102 | hsa-miR-105-5p | MIMAT0019023 | hsa-miR-4489 |
| MIMAT0000103 | hsa-miR-106a-5p | MIMAT0019024 | hsa-miR-548a1 |
| MIMAT0000222 | hsa-miR-192-5p | MIMAT0019025 | hsa-miR-4490 |
| MIMAT0000226 | hsa-miR-196a-5p | MIMAT0019026 | hsa-miR-4491 |
| MIMAT0000227 | hsa-miR-197-3p | MIMAT0019027 | hsa-miR-4492 |
| MIMAT0000231 | hsa-miR-199a-5p | MIMAT0019028 | hsa-miR-4493 |
| MIMAT0000232 | hsa-miR-199a-3p | MIMAT0019029 | hsa-miR-4494 |
| MIMAT0000241 | hsa-miR-208a-3p | MIMAT0019030 | hsa-miR-4495 |
| MIMAT0000242 | hsa-miR-129-5p | MIMAT0019031 | hsa-miR-4496 |
| MIMAT0000243 | hsa-miR-148a-3p | MIMAT0019032 | hsa-miR-4497 |
| MIMAT0000244 | hsa-miR-30c-5p | MIMAT0019033 | hsa-miR-4498 |
| MIMAT0000245 | hsa-miR-30d-5p | MIMAT0019034 | hsa-miR-4419b |
| MIMAT0000250 | hsa-miR-139-5p | MIMAT0019035 | hsa-miR-4499 |
| MIMAT0000251 | hsa-miR-147a | MIMAT0019036 | hsa-miR-4500 |
| MIMAT0000252 | hsa-miR-7-5p | MIMAT0019037 | hsa-miR-4501 |
| MIMAT0000253 | hsa-miR-10a-5p | MIMAT0019038 | hsa-miR-4502 |
| MIMAT0000254 | hsa-miR-10b-5p | MIMAT0019039 | hsa-miR-4503 |
| MIMAT0000255 | hsa-miR-34a-5p | MIMAT0019040 | hsa-miR-4504 |
| MIMAT0000256 | hsa-miR-181a-5p | MIMAT0019041 | hsa-miR-4505 |
| MIMAT0000257 | hsa-miR-181b-5p | MIMAT0019042 | hsa-miR-4506 |
| MIMAT0000258 | hsa-miR-181c-5p | MIMAT0019043 | hsa-miR-2392 |
| MIMAT0000259 | hsa-miR-182-5p | MIMAT0019044 | hsa-miR-4507 |
| MIMAT0000260 | hsa-miR-182-3p | MIMAT0019045 | hsa-miR-4508 |
| MIMAT0000261 | hsa-miR-183-5p | MIMAT0019046 | hsa-miR-4509 |
| MIMAT0000262 | hsa-miR-187-3p | MIMAT0019047 | hsa-miR-4510 |
| MIMAT0000263 | hsa-miR-199b-5p | MIMAT0019048 | hsa-miR-4511 |
| MIMAT0000264 | hsa-miR-203a-3p | MIMAT0019049 | hsa-miR-4512 |
| MIMAT0000265 | hsa-miR-204-5p | MIMAT0019050 | hsa-miR-4513 |
| MIMAT0000266 | hsa-miR-205-5p | MIMAT0019051 | hsa-miR-4514 |
| MIMAT0000268 | hsa-miR-211-5p | MIMAT0019052 | hsa-miR-4515 |

**[Table 1-3]**

| | | | |
|---|---|---|---|
| MIMAT0000269 | hsa-miR-212-3p | MIMAT0019053 | hsa-miR-4516 |
| MIMAT0000270 | hsa-miR-181a-3p | MIMAT0019054 | hsa-miR-4517 |
| MIMAT0000271 | hsa-miR-214-3p | MIMAT0019055 | hsa-miR-4518 |
| MIMAT0000273 | hsa-miR-216a-5p | MIMAT0019056 | hsa-miR-4519 |
| MIMAT0000275 | hsa-miR-218-5p | MIMAT0019058 | hsa-miR-4521 |
| MIMAT0000276 | hsa-miR-219a-5p | MIMAT0019059 | hsa-miR-1269b |
| MIMAT0000278 | hsa-miR-221-3p | MIMAT0019060 | hsa-miR-4522 |
| MIMAT0000279 | hsa-miR-222-3p | MIMAT0019061 | hsa-miR-4523 |
| MIMAT0000280 | hsa-miR-223-3p | MIMAT0019064 | hsa-miR-4525 |
| MIMAT0000281 | hsa-miR-224-5p | MIMAT0019065 | hsa-miR-4526 |
| MIMAT0000318 | hsa-miR-200b-3p | MIMAT0019066 | hsa-miR-4527 |
| MIMAT0000414 | hsa-let-7g-5p | MIMAT0019067 | hsa-miR-4528 |
| MIMAT0000415 | hsa-let-7i-5p | MIMAT0019068 | hsa-miR-4529-3p |
| MIMAT0000416 | hsa-miR-1-3p | MIMAT0019069 | hsa-miR-4530 |
| MIMAT0000417 | hsa-miR-15b-5p | MIMAT0019070 | hsa-miR-4531 |
| MIMAT0000418 | hsa-miR-23b-3p | MIMAT0019071 | hsa-miR-4532 |
| MIMAT0000419 | hsa-miR-27b-3p | MIMAT0019072 | hsa-miR-4533 |
| MIMAT0000420 | hsa-miR-30b-5p | MIMAT0019073 | hsa-miR-4534 |
| MIMAT0000421 | hsa-miR-122-5p | MIMAT0019074 | hsa-miR-378i |
| MIMAT0000422 | hsa-miR-124-3p | MIMAT0019075 | hsa-miR-4535 |
| MIMAT0000423 | hsa-miR-125b-5p | MIMAT0019077 | hsa-miR-1587 |
| MIMAT0000424 | hsa-miR-128-3p | MIMAT0019079 | hsa-miR-548an |
| MIMAT0000425 | hsa-miR-130a-3p | MIMAT0019080 | hsa-miR-4537 |
| MIMAT0000426 | hsa-miR-132-3p | MIMAT0019081 | hsa-miR-4538 |
| MIMAT0000428 | hsa-miR-135a-5p | MIMAT0019082 | hsa-miR-4539 |
| MIMAT0000430 | hsa-miR-138-5p | MIMAT0019083 | hsa-miR-4540 |
| MIMAT0000431 | hsa -miR-14 0-5p | MIMAT0019197 | hsa-miR-3117-5p |
| MIMAT0000432 | hsa -miR-141-3p | MIMAT0019198 | hsa-miR-3120-5p |
| MIMAT0000435 | hsa-miR-143-3p | MIMAT0019199 | hsa-miR-3121-5p |
| MIMAT0000436 | hsa-miR-144-3p | MIMAT0019200 | hsa-miR-3124-3p |
| MIMAT0000437 | hsa-miR-145-5p | MIMAT0019201 | hsa-miR-3127-3p |
| MIMAT0000440 | hsa-miR-191-5p | MIMAT0019202 | hsa-miR-3129-3p |
| MIMAT0000441 | hsa-miR-9-5p | MIMAT0019203 | hsa-miR-3136-3p |
| MIMAT0000442 | hsa-miR-9-3p | MIMAT0019204 | hsa-miR-3140-5p |
| MIMAT0000443 | hsa-miR-125a-5p | MIMAT0019205 | hsa-miR-3145-5p |
| MIMAT0000444 | hsa-miR-126-5p | MIMAT0019206 | hsa-miR-3150a-5p |

**[Table 1-4]**

| | | | |
|---|---|---|---|
| MIMAT0000445 | hsa-miR-126-3p | MIMAT0019207 | hsa-miR-3152-5p |
| MIMAT0000446 | hsa-miR-127-3p | MIMAT0019208 | hsa-miR-3074-5p |
| MIMAT0000448 | hsa-miR-136-5p | MIMAT0019209 | hsa-miR-3156-3p |
| MIMAT0000449 | hsa-miR-146a-5p | MIMAT0019210 | hsa-miR-3157-3p |
| MIMAT0000450 | hsa-miR-149-5p | MIMAT0019211 | hsa-miR-3158-5p |
| MIMAT0000451 | hsa-miR-150-5p | MIMAT0019212 | hsa-miR-3160-5p |
| MIMAT0000452 | hsa-miR-154-5p | MIMAT0019213 | hsa-miR-3162-3p |
| MIMAT0000453 | hsa-miR-154-3p | MIMAT0019214 | hsa-miR-3173-5p |
| MIMAT0000455 | hsa-miR-185-5p | MIMAT0019215 | hsa-miR-3177-5p |
| MIMAT0000456 | hsa-miR-186-5p | MIMAT0019216 | hsa-miR-3187-5p |
| MIMAT0000457 | hsa-miR-188-5p | MIMAT0019217 | hsa-miR-3189-5p |
| MIMAT0000458 | hsa-miR-190a-5p | MIMAT0019218 | hsa-miR-3194-3p |
| MIMAT0000459 | hsa-miR-193a-3p | MIMAT0019219 | hsa-miR-3619-3p |
| MIMAT0000460 | hsa-miR-194-5p | MIMAT0019220 | hsa-miR-3664-3p |
| MIMAT0000461 | hsa-miR-195-5p | MIMAT0019221 | hsa-miR-3677-5p |
| MIMAT0000510 | hsa-miR-320a | MIMAT0019222 | hsa-miR-3682-5p |
| MIMAT0000617 | hsa-miR-200c-3p | MIMAT0019223 | hsa-miR-3688-5p |
| MIMAT0000646 | hsa-miR-155-5p | MIMAT0019224 | hsa-miR-3691-3p |
| MIMAT0000680 | hsa-miR-106b-5p | MIMAT0019225 | hsa-miR-3913-3p |
| MIMAT0000681 | hsa-miR-29c-3p | MIMAT0019226 | hsa-miR-3150b-5p |
| MIMAT0000682 | hsa-miR-200a-3p | MIMAT0019227 | hsa-miR-3922-5p |
| MIMAT0000683 | hsa-miR-302a-5p | MIMAT0019228 | hsa-miR-3925-3p |
| MIMAT0000684 | hsa-miR-302a-3p | MIMAT0019229 | hsa-miR-3940-5p |
| MIMAT0000685 | hsa-miR-34b-5p | MIMAT0019230 | hsa-miR-3942-3p |
| MIMAT0000686 | hsa-miR-34c-5p | MIMAT0019231 | hsa-miR-3944-5p |
| MIMAT0000687 | hsa-miR-299-3p | MIMAT0019232 | hsa-miR-4423-5p |
| MIMAT0000688 | hsa-miR-301a-3p | MIMAT0019233 | hsa-miR-4446-5p |
| MIMAT0000689 | hsa-miR-99b-5p | MIMAT0019234 | hsa-miR-4474-5p |
| MIMAT0000690 | hsa-miR-296-5p | MIMAT0019236 | hsa-miR-4529-5p |
| MIMAT0000691 | hsa-miR-130b-3p | MIMAT0019337 | hsa-miR-3960 |
| MIMAT0000692 | hsa-miR-30e-5p | MIMAT0019357 | hsa-miR-3972 |
| MIMAT0000693 | hsa-miR-30e-3p | MIMAT0019358 | hsa-miR-3973 |
| MIMAT0000703 | hsa-miR-361-5p | MIMAT0019359 | hsa-miR-3974 |
| MIMAT0000705 | hsa-miR-362-5p | MIMAT0019360 | hsa-miR-3975 |
| MIMAT0000707 | hsa-miR-363-3p | MIMAT0019361 | hsa-miR-3976 |
| MIMAT0000710 | hsa-miR-365a-3p | MIMAT0019362 | hsa-miR-3977 |

**[Table 1-5]**

| | | | |
|---|---|---|---|
| MIMAT0000714 | hsa-miR-302b-5p | MIMAT0019363 | hsa-miR-3978 |
| MIMAT0000715 | hsa-miR-302b-3p | MIMAT0019689 | hsa-miR-4633-5p |
| MIMAT0000716 | hsa-miR-302c-5p | MIMAT0019690 | hsa-miR-4633-3p |
| MIMAT0000717 | hsa-miR-302c-3p | MIMAT0019691 | hsa-miR-4634 |
| MIMAT0000718 | hsa-miR-302d-3p | MIMAT0019692 | hsa-miR-4635 |
| MIMAT0000719 | hsa-miR-367-3p | MIMAT0019693 | hsa-miR-4636 |
| MIMAT0000720 | hsa-miR-376c-3p | MIMAT0019694 | hsa-miR-4637 |
| MIMAT0000721 | hsa-miR-369-3p | MIMAT0019695 | hsa-miR-4638-5p |
| MIMAT0000723 | hsa-miR-371a-3p | MIMAT0019696 | hsa-miR-4638-3p |
| MIMAT0000725 | hsa-miR-373-5p | MIMAT0019697 | hsa-miR-4639-5p |
| MIMAT0000726 | hsa-miR-373-3p | MIMAT0019698 | hsa-miR-4639-3p |
| MIMAT0000727 | hsa-miR-374a-5p | MIMAT0019699 | hsa-miR-4640-5p |
| MIMAT0000729 | hsa-miR-376a-3p | MIMAT0019700 | hsa-miR-4640-3p |
| MIMAT0000730 | hsa-miR-377-3p | MIMAT0019701 | hsa-miR-4641 |
| MIMAT0000731 | hsa-miR-378a-5p | MIMAT0019702 | hsa-miR-4642 |
| MIMAT0000732 | hsa-miR-378a-3p | MIMAT0019703 | hsa-miR-4643 |
| MIMAT0000733 | hsa-miR-379-5p | MIMAT0019704 | hsa-miR-4644 |
| MIMAT0000734 | hsa-miR-380-5p | MIMAT0019705 | hsa-miR-4645-5p |
| MIMAT0000735 | hsa-miR-380-3p | MIMAT0019706 | hsa-miR-4645-3p |
| MIMAT0000736 | hsa-miR-381-3p | MIMAT0019707 | hsa-miR-4646-5p |
| MIMAT0000737 | hsa-miR-382-5p | MIMAT0019708 | hsa-miR-4646-3p |
| MIMAT0000750 | hsa-miR-340-3p | MIMAT0019709 | hsa-miR-4647 |
| MIMAT0000751 | hsa-miR-330-3p | MIMAT0019710 | hsa-miR-4648 |
| MIMAT0000753 | hsa-miR-342-3p | MIMAT0019711 | hsa-miR-4649-5p |
| MIMAT0000754 | hsa-miR-337-3p | MIMAT0019712 | hsa-miR-4649-3p |
| MIMAT0000755 | hsa-miR-323a-3p | MIMAT0019713 | hsa-miR-4650-5p |
| MIMAT0000757 | hsa-miR-151a-3p | MIMAT0019714 | hsa-miR-4650-3p |
| MIMAT0000758 | hsa-miR-135b-5p | MIMAT0019715 | hsa-miR-4651 |
| MIMAT0000759 | hsa-miR-148b-3p | MIMAT0019716 | hsa-miR-4652-5p |
| MIMAT0000760 | hsa-miR-331-3p | MIMAT0019717 | hsa-miR-4652-3p |
| MIMAT0000763 | hsa-miR-338-3p | MIMAT0019718 | hsa-miR-4653-5p |
| MIMAT0000764 | hsa-miR-339-5p | MIMAT0019719 | hsa-miR-4653-3p |
| MIMAT0000765 | hsa-miR-335-5p | MIMAT0019720 | hsa-miR-4654 |
| MIMAT0000772 | hsa-miR-345-5p | MIMAT0019721 | hsa-miR-4655-5p |
| MIMAT0001080 | hsa-miR-196b-5p | MIMAT0019722 | hsa-miR-4655-3p |
| MIMAT0001340 | hsa-miR-423-3p | MIMAT0019723 | hsa-miR-4656 |

**[Table 1-6]**

| | | | |
|---|---|---|---|
| MIMAT0001341 | hsa-miR-424-5p | MIMAT0019724 | hsa-miR-4657 |
| MIMAT0001343 | hsa-miR-425-3p | MIMAT0019725 | hsa-miR-4658 |
| MIMAT0001412 | hsa-miR-18b-5p | MIMAT0019726 | hsa-miR-4659a-5p |
| MIMAT0001413 | hsa-miR-20b-5p | MIMAT0019727 | hsa-miR-4659a-3p |
| MIMAT0001541 | hsa-miR-449a | MIMAT0019728 | hsa-miR-4660 |
| MIMAT0001545 | hsa-miR-450a-5p | MIMAT0019729 | hsa-miR-4661-5p |
| MIMAT0001618 | hsa-miR-191-3p | MIMAT0019730 | hsa-miR-4661-3p |
| MIMAT0001620 | hsa-miR-200a-5p | MIMAT0019731 | hsa-miR-4662a-5p |
| MIMAT0001625 | hsa-miR-431-5p | MIMAT0019732 | hsa-miR-4662a-3p |
| MIMAT0001630 | hsa-miR-323b-5p | MIMAT0019733 | hsa-miR-4659b-5p |
| MIMAT0001631 | hsa-miR-451a | MIMAT0019734 | hsa-miR-4659b-3p |
| MIMAT0001635 | hsa-miR-452-5p | MIMAT0019735 | hsa-miR-4663 |
| MIMAT0001636 | hsa-miR-452-3p | MIMAT0019736 | hsa-miR-4662b |
| MIMAT0002172 | hsa-miR-376b-3p | MIMAT0019737 | hsa-miR-4664-5p |
| MIMAT0002173 | hsa-miR-483-3p | MIMAT0019738 | hsa-miR-4664-3p |
| MIMAT0002177 | hsa-miR-486-5p | MIMAT0019739 | hsa-miR-4665-5p |
| MIMAT0002178 | hsa-miR-487a-3p | MIMAT0019740 | hsa-miR-4665-3p |
| MIMAT0002804 | hsa-miR-488-5p | MIMAT0019743 | hsa-miR-4667-5p |
| MIMAT0002806 | hsa-miR-490-3p | MIMAT0019744 | hsa-miR-4667-3p |
| MIMAT0002807 | hsa-miR-491-5p | MIMAT0019745 | hsa-miR-4668-5p |
| MIMAT0002809 | hsa-miR-146b-5p | MIMAT0019746 | hsa-miR-4668-3p |
| MIMAT0002810 | hsa-miR-202-5p | MIMAT0019747 | hsa-miR-219b-5p |
| MIMAT0002811 | hsa-miR-202-3p | MIMAT0019748 | hsa-miR-219b-3p |
| MIMAT0002813 | hsa-miR-493-5p | MIMAT0019749 | hsa-miR-4669 |
| MIMAT0002814 | hsa-miR-432-5p | MIMAT0019750 | hsa-miR-4670-5p |
| MIMAT0002815 | hsa-miR-432-3p | MIMAT0019751 | hsa-miR-4670-3p |
| MIMAT0002817 | hsa-miR-495-3p | MIMAT0019752 | hsa-miR-4671-5p |
| MIMAT0002819 | hsa-miR-193b-3p | MIMAT0019753 | hsa-miR-4671-3p |
| MIMAT0002820 | hsa-miR-497-5p | MIMAT0019754 | hsa-miR-4672 |
| MIMAT0002828 | hsa-miR-519e-5p | MIMAT0019755 | hsa-miR-4673 |
| MIMAT0002829 | hsa-miR-519e-3p | MIMAT0019756 | hsa-miR-4674 |
| MIMAT0002831 | hsa-miR-519c-5p | MIMAT0019757 | hsa-miR-4675 |
| MIMAT0002832 | hsa-miR-519c-3p | MIMAT0019758 | hsa-miR-4676-5p |
| MIMAT0002833 | hsa-miR-520a-5p | MIMAT0019759 | hsa-miR-4676-3p |
| MIMAT0002834 | hsa-miR-520a-3p | MIMAT0019760 | hsa-miR-4677-5p |
| MIMAT0002835 | hsa-miR-526b-5p | MIMAT0019761 | hsa-miR-4677-3p |

**[Table 1-7]**

| | | | |
|---|---|---|---|
| MIMAT0002836 | hsa-miR-526b-3p | MIMAT0019762 | hsa-miR-4678 |
| MIMAT0002837 | hsa-miR-519b-3p | MIMAT0019763 | hsa-miR-4679 |
| MIMAT0002838 | hsa-miR-525-5p | MIMAT0019764 | hsa-miR-4680-5p |
| MIMAT0002839 | hsa-miR-525-3p | MIMAT0019765 | hsa-miR-4680-3p |
| MIMAT0002840 | hsa-miR-523-3p | MIMAT0019766 | hsa-miR-4681 |
| MIMAT0002841 | hsa-miR-518f-5p | MIMAT0019767 | hsa-miR-4682 |
| MIMAT0002842 | hsa-miR-518f-3p | MIMAT0019768 | hsa-miR-4683 |
| MIMAT0002846 | hsa-miR-520c-3p | MIMAT0019769 | hsa-miR-4684-5p |
| MIMAT0002847 | hsa-miR-518c-5p | MIMAT0019770 | hsa-miR-4684-3p |
| MIMAT0002848 | hsa-miR-518c-3p | MIMAT0019771 | hsa-miR-4685-5p |
| MIMAT0002849 | hsa-miR-524-5p | MIMAT0019772 | hsa-miR-4685-3p |
| MIMAT0002850 | hsa-miR-524-3p | MIMAT0019773 | hsa-miR-4686 |
| MIMAT0002851 | hsa-miR-517-5p | MIMAT0019774 | hsa-miR-4687-5p |
| MIMAT0002852 | hsa-miR-517a-3p | MIMAT0019775 | hsa-miR-4687-3p |
| MIMAT0002855 | hsa-miR-520d-5p | MIMAT0019776 | hsa-miR-1343-3p |
| MIMAT0002856 | hsa-miR-520d-3p | MIMAT0019777 | hsa-miR-4688 |
| MIMAT0002857 | hsa-miR-517b-3p | MIMAT0019778 | hsa-miR-4689 |
| MIMAT0002859 | hsa-miR-516b-5p | MIMAT0019779 | hsa-miR-4690-5p |
| MIMAT0002860 | hsa-miR-516b-3p | MIMAT0019780 | hsa-miR-4690-3p |
| MIMAT0002861 | hsa-miR-518e-3p | MIMAT0019781 | hsa-miR-4691-5p |
| MIMAT0002863 | hsa-miR-518a-3p | MIMAT0019782 | hsa-miR-4691-3p |
| MIMAT0002864 | hsa-miR-518d-3p | MIMAT0019783 | hsa-miR-4692 |
| MIMAT0002866 | hsa-miR-517c-3p | MIMAT0019784 | hsa-miR-4693-5p |
| MIMAT0002868 | hsa-miR-522-3p | MIMAT0019785 | hsa-miR-4693-3p |
| MIMAT0002869 | hsa-miR-519a-3p | MIMAT0019786 | hsa-miR-4694-5p |
| MIMAT0002870 | hsa-miR-499a-5p | MIMAT0019787 | hsa-miR-4694-3p |
| MIMAT0002871 | hsa-miR-500a-3p | MIMAT0019788 | hsa-miR-4695-5p |
| MIMAT0002872 | hsa-miR-501-5p | MIMAT0019789 | hsa-miR-4695-3p |
| MIMAT0002873 | hsa-miR-502-5p | MIMAT0019790 | hsa-miR-4696 |
| MIMAT0002874 | hsa-miR-503-5p | MIMAT0019791 | hsa-miR-4697-5p |
| MIMAT0002876 | hsa-miR-505-3p | MIMAT0019792 | hsa-miR-4697-3p |
| MIMAT0002877 | hsa-miR-513a-5p | MIMAT0019793 | hsa-miR-4698 |
| MIMAT0002878 | hsa-miR-506-3p | MIMAT0019794 | hsa-miR-4699-5p |
| MIMAT0002880 | hsa-miR-508-3p | MIMAT0019795 | hsa-miR-4699-3p |
| MIMAT0002881 | hsa-miR-509-3p | MIMAT0019796 | hsa-miR-4700-5p |
| MIMAT0002883 | hsa-miR-514a-3p | MIMAT0019797 | hsa-miR-4700-3p |

**[Table 1-8]**

| | | | |
|---|---|---|---|
| MIMAT0002888 | hsa-miR-532-5p | MIMAT0019798 | hsa-miR-4701-5p |
| MIMAT0002891 | hsa-miR-18a-3p | MIMAT0019799 | hsa-miR-4701-3p |
| MIMAT0003150 | hsa-miR-455-5p | MIMAT0019801 | hsa-miR-4703-5p |
| MIMAT0003161 | hsa-miR-493-3p | MIMAT0019802 | hsa-miR-4703-3p |
| MIMAT0003163 | hsa-miR-539-5p | MIMAT0019803 | hsa-miR-4704-5p |
| MIMAT0003164 | hsa-miR-544a | MIMAT0019804 | hsa-miR-4704-3p |
| MIMAT0003165 | hsa-miR-545-3p | MIMAT0019805 | hsa-miR-4705 |
| MIMAT0003218 | hsa-miR-92b-3p | MIMAT0019806 | hsa-miR-4706 |
| MIMAT0003220 | hsa-miR-556-5p | MIMAT0019807 | hsa-miR-4707-5p |
| MIMAT0003225 | hsa-miR-561-3p | MIMAT0019808 | hsa-miR-4707-3p |
| MIMAT0003233 | hsa-miR-5 51b-3p | MIMAT0019809 | hsa-miR-4708-5p |
| MIMAT0003235 | hsa-miR-570-3p | MIMAT0019810 | hsa-miR-4708-3p |
| MIMAT0003239 | hsa-miR-574-3p | MIMAT0019811 | hsa-miR-4709-5p |
| MIMAT0003241 | hsa-miR-576-5p | MIMAT0019812 | hsa-miR-4709-3p |
| MIMAT0003247 | hsa-miR-582-5p | MIMAT0019815 | hsa-miR-4710 |
| MIMAT0003249 | hsa-miR-584-5p | MIMAT0019816 | hsa-miR-4711-5p |
| MIMAT0003251 | hsa-miR-548a-3p | MIMAT0019817 | hsa-miR-4711-3p |
| MIMAT0003254 | hsa-miR-548b-3p | MIMAT0019818 | hsa-miR-4712-5p |
| MIMAT0003256 | hsa-miR-589-3p | MIMAT0019819 | hsa-miR-4712-3p |
| MIMAT0003257 | hsa-miR-550a-3p | MIMAT0019820 | hsa-miR-4713-5p |
| MIMAT0003258 | hsa-miR-590-5p | MIMAT0019821 | hsa-miR-4713-3p |
| MIMAT0003261 | hsa-miR-593-5p | MIMAT0019822 | hsa-miR-4714-5p |
| MIMAT0003283 | hsa-miR-615-3p | MIMAT0019823 | hsa-miR-4714-3p |
| MIMAT0003284 | hsa-miR-616-5p | MIMAT0019824 | hsa-miR-4715-5p |
| MIMAT0003285 | hsa-miR-548c-3p | MIMAT0019825 | hsa-miR-4715-3p |
| MIMAT0003293 | hsa-miR-624-5p | MIMAT0019826 | hsa-miR-4716-5p |
| MIMAT0003294 | hsa-miR-625-5p | MIMAT0019827 | hsa-miR-4716-3p |
| MIMAT0003297 | hsa-miR-628-3p | MIMAT0019829 | hsa-miR-4717-5p |
| MIMAT0003298 | hsa-miR-629-3p | MIMAT0019830 | hsa-miR-4717-3p |
| MIMAT0003301 | hsa-miR-33b-5p | MIMAT0019831 | hsa-miR-4718 |
| MIMAT0003312 | hsa-miR-642a-5p | MIMAT0019832 | hsa-miR-4719 |
| MIMAT0003314 | hsa-miR-644a | MIMAT0019833 | hsa-miR-4720-5p |
| MIMAT0003322 | hsa-miR-652-3p | MIMAT0019834 | hsa-miR-4720-3p |
| MIMAT0003323 | hsa-miR-548d-3p | MIMAT0019835 | hsa-miR-4721 |
| MIMAT0003326 | hsa-miR-663a | MIMAT0019836 | hsa-miR-4722-5p |
| MIMAT0003327 | hsa-miR-449b-5p | MIMAT0019837 | hsa-miR-4722-3p |

**[Table 1-9]**

| | | | |
|---|---|---|---|
| MIMAT0003329 | hsa-miR-411-5p | MIMAT0019838 | hsa-miR-4723-5p |
| MIMAT0003330 | hsa-miR-654-5p | MIMAT0019839 | hsa-miR-4723-3p |
| MIMAT0003333 | hsa-miR-549a | MIMAT0019840 | hsa-miR-451b |
| MIMAT0003337 | hsa-miR-659-3p | MIMAT0019841 | hsa-miR-4724-5p |
| MIMAT0003338 | hsa-miR-660-5p | MIMAT0019842 | hsa-miR-4724-3p |
| MIMAT0003385 | hsa-miR-363-5p | MIMAT0019843 | hsa-miR-4725-5p |
| MIMAT0003386 | hsa-miR-376a-5p | MIMAT0019844 | hsa-miR-4725-3p |
| MIMAT0003393 | hsa-miR-425-5p | MIMAT0019845 | hsa-miR-4726-5p |
| MIMAT0003879 | hsa-miR-758-3p | MIMAT0019846 | hsa-miR-4726-3p |
| MIMAT0003880 | hsa-miR-671-5p | MIMAT0019847 | hsa-miR-4727-5p |
| MIMAT0003884 | hsa-miR-454-5p | MIMAT0019848 | hsa-miR-4727-3p |
| MIMAT0003885 | hsa-miR-454-3p | MIMAT0019849 | hsa-miR-4728-5p |
| MIMAT0003888 | hsa-miR-766-3p | MIMAT0019850 | hsa-miR-4728-3p |
| MIMAT0004284 | hsa-miR-675-5p | MIMAT0019851 | hsa-miR-4729 |
| MIMAT0004481 | hsa-let-7a-3p | MIMAT0019852 | hsa-miR-4730 |
| MIMAT0004482 | hsa-let-7b-3p | MIMAT0019853 | hsa-miR-4731-5p |
| MIMAT0004484 | hsa-let-7d-3p | MIMAT0019854 | hsa-miR-4731-3p |
| MIMAT0004485 | hsa-let-7e-3p | MIMAT0019855 | hsa-miR-4732-5p |
| MIMAT0004486 | hsa-let-7f-1-3p | MIMAT0019856 | hsa-miR-4732-3p |
| MIMAT0004487 | hsa-let-7f-2-3p | MIMAT0019857 | hsa-miR-4733-5p |
| MIMAT0004488 | hsa-miR-15a-3p | MIMAT0019858 | hsa-miR-4733-3p |
| MIMAT0004489 | hsa-miR-16-1-3p | MIMAT0019859 | hsa-miR-4734 |
| MIMAT0004490 | hsa-miR-19a-5p | MIMAT0019860 | hsa-miR-4735-5p |
| MIMAT0004491 | hsa-miR-19b-1-5p | MIMAT0019861 | hsa-miR-4735-3p |
| MIMAT0004492 | hsa-miR-19b-2-5p | MIMAT0019862 | hsa-miR-4736 |
| MIMAT0004493 | hsa-miR-20a-3p | MIMAT0019863 | hsa-miR-4737 |
| MIMAT0004494 | hsa-miR-21-3p | MIMAT0019864 | hsa-miR-3064-5p |
| MIMAT0004495 | hsa-miR-22-5p | MIMAT0019865 | hsa-miR-3064-3p |
| MIMAT0004496 | hsa-miR-23a-5p | MIMAT0019866 | hsa-miR-4738-5p |
| MIMAT0004497 | hsa-miR-24-2-5p | MIMAT0019867 | hsa-miR-4738-3p |
| MIMAT0004498 | hsa-miR-25-5p | MIMAT0019868 | hsa-miR-4739 |
| MIMAT0004499 | hsa-miR-26a-1-3p | MIMAT0019869 | hsa-miR-4740-5p |
| MIMAT0004500 | hsa-miR-26b-3p | MIMAT0019870 | hsa-miR-4740-3p |
| MIMAT0004501 | hsa-miR-27a-5p | MIMAT0019871 | hsa-miR-4741 |
| MIMAT0004503 | hsa-miR-29a-5p | MIMAT0019872 | hsa-miR-4742-5p |
| MIMAT0004504 | hsa-miR-31-3p | MIMAT0019873 | hsa-miR-4742-3p |

**[Table 1-10]**

| | | | |
|---|---|---|---|
| MIMAT0004505 | hsa-miR-32-3p | MIMAT0019875 | hsa-miR-4744 |
| MIMAT0004506 | hsa-miR-33a-3p | MIMAT0019876 | hsa-miR-3591-5p |
| MIMAT0004507 | hsa-miR-92a-1-5p | MIMAT0019877 | hsa-miR-3591-3p |
| MIMAT0004508 | hsa-miR-92a-2-5p | MIMAT0019878 | hsa-miR-4745-5p |
| MIMAT0004509 | hsa-miR-93-3p | MIMAT0019879 | hsa-miR-4745-3p |
| MIMAT0004510 | hsa-miR-96-3p | MIMAT0019880 | hsa-miR-4746-5p |
| MIMAT0004511 | hsa-miR-99a-3p | MIMAT0019881 | hsa-miR-4746-3p |
| MIMAT0004512 | hsa-miR-100-3p | MIMAT0019882 | hsa-miR-4747-5p |
| MIMAT0004513 | hsa-miR-101-5p | MIMAT0019883 | hsa-miR-4747-3p |
| MIMAT0004514 | hsa-miR-29b-1-5p | MIMAT0019884 | hsa-miR-4748 |
| MIMAT0004515 | hsa-miR-29b-2-5p | MIMAT0019885 | hsa-miR-4749-5p |
| MIMAT0004516 | hsa-miR-105-3p | MIMAT0019886 | hsa-miR-4749-3p |
| MIMAT0004517 | hsa-miR-106a-3p | MIMAT0019888 | hsa-miR-4751 |
| MIMAT0004518 | hsa-miR-16-2-3p | MIMAT0019889 | hsa-miR-4752 |
| MIMAT0004543 | hsa-miR-192-3p | MIMAT0019890 | hsa-miR-4753-5p |
| MIMAT0004548 | hsa-miR-129-1-3p | MIMAT0019891 | hsa-miR-4753-3p |
| MIMAT0004549 | hsa-miR-148a-5p | MIMAT0019892 | hsa-miR-371b-5p |
| MIMAT0004550 | hsa-miR-30c-2-3p | MIMAT0019893 | hsa-miR-371b-3p |
| MIMAT0004551 | hsa-miR-30d-3p | MIMAT0019894 | hsa-miR-4754 |
| MIMAT0004553 | hsa-miR-7-1-3p | MIMAT0019895 | hsa-miR-4755-5p |
| MIMAT0004554 | hsa-miR-7-2-3p | MIMAT0019896 | hsa-miR-4755-3p |
| MIMAT0004555 | hsa-miR-10a-3p | MIMAT0019899 | hsa-miR-4756-5p |
| MIMAT0004556 | hsa-miR-10b-3p | MIMAT0019900 | hsa-miR-4756-3p |
| MIMAT0004557 | hsa-miR-34a-3p | MIMAT0019901 | hsa-miR-4757-5p |
| MIMAT0004558 | hsa-miR-181a-2-3p | MIMAT0019902 | hsa-miR-4757-3p |
| MIMAT0004559 | hsa-miR-181c-3p | MIMAT0019903 | hsa-miR-4758-5p |
| MIMAT0004560 | hsa-miR-183-3p | MIMAT0019904 | hsa-miR-4758-3p |
| MIMAT0004561 | hsa-miR-187-5p | MIMAT0019905 | hsa-miR-4759 |
| MIMAT0004562 | hsa-miR-196a-3p | MIMAT0019906 | hsa-miR-4760-5p |
| MIMAT0004564 | hsa-miR-214-5p | MIMAT0019907 | hsa-miR-4760-3p |
| MIMAT0004565 | hsa-miR-218-1-3p | MIMAT0019908 | hsa-miR-4761-5p |
| MIMAT0004566 | hsa-miR-218-2-3p | MIMAT0019909 | hsa-miR-4761-3p |
| MIMAT0004568 | hsa-miR-221-5p | MIMAT0019910 | hsa-miR-4762-5p |
| MIMAT0004569 | hsa-miR-222-5p | MIMAT0019911 | hsa-miR-4762-3p |
| MIMAT0004570 | hsa-miR-223-5p | MIMAT0019912 | hsa-miR-4763-5p |
| MIMAT0004571 | hsa-miR-200b-5p | MIMAT0019913 | hsa-miR-4763-3p |

**[Table 1-11]**

| | | | |
|---|---|---|---|
| MIMAT0004584 | hsa-let-7g-3p | MIMAT0019914 | hsa-miR-4764-5p |
| MIMAT0004585 | hsa-let-7i-3p | MIMAT0019915 | hsa-miR-4764-3p |
| MIMAT0004586 | hsa-miR-15b-3p | MIMAT0019916 | hsa-miR-4765 |
| MIMAT0004587 | hsa-miR-23b-5p | MIMAT0019917 | hsa-miR-4766-5p |
| MIMAT0004588 | hsa-miR-27b-5p | MIMAT0019918 | hsa-miR-4766-3p |
| MIMAT0004589 | hsa-miR-30b-3p | MIMAT0019919 | hsa-miR-4767 |
| MIMAT0004590 | hsa-miR-122-3p | MIMAT0019920 | hsa-miR-4768-5p |
| MIMAT0004591 | hsa-miR-124-5p | MIMAT0019921 | hsa-miR-4768-3p |
| MIMAT0004592 | hsa-miR-125b-1-3p | MIMAT0019922 | hsa-miR-4769-5p |
| MIMAT0004593 | hsa-miR-130a-5p | MIMAT0019923 | hsa-miR-4769-3p |
| MIMAT0004594 | hsa-miR-132-5p | MIMAT0019924 | hsa-miR-4770 |
| MIMAT0004595 | hsa-miR-135a-3p | MIMAT0019925 | hsa-miR-4771 |
| MIMAT0004596 | hsa-miR-138-2-3p | MIMAT0019926 | hsa-miR-4772-5p |
| MIMAT0004598 | hsa-miR-141-5p | MIMAT0019927 | hsa-miR-4772-3p |
| MIMAT0004599 | hsa-miR-143-5p | MIMAT0019928 | hsa-miR-4773 |
| MIMAT0004600 | hsa-miR-144-5p | MIMAT0019929 | hsa-miR-4774-5p |
| MIMAT0004601 | hsa-miR-145-3p | MIMAT0019930 | hsa-miR-4774-3p |
| MIMAT0004603 | hsa-miR-125b-2-3p | MIMAT0019931 | hsa-miR-4775 |
| MIMAT0004605 | hsa-miR-129-2-3p | MIMAT0019932 | hsa-miR-4776-5p |
| MIMAT0004606 | hsa-miR-136-3p | MIMAT0019933 | hsa-miR-4776-3p |
| MIMAT0004607 | hsa-miR-138-1-3p | MIMAT0019934 | hsa-miR-4777-5p |
| MIMAT0004608 | hsa-miR-146a-3p | MIMAT0019935 | hsa-miR-4777-3p |
| MIMAT0004609 | hsa-miR-149-3p | MIMAT0019936 | hsa-miR-4778-5p |
| MIMAT0004610 | hsa-miR-150-3p | MIMAT0019937 | hsa-miR-4778-3p |
| MIMAT0004611 | hsa-miR-185-3p | MIMAT0019938 | hsa-miR-4779 |
| MIMAT0004612 | hsa-miR-186-3p | MIMAT0019939 | hsa-miR-4780 |
| MIMAT0004615 | hsa-miR-195-3p | MIMAT0019940 | hsa-miR-4436b-5p |
| MIMAT0004657 | hsa-miR-200c-5p | MIMAT0019941 | hsa-miR-4436b-3p |
| MIMAT0004658 | hsa-miR-155-3p | MIMAT0019942 | hsa-miR-4781-5p |
| MIMAT0004671 | hsa-miR-194-3p | MIMAT0019943 | hsa-miR-4781-3p |
| MIMAT0004672 | hsa-miR-106b-3p | MIMAT0019944 | hsa-miR-4782-5p |
| MIMAT0004673 | hsa-miR-29c-5p | MIMAT0019945 | hsa-miR-4782-3p |
| MIMAT0004674 | hsa-miR-30c-1-3p | MIMAT0019946 | hsa-miR-4783-5p |
| MIMAT0004676 | hsa-miR-34b-3p | MIMAT0019947 | hsa-miR-4783-3p |
| MIMAT0004678 | hsa-miR-99b-3p | MIMAT0019948 | hsa-miR-4784 |
| MIMAT0004680 | hsa-miR-130b-5p | MIMAT0019949 | hsa-miR-4785 |

**[Table 1-12]**

| | | | |
|---|---|---|---|
| MIMAT0004681 | hsa-miR-26a-2-3p | MIMAT0019950 | hsa-miR-1245b-5p |
| MIMAT0004685 | hsa-miR-302d-5p | MIMAT0019951 | hsa-miR-1245b-3p |
| MIMAT0004686 | hsa-miR-367-5p | MIMAT0019952 | hsa-miR-2467-5p |
| MIMAT0004687 | hsa-miR-371a-5p | MIMAT0019953 | hsa-miR-2467-3p |
| MIMAT0004688 | hsa-miR-374a-3p | MIMAT0019954 | hsa-miR-4786-5p |
| MIMAT0004689 | hsa-miR-377-5p | MIMAT0019955 | hsa-miR-4786-3p |
| MIMAT0004690 | hsa-miR-379-3p | MIMAT0019956 | hsa-miR-4787-5p |
| MIMAT0004692 | hsa-miR-340-5p | MIMAT0019957 | hsa-miR-4787-3p |
| MIMAT0004696 | hsa-miR-323a-5p | MIMAT0019958 | hsa-miR-4788 |
| MIMAT0004697 | hsa-miR-151a-5p | MIMAT0019959 | hsa-miR-4789-5p |
| MIMAT0004698 | hsa-miR-135b-3p | MIMAT0019960 | hsa-miR-4789-3p |
| MIMAT0004699 | hsa-miR-148b-5p | MIMAT0019961 | hsa-miR-4790-5p |
| MIMAT0004703 | hsa-miR-335-3p | MIMAT0019962 | hsa-miR-4790-3p |
| MIMAT0004749 | hsa-miR-424-3p | MIMAT0019963 | hsa-miR-4791 |
| MIMAT0004751 | hsa-miR-18b-3p | MIMAT0019964 | hsa-miR-4792 |
| MIMAT0004752 | hsa-miR-20b-3p | MIMAT0019965 | hsa-miR-4793-5p |
| MIMAT0004757 | hsa-miR-431-3p | MIMAT0019966 | hsa-miR-4793-3p |
| MIMAT0004763 | hsa-miR-488-3p | MIMAT0019967 | hsa-miR-4794 |
| MIMAT0004767 | hsa-miR-193b-5p | MIMAT0019968 | hsa-miR-4795-5p |
| MIMAT0004768 | hsa-miR-497-3p | MIMAT0019969 | hsa-miR-4795-3p |
| MIMAT0004772 | hsa-miR-499a-3p | MIMAT0019970 | hsa-miR-4796-5p |
| MIMAT0004773 | hsa-miR-500a-5p | MIMAT0019971 | hsa-miR-4796-3p |
| MIMAT0004776 | hsa-miR-505-5p | MIMAT0019972 | hsa-miR-4797-5p |
| MIMAT0004777 | hsa-miR-513a-3p | MIMAT0019973 | hsa-miR-4797-3p |
| MIMAT0004785 | hsa-miR-545-5p | MIMAT0019974 | hsa-miR-4798-5p |
| MIMAT0004792 | hsa-miR-92b-5p | MIMAT0019975 | hsa-miR-4798-3p |
| MIMAT0004794 | hsa-miR-551b-5p | MIMAT0019976 | hsa-miR-4799-5p |
| MIMAT0004799 | hsa-miR-589-5p | MIMAT0019977 | hsa-miR-4799-3p |
| MIMAT0004800 | hsa-miR-550a-5p | MIMAT0019978 | hsa-miR-4800-5p |
| MIMAT0004802 | hsa-miR-593-3p | MIMAT0019979 | hsa-miR-4800-3p |
| MIMAT0004805 | hsa-miR-616-3p | MIMAT0019980 | hsa-miR-4801 |
| MIMAT0004807 | hsa-miR-624-3p | MIMAT0019981 | hsa-miR-4802-5p |
| MIMAT0004808 | hsa-miR-625-3p | MIMAT0019982 | hsa-miR-4802-3p |
| MIMAT0004810 | hsa-miR-629-5p | MIMAT0019983 | hsa-miR-4803 |
| MIMAT0004811 | hsa-miR-33b-3p | MIMAT0019984 | hsa-miR-4804-5p |
| MIMAT0004813 | hsa-miR-411-3p | MIMAT0019985 | hsa-miR-4804-3p |

**[Table 1-13]**

| | | | |
|---|---|---|---|
| MIMAT0004916 | hsa-miR-888-5p | MIMAT0020541 | hsa-miR-5047 |
| MIMAT0004917 | hsa-miR-888-3p | MIMAT0020600 | hsa-miR-5095 |
| MIMAT0004919 | hsa-miR-541-5p | MIMAT0020601 | hsa-miR-1273f |
| MIMAT0004920 | hsa-miR-541-3p | MIMAT0020603 | hsa-miR-5096 |
| MIMAT0004926 | hsa-miR-708-5p | MIMAT0020924 | hsa-miR-642a-3p |
| MIMAT0004927 | hsa-miR-708-3p | MIMAT0020925 | hsa-miR-550a-3-5p |
| MIMAT0004945 | hsa-miR-744-5p | MIMAT0020957 | hsa-miR-548ah-3p |
| MIMAT0004946 | hsa-miR-744-3p | MIMAT0020958 | hsa-miR-4482-3p |
| MIMAT0004949 | hsa-miR-877-5p | MIMAT0020959 | hsa-miR-4536-3p |
| MIMAT0004950 | hsa-miR-877-3p | MIMAT0021017 | hsa-miR-4999-5p |
| MIMAT0004953 | hsa-miR-873-5p | MIMAT0021018 | hsa-miR-4999-3p |
| MIMAT0004955 | hsa-miR-374b-5p | MIMAT0021019 | hsa-miR-5000-5p |
| MIMAT0004956 | hsa-miR-374b-3p | MIMAT0021020 | hsa-miR-5000-3p |
| MIMAT0004958 | hsa-miR-301b-3p | MIMAT0021021 | hsa-miR-5001-5p |
| MIMAT0004980 | hsa-miR-937-3p | MIMAT0021022 | hsa-miR-5001-3p |
| MIMAT0004982 | hsa-miR-939-5p | MIMAT0021023 | hsa-miR-5002-5p |
| MIMAT0005449 | hsa-miR-523-5p | MIMAT0021024 | hsa-miR-5002-3p |
| MIMAT0005450 | hsa-miR-518e-5p | MIMAT0021025 | hsa-miR-5003-5p |
| MIMAT0005451 | hsa-miR-522-5p | MIMAT0021026 | hsa-miR-5003-3p |
| MIMAT0005452 | hsa-miR-519a-5p | MIMAT0021027 | hsa-miR-5004-5p |
| MIMAT0005576 | hsa-miR-1226-5p | MIMAT0021028 | hsa-miR-5004-3p |
| MIMAT0005577 | hsa-miR-1226-3p | MIMAT0021029 | hsa-miR-548ao-5p |
| MIMAT0005580 | hsa-miR-1227-3p | MIMAT0021030 | hsa-miR-548ao-3p |
| MIMAT0005582 | hsa-miR-1228-5p | MIMAT0021033 | hsa-miR-5006-5p |
| MIMAT0005583 | hsa-miR-1228-3p | MIMAT0021034 | hsa-miR-5006-3p |
| MIMAT0005584 | hsa-miR-1229-3p | MIMAT0021035 | hsa-miR-5007-5p |
| MIMAT0005588 | hsa-miR-1233-3p | MIMAT0021036 | hsa-miR-5007-3p |
| MIMAT0005589 | hsa-miR-1234-3p | MIMAT0021037 | hsa-miR-548ap-5p |
| MIMAT0005591 | hsa-miR-1236-3p | MIMAT0021038 | hsa-miR-548ap-3p |
| MIMAT0005592 | hsa-miR-1237-3p | MIMAT0021039 | hsa-miR-5008-5p |
| MIMAT0005593 | hsa-miR-1238-3p | MIMAT0021040 | hsa-miR-5008-3p |
| MIMAT0005789 | hsa-miR-513c-5p | MIMAT0021041 | hsa-miR-5009-5p |
| MIMAT0005796 | hsa-miR-1271-5p | MIMAT0021042 | hsa-miR-5009-3p |
| MIMAT0005798 | hsa-miR-1185-5p | MIMAT0021043 | hsa-miR-5010-5p |
| MIMAT0005823 | hsa-miR-1178-3p | MIMAT0021044 | hsa-miR-5010-3p |
| MIMAT0005876 | hsa-miR-1285-3p | MIMAT0021045 | hsa-miR-5011-5p |

**[Table 1-14]**

| | | | |
|---|---|---|---|
| MIMAT0005885 | hsa-miR-1295a | MIMAT0021046 | hsa-miR-5011-3p |
| MIMAT0005892 | hsa-miR-1304-5p | MIMAT0021079 | hsa-miR-5087 |
| MIMAT0005897 | hsa-miR-1245a | MIMAT0021080 | hsa-miR-5088-5p |
| MIMAT0005899 | hsa-miR-1247-5p | MIMAT0021082 | hsa-miR-5090 |
| MIMAT0005901 | hsa-miR-1249-3p | MIMAT0021083 | hsa-miR-5091 |
| MIMAT0005911 | hsa-miR-1260a | MIMAT0021084 | hsa-miR-5092 |
| MIMAT0005912 | hsa-miR-548g-3p | MIMAT0021085 | hsa-miR-5093 |
| MIMAT0005919 | hsa-miR-548o-3p | MIMAT0021086 | hsa-miR-5094 |
| MIMAT0005922 | hsa-miR-1268a | MIMAT0021116 | hsa-miR-5186 |
| MIMAT0005923 | hsa-miR-1269a | MIMAT0021117 | hsa-miR-5187-5p |
| MIMAT0005926 | hsa-miR-1273a | MIMAT0021118 | hsa-miR-5187-3p |
| MIMAT0005928 | hsa-miR-548h-5p | MIMAT0021119 | hsa-miR-5188 |
| MIMAT0005933 | hsa-miR-1277-3p | MIMAT0021120 | hsa-miR-5189-5p |
| MIMAT0005943 | hsa-miR-1292-5p | MIMAT0021121 | hsa-miR-5190 |
| MIMAT0005945 | hsa-miR-1255b-5p | MIMAT0021122 | hsa-miR-5191 |
| MIMAT0005948 | hsa-miR-664a-5p | MIMAT0021123 | hsa-miR-5192 |
| MIMAT0005949 | hsa-miR-664a-3p | MIMAT0021124 | hsa-miR-5193 |
| MIMAT0005950 | hsa-miR-1306-3p | MIMAT0021125 | hsa-miR-5194 |
| MIMAT0005951 | hsa-miR-1307-3p | MIMAT0021126 | hsa-miR-5195-5p |
| MIMAT0006790 | hsa-miR-675-3p | MIMAT0021127 | hsa-miR-5195-3p |
| MIMAT0007402 | hsa-miR-103b | MIMAT0021128 | hsa-miR-5196-5p |
| MIMAT0007882 | hsa-miR-1909-5p | MIMAT0021129 | hsa-miR-5196-3p |
| MIMAT0007883 | hsa-miR-1909-3p | MIMAT0021130 | hsa-miR-5197-5p |
| MIMAT0007885 | hsa-miR-1911-5p | MIMAT0021131 | hsa-miR-5197-3p |
| MIMAT0007886 | hsa-miR-1911-3p | MIMAT0022255 | hsa-miR-4524b-5p |
| MIMAT0007889 | hsa-miR-1914-5p | MIMAT0022256 | hsa-miR-4524b-3p |
| MIMAT0007890 | hsa-miR-1914-3p | MIMAT0022257 | hsa-miR-5571-5p |
| MIMAT0007891 | hsa-miR-1915-5p | MIMAT0022258 | hsa-miR-5571-3p |
| MIMAT0007892 | hsa-miR-1915-3p | MIMAT0022259 | hsa-miR-5100 |
| MIMAT0009196 | hsa-miR-103a-2-5p | MIMAT0022260 | hsa-miR-5572 |
| MIMAT0009197 | hsa-miR-205-3p | MIMAT0022263 | hsa-miR-548aq-5p |
| MIMAT0009198 | hsa-miR-224-3p | MIMAT0022264 | hsa-miR-548aq-3p |
| MIMAT0009199 | hsa-miR-365a-5p | MIMAT0022265 | hsa-miR-548ar-5p |
| MIMAT0009201 | hsa-miR-196b-3p | MIMAT0022266 | hsa-miR-548ar-3p |
| MIMAT0009203 | hsa-miR-449b-3p | MIMAT0022267 | hsa-miR-548as-5p |
| MIMAT0010195 | hsa-let-7a-2-3p | MIMAT0022268 | hsa-miR-548as-3p |

**[Table 1-15]**

| | | | |
|---|---|---|---|
| MIMAT0010251 | hsa-miR-449c-5p | MIMAT0022269 | hsa-miR-5579-5p |
| MIMAT0011156 | hsa -miR-2114-5p | MIMAT0022270 | hsa-miR-5579-3p |
| MIMAT0011157 | hsa -miR-2114-3p | MIMAT0022273 | hsa-miR-5580-5p |
| MIMAT0011158 | hsa -miR-2115-5p | MIMAT0022274 | hsa-miR-5580-3p |
| MIMAT0011159 | hsa -miR-2115-3p | MIMAT0022275 | hsa-miR-5581-5p |
| MIMAT0011160 | hsa -miR-2116-5p | MIMAT0022276 | hsa-miR-5581-3p |
| MIMAT0011161 | hsa -miR-2116-3p | MIMAT0022277 | hsa-miR-548at-5p |
| MIMAT0011777 | hsa-miR-2277-3p | MIMAT0022278 | hsa-miR-548at-3p |
| MIMAT0013515 | hsa-miR-2681-5p | MIMAT0022279 | hsa-miR-5582-5p |
| MIMAT0013516 | hsa-miR-2681-3p | MIMAT0022280 | hsa-miR-5582-3p |
| MIMAT0013517 | hsa-miR-2682-5p | MIMAT0022281 | hsa-miR-5583-5p |
| MIMAT0013518 | hsa-miR-2682-3p | MIMAT0022282 | hsa-miR-5583-3p |
| MIMAT0013771 | hsa-miR-449c-3p | MIMAT0022283 | hsa-miR-5584-5p |
| MIMAT0014979 | hsa-miR-3117-3p | MIMAT0022284 | hsa-miR-5584-3p |
| MIMAT0014982 | hsa-miR-3120-3p | MIMAT0022285 | hsa-miR-5585-5p |
| MIMAT0014983 | hsa-miR-3121-3p | MIMAT0022286 | hsa-miR-5585-3p |
| MIMAT0014986 | hsa-miR-3124-5p | MIMAT0022287 | hsa-miR-5586-5p |
| MIMAT0014990 | hsa-miR-3127-5p | MIMAT0022288 | hsa-miR-5586-3p |
| MIMAT0014992 | hsa-miR-3129-5p | MIMAT0022289 | hsa-miR-5587-5p |
| MIMAT0015001 | hsa-miR-3135a | MIMAT0022290 | hsa-miR-5587-3p |
| MIMAT0015003 | hsa -miR-3136-5p | MIMAT0022291 | hsa-miR-548au-5p |
| MIMAT0015008 | hsa-miR-3140-3p | MIMAT0022292 | hsa-miR-548au-3p |
| MIMAT0015009 | hsa-miR-548t-5p | MIMAT0022293 | hsa-miR-1295b-5p |
| MIMAT0015016 | hsa -miR-3145-3p | MIMAT0022294 | hsa-miR-1295b-3p |
| MIMAT0015023 | hsa-miR-3150a-3p | MIMAT0022295 | hsa-miR-5588-5p |
| MIMAT0015025 | hsa-miR-3152-3p | MIMAT0022296 | hsa-miR-5588-3p |
| MIMAT0015027 | hsa-miR-3074-3p | MIMAT0022297 | hsa-miR-5589-5p |
| MIMAT0015029 | hsa-miR-3155a | MIMAT0022298 | hsa-miR-5589-3p |
| MIMAT0015030 | hsa-miR-3156-5p | MIMAT0022299 | hsa-miR-5590-5p |
| MIMAT0015031 | hsa-miR-3157-5p | MIMAT0022300 | hsa-miR-5590-3p |
| MIMAT0015032 | hsa-miR-3158-3p | MIMAT0022301 | hsa-miR-5591-5p |
| MIMAT0015034 | hsa-miR-3160-3p | MIMAT0022302 | hsa-miR-5591-3p |
| MIMAT0015036 | hsa-miR-3162-5p | MIMAT0022303 | hsa-miR-548av-5p |
| MIMAT0015048 | hsa-miR-3173-3p | MIMAT0022304 | hsa-miR-548av-3p |
| MIMAT0015054 | hsa-miR-3177-3p | MIMAT0022468 | hsa-miR-5680 |
| MIMAT0015064 | hsa-miR-3184-5p | MIMAT0022469 | hsa-miR-5681a |

**[Table 1-16]**

| | | | |
|---|---|---|---|
| MIMAT0015069 | hsa-miR-3187-3p | MIMAT0022470 | hsa-miR-5682 |
| MIMAT0015071 | hsa-miR-3189-3p | MIMAT0022471 | hsa-miR-548aw |
| MIMAT0015073 | hsa-miR-3190-5p | MIMAT0022472 | hsa-miR-5683 |
| MIMAT0015075 | hsa-miR-3191-3p | MIMAT0022473 | hsa-miR-5684 |
| MIMAT0015078 | hsa-miR-3194-5p | MIMAT0022474 | hsa-miR-548ax |
| MIMAT0015081 | hsa-miR-548x-3p | MIMAT0022475 | hsa-miR-5685 |
| MIMAT0015085 | hsa-miR-3200-3p | MIMAT0022476 | hsa-miR-5692c |
| MIMAT0016895 | hsa-miR-2355-5p | MIMAT0022478 | hsa-miR-5687 |
| MIMAT0017983 | hsa-miR-3606-5p | MIMAT0022479 | hsa-miR-5688 |
| MIMAT0017997 | hsa-miR-3617-5p | MIMAT0022480 | hsa-miR-5681b |
| MIMAT0017999 | hsa-miR-3619-5p | MIMAT0022481 | hsa-miR-5689 |
| MIMAT0018001 | hsa-miR-3620-3p | MIMAT0022482 | hsa-miR-5690 |
| MIMAT0018073 | hsa-miR-3653-3p | MIMAT0022483 | hsa-miR-5691 |
| MIMAT0018086 | hsa-miR-3664-5p | MIMAT0022484 | hsa-miR-5692a |
| MIMAT0018101 | hsa-miR-3677-3p | MIMAT0022485 | hsa-miR-4666b |
| MIMAT0018106 | hsa-miR-3680-5p | MIMAT0022486 | hsa-miR-5693 |
| MIMAT0018107 | hsa-miR-3680-3p | MIMAT0022487 | hsa-miR-5694 |
| MIMAT0018108 | hsa-miR-3681-5p | MIMAT0022488 | hsa-miR-5695 |
| MIMAT0018109 | hsa-miR-3681-3p | MIMAT0022489 | hsa-miR-5696 |
| MIMAT0018110 | hsa-miR-3682-3p | MIMAT0022490 | hsa-miR-5697 |
| MIMAT0018116 | hsa-miR-3688-3p | MIMAT0022491 | hsa-miR-5698 |
| MIMAT0018120 | hsa-miR-3691-5p | MIMAT0022492 | hsa-miR-5699-3p |
| MIMAT0018121 | hsa-miR-3692-5p | MIMAT0022493 | hsa-miR-5700 |
| MIMAT0018122 | hsa-miR-3692-3p | MIMAT0022494 | hsa-miR-5701 |
| MIMAT0018180 | hsa-miR-3689b-5p | MIMAT0022495 | hsa-miR-5702 |
| MIMAT0018181 | hsa-miR-3689b-3p | MIMAT0022496 | hsa-miR-5703 |
| MIMAT0018187 | hsa-miR-3913-5p | MIMAT0022497 | hsa-miR-5692b |
| MIMAT0018194 | hsa-miR-3150b-3p | MIMAT0022498 | hsa-miR-5704 |
| MIMAT0018197 | hsa-miR-3922-3p | MIMAT0022499 | hsa-miR-5705 |
| MIMAT0018200 | hsa-miR-3925-5p | MIMAT0022500 | hsa-miR-5706 |
| MIMAT0018202 | hsa-miR-3927-3p | MIMAT0022501 | hsa-miR-5707 |
| MIMAT0018203 | hsa-miR-676-5p | MIMAT0022502 | hsa-miR-5708 |
| MIMAT0018204 | hsa-miR-676-3p | MIMAT0022691 | hsa-miR-197-5p |
| MIMAT0018349 | hsa-miR-3934-5p | MIMAT0022692 | hsa-miR-181b-3p |
| MIMAT0018356 | hsa-miR-3940-3p | MIMAT0022693 | hsa-miR-204-3p |
| MIMAT0018358 | hsa-miR-3942-5p | MIMAT0022694 | hsa-miR-211-3p |

**[Table 1-17]**

| | | | |
|---|---|---|---|
| MIMAT0018360 | hsa-miR-3944-3p | MIMAT0022695 | hsa-miR-212-5p |
| MIMAT0018443 | hsa-miR-374c-5p | MIMAT0022696 | hsa-miR-301a-5p |
| MIMAT0018444 | hsa-miR-642b-3p | MIMAT0022697 | hsa-miR-382-3p |
| MIMAT0018445 | hsa-miR-550b-3p | MIMAT0022698 | hsa-miR-345-3p |
| MIMAT0018946 | hsa-miR-548ad-3p | MIMAT0022700 | hsa-miR-450a-1-3p |
| MIMAT0018949 | hsa-miR-4433a-3p | MIMAT0022701 | hsa-miR-506-5p |
| MIMAT0018954 | hsa-miR-548ae-3p | MIMAT0022702 | hsa-miR-514a-5p |
| MIMAT0018963 | hsa-miR-4445-5p | MIMAT0022705 | hsa-miR-539-3p |
| MIMAT0018964 | hsa-miR-4445-3p | MIMAT0022706 | hsa-miR-561-5p |
| MIMAT0018972 | hsa-miR-548ah-5p | MIMAT0022707 | hsa-miR-570-5p |
| MIMAT0018990 | hsa-miR-548aj-3p | MIMAT0022708 | hsa-miR-584-3p |
| MIMAT0019016 | hsa-miR-4482-5p | MIMAT0022709 | hsa-miR-652-5p |
| MIMAT0019019 | hsa-miR-4485-3p | MIMAT0022710 | hsa-miR-659-5p |
| MIMAT0019057 | hsa-miR-4520-3p | MIMAT0022711 | hsa-miR-660-3p |
| MIMAT0019062 | hsa-miR-4524a-5p | MIMAT0022712 | hsa-miR-1271-3p |
| MIMAT0019063 | hsa-miR-4524a-3p | MIMAT0022713 | hsa-miR-1185-2-3p |
| MIMAT0019076 | hsa-miR-548am-3p | MIMAT0022714 | hsa-miR-766-5p |
| MIMAT0019078 | hsa-miR-4536-5p | MIMAT0022717 | hsa-miR-873-3p |
| MIMAT0019235 | hsa-miR-4520-5p | MIMAT0022719 | hsa-miR-1285-5p |
| MIMAT0019688 | hsa-miR-4632-3p | MIMAT0022720 | hsa-miR-1304-3p |
| MIMAT0019741 | hsa-miR-4666a-5p | MIMAT0022721 | hsa-miR-1247-3p |
| MIMAT0019742 | hsa-miR-4666a-3p | MIMAT0022722 | hsa-miR-548g-5p |
| MIMAT0019813 | hsa-miR-203b-5p | MIMAT0022723 | hsa-miR-548h-3p |
| MIMAT0019814 | hsa-miR-203b-3p | MIMAT0022724 | hsa-miR-1277-5p |
| MIMAT0019828 | hsa-miR-3529-5p | MIMAT0022725 | hsa-miR-1255b-2-3p |
| MIMAT0019874 | hsa-miR-4743-5p | MIMAT0022726 | hsa-miR-1306-5p |
| MIMAT0019887 | hsa-miR-4750-5p | MIMAT0022727 | hsa-miR-1307-5p |
| MIMAT0019897 | hsa-miR-499b-5p | MIMAT0022728 | hsa-miR-513c-3p |
| MIMAT0019898 | hsa-miR-499b-3p | MIMAT0022730 | hsa-miR-548t-3p |
| MIMAT0020300 | hsa-miR-4520-2-3p | MIMAT0022731 | hsa-miR-3184-3p |
| MIMAT0020602 | hsa-miR-1273g-5p | MIMAT0022732 | hsa-miR-3191-5p |
| MIMAT0020956 | hsa-miR-4433a-5p | MIMAT0022733 | hsa-miR-548x-5p |
| MIMAT0021081 | hsa-miR-5089-5p | MIMAT0022735 | hsa-miR-374c-3p |
| MIMAT002227 1 | hsa-miR-664b-5p | MIMAT0022736 | hsa-miR-642b-5p |
| MIMAT0022272 | hsa-miR-664b-3p | MIMAT0022737 | hsa-miR-550b-2-5p |
| MIMAT0000064 | hsa-let-7c-5p | MIMAT0022738 | hsa-miR-548o-5p |

**[Table 1-18]**

| | | | |
|---|---|---|---|
| MIMAT0000094 | hsa-miR-95-3p | MIMAT0022739 | hsa-miR-548aj-5p |
| MIMAT0000104 | hsa-miR-107 | MIMAT0022740 | hsa-miR-548am-5p |
| MIMAT0000228 | hsa-miR-198 | MIMAT0022741 | hsa-miR-3529-3p |
| MIMAT0000267 | hsa-miR-210-3p | MIMAT0022742 | hsa-miR-1273g-3p |
| MIMAT0000272 | hsa-miR-215-5p | MIMAT0022833 | hsa-miR-365b-5p |
| MIMAT0000274 | hsa-miR-217 | MIMAT0022834 | hsa-miR-365b-3p |
| MIMAT0000427 | hsa-miR-133a-3p | MIMAT0022838 | hsa-miR-1185-1-3p |
| MIMAT0000429 | hsa-miR-137 | MIMAT0022839 | hsa-miR-3190-3p |
| MIMAT0000433 | hsa-miR-142-5p | MIMAT0022842 | hsa-miR-98-3p |
| MIMAT0000434 | hsa-miR-142-3p | MIMAT0022844 | hsa-miR-216a-3p |
| MIMAT0000438 | hsa-miR-152-3p | MIMAT0022861 | hsa-miR-376c-5p |
| MIMAT0000439 | hsa-miR-153-3p | MIMAT0022862 | hsa-miR-381-5p |
| MIMAT0000447 | hsa-miR-134-5p | MIMAT0022923 | hsa-miR-376b-5p |
| MIMAT0000454 | hsa-miR-184 | MIMAT0022924 | hsa-miR-495-5p |
| MIMAT0000462 | hsa-miR-206 | MIMAT0022925 | hsa-miR-503-3p |
| MIMAT0000722 | hsa-miR-370-3p | MIMAT0022928 | hsa-miR-376a-2-5p |
| MIMAT0000724 | hsa-miR-372-3p | MIMAT0022929 | hsa-miR-758-5p |
| MIMAT0000728 | hsa-miR-375 | MIMAT0022938 | hsa-miR-937-5p |
| MIMAT0000738 | hsa-miR-383-5p | MIMAT0022939 | hsa-miR-939-3p |
| MIMAT0000752 | hsa-miR-328-3p | MIMAT0022940 | hsa-miR-1178-5p |
| MIMAT0000756 | hsa-miR-326 | MIMAT0022941 | hsa-miR-1227-5p |
| MIMAT0000761 | hsa-miR-324-5p | MIMAT0022942 | hsa-miR-1229-5p |
| MIMAT0000762 | hsa-miR-324-3p | MIMAT0022943 | hsa-miR-1233-5p |
| MIMAT0000770 | hsa-miR-133b | MIMAT0022945 | hsa-miR-1236-5p |
| MIMAT0000771 | hsa-miR-325 | MIMAT0022946 | hsa-miR-1237-5p |
| MIMAT0000773 | hsa-miR-346 | MIMAT0022947 | hsa-miR-1238-5p |
| MIMAT0001075 | hsa-miR-384 | MIMAT0022948 | hsa-miR-1292-3p |
| MIMAT0001339 | hsa-miR-422a | MIMAT0022965 | hsa-miR-3606-3p |
| MIMAT0001532 | hsa-miR-448 | MIMAT0022966 | hsa-miR-3617-3p |
| MIMAT0001536 | hsa-miR-429 | MIMAT0022967 | hsa-miR-3620-5p |
| MIMAT0001621 | hsa-miR-369-5p | MIMAT0022970 | hsa-miR-3927-5p |
| MIMAT0001627 | hsa-miR-433-3p | MIMAT0022975 | hsa-miR-3934-3p |
| MIMAT0001629 | hsa-miR-329-3p | MIMAT0022977 | hsa-miR-4632-5p |
| MIMAT0001638 | hsa-miR-409-5p | MIMAT0022978 | hsa-miR-4743-3p |
| MIMAT0001639 | hsa-miR-409-3p | MIMAT0022979 | hsa-miR-4750-3p |
| MIMAT0002170 | hsa-miR-412-3p | MIMAT0022984 | hsa-miR-5089-3p |

**[Table 1-19]**

| | | | |
|---|---|---|---|
| MIMAT0002171 | hsa-miR-410-3p | MIMAT0023116 | hsa-miR-5739 |
| MIMAT0002174 | hsa-miR-484 | MIMAT0023252 | hsa-miR-5787 |
| MIMAT0002175 | hsa-miR-485-5p | MIMAT0023693 | hsa-miR-6068 |
| MIMAT0002176 | hsa-miR-485-3p | MIMAT0023694 | hsa-miR-6069 |
| MIMAT0002805 | hsa-miR-489-3p | MIMAT0023695 | hsa-miR-6070 |
| MIMAT0002808 | hsa-miR-511-5p | MIMAT0023696 | hsa-miR-6071 |
| MIMAT0002812 | hsa-miR-492 | MIMAT0023697 | hsa-miR-6072 |
| MIMAT0002816 | hsa-miR-494-3p | MIMAT0023698 | hsa-miR-6073 |
| MIMAT0002818 | hsa-miR-496 | MIMAT0023699 | hsa-miR-6074 |
| MIMAT0002821 | hsa-miR-181d-5p | MIMAT0023700 | hsa-miR-6075 |
| MIMAT0002822 | hsa-miR-512-5p | MIMAT0023701 | hsa-miR-6076 |
| MIMAT0002823 | hsa-miR-512-3p | MIMAT0023702 | hsa-miR-6077 |
| MIMAT0002824 | hsa-miR-498 | MIMAT0023703 | hsa-miR-6078 |
| MIMAT0002825 | hsa-miR-520e | MIMAT0023704 | hsa-miR-6079 |
| MIMAT0002826 | hsa-miR-515-5p | MIMAT0023705 | hsa-miR-6080 |
| MIMAT0002827 | hsa-miR-515-3p | MIMAT0023706 | hsa-miR-6081 |
| MIMAT0002830 | hsa-miR-520f-3p | MIMAT0023707 | hsa-miR-6082 |
| MIMAT0002843 | hsa-miR-520b | MIMAT0023708 | hsa-miR-6083 |
| MIMAT0002844 | hsa-miR-518b | MIMAT0023709 | hsa-miR-6084 |
| MIMAT0002845 | hsa-miR-526a | MIMAT0023710 | hsa-miR-6085 |
| MIMAT0002853 | hsa-miR-519d-3p | MIMAT0023711 | hsa-miR-6086 |
| MIMAT0002854 | hsa-miR-521 | MIMAT0023712 | hsa-miR-6087 |
| MIMAT0002858 | hsa-miR-520g-3p | MIMAT0023713 | hsa-miR-6088 |
| MIMAT0002862 | hsa-miR-527 | MIMAT0023714 | hsa-miR-6089 |
| MIMAT0002867 | hsa-miR-520h | MIMAT0023715 | hsa-miR-6090 |
| MIMAT0002875 | hsa-miR-504-5p | MIMAT0024597 | hsa-miR-6124 |
| MIMAT0002879 | hsa-miR-507 | MIMAT0024598 | hsa-miR-6125 |
| MIMAT0002882 | hsa-miR-510-5p | MIMAT0024599 | hsa-miR-6126 |
| MIMAT0002890 | hsa-miR-299-5p | MIMAT0024610 | hsa-miR-6127 |
| MIMAT0003180 | hsa-miR-487b-3p | MIMAT0024611 | hsa-miR-6128 |
| MIMAT0003214 | hsa-miR-551a | MIMAT0024612 | hsa-miR-378j |
| MIMAT0003215 | hsa-miR-552-3p | MIMAT0024613 | hsa-miR-6129 |
| MIMAT0003216 | hsa-miR-553 | MIMAT0024614 | hsa-miR-6130 |
| MIMAT0003217 | hsa-miR-554 | MIMAT0024615 | hsa-miR-6131 |
| MIMAT0003219 | hsa-miR-555 | MIMAT0024616 | hsa-miR-6132 |
| MIMAT0003221 | hsa-miR-557 | MIMAT0024617 | hsa-miR-6133 |

**[Table 1-20]**

| | | | |
|---|---|---|---|
| MIMAT0003222 | hsa-miR-558 | MIMAT0024618 | hsa-miR-6134 |
| MIMAT0003223 | hsa-miR-559 | MIMAT0024782 | hsa-miR-6165 |
| MIMAT0003226 | hsa-miR-562 | MIMAT0025450 | hsa-miR-6499-5p |
| MIMAT0003227 | hsa-miR-563 | MIMAT0025451 | hsa-miR-6499-3p |
| MIMAT0003228 | hsa-miR-564 | MIMAT0025452 | hsa-miR-548ay-5p |
| MIMAT0003230 | hsa-miR-566 | MIMAT0025453 | hsa-miR-548ay-3p |
| MIMAT0003231 | hsa-miR-567 | MIMAT0025454 | hsa-miR-6500-5p |
| MIMAT0003232 | hsa-miR-568 | MIMAT0025455 | hsa-miR-6500-3p |
| MIMAT0003234 | hsa-miR-569 | MIMAT0025456 | hsa-miR-548az-5p |
| MIMAT0003236 | hsa-miR-571 | MIMAT0025457 | hsa-miR-548az-3p |
| MIMAT0003237 | hsa-miR-572 | MIMAT0025458 | hsa-miR-6501-5p |
| MIMAT0003238 | hsa-miR-573 | MIMAT0025459 | hsa-miR-6501-3p |
| MIMAT0003240 | hsa-miR-575 | MIMAT0025460 | hsa-miR-6502-5p |
| MIMAT0003242 | hsa-miR-577 | MIMAT0025461 | hsa-miR-6502-3p |
| MIMAT0003243 | hsa-miR-578 | MIMAT0025462 | hsa-miR-6503-5p |
| MIMAT0003244 | hsa-miR-579-3p | MIMAT0025463 | hsa-miR-6503-3p |
| MIMAT0003245 | hsa-miR-580-3p | MIMAT0025464 | hsa-miR-6504-5p |
| MIMAT0003246 | hsa-miR-581 | MIMAT0025465 | hsa-miR-6504-3p |
| MIMAT0003248 | hsa-miR-583 | MIMAT0025466 | hsa-miR-6505-5p |
| MIMAT0003250 | hsa-miR-585-3p | MIMAT0025467 | hsa-miR-6505-3p |
| MIMAT0003252 | hsa-miR-586 | MIMAT0025468 | hsa-miR-6506-5p |
| MIMAT0003253 | hsa-miR-587 | MIMAT0025469 | hsa-miR-6506-3p |
| MIMAT0003255 | hsa-miR-588 | MIMAT0025470 | hsa-miR-6507-5p |
| MIMAT0003259 | hsa-miR-591 | MIMAT0025471 | hsa-miR-6507-3p |
| MIMAT0003260 | hsa-miR-592 | MIMAT0025472 | hsa-miR-6508-5p |
| MIMAT0003263 | hsa-miR-595 | MIMAT0025473 | hsa-miR-6508-3p |
| MIMAT0003264 | hsa-miR-596 | MIMAT0025474 | hsa-miR-6509-5p |
| MIMAT0003265 | hsa-miR-597-5p | MIMAT0025475 | hsa-miR-6509-3p |
| MIMAT0003266 | hsa-miR-598-3p | MIMAT0025476 | hsa-miR-6510-5p |
| MIMAT0003267 | hsa-miR-599 | MIMAT0025477 | hsa-miR-6510-3p |
| MIMAT0003268 | hsa-miR-600 | MIMAT0025478 | hsa-miR-6511a-5p |
| MIMAT0003269 | hsa-miR-601 | MIMAT0025479 | hsa-miR-6511a-3p |
| MIMAT0003270 | hsa-miR-602 | MIMAT0025480 | hsa-miR-6512-5p |
| MIMAT0003271 | hsa-miR-603 | MIMAT0025481 | hsa-miR-6512-3p |
| MIMAT0003272 | hsa-miR-604 | MIMAT0025482 | hsa-miR-6513-5p |
| MIMAT0003273 | hsa-miR-605-5p | MIMAT0025483 | hsa-miR-6513-3p |

**[Table 1-21]**

| | | | |
|---|---|---|---|
| MIMAT0003274 | hsa-miR-606 | MIMAT0025484 | hsa-miR-6514-5p |
| MIMAT0003275 | hsa-miR-607 | MIMAT0025485 | hsa-miR-6514-3p |
| MIMAT0003276 | hsa-miR-608 | MIMAT0025486 | hsa-miR-6515-5p |
| MIMAT0003277 | hsa-miR-609 | MIMAT0025487 | hsa-miR-6515-3p |
| MIMAT0003278 | hsa-miR-610 | MIMAT0025841 | hsa-miR-6715a-3p |
| MIMAT0003279 | hsa-miR-611 | MIMAT0025842 | hsa-miR-6715b-5p |
| MIMAT0003280 | hsa-miR-612 | MIMAT0025843 | hsa-miR-6715b-3p |
| MIMAT0003281 | hsa-miR-613 | MIMAT0025844 | hsa -miR-6716-5p |
| MIMAT0003282 | hsa-miR-614 | MIMAT0025845 | hsa -miR-6716-3p |
| MIMAT0003286 | hsa-miR-617 | MIMAT0025846 | hsa-miR-6717-5p |
| MIMAT0003287 | hsa-miR-618 | MIMAT0025847 | hsa-miR-6511b-5p |
| MIMAT0003288 | hsa-miR-619-3p | MIMAT0025848 | hsa-miR-6511b-3p |
| MIMAT0003289 | hsa-miR-620 | MIMAT0025849 | hsa -miR-6718-5p |
| MIMAT0003290 | hsa-miR-621 | MIMAT0025850 | hsa -miR-6719-3p |
| MIMAT0003291 | hsa-miR-622 | MIMAT0025851 | hsa-miR-672 0-3p |
| MIMAT0003292 | hsa-miR-623 | MIMAT0025852 | hsa-miR-6721-5p |
| MIMAT0003295 | hsa-miR-626 | MIMAT0025853 | hsa-miR-6722-5p |
| MIMAT0003296 | hsa-miR-627-5p | MIMAT0025854 | hsa-miR-6722-3p |
| MIMAT0003299 | hsa-miR-630 | MIMAT0025855 | hsa-miR-6723-5p |
| MIMAT0003300 | hsa-miR-631 | MIMAT0025856 | hsa-miR-6724-5p |
| MIMAT0003302 | hsa-miR-632 | MIMAT0025857 | hsa-miR-892c-5p |
| MIMAT0003303 | hsa-miR-633 | MIMAT0025858 | hsa-miR-892c-3p |
| MIMAT0003304 | hsa-miR-634 | MIMAT0026472 | hsa-let-7c-3p |
| MIMAT0003305 | hsa-miR-635 | MIMAT0026473 | hsa-miR-95-5p |
| MIMAT0003306 | hsa-miR-636 | MIMAT0026474 | hsa-miR-208a-5p |
| MIMAT0003307 | hsa-miR-637 | MIMAT0026475 | hsa-miR-210-5p |
| MIMAT0003308 | hsa-miR-638 | MIMAT0026476 | hsa-miR-215-3p |
| MIMAT0003309 | hsa-miR-639 | MIMAT0026477 | hsa-miR-128-1-5p |
| MIMAT0003310 | hsa-miR-640 | MIMAT0026478 | hsa-miR-133a-5p |
| MIMAT0003311 | hsa-miR-641 | MIMAT0026479 | hsa-miR-152-5p |
| MIMAT0003313 | hsa-miR-643 | MIMAT0026480 | hsa-miR-153-5p |
| MIMAT0003315 | hsa-miR-645 | MIMAT0026481 | hsa-miR-134-3p |
| MIMAT0003316 | hsa-miR-646 | MIMAT0026482 | hsa-miR-190a-3p |
| MIMAT0003317 | hsa-miR-647 | MIMAT0026483 | hsa-miR-370-5p |
| MIMAT0003318 | hsa-miR-648 | MIMAT0026484 | hsa-miR-372-5p |
| MIMAT0003319 | hsa-miR-649 | MIMAT0026485 | hsa-miR-383-3p |

**[Table 1-22]**

| | | | |
|---|---|---|---|
| MIMAT0003320 | hsa-miR-650 | MIMAT0026486 | hsa-miR-328-5p |
| MIMAT0003321 | hsa-miR-651-5p | MIMAT0026554 | hsa-miR-433-5p |
| MIMAT0003324 | hsa-miR-661 | MIMAT0026555 | hsa-miR-329-5p |
| MIMAT0003325 | hsa-miR-662 | MIMAT0026557 | hsa-miR-412-5p |
| MIMAT0003328 | hsa-miR-653-5p | MIMAT0026558 | hsa-miR-410-5p |
| MIMAT0003331 | hsa-miR-655-3p | MIMAT0026559 | hsa-miR-487a-5p |
| MIMAT0003332 | hsa-miR-656-3p | MIMAT0026605 | hsa-miR-489-5p |
| MIMAT0003335 | hsa-miR-657 | MIMAT0026606 | hsa-miR-311-3p |
| MIMAT0003336 | hsa-miR-658 | MIMAT0026607 | hsa-miR-494-5p |
| MIMAT0003339 | hsa-miR-421 | MIMAT0026608 | hsa-miR-181d-3p |
| MIMAT0003340 | hsa-miR-542-5p | MIMAT0026609 | hsa-miR-520f-5p |
| MIMAT0003389 | hsa-miR-542-3p | MIMAT0026610 | hsa-miR-519d-5p |
| MIMAT0003881 | hsa-miR-668-3p | MIMAT0026611 | hsa-miR-520g-5p |
| MIMAT0003882 | hsa-miR-767-5p | MIMAT0026612 | hsa-miR-504-3p |
| MIMAT0003883 | hsa-miR-767-3p | MIMAT0026613 | hsa-miR-510-3p |
| MIMAT0003886 | hsa-miR-769-5p | MIMAT0026614 | hsa-miR-487b-5p |
| MIMAT0003887 | hsa-miR-769-3p | MIMAT0026615 | hsa-miR-552-5p |
| MIMAT0003945 | hsa-miR-765 | MIMAT0026616 | hsa-miR-579-5p |
| MIMAT0003948 | hsa-miR-770-5p | MIMAT0026617 | hsa-miR-580-5p |
| MIMAT0004185 | hsa-miR-802 | MIMAT0026618 | hsa-miR-585-5p |
| MIMAT0004450 | hsa-miR-297 | MIMAT0026619 | hsa-miR-597-3p |
| MIMAT0004502 | hsa-miR-28-3p | MIMAT0026620 | hsa-miR-598-5p |
| MIMAT0004552 | hsa-miR-139-3p | MIMAT0026621 | hsa-miR-605-3p |
| MIMAT0004563 | hsa-miR-199b-3p | MIMAT0026622 | hsa-miR-619-5p |
| MIMAT0004567 | hsa-miR-219a-1-3p | MIMAT0026623 | hsa-miR-627-3p |
| MIMAT0004597 | hsa-miR-140-3p | MIMAT0026624 | hsa-miR-651-3p |
| MIMAT0004602 | hsa-miR-125a-3p | MIMAT0026625 | hsa-miR-653-3p |
| MIMAT0004604 | hsa-miR-127-5p | MIMAT0026626 | hsa-miR-655-5p |
| MIMAT0004613 | hsa-miR-188-3p | MIMAT0026627 | hsa-miR-656-5p |
| MIMAT0004614 | hsa-miR-193a-5p | MIMAT0026636 | hsa-miR-668-5p |
| MIMAT0004675 | hsa-miR-219a-2-3p | MIMAT0026637 | hsa-miR-1296-3p |
| MIMAT0004677 | hsa-miR-34c-3p | MIMAT0026638 | hsa-miR-1468-3p |
| MIMAT0004679 | hsa-miR-296-3p | MIMAT0026639 | hsa-miR-1301-5p |
| MIMAT0004682 | hsa-miR-361-3p | MIMAT0026640 | hsa-miR-670-3p |
| MIMAT0004683 | hsa-miR-362-3p | MIMAT0026641 | hsa-miR-1298-3p |
| MIMAT0004693 | hsa-miR-330-5p | MIMAT0026717 | hsa-miR-891a-3p |

**[Table 1-23]**

| | | | |
|---|---|---|---|
| MIMAT0004694 | hsa-miR-342-5p | MIMAT0026718 | hsa-miR-874-5p |
| MIMAT0004695 | hsa-miR-337-5p | MIMAT0026719 | hsa-miR-889-5p |
| MIMAT0004700 | hsa-miR-331-5p | MIMAT0026720 | hsa-miR-887-5p |
| MIMAT0004701 | hsa-miR-338-5p | MIMAT0026721 | hsa-miR-216b-3p |
| MIMAT0004702 | hsa-miR-339-3p | MIMAT0026722 | hsa-miR-208b-5p |
| MIMAT0004748 | hsa-miR-423-5p | MIMAT0026734 | hsa-miR-942-3p |
| MIMAT0004761 | hsa-miR-483-5p | MIMAT0026735 | hsa-miR-1180-5p |
| MIMAT0004762 | hsa-miR-486-3p | MIMAT0026736 | hsa-miR-548e-5p |
| MIMAT0004764 | hsa-miR-490-5p | MIMAT0026737 | hsa-miR-548j-3p |
| MIMAT0004765 | hsa-miR-491-3p | MIMAT0026738 | hsa-miR-1287-3p |
| MIMAT0004766 | hsa-miR-146b-3p | MIMAT0026739 | hsa-miR-548f-5p |
| MIMAT0004770 | hsa-miR-516a-5p | MIMAT0026740 | hsa-miR-1250-3p |
| MIMAT0004774 | hsa-miR-501-3p | MIMAT0026741 | hsa-miR-1251-3p |
| MIMAT0004775 | hsa-miR-502-3p | MIMAT0026742 | hsa-miR-1266-3p |
| MIMAT0004778 | hsa-miR-508-5p | MIMAT0026743 | hsa-miR-1288-5p |
| MIMAT0004779 | hsa-miR-509-5p | MIMAT0026744 | hsa-miR-1252-3p |
| MIMAT0004780 | hsa-miR-532-3p | MIMAT0026749 | hsa-miR-513b-3p |
| MIMAT0004784 | hsa-miR-455-3p | MIMAT0026765 | hsa-miR-1537-5p |
| MIMAT0004793 | hsa-miR-556-3p | MIMAT0026916 | hsa-miR-1908-3p |
| MIMAT0004795 | hsa-miR-574-5p | MIMAT0026917 | hsa-miR-1910-3p |
| MIMAT0004796 | hsa-miR-576-3p | MIMAT0026921 | hsa-miR-2276-5p |
| MIMAT0004797 | hsa-miR-582-3p | MIMAT0027026 | hsa-miR-3151-3p |
| MIMAT0004798 | hsa-miR-548b-5p | MIMAT0027027 | hsa-miR-3192-3p |
| MIMAT0004801 | hsa-miR-590-3p | MIMAT0027032 | hsa-miR-500b-3p |
| MIMAT0004803 | hsa-miR-548a-5p | MIMAT0027036 | hsa-miR-3912-5p |
| MIMAT0004804 | hsa-miR-615-5p | MIMAT0027037 | hsa-miR-3928-5p |
| MIMAT0004806 | hsa-miR-548c-5p | MIMAT0027038 | hsa-miR-1343-5p |
| MIMAT0004809 | hsa-miR-628-5p | MIMAT0027041 | hsa-miR-5088-3p |
| MIMAT0004812 | hsa-miR-548d-5p | MIMAT0027088 | hsa-miR-5189-3p |
| MIMAT0004814 | hsa-miR-654-3p | MIMAT0027103 | hsa-miR-5699-5p |
| MIMAT0004819 | hsa-miR-671-3p | MIMAT0027345 | hsa-miR-6720-5p |
| MIMAT0004901 | hsa-miR-298 | MIMAT0027353 | hsa-miR-6726-5p |
| MIMAT0004902 | hsa-miR-891a-5p | MIMAT0027354 | hsa-miR-6726-3p |
| MIMAT0004903 | hsa-miR-300 | MIMAT0027355 | hsa-miR-6727-5p |
| MIMAT0004907 | hsa-miR-892a | MIMAT0027356 | hsa-miR-6727-3p |
| MIMAT0004909 | hsa-miR-450b-5p | MIMAT0027357 | hsa-miR-6728-5p |

**[Table 1-24]**

| | | | |
|---|---|---|---|
| MIMAT0004910 | hsa-miR-450b-3p | MIMAT0027358 | hsa-miR-6728-3p |
| MIMAT0004911 | hsa-miR-874-3p | MIMAT0027359 | hsa-miR-6729-5p |
| MIMAT0004912 | hsa-miR-890 | MIMAT0027360 | hsa-miR-6729-3p |
| MIMAT0004913 | hsa-miR-891b | MIMAT0027361 | hsa-miR-6730-5p |
| MIMAT0004918 | hsa-miR-892b | MIMAT0027362 | hsa-miR-6730-3p |
| MIMAT0004921 | hsa-miR-889-3p | MIMAT0027363 | hsa-miR-6731-5p |
| MIMAT0004922 | hsa-miR-875-5p | MIMAT0027364 | hsa-miR-6731-3p |
| MIMAT0004923 | hsa-miR-875-3p | MIMAT0027365 | hsa-miR-6732-5p |
| MIMAT0004924 | hsa-miR-876-5p | MIMAT0027366 | hsa-miR-6732-3p |
| MIMAT0004925 | hsa-miR-876-3p | MIMAT0027367 | hsa-miR-6733-5p |
| MIMAT0004928 | hsa-miR-147b | MIMAT0027368 | hsa-miR-6733-3p |
| MIMAT0004929 | hsa-miR-190b | MIMAT0027369 | hsa-miR-6734-5p |
| MIMAT0004947 | hsa-miR-885-5p | MIMAT0027370 | hsa-miR-6734-3p |
| MIMAT0004948 | hsa-miR-885-3p | MIMAT0027371 | hsa-miR-6735-5p |
| MIMAT0004951 | hsa-miR-887-3p | MIMAT0027372 | hsa-miR-6735-3p |
| MIMAT0004952 | hsa-miR-665 | MIMAT0027373 | hsa-miR-6736-5p |
| MIMAT0004954 | hsa-miR-543 | MIMAT0027374 | hsa-miR-6736-3p |
| MIMAT0004957 | hsa-miR-760 | MIMAT0027375 | hsa-miR-6737-5p |
| MIMAT0004959 | hsa-miR-216b-5p | MIMAT0027376 | hsa-miR-6737-3p |
| MIMAT0004960 | hsa-miR-208b-3p | MIMAT0027377 | hsa-miR-6738-5p |
| MIMAT0004970 | hsa-miR-920 | MIMAT0027378 | hsa-miR-6738-3p |
| MIMAT0004971 | hsa-miR-921 | MIMAT0027379 | hsa-miR-6739-5p |
| MIMAT0004972 | hsa-miR-922 | MIMAT0027380 | hsa-miR-6739-3p |
| MIMAT0004974 | hsa-miR-924 | MIMAT0027381 | hsa-miR-6740-5p |
| MIMAT0004975 | hsa-miR-509-3-5p | MIMAT0027382 | hsa-miR-6740-3p |
| MIMAT0004976 | hsa-miR-933 | MIMAT0027383 | hsa-miR-6741-5p |
| MIMAT0004977 | hsa-miR-934 | MIMAT0027384 | hsa-miR-6741-3p |
| MIMAT0004978 | hsa-miR-935 | MIMAT0027385 | hsa-miR-6742-5p |
| MIMAT0004979 | hsa-miR-936 | MIMAT0027386 | hsa-miR-6742-3p |
| MIMAT0004981 | hsa-miR-938 | MIMAT0027387 | hsa-miR-6743-5p |
| MIMAT0004983 | hsa-miR-940 | MIMAT0027388 | hsa-miR-6743-3p |
| MIMAT0004984 | hsa-miR-941 | MIMAT0027389 | hsa-miR-6744-5p |
| MIMAT0004985 | hsa-miR-942-5p | MIMAT0027390 | hsa-miR-6744-3p |
| MIMAT0004986 | hsa-miR-943 | MIMAT0027391 | hsa-miR-6745 |
| MIMAT0004987 | hsa-miR-944 | MIMAT0027392 | hsa-miR-6746-5p |
| MIMAT0005454 | hsa-miR-519b-5p | MIMAT0027393 | hsa-miR-6746-3p |

**[Table 1-25]**

| | | | |
|---|---|---|---|
| MIMAT0005455 | hsa-miR-520c-5p | MIMAT0027394 | hsa-miR-6747-5p |
| MIMAT0005456 | hsa-miR-518d-5p | MIMAT0027395 | hsa-miR-6747-3p |
| MIMAT0005457 | hsa-miR-518a-5p | MIMAT0027396 | hsa-miR-6748-5p |
| MIMAT0005458 | hsa-miR-1224-5p | MIMAT0027397 | hsa-miR-6748-3p |
| MIMAT0005459 | hsa-miR-1224-3p | MIMAT0027398 | hsa-miR-6749-5p |
| MIMAT0005572 | hsa-miR-1225-5p | MIMAT0027399 | hsa-miR-6749-3p |
| MIMAT0005573 | hsa-miR-1225-3p | MIMAT0027400 | hsa-miR-6750-5p |
| MIMAT0005586 | hsa-miR-1231 | MIMAT0027401 | hsa-miR-6750-3p |
| MIMAT0005788 | hsa-miR-513b-5p | MIMAT0027402 | hsa-miR-6751-5p |
| MIMAT0005791 | hsa-miR-1264 | MIMAT0027403 | hsa-miR-6751-3p |
| MIMAT0005792 | hsa-miR-320b | MIMAT0027404 | hsa-miR-6752-5p |
| MIMAT0005793 | hsa-miR-320c | MIMAT0027405 | hsa-miR-6752-3p |
| MIMAT0005794 | hsa-miR-1296-5p | MIMAT0027406 | hsa-miR-6753-5p |
| MIMAT0005795 | hsa-miR-1323 | MIMAT0027407 | hsa-miR-6753-3p |
| MIMAT0005797 | hsa-miR-1301-3p | MIMAT0027408 | hsa-miR-6754-5p |
| MIMAT0005799 | hsa-miR-1283 | MIMAT0027409 | hsa-miR-6754-3p |
| MIMAT0005800 | hsa-miR-1298-5p | MIMAT0027410 | hsa-miR-6755-5p |
| MIMAT0005824 | hsa-miR-1179 | MIMAT0027411 | hsa-miR-6755-3p |
| MIMAT0005825 | hsa-miR-1180-3p | MIMAT0027412 | hsa-miR-6756-5p |
| MIMAT0005826 | hsa-miR-1181 | MIMAT0027413 | hsa-miR-6756-3p |
| MIMAT0005827 | hsa-miR-1182 | MIMAT0027414 | hsa-miR-6757-5p |
| MIMAT0005828 | hsa-miR-1183 | MIMAT0027415 | hsa-miR-6757-3p |
| MIMAT0005829 | hsa-miR-1184 | MIMAT0027416 | hsa-miR-6758-5p |
| MIMAT0005863 | hsa-miR-1200 | MIMAT0027417 | hsa-miR-6758-3p |
| MIMAT0005865 | hsa-miR-1202 | MIMAT0027418 | hsa-miR-6759-5p |
| MIMAT0005866 | hsa-miR-1203 | MIMAT0027419 | hsa-miR-6759-3p |
| MIMAT0005867 | hsa-miR-663b | MIMAT0027420 | hsa-miR-6760-5p |
| MIMAT0005868 | hsa-miR-1204 | MIMAT0027421 | hsa-miR-6760-3p |
| MIMAT0005869 | hsa-miR-1205 | MIMAT0027422 | hsa-miR-6761-5p |
| MIMAT0005870 | hsa-miR-1206 | MIMAT0027423 | hsa-miR-6761-3p |
| MIMAT0005871 | hsa-miR-1207-5p | MIMAT0027424 | hsa-miR-6762-5p |
| MIMAT0005872 | hsa-miR-1207-3p | MIMAT0027425 | hsa-miR-6762-3p |
| MIMAT0005873 | hsa-miR-1208 | MIMAT0027426 | hsa-miR-6763-5p |
| MIMAT0005874 | hsa-miR-548e-3p | MIMAT0027427 | hsa-miR-6763-3p |
| MIMAT0005875 | hsa-miR-548j-5p | MIMAT0027428 | hsa-miR-6764-5p |
| MIMAT0005877 | hsa-miR-1286 | MIMAT0027429 | hsa-miR-6764-3p |

**[Table 1-26]**

| | | | |
|---|---|---|---|
| MIMAT0005878 | hsa-miR-1287-5p | MIMAT0027430 | hsa-miR-6765-5p |
| MIMAT0005879 | hsa-miR-1289 | MIMAT0027431 | hsa-miR-6765-3p |
| MIMAT0005880 | hsa-miR-1290 | MIMAT0027432 | hsa-miR-6766-5p |
| MIMAT0005881 | hsa-miR-1291 | MIMAT0027433 | hsa-miR-6766-3p |
| MIMAT0005882 | hsa-miR-548k | MIMAT0027434 | hsa-miR-6767-5p |
| MIMAT0005883 | hsa-miR-1293 | MIMAT0027435 | hsa-miR-6767-3p |
| MIMAT0005884 | hsa-miR-1294 | MIMAT0027436 | hsa-miR-6768-5p |
| MIMAT0005886 | hsa-miR-1297 | MIMAT0027437 | hsa-miR-6768-3p |
| MIMAT0005887 | hsa-miR-1299 | MIMAT0027438 | hsa-miR-6769a-5p |
| MIMAT0005889 | hsa-miR-5481 | MIMAT0027439 | hsa-miR-6769a-3p |
| MIMAT0005890 | hsa-miR-1302 | MIMAT0027440 | hsa-miR-6770-5p |
| MIMAT0005891 | hsa-miR-1303 | MIMAT0027441 | hsa-miR-6770-3p |
| MIMAT0005893 | hsa-miR-1305 | MIMAT0027442 | hsa-miR-6771-5p |
| MIMAT0005894 | hsa-miR-1243 | MIMAT0027443 | hsa-miR-6771-3p |
| MIMAT0005895 | hsa-miR-548f-3p | MIMAT0027444 | hsa-miR-6772-5p |
| MIMAT0005896 | hsa-miR-1244 | MIMAT0027445 | hsa-miR-6772-3p |
| MIMAT0005898 | hsa-miR-1246 | MIMAT0027446 | hsa-miR-6773-5p |
| MIMAT0005900 | hsa-miR-1248 | MIMAT0027447 | hsa-miR-6773-3p |
| MIMAT0005902 | hsa-miR-1250-5p | MIMAT0027448 | hsa-miR-6774-5p |
| MIMAT0005903 | hsa-miR-1251-5p | MIMAT0027449 | hsa-miR-6774-3p |
| MIMAT0005904 | hsa-miR-1253 | MIMAT0027450 | hsa-miR-6775-5p |
| MIMAT0005905 | hsa-miR-1254 | MIMAT0027451 | hsa-miR-6775-3p |
| MIMAT0005906 | hsa-miR-1255a | MIMAT0027452 | hsa-miR-6776-5p |
| MIMAT0005907 | hsa-miR-1256 | MIMAT0027453 | hsa-miR-6776-3p |
| MIMAT0005908 | hsa-miR-1257 | MIMAT0027454 | hsa-miR-6777-5p |
| MIMAT0005909 | hsa-miR-1258 | MIMAT0027455 | hsa-miR-6777-3p |
| MIMAT0005913 | hsa-miR-1261 | MIMAT0027456 | hsa-miR-6778-5p |
| MIMAT0005914 | hsa-miR-1262 | MIMAT0027457 | hsa-miR-6778-3p |
| MIMAT0005915 | hsa-miR-1263 | MIMAT0027458 | hsa-miR-6779-5p |
| MIMAT0005916 | hsa-miR-548n | MIMAT0027459 | hsa-miR-6779-3p |
| MIMAT0005917 | hsa-miR-548m | MIMAT0027460 | hsa-miR-6780a-5p |
| MIMAT0005918 | hsa-miR-1265 | MIMAT0027461 | hsa-miR-6780a-3p |
| MIMAT0005920 | hsa-miR-1266-5p | MIMAT0027462 | hsa-miR-6781-5p |
| MIMAT0005921 | hsa-miR-1267 | MIMAT0027463 | hsa-miR-6781-3p |
| MIMAT0005924 | hsa-miR-1270 | MIMAT0027464 | hsa-miR-6782-5p |
| MIMAT0005925 | hsa-miR-1272 | MIMAT0027465 | hsa-miR-6782-3p |

**[Table 1-27]**

| | | | |
|---|---|---|---|
| MIMAT0005929 | hsa-miR-1275 | MIMAT0027466 | hsa-miR-6783-5p |
| MIMAT0005930 | hsa-miR-1276 | MIMAT0027467 | hsa-miR-6783-3p |
| MIMAT0005931 | hsa-miR-302e | MIMAT0027468 | hsa-miR-6784-5p |
| MIMAT0005932 | hsa-miR-302f | MIMAT0027469 | hsa-miR-6784-3p |
| MIMAT0005934 | hsa-miR-548p | MIMAT0027470 | hsa-miR-6785-5p |
| MIMAT0005935 | hsa-miR-548i | MIMAT0027471 | hsa-miR-6785-3p |
| MIMAT0005936 | hsa-miR-1278 | MIMAT0027472 | hsa-miR-6786-5p |
| MIMAT0005937 | hsa-miR-1279 | MIMAT0027473 | hsa-miR-6786-3p |
| MIMAT0005939 | hsa-miR-1281 | MIMAT0027474 | hsa-miR-6787-5p |
| MIMAT0005940 | hsa-miR-1282 | MIMAT0027475 | hsa-miR-6787-3p |
| MIMAT0005941 | hsa-miR-1284 | MIMAT0027476 | hsa-miR-6788-5p |
| MIMAT0005942 | hsa-miR-1288-3p | MIMAT0027477 | hsa-miR-6788-3p |
| MIMAT0005944 | hsa-miR-1252-5p | MIMAT0027478 | hsa-miR-6789-5p |
| MIMAT0005952 | hsa-miR-1321 | MIMAT0027479 | hsa-miR-6789-3p |
| MIMAT0005953 | hsa-miR-1322 | MIMAT0027480 | hsa-miR-6790-5p |
| MIMAT0005955 | hsa-miR-1197 | MIMAT0027481 | hsa-miR-6790-3p |
| MIMAT0005956 | hsa-miR-1324 | MIMAT0027482 | hsa-miR-6791-5p |
| MIMAT0006764 | hsa-miR-320d | MIMAT0027483 | hsa-miR-6791-3p |
| MIMAT0006765 | hsa-miR-1825 | MIMAT0027484 | hsa-miR-6792-5p |
| MIMAT0006767 | hsa-miR-1827 | MIMAT0027485 | hsa-miR-6792-3p |
| MIMAT0006778 | hsa-miR-516a-3p | MIMAT0027486 | hsa-miR-6793-5p |
| MIMAT0006789 | hsa -miR-1468-5p | MIMAT0027487 | hsa-miR-6793-3p |
| MIMAT0007347 | hsa-miR-1469 | MIMAT0027488 | hsa-miR-6794-5p |
| MIMAT0007348 | hsa-miR-1470 | MIMAT0027489 | hsa-miR-6794-3p |
| MIMAT0007349 | hsa-miR-1471 | MIMAT0027490 | hsa-miR-6795-5p |
| MIMAT0007399 | hsa-miR-1537-3p | MIMAT0027491 | hsa-miR-6795-3p |
| MIMAT0007400 | hsa-miR-1538 | MIMAT0027492 | hsa-miR-6796-5p |
| MIMAT0007401 | hsa-miR-1539 | MIMAT0027493 | hsa-miR-6796-3p |
| MIMAT0007881 | hsa-miR-1908-5p | MIMAT0027494 | hsa-miR-6797-5p |
| MIMAT0007884 | hsa-miR-1910-5p | MIMAT0027495 | hsa-miR-6797-3p |
| MIMAT0007887 | hsa-miR-1912 | MIMAT0027496 | hsa-miR-6798-5p |
| MIMAT0007888 | hsa-miR-1913 | MIMAT0027497 | hsa-miR-6798-3p |
| MIMAT0009206 | hsa-miR-2113 | MIMAT0027498 | hsa-miR-6799-5p |
| MIMAT0009447 | hsa-miR-1972 | MIMAT0027499 | hsa-miR-6799-3p |
| MIMAT0009448 | hsa-miR-1973 | MIMAT0027500 | hsa-miR-6800-5p |
| MIMAT0009451 | hsa-miR-1976 | MIMAT0027501 | hsa-miR-6800-3p |

**[Table 1-28]**

| | | | |
|---|---|---|---|
| MIMAT0009977 | hsa-miR-2052 | MIMAT0027502 | hsa-miR-6801-5p |
| MIMAT0009978 | hsa-miR-2053 | MIMAT0027503 | hsa-miR-6801-3p |
| MIMAT0009979 | hsa-miR-2054 | MIMAT0027504 | hsa-miR-6802-5p |
| MIMAT0010133 | hsa-miR-2110 | MIMAT0027505 | hsa-miR-6802-3p |
| MIMAT0010214 | hsa-miR-151b | MIMAT0027506 | hsa-miR-6803-5p |
| MIMAT0010313 | hsa-miR-762 | MIMAT0027507 | hsa-miR-6803-3p |
| MIMAT0010357 | hsa-miR-670-5p | MIMAT0027508 | hsa-miR-6804-5p |
| MIMAT0010364 | hsa-miR-761 | MIMAT0027509 | hsa-miR-6804-3p |
| MIMAT0010367 | hsa-miR-764 | MIMAT0027510 | hsa-miR-6805-5p |
| MIMAT0010497 | hsa-miR-759 | MIMAT0027511 | hsa-miR-6805-3p |
| MIMAT0011162 | hsa-miR-2117 | MIMAT0027512 | hsa-miR-6806-5p |
| MIMAT0011163 | hsa-miR-548q | MIMAT0027513 | hsa-miR-6806-3p |
| MIMAT0011775 | hsa-miR-2276-3p | MIMAT0027514 | hsa-miR-6807-5p |
| MIMAT0011778 | hsa-miR-2278 | MIMAT0027515 | hsa-miR-6807-3p |
| MIMAT0012734 | hsa-miR-711 | MIMAT0027516 | hsa-miR-6808-5p |
| MIMAT0012735 | hsa-miR-718 | MIMAT0027517 | hsa-miR-6808-3p |
| MIMAT0013802 | hsa-miR-2861 | MIMAT0027518 | hsa-miR-6809-5p |
| MIMAT0013863 | hsa-miR-2909 | MIMAT0027519 | hsa-miR-6809-3p |
| MIMAT0014977 | hsa-miR-3115 | MIMAT0027520 | hsa-miR-6810-5p |
| MIMAT0014978 | hsa-miR-3116 | MIMAT0027521 | hsa-miR-6810-3p |
| MIMAT0014980 | hsa-miR-3118 | MIMAT0027522 | hsa-miR-6811-5p |
| MIMAT0014981 | hsa-miR-3119 | MIMAT0027523 | hsa-miR-6811-3p |
| MIMAT0014984 | hsa-miR-3122 | MIMAT0027524 | hsa-miR-6812-5p |
| MIMAT0014985 | hsa-miR-3123 | MIMAT0027525 | hsa-miR-6812-3p |
| MIMAT0014987 | hsa-miR-548s | MIMAT0027526 | hsa-miR-6813-5p |
| MIMAT0014988 | hsa-miR-3125 | MIMAT0027527 | hsa-miR-6813-3p |
| MIMAT0014989 | hsa-miR-3126-5p | MIMAT0027528 | hsa -miR-6814-5p |
| MIMAT0014991 | hsa-miR-3128 | MIMAT0027529 | hsa -miR-6814-3p |
| MIMAT0014994 | hsa-miR-3130-3p | MIMAT0027530 | hsa-miR-6815-5p |
| MIMAT0014995 | hsa-miR-3130-5p | MIMAT0027531 | hsa-miR-6815-3p |
| MIMAT0014996 | hsa-miR-3131 | MIMAT0027532 | hsa-miR-6816-5p |
| MIMAT0014997 | hsa-miR-3132 | MIMAT0027533 | hsa-miR-6816-3p |
| MIMAT0014998 | hsa-miR-3133 | MIMAT0027534 | hsa-miR-6817-5p |
| MIMAT0014999 | hsa-miR-378b | MIMAT0027535 | hsa-miR-6817-3p |
| MIMAT0015000 | hsa-miR-3134 | MIMAT0027536 | hsa-miR-6818-5p |
| MIMAT0015002 | hsa-miR-466 | MIMAT0027537 | hsa-miR-6818-3p |

**[Table 1-29]**

| | | | |
|---|---|---|---|
| MIMAT0015004 | hsa-miR-544b | MIMAT0027538 | hsa-miR-6819-5p |
| MIMAT0015005 | hsa-miR-3137 | MIMAT0027539 | hsa-miR-6819-3p |
| MIMAT0015006 | hsa-miR-3138 | MIMAT0027540 | hsa-miR-6820-5p |
| MIMAT0015007 | hsa-miR-3139 | MIMAT0027541 | hsa-miR-6820-3p |
| MIMAT0015010 | hsa-miR-3141 | MIMAT0027542 | hsa-miR-6821-5p |
| MIMAT0015011 | hsa-miR-3142 | MIMAT0027543 | hsa-miR-6821-3p |
| MIMAT0015012 | hsa-miR-3143 | MIMAT0027544 | hsa-miR-6822-5p |
| MIMAT0015013 | hsa-miR-548u | MIMAT0027545 | hsa-miR-6822-3p |
| MIMAT0015014 | hsa-miR-3144-5p | MIMAT0027546 | hsa-miR-6823-5p |
| MIMAT0015015 | hsa-miR-3144-3p | MIMAT0027547 | hsa-miR-6823-3p |
| MIMAT0015017 | hsa-miR-1273c | MIMAT0027548 | hsa-miR-6824-5p |
| MIMAT0015018 | hsa-miR-3146 | MIMAT0027549 | hsa-miR-6824-3p |
| MIMAT0015019 | hsa-miR-3147 | MIMAT0027550 | hsa-miR-6825-5p |
| MIMAT0015020 | hsa-miR-548v | MIMAT0027551 | hsa-miR-6825-3p |
| MIMAT0015021 | hsa-miR-3148 | MIMAT0027552 | hsa-miR-6826-5p |
| MIMAT0015022 | hsa-miR-3149 | MIMAT0027553 | hsa-miR-6826-3p |
| MIMAT0015024 | hsa-miR-3151-5p | MIMAT0027554 | hsa-miR-6827-5p |
| MIMAT0015026 | hsa-miR-3153 | MIMAT0027555 | hsa-miR-6827-3p |
| MIMAT0015028 | hsa-miR-3154 | MIMAT0027556 | hsa-miR-6828-5p |
| MIMAT0015033 | hsa-miR-3159 | MIMAT0027557 | hsa-miR-6828-3p |
| MIMAT0015035 | hsa-miR-3161 | MIMAT0027558 | hsa-miR-6829-5p |
| MIMAT0015037 | hsa-miR-3163 | MIMAT0027559 | hsa-miR-6829-3p |
| MIMAT0015038 | hsa-miR-3164 | MIMAT0027560 | hsa-miR-6830-5p |
| MIMAT0015039 | hsa-miR-3165 | MIMAT0027561 | hsa-miR-6830-3p |
| MIMAT0015040 | hsa-miR-3166 | MIMAT0027562 | hsa-miR-6831-5p |
| MIMAT0015041 | hsa-miR-1260b | MIMAT0027563 | hsa-miR-6831-3p |
| MIMAT0015042 | hsa-miR-3167 | MIMAT0027564 | hsa-miR-6832-5p |
| MIMAT0015043 | hsa-miR-3168 | MIMAT0027565 | hsa-miR-6832-3p |
| MIMAT0015044 | hsa-miR-3169 | MIMAT0027566 | hsa-miR-6833-5p |
| MIMAT0015045 | hsa-miR-3170 | MIMAT0027567 | hsa-miR-6833-3p |
| MIMAT0015046 | hsa-miR-3171 | MIMAT0027568 | hsa-miR-6834-5p |
| MIMAT0015049 | hsa-miR-1193 | MIMAT0027569 | hsa-miR-6834-3p |
| MIMAT0015050 | hsa-miR-323b-3p | MIMAT0027570 | hsa-miR-6835-5p |
| MIMAT0015051 | hsa-miR-3174 | MIMAT0027571 | hsa-miR-6835-3p |
| MIMAT0015052 | hsa-miR-3175 | MIMAT0027572 | hsa-miR-6780b-5p |
| MIMAT0015053 | hsa-miR-3176 | MIMAT0027573 | hsa-miR-6780b-3p |

**[Table 1-30]**

| | | | |
|---|---|---|---|
| MIMAT0015055 | hsa-miR-3178 | MIMAT0027574 | hsa-miR-6836-5p |
| MIMAT0015056 | hsa-miR-3179 | MIMAT0027575 | hsa-miR-6836-3p |
| MIMAT0015057 | hsa-miR-3180-5p | MIMAT0027576 | hsa-miR-6837-5p |
| MIMAT0015058 | hsa-miR-3180-3p | MIMAT0027577 | hsa-miR-6837-3p |
| MIMAT0015060 | hsa-miR-548w | MIMAT0027578 | hsa-miR-6838-5p |
| MIMAT0015061 | hsa-miR-3181 | MIMAT0027579 | hsa-miR-6838-3p |
| MIMAT0015062 | hsa-miR-3182 | MIMAT0027580 | hsa-miR-6839-5p |
| MIMAT0015063 | hsa-miR-3183 | MIMAT0027581 | hsa-miR-6839-3p |
| MIMAT0015065 | hsa-miR-3185 | MIMAT0027582 | hsa-miR-6840-5p |
| MIMAT0015066 | hsa-miR-3065-5p | MIMAT0027583 | hsa-miR-6840-3p |
| MIMAT0015067 | hsa-miR-3186-5p | MIMAT0027584 | hsa-miR-6841-5p |
| MIMAT0015068 | hsa-miR-3186-3p | MIMAT0027585 | hsa-miR-6841-3p |
| MIMAT0015070 | hsa-miR-3188 | MIMAT0027586 | hsa-miR-6842-5p |
| MIMAT0015072 | hsa-miR-320e | MIMAT0027587 | hsa-miR-6842-3p |
| MIMAT0015076 | hsa-miR-3192-5p | MIMAT0027588 | hsa-miR-6843-3p |
| MIMAT0015077 | hsa-miR-3193 | MIMAT0027589 | hsa-miR-6844 |
| MIMAT0015079 | hsa-miR-3195 | MIMAT0027590 | hsa-miR-6845-5p |
| MIMAT0015080 | hsa-miR-3196 | MIMAT0027591 | hsa-miR-6845-3p |
| MIMAT0015082 | hsa-miR-3197 | MIMAT0027592 | hsa-miR-6846-5p |
| MIMAT0015083 | hsa-miR-3198 | MIMAT0027593 | hsa-miR-6846-3p |
| MIMAT0015084 | hsa-miR-3199 | MIMAT0027594 | hsa-miR-6847-5p |
| MIMAT0015086 | hsa-miR-3201 | MIMAT0027595 | hsa-miR-6847-3p |
| MIMAT0015087 | hsa-miR-514b-5p | MIMAT0027596 | hsa-miR-6848-5p |
| MIMAT0015088 | hsa-miR-514b-3p | MIMAT0027597 | hsa-miR-6848-3p |
| MIMAT0015089 | hsa-miR-3202 | MIMAT0027598 | hsa-miR-6849-5p |
| MIMAT0015090 | hsa-miR-1273d | MIMAT0027599 | hsa-miR-6849-3p |
| MIMAT0015377 | hsa-miR-3126-3p | MIMAT0027600 | hsa-miR-6850-5p |
| MIMAT0015378 | hsa-miR-3065-3p | MIMAT0027601 | hsa-miR-6850-3p |
| MIMAT0016844 | hsa-miR-4295 | MIMAT0027602 | hsa-miR-6851-5p |
| MIMAT0016845 | hsa-miR-4296 | MIMAT0027603 | hsa-miR-6851-3p |
| MIMAT0016846 | hsa-miR-4297 | MIMAT0027604 | hsa-miR-6852-5p |
| MIMAT0016847 | hsa-miR-378c | MIMAT0027605 | hsa-miR-6852-3p |
| MIMAT0016848 | hsa-miR-4293 | MIMAT0027606 | hsa-miR-6853-5p |
| MIMAT0016849 | hsa-miR-4294 | MIMAT0027607 | hsa-miR-6853-3p |
| MIMAT0016850 | hsa-miR-4301 | MIMAT0027608 | hsa-miR-6854-5p |
| MIMAT0016851 | hsa-miR-4299 | MIMAT0027609 | hsa-miR-6854-3p |

**[Table 1-31]**

| | | | |
|---|---|---|---|
| MIMAT0016852 | hsa-miR-4298 | MIMAT0027610 | hsa-miR-6855-5p |
| MIMAT0016853 | hsa-miR-4300 | MIMAT0027611 | hsa-miR-6855-3p |
| MIMAT0016854 | hsa-miR-4304 | MIMAT0027612 | hsa-miR-6856-5p |
| MIMAT0016855 | hsa-miR-4302 | MIMAT0027613 | hsa-miR-6856-3p |
| MIMAT0016856 | hsa-miR-4303 | MIMAT0027614 | hsa-miR-6857-5p |
| MIMAT0016857 | hsa-miR-4305 | MIMAT0027615 | hsa-miR-6857-3p |
| MIMAT0016858 | hsa-miR-4306 | MIMAT0027616 | hsa-miR-6858-5p |
| MIMAT0016859 | hsa-miR-4309 | MIMAT0027617 | hsa-miR-6858-3p |
| MIMAT0016860 | hsa-miR-4307 | MIMAT0027618 | hsa-miR-6859-5p |
| MIMAT0016861 | hsa-miR-4308 | MIMAT0027619 | hsa-miR-6859-3p |
| MIMAT0016862 | hsa-miR-4310 | MIMAT0027620 | hsa-miR-6769b-5p |
| MIMAT0016863 | hsa-miR-4311 | MIMAT0027621 | hsa-miR-6769b-3p |
| MIMAT0016864 | hsa-miR-4312 | MIMAT0027622 | hsa-miR-6860 |
| MIMAT0016865 | hsa-miR-4313 | MIMAT0027623 | hsa-miR-6861-5p |
| MIMAT0016866 | hsa-miR-4315 | MIMAT0027624 | hsa-miR-6861-3p |
| MIMAT0016867 | hsa-miR-4316 | MIMAT0027625 | hsa-miR-6862-5p |
| MIMAT0016868 | hsa-miR-4314 | MIMAT0027626 | hsa-miR-6862-3p |
| MIMAT0016869 | hsa-miR-4318 | MIMAT0027627 | hsa-miR-6863 |
| MIMAT0016870 | hsa-miR-4319 | MIMAT0027628 | hsa-miR-6864-5p |
| MIMAT0016871 | hsa-miR-4320 | MIMAT0027629 | hsa-miR-6864-3p |
| MIMAT0016872 | hsa-miR-4317 | MIMAT0027630 | hsa-miR-6865-5p |
| MIMAT0016873 | hsa-miR-4322 | MIMAT0027631 | hsa-miR-6865-3p |
| MIMAT0016874 | hsa-miR-4321 | MIMAT0027632 | hsa-miR-6866-5p |
| MIMAT0016875 | hsa-miR-4323 | MIMAT0027633 | hsa-miR-6866-3p |
| MIMAT0016876 | hsa-miR-4324 | MIMAT0027634 | hsa-miR-6867-5p |
| MIMAT0016877 | hsa-miR-4256 | MIMAT0027635 | hsa-miR-6867-3p |
| MIMAT0016878 | hsa-miR-4257 | MIMAT0027636 | hsa-miR-6868-5p |
| MIMAT0016879 | hsa-miR-4258 | MIMAT0027637 | hsa-miR-6868-3p |
| MIMAT0016880 | hsa-miR-4259 | MIMAT0027638 | hsa-miR-6869-5p |
| MIMAT0016881 | hsa-miR-4260 | MIMAT0027639 | hsa-miR-6869-3p |
| MIMAT0016882 | hsa-miR-4253 | MIMAT0027640 | hsa-miR-6870-5p |
| MIMAT0016883 | hsa-miR-4251 | MIMAT0027641 | hsa-miR-6870-3p |
| MIMAT0016884 | hsa-miR-4254 | MIMAT0027642 | hsa-miR-6871-5p |
| MIMAT0016885 | hsa-miR-4255 | MIMAT0027643 | hsa-miR-6871-3p |
| MIMAT0016886 | hsa-miR-4252 | MIMAT0027644 | hsa-miR-6872-5p |
| MIMAT0016887 | hsa-miR-4325 | MIMAT0027645 | hsa-miR-6872-3p |

**[Table 1-32]**

| | | | |
|---|---|---|---|
| MIMAT0016888 | hsa-miR-4326 | MIMAT0027646 | hsa-miR-6873-5p |
| MIMAT0016889 | hsa-miR-4327 | MIMAT0027647 | hsa-miR-6873-3p |
| MIMAT0016890 | hsa-miR-4261 | MIMAT0027648 | hsa-miR-6874-5p |
| MIMAT0016891 | hsa-miR-4265 | MIMAT0027649 | hsa-miR-6874-3p |
| MIMAT0016892 | hsa-miR-4266 | MIMAT0027650 | hsa-miR-6875-5p |
| MIMAT0016893 | hsa-miR-4267 | MIMAT0027651 | hsa-miR-6875-3p |
| MIMAT0016894 | hsa-miR-4262 | MIMAT0027652 | hsa-miR-6876-5p |
| MIMAT0016896 | hsa-miR-4268 | MIMAT0027653 | hsa-miR-6876-3p |
| MIMAT0016897 | hsa-miR-4269 | MIMAT0027654 | hsa-miR-6877-5p |
| MIMAT0016898 | hsa-miR-4263 | MIMAT0027655 | hsa-miR-6877-3p |
| MIMAT0016899 | hsa-miR-4264 | MIMAT0027656 | hsa-miR-6878-5p |
| MIMAT0016900 | hsa-miR-4270 | MIMAT0027657 | hsa-miR-6878-3p |
| MIMAT0016901 | hsa-miR-4271 | MIMAT0027658 | hsa-miR-6879-5p |
| MIMAT0016902 | hsa-miR-4272 | MIMAT0027659 | hsa-miR-6879-3p |
| MIMAT0016903 | hsa-miR-4273 | MIMAT0027660 | hsa-miR-6880-5p |
| MIMAT0016904 | hsa-miR-4276 | MIMAT0027661 | hsa-miR-6880-3p |
| MIMAT0016905 | hsa-miR-4275 | MIMAT0027662 | hsa-miR-6881-5p |
| MIMAT0016906 | hsa-miR-4274 | MIMAT0027663 | hsa-miR-6881-3p |
| MIMAT0016907 | hsa-miR-4281 | MIMAT0027664 | hsa-miR-6882-5p |
| MIMAT0016908 | hsa-miR-4277 | MIMAT0027665 | hsa-miR-6882-3p |
| MIMAT0016909 | hsa-miR-4279 | MIMAT0027666 | hsa-miR-6883-5p |
| MIMAT0016910 | hsa-miR-4278 | MIMAT0027667 | hsa-miR-6883-3p |
| MIMAT0016911 | hsa-miR-4280 | MIMAT0027668 | hsa-miR-6884-5p |
| MIMAT0016912 | hsa-miR-4282 | MIMAT0027669 | hsa-miR-6884-3p |
| MIMAT0016913 | hsa-miR-4285 | MIMAT0027670 | hsa-miR-6885-5p |
| MIMAT0016914 | hsa-miR-4283 | MIMAT0027671 | hsa-miR-6885-3p |
| MIMAT0016915 | hsa-miR-4284 | MIMAT0027672 | hsa-miR-6886-5p |
| MIMAT0016916 | hsa-miR-4286 | MIMAT0027673 | hsa-miR-6886-3p |
| MIMAT0016917 | hsa-miR-4287 | MIMAT0027674 | hsa-miR-6887-5p |
| MIMAT0016918 | hsa-miR-4288 | MIMAT0027675 | hsa-miR-6887-3p |
| MIMAT0016919 | hsa-miR-4292 | MIMAT0027676 | hsa-miR-6888-5p |
| MIMAT0016920 | hsa-miR-4289 | MIMAT0027677 | hsa-miR-6888-3p |
| MIMAT0016921 | hsa-miR-4290 | MIMAT0027678 | hsa-miR-6889-5p |
| MIMAT0016922 | hsa-miR-4291 | MIMAT0027679 | hsa-miR-6889-3p |
| MIMAT0016923 | hsa-miR-4329 | MIMAT0027680 | hsa-miR-6890-5p |
| MIMAT0016924 | hsa-miR-4330 | MIMAT0027681 | hsa-miR-6890-3p |

**[Table 1-33]**

| | | | |
|---|---|---|---|
| MIMAT0016925 | hsa-miR-500b-5p | MIMAT0027682 | hsa-miR-6891-5p |
| MIMAT0016926 | hsa-miR-4328 | MIMAT0027683 | hsa-miR-6891-3p |
| MIMAT0017352 | hsa-miR-2277-5p | MIMAT0027684 | hsa-miR-6892-5p |
| MIMAT0017392 | hsa-miR-3200-5p | MIMAT0027685 | hsa-miR-6892-3p |
| MIMAT0017950 | hsa-miR-2355-3p | MIMAT0027686 | hsa-miR-6893-5p |
| MIMAT0017981 | hsa-miR-3605-5p | MIMAT0027687 | hsa-miR-6893-3p |
| MIMAT0017982 | hsa-miR-3605-3p | MIMAT0027688 | hsa-miR-6894-5p |
| MIMAT0017984 | hsa-miR-3607-5p | MIMAT0027689 | hsa-miR-6894-3p |
| MIMAT0017985 | hsa-miR-3607-3p | MIMAT0027690 | hsa-miR-6895-5p |
| MIMAT0017986 | hsa-miR-3609 | MIMAT0027691 | hsa-miR-6895-3p |
| MIMAT0017987 | hsa-miR-3610 | MIMAT0028109 | hsa-miR-7106-5p |
| MIMAT0017988 | hsa-miR-3611 | MIMAT0028110 | hsa-miR-7106-3p |
| MIMAT0017989 | hsa-miR-3612 | MIMAT0028111 | hsa-miR-7107-5p |
| MIMAT0017990 | hsa-miR-3613-5p | MIMAT0028112 | hsa-miR-7107-3p |
| MIMAT0017991 | hsa-miR-3613-3p | MIMAT0028113 | hsa-miR-7108-5p |
| MIMAT0017992 | hsa-miR-3614-5p | MIMAT0028114 | hsa-miR-7108-3p |
| MIMAT0017993 | hsa-miR-3614-3p | MIMAT0028115 | hsa-miR-7109-5p |
| MIMAT0017994 | hsa-miR-3615 | MIMAT0028116 | hsa-miR-7109-3p |
| MIMAT0017995 | hsa-miR-3616-5p | MIMAT0028117 | hsa-miR-7110-5p |
| MIMAT0017996 | hsa-miR-3616-3p | MIMAT0028118 | hsa-miR-7110-3p |
| MIMAT0017998 | hsa-miR-3618 | MIMAT0028119 | hsa-miR-7111-5p |
| MIMAT0018000 | hsa-miR-23c | MIMAT0028120 | hsa-miR-7111-3p |
| MIMAT0018002 | hsa-miR-3621 | MIMAT0028121 | hsa-miR-7112-5p |
| MIMAT0018003 | hsa-miR-3622a-5p | MIMAT0028122 | hsa-miR-7112-3p |
| MIMAT0018004 | hsa-miR-3622a-3p | MIMAT0028123 | hsa-miR-7113-5p |
| MIMAT0018005 | hsa-miR-3622b-5p | MIMAT0028124 | hsa-miR-7113-3p |
| MIMAT0018006 | hsa-miR-3622b-3p | MIMAT0028125 | hsa-miR-7114-5p |
| MIMAT0018065 | hsa-miR-3646 | MIMAT0028126 | hsa-miR-7114-3p |
| MIMAT0018068 | hsa-miR-3648 | MIMAT0028211 | hsa-miR-7150 |
| MIMAT0018069 | hsa-miR-3649 | MIMAT0028212 | hsa-miR-7151-5p |
| MIMAT0018070 | hsa-miR-3650 | MIMAT0028213 | hsa-miR-7151-3p |
| MIMAT0018071 | hsa-miR-3651 | MIMAT0028214 | hsa-miR-7152-5p |
| MIMAT0018072 | hsa-miR-3652 | MIMAT0028215 | hsa-miR-7152-3p |
| MIMAT0018074 | hsa-miR-3654 | MIMAT0028216 | hsa-miR-7153-5p |
| MIMAT0018075 | hsa-miR-3655 | MIMAT0028217 | hsa-miR-7153-3p |
| MIMAT0018076 | hsa-miR-3656 | MIMAT0028218 | hsa-miR-7154-5p |

**[Table 1-34]**

| | | | |
|---|---|---|---|
| MIMAT0018077 | hsa-miR-3657 | MIMAT0028219 | hsa-miR-7154-3p |
| MIMAT0018078 | hsa-miR-3658 | MIMAT0028220 | hsa-miR-7155-5p |
| MIMAT0018079 | hsa-miR-1273e | MIMAT0028221 | hsa-miR-7155-3p |
| MIMAT0018080 | hsa-miR-3659 | MIMAT0028222 | hsa-miR-7156-5p |
| MIMAT0018081 | hsa-miR-3660 | MIMAT0028223 | hsa-miR-7156-3p |
| MIMAT0018082 | hsa-miR-3661 | MIMAT0028224 | hsa-miR-7157-5p |
| MIMAT0018083 | hsa-miR-3662 | MIMAT0028225 | hsa-miR-7157-3p |
| MIMAT0018084 | hsa-miR-3663-5p | MIMAT0028226 | hsa-miR-7158-5p |
| MIMAT0018085 | hsa-miR-3663-3p | MIMAT0028227 | hsa-miR-7158-3p |
| MIMAT0018087 | hsa-miR-3665 | MIMAT0028228 | hsa-miR-7159-5p |
| MIMAT0018088 | hsa-miR-3666 | MIMAT0028229 | hsa-miR-7159-3p |
| MIMAT0018089 | hsa-miR-3667-5p | MIMAT0028230 | hsa-miR-7160-5p |
| MIMAT0018090 | hsa-miR-3667-3p | MIMAT0028231 | hsa-miR-7160-3p |
| MIMAT0018091 | hsa-miR-3668 | MIMAT0028232 | hsa-miR-7161-5p |
| MIMAT0018093 | hsa-miR-3670 | MIMAT0028233 | hsa-miR-7161-3p |
| MIMAT0018094 | hsa-miR-3671 | MIMAT0028234 | hsa-miR-7162-5p |
| MIMAT0018095 | hsa-miR-3672 | MIMAT0028235 | hsa-miR-7162-3p |
| MIMAT0018097 | hsa-miR-3674 | MIMAT0029310 | hsa-miR-7515 |
| MIMAT0018098 | hsa-miR-3675-5p | MIMAT0029782 | hsa-miR-7641 |
| MIMAT0018099 | hsa-miR-3675-3p | MIMAT0030017 | hsa-miR-7702 |
| MIMAT0018102 | hsa-miR-3678-5p | MIMAT0030018 | hsa-miR-7703 |
| MIMAT0018103 | hsa-miR-3678-3p | MIMAT0030019 | hsa-miR-7704 |
| MIMAT0018104 | hsa-miR-3679-5p | MIMAT0030020 | hsa-miR-7705 |
| MIMAT0018105 | hsa-miR-3679-3p | MIMAT0030021 | hsa-miR-7706 |
| MIMAT0018111 | hsa-miR-3683 | MIMAT0030411 | hsa-miR-7843-5p |
| MIMAT0018112 | hsa-miR-3684 | MIMAT0030412 | hsa-miR-7843-3p |
| MIMAT0018113 | hsa-miR-3685 | MIMAT0030413 | hsa-miR-4433b-5p |
| MIMAT0018114 | hsa-miR-3686 | MIMAT0030414 | hsa-miR-4433b-3p |
| MIMAT0018115 | hsa-miR-3687 | MIMAT0030415 | hsa-miR-1273h-5p |
| MIMAT0018117 | hsa-miR-3689a-5p | MIMAT0030416 | hsa-miR-1273h-3p |
| MIMAT0018118 | hsa-miR-3689a-3p | MIMAT0030417 | hsa-miR-6516-5p |
| MIMAT0018119 | hsa-miR-3690 | MIMAT0030418 | hsa-miR-6516-3p |
| MIMAT0018164 | hsa-miR-3713 | MIMAT0030419 | hsa-miR-7844-5p |
| MIMAT0018165 | hsa-miR-3714 | MIMAT0030420 | hsa-miR-7845-5p |
| MIMAT0018178 | hsa-miR-3180 | MIMAT0030421 | hsa-miR-7846-3p |
| MIMAT0018179 | hsa-miR-3907 | MIMAT0030422 | hsa-miR-7847-3p |

**[Table 1-35]**

| | | | |
|---|---|---|---|
| MIMAT0018182 | hsa-miR-3908 | MIMAT0030423 | hsa-miR-7848-3p |
| MIMAT0018183 | hsa-miR-3909 | MIMAT0030424 | hsa-miR-7849-3p |
| MIMAT0018184 | hsa-miR-3910 | MIMAT0030425 | hsa-miR-7850-5p |
| MIMAT0018185 | hsa-miR-3911 | MIMAT0030426 | hsa-miR-7851-3p |
| MIMAT0018186 | hsa-miR-3912-3p | MIMAT0030427 | hsa-miR-7852-3p |
| MIMAT0018188 | hsa-miR-3914 | MIMAT0030428 | hsa-miR-7853-5p |
| MIMAT0018189 | hsa-miR-3915 | MIMAT0030429 | hsa-miR-7854-3p |
| MIMAT0018190 | hsa-miR-3916 | MIMAT0030430 | hsa-miR-7855-5p |
| MIMAT0018191 | hsa-miR-3917 | MIMAT0030431 | hsa-miR-7856-5p |
| MIMAT0018192 | hsa-miR-3918 | MIMAT0030979 | hsa-miR-8052 |
| MIMAT0018193 | hsa-miR-3919 | MIMAT0030980 | hsa-miR-8053 |
| MIMAT0018195 | hsa-miR-3920 | MIMAT0030981 | hsa-miR-8054 |
| MIMAT0018196 | hsa-miR-3921 | MIMAT0030982 | hsa-miR-8055 |
| MIMAT0018198 | hsa-miR-3923 | MIMAT0030983 | hsa-miR-8056 |
| MIMAT0018199 | hsa-miR-3924 | MIMAT0030984 | hsa-miR-8057 |
| MIMAT0018201 | hsa-miR-3926 | MIMAT0030985 | hsa-miR-8058 |
| MIMAT0018205 | hsa-miR-3928-3p | MIMAT0030986 | hsa-miR-8059 |
| MIMAT0018206 | hsa-miR-3929 | MIMAT0030987 | hsa-miR-8060 |
| MIMAT0018350 | hsa-miR-3935 | MIMAT0030988 | hsa-miR-8061 |
| MIMAT0018351 | hsa-miR-3936 | MIMAT0030989 | hsa-miR-8062 |
| MIMAT0018352 | hsa-miR-3937 | MIMAT0030990 | hsa-miR-8063 |
| MIMAT0018353 | hsa-miR-3938 | MIMAT0030991 | hsa-miR-8064 |
| MIMAT0018354 | hsa-miR-548y | MIMAT0030992 | hsa-miR-8065 |
| MIMAT0018355 | hsa-miR-3939 | MIMAT0030993 | hsa-miR-8066 |
| MIMAT0018357 | hsa-miR-3941 | MIMAT0030994 | hsa-miR-8067 |
| MIMAT0018359 | hsa-miR-3943 | MIMAT0030995 | hsa-miR-8068 |
| MIMAT0018361 | hsa-miR-3945 | MIMAT0030996 | hsa-miR-8069 |
| MIMAT0018446 | hsa-miR-548z | MIMAT0030997 | hsa-miR-8070 |
| MIMAT0018447 | hsa-miR-548aa | MIMAT0030998 | hsa-miR-8071 |
| MIMAT0018925 | hsa-miR-1268b | MIMAT0030999 | hsa-miR-8072 |
| MIMAT0018926 | hsa-miR-378d | MIMAT0031000 | hsa-miR-8073 |
| MIMAT0018927 | hsa-miR-378e | MIMAT0031001 | hsa-miR-8074 |
| MIMAT0018928 | hsa-miR-548ab | MIMAT0031002 | hsa-miR-8075 |
| MIMAT0018929 | hsa-miR-4417 | MIMAT0031003 | hsa-miR-8076 |
| MIMAT0018930 | hsa-miR-4418 | MIMAT0031004 | hsa-miR-8077 |
| MIMAT0018931 | hsa-miR-4419a | MIMAT0031005 | hsa-miR-8078 |

**[Table 1-36]**

| | | | |
|---|---|---|---|
| MIMAT0018932 | hsa-miR-378f | MIMAT0031006 | hsa-miR-8079 |
| MIMAT0018933 | hsa-miR-4420 | MIMAT0031007 | hsa-miR-8080 |
| MIMAT0018934 | hsa-miR-4421 | MIMAT0031008 | hsa-miR-8081 |
| MIMAT0018935 | hsa-miR-4422 | MIMAT0031009 | hsa-miR-8082 |
| MIMAT0018936 | hsa-miR-4423-3p | MIMAT0031010 | hsa-miR-8083 |
| MIMAT0018937 | hsa-miR-378g | MIMAT0031011 | hsa-miR-8084 |
| MIMAT0018938 | hsa-miR-548ac | MIMAT0031012 | hsa-miR-8085 |
| MIMAT0018939 | hsa-miR-4424 | MIMAT0031013 | hsa-miR-8086 |
| MIMAT0018940 | hsa-miR-4425 | MIMAT0031014 | hsa-miR-8087 |
| MIMAT0018941 | hsa-miR-4426 | MIMAT0031015 | hsa-miR-8088 |
| MIMAT0018942 | hsa-miR-4427 | MIMAT0031016 | hsa-miR-8089 |
| MIMAT0018943 | hsa-miR-4428 | MIMAT0031074 | hsa-miR-450a-2-3p |
| MIMAT0018944 | hsa-miR-4429 | MIMAT0031095 | hsa-miR-128-2-5p |
| MIMAT0018945 | hsa-miR-4430 | MIMAT0031119 | hsa-miR-1199-5p |
| MIMAT0018947 | hsa-miR-4431 | MIMAT0031120 | hsa-miR-1199-3p |
| MIMAT0018948 | hsa-miR-4432 | MIMAT0031175 | hsa-miR-548ba |
| MIMAT0018950 | hsa-miR-4434 | MIMAT0031176 | hsa-miR-7973 |
| MIMAT0018951 | hsa-miR-4435 | MIMAT0031177 | hsa-miR-7974 |
| MIMAT0018952 | hsa-miR-4436a | MIMAT0031178 | hsa-miR-7975 |
| MIMAT0018953 | hsa-miR-4437 | MIMAT0031179 | hsa-miR-7976 |
| MIMAT0018956 | hsa-miR-4438 | MIMAT0031180 | hsa-miR-7977 |
| MIMAT0018957 | hsa-miR-4439 | MIMAT0031181 | hsa-miR-7978 |
| MIMAT0018958 | hsa-miR-4440 | MIMAT0031890 | hsa-miR-203a-5p |
| MIMAT0018959 | hsa-miR-4441 | MIMAT0031892 | hsa-miR-1-5p |
| MIMAT0018960 | hsa-miR-4442 | MIMAT0031893 | hsa-miR-181b-2-3p |
| MIMAT0018961 | hsa-miR-4443 | MIMAT0032026 | hsa-miR-301b-5p |
| MIMAT0018962 | hsa-miR-4444 | MIMAT0032029 | hsa-miR-1249-5p |
| MIMAT0018965 | hsa-miR-4446-3p | MIMAT0032110 | hsa-miR-3653-5p |
| MIMAT0018966 | hsa-miR-4447 | MIMAT0032114 | hsa-miR-548ad-5p |
| MIMAT0018967 | hsa-miR-4448 | MIMAT0032115 | hsa-miR-548ae-5p |
| MIMAT0018968 | hsa-miR-4449 | MIMAT0032116 | hsa-miR-4485-5p |
| MIMAT0018969 | hsa-miR-548ag | MIMAT0033692 | hsa-miR-8485 |
| MIMAT0018971 | hsa-miR-4450 | MIMAT0035542 | hsa-miR-9500 |
| MIMAT0018973 | hsa-miR-4451 | MIMAT0035703 | hsa-miR-548bb-5p |
| MIMAT0018974 | hsa-miR-4452 | MIMAT0035704 | hsa-miR-548bb-3p |
| MIMAT0018975 | hsa-miR-4453 | | |

**[Table 1-37]**

| | |
|---|---|
| MIMAT0018976 | hsa-miR-4454 |
| MIMAT0018977 | hsa-miR-4455 |
| MIMAT0018978 | hsa-miR-4456 |
| MIMAT0018979 | hsa-miR-4457 |
| MIMAT0018980 | hsa-miR-4458 |

As used herein, "long non-coding RNA (lncRNA)" refers to an RNA of 200 nt or greater that functions without being translated into a protein. Specific information (sequence and the like) of lncRNAs is available from, for example, RNAcentral (http://rnacentral.org/). For example, mature microRNAs in humans include those in the following tables.

As used herein, "ribosome RNA (rRNA)" refers to an RNA constituting a ribosome. Specific information (sequence and the like) of rRNAs is available from, for example, NCBI (https://www.ncbi.nlm.nih.gov/). For example, mature microRNAs in humans include those in the following tables.

As used herein, "transfer RNA (tRNA)" refers to a tRNA that is known to be aminoacylated by an aminoacyl tRNA synthetase. Specific information (sequence and the like) of tRNAs is available from, for example, NCBI (https://www.ncbi.nlm.nih.gov/). For example, mature microRNAs in humans include those in the following tables.

As used herein, "modification" used in the context of a nucleic acid refers to a substitution of a constituent unit of a nucleic acid or a part of all of the terminal thereof with another group of atoms, or addition of a functional group. A collection of modifications of an RNA is also known as "RNA Modomics", "RNA Mod", or the like, which are also known as epitranscriptome because an RNA is a transcript. These terms are used synonymously herein.

Examples of RNA modifications include, but are not limited to, those listed in the following tables. It is understood that anything can be used, as long as it falls under a modification.

**[Table 2-1]**

| **Name** | **Abbreviation** |
|---|---|
| **1,2'-O-dimethyladenosine** | m1Am |
| **1,2'-O-dimethylguanosine** | m1Gm |
| **1,2'-O-dimethylinosine** | m1Im |
| **l-methyl-3-(3-amino-3-carboxypropyl)pseudouridine** | mlacp3Y |
| **1-methyladenosine** | m1A |
| **1-methylguanosine** | m1G |
| **1-methylinosine** | m1I |
| **1-methylpseudouridine** | m1Y |
| **2,8-dimethyladenosine** | m2, 8A |
| **2-gelanylthiouridine** | ges2U |
| **2-lysidine** | k2C |
| **2-methyladenosine** | m2A |
| **2-methylthio cyclic N6-threonylcarbamoyladenosine** | ms2ct6A |
| **2-methylthio-N6-(cis-hydroxyisopentenyl)adenosine** | ms2io6A |
| **2-methylthio-N6-hydroxynorvalylcarbamoyladenosine** | ms2hn6A |
| **2-methylthio-N6-isopentenyladenosine** | ms2i6A |
| **2-methylthio-N6-methyladenosine** | ms2m6A |
| **2-methylthio-N6-threonylcarbamoyladenosine** | ms2t6A |
| **2-selenouridine** | se2U |
| **2-thio-2'-O-methyluridine** | s2Um |
| **2-thiocytidine** | s2C |
| **2-thiouridine** | s2U |
| **2'-O-methyladenosine** | Am |
| **2'-O-methylcytidine** | Cm |
| **2'-O-methylguanosine** | Gm |
| **2'-O-methylinosine** | Im |
| **2'-O-methylpseudouridine** | Ym |
| **2'-O-methyluridine** | Um |
| **2'-O-methyluridine 5-oxyacetic acid methyl ester** | mcmo5Um |
| **2'-O-ribosyladenosine (phosphoric acid)** | Ar (p) |
| **2'-O-ribosylguanosine (phosphoric acid)** | Gr (p) |
| **2'3'-cyclic phosphoric acid end** | (pN) 2'3'>p |
| **3,2'-O-dimethyluridine** | m3Um |

**[Table 2-2]**

| | |
|---|---|
| **3-(3-amino-3-carboxypropyl)-5,6-dihydrouridine** | acp3D |
| **3-(3-amino-3-carboxypropyl)pseudouridine** | acp3Y |
| **3-(3-amino-3-carboxypropyl)uridine** | acp3U |
| **3-methylcytidine** | m3C |
| **3-methylpseudouridine** | m3Y |
| **3-methyluridine** | m3U |
| **4-dimethylwyosine** | imG-14 |
| **4-thiouridine** | s4U |
| **5,2'-O-dimethylcytidine** | m5Cm |
| **5,2'-O-dimethyluridine** | m5Um |
| **5-(carboxyhydroxymethyl)-2'-O-methyluridine methyl ester** | mchm5Um |
| **5-(carboxyhydroxymethyl)uridine methyl ester** | mchm5U |
| **5-(isopentenylaminomethyl)-2-thiouridine** | inm5s2U |
| **5-(isopentenylaminomethyl)-2'-O-methyluridine** | inm5Um |
| **5-(isopentenylaminomethyl)uridine** | inm5U |
| **5-aminomethyl-2-gelanylthiouridine** | nm5ges2U |
| **5-aminomethyl-2-selenouridine** | nm5se2U |
| **5-aminomethyl-2-thiouridine** | nm5s2U |
| **5-aminomethyluridine** | nm5U |
| **5-carbamoylhydroxymethyluridine** | nchm5U |
| **5-carbamoylmethyl-2-thiouridine** | ncm5s2U |
| **5-carbamoylmethyl-2'-O-methyluridine** | ncm5Um |
| **5-carbamoylmethyluridine** | ncm5U |
| **5-carboxyhydroxymethyluridine** | chm5U |
| **5-carboxymethyl-2-thiouridine** | cm5s2U |
| **5-carboxymethylaminomethyl-2-gelanylthiouridine** | cmnm5ges2U |
| **5-carboxymethylaminomethyl-2-selenouridine** | cmnm5s e 2U |
| **5-carboxymethylaminomethyl-2-thiouridine** | cmnm5 s 2U |
| **5-carboxymethylaminomethyl-2'-O-methyluridine** | cmnm5Um |
| **5-carboxymethylaminomethyluridine** | cmnm5U |
| **5-carboxymethyluridine** | cm5U |
| **5-cyanomethyluridine** | cnm5U |
| **5-formyl-2'-O-methylcytidine** | f5Cm |
| **5-formylcytidine** | f5C |
| **5-hydroxycytidine** | ho5C |

**[Table 2-3]**

| | |
|---|---|
| **5-hydroxymethylcytidine** | hm5C |
| **5-hydroxyuridine** | ho5U |
| **5-methoxycarbonylmethyl-2-thiouridine** | mcm5s2U |
| **5-methoxycarbonylmethyl-2'-O-methyluridine** | mcm5Um |
| **5-methoxycarbonylmethyluridine** | mcm5U |
| **5-methoxyuridine** | mo5U |
| **5-methyl-2-thiouridine** | m5s2U |
| **5-methyaminomethyl-2-gelanylthiouridine** | mnm5ges2U |
| **5-methylaminomethyl-2-selenouridine** | mnm5se2U |
| **5-methylaminomethyl-2-thiouridine** | mnm5s2U |
| **5-methylaminomethyluridine** | mnm5U |
| **5-methylcytidine** | m5C |
| **5-methyldihydrouridine** | m5D |
| **5-methyluridine** | m5U |
| **5-taurinomethyl-2-thiouridine** | tm5s2U |
| **5-taurinomethyluridine** | tm5U |
| **5'(3'-dephospho-CoA)** | CoA (pN) |
| **5'(3'-dephosphoacetyl-CoA)** | acCoA (pN) |
| **5'(3'-dephosphomalonyl-CoA)** | malonyl-CoA (pN) |
| **5'(3'-dephosphosuccinyl-CoA)** | succinyl-CoA (pN) |
| **5' diphosphate end** | p (pN) |
| **5 'hydroxyl end** | 5'-OH-N |
| **5' monophosphate end** | (pN) |
| **5' nicotinamide adenine dinucleotide** | NAD (pN) |
| **5' triphosphate end** | pp (pN) |
| **7-aminocarboxypropyl-dimethylwyosine** | yW- 86 |
| **7-aminocarboxypropylwyosine** | yW-72 |
| **7-aminoacarboxypropylwyosine methyl ester** | yW-58 |
| **7-aminomethyl-7-deazaguanosine** | preQltRNA |
| **7-cyano-7-deazaguanosine** | preQ0tRNA |
| **7-methylguanosine** | m7G |
| **7-methylguanosine cap (cap 0)** | m7Gpp (pN) |
| **8-methyladenosine** | m8A |
| **N2,2'-O-dimethylguanosine** | m2Gm |
| **N2,7,2'-O-trimethylguanosine** | m2, 7Gm |

**[Table 2-4]**

| | |
|---|---|
| **N2,7-dimethylguanosine** | m2, 7G |
| **N2,7-dimethylguanosine cap (cap DMG)** | m2, 7Gpp (pN) |
| **N2,N2,2'-O-trimethylguanosine** | m2,2Gm |
| **N2,N2,7-trimethylguanosine** | m2, 2, 7G |
| **N2,N2,7-trimethylguanosine cap (cap TMG)** | m2, 2, 7Gpp (pN) |
| **N2,N2-dimethylguanosine** | m2, 2G |
| **N2-methylguanosine** | m2G |
| **N4,2'-O-dimethylcytidine** | m4Cm |
| **N4,N4,2'-O-trimethylcytidine** | m4, 4Cm |
| **N4,N4-dimethylcytidine** | m4, 4C |
| **N4-acetyl-2'-O-methylcytidine** | ac4Cm |
| **N4-acetylcytidine** | ac4C |
| **N4-methylcytidine** | m4C |
| **N6,2'-O-dimethyladenosine** | m6Am |
| **N6,N6-2'-O-trimethyladenosine** | m6, 6Am |
| **N6,N6-dimethyladenosine** | m6, 6A |
| **N6-(cis-hydroxyisopentenyl)adenosine** | io6A |
| **N6-acetyladenosine** | ac6A |
| **N6-formyladenosine** | f6A |
| **N6-glycinylcarbamoyladenosine** | g6A |
| **N6-hydroxymethyladenosine** | hm6A |
| **N6-hydroxynorvalylcarbamoyladenosine** | hn6A |
| **N6-isopentenyladenosi** ne | i6A |
| **N6-methyl-N6-threonylcarbamoyladenosine** | m6t6A |
| **N6-methyladenosine** | m6A |
| **N6-threonylcarbamoyladenosine** | t6A |
| **Q base** | Qbase |
| **Adenosine** | A |
| **Agmatidine** | C+ |
| **α-dimethylmonophosphate cap** | mm (pN) |
| **α-methylmonophosphate cap** | m (pN) |
| **Archaeosine** | G+ |
| **Cyclic N6-threnonylcarbamoyladenosine** | ct 6A |
| **Cytidine** | C |
| **Dihydrouridine** | D |

**[Table 2-5]**

| | |
|---|---|
| **Epoxyqueuosine** | oQtRNA |
| **Galactosyl-queuosine** | galQtRNA |
| **γ-methyltriphosphate cap** | mpp (pN) |
| **Glutamyl-queuosine** | gluQtRNA |
| **Guanosine** | G |
| **Guanosine added to any nucleotide** | pG (pN) |
| **Guanylated 5' end (cap G)** | Gpp (pN) |
| **Hydroxy-N6-threonylcarbamoyladenosine** | ht6A |
| **Hydroxywybutosine** | OHyW |
| **Inosine** | I |
| **Isowyosine** | imG2 |
| **Mannosyl-queuosine** | manQtRNA |
| **Methylated undermodified hydroxywybutosine** | OHyWy |
| **Methylwybutosine** | mimG |
| **Peroxywybutosine** | o2yW |
| **Pre-Q0 base** | preQ0base |
| **Pre-Q1 base** | preQlbase |
| **Pseudouridine** | Y |
| **Queuosine** | QtRNA |
| **Undermodified hydroxywybutosine** | OHyWx |
| **Uridine** | U |
| **Uridine 5-oxyacetic acid** | cmo5U |
| **Uridine 5-oxyacetic acid methyl ester** | mcmo5U |
| **Wybutosine** | yW |
| **Wyosine** | imG |

These modifications can be distinguished by any method that is known in the art, such as mass spectrometry, specific chemical reaction, or comparison with a standard synthetic product (e.g., comparison of time of retention by LC), and optionally utilizing information that has been accumulated up to that point.

As used herein, "methylation", in the context of a nucleic acid, refers to methylation of any location of any type of nucleotide and is typically methylation of adenine (e.g., position 6; m6A, position 1; m1A) or methylation of cytosine (e.g., position 5; m5C, position 3; m3C). A detected modified site can be identified using a methodology that is known in the art. For example, each of m1A and m6A and m3C and m5C can be determined by chemical modifications. For example, it is possible to determine whether a behavior according to measurement by MALDI and chemical modification is correct by utilizing a standard synthetic RNA.

In addition, majority of types of RNA modifications found in for example tRNA, rRNA, mRNA, or the like can be distinguished as a difference in the mass number. For example, modifications can be theoretically identified by creating a difference in the mass number with a chemical modification. However, modifications with the same mass number can be distinguished using other approaches that are known in the art.

As used herein, "measurement" is used in the meaning that is commonly used in the art, referring to determining what the amount of a certain subject is. As used herein, "detection" is used in the meaning that is commonly used in the art, referring to investigating and finding a substance, component, or the like. "Identification" refers to an act of searching for where a certain subject belongs from among known classifications that are associated therewith. When used in the field of chemistry, identification refers to determining the identity of a target subject as a chemical substance (e.g., determining a chemical structure). "Quantification" refers to determination of the amount of a target substance.

As used herein, the "amount" of an analyte in a sample generally refers to an absolute value reflecting the mass of the analyte that can be detected in a volume of sample. However, amount is also intended as a relative amount as compared to the amount of another analyte. For example, the amount of an analyte in a sample can be an amount that is greater than a control level or a normal level of an analyte that is generally present in a sample.

The term "about", when used herein in relation to a quantitative measurement excluding measurement of the mass of an ion, refers to the indicated value plus or minus 10%. Even if "about" is not explicitly indicated, a value can be interpreted in the same manner as if the term "about" is used. Mass spectrometers can vary slightly in the determination of mass of a given analyte. The term "about" in relation to the mass of ions or the mass/charge ratio of ions refers to +/- 0.5 atom mass unit.

As used herein, "subject" refers to a subject targeted for the analysis, diagnosis, detection, or the like of the invention (e.g., food, organism such as a human or microorganism, cell, blood, or serum retrieved from an organism, or the like).

As used herein, "organ" refers to a constituent unit of a body of a multicellular organism such as an animal or plant among organisms, which is morphologically distinct from the surroundings and serves as a set of functions as a whole. Representative examples thereof include, but are not limited to, a liver, spleen, and lymph node, as well as other organs such as the kidney, lung, adrenal gland, pancreas, and heart.

As used herein, "biomarker" is an indicator for evaluating a condition or action of a subject. Unless specifically noted otherwise, "biomarker" is also referred to as "marker" herein.

The detecting agent or detection means of the invention can be a complex or complex molecule prepared by coupling, to a portion that is made detectable (e.g., antibody or the like), another substance (e.g., label or the like). As used herein, "complex" or "complex molecule" refers to any construct including two or more portions. For example, if one of the portions is a polypeptide, the other portion can be a polypeptide or other substances (e.g., substrate, saccharide, lipid, nucleic acid, other carbohydrate, or the like). The two or more portions constituting a complex herein can be bound by a covalent bond or other bonds (e.g., hydrogen bond, ion bond, hydrophobic interaction, van der Waals force, or the like). If two or more portions are polypeptides, the complex can be referred to as a chimeric polypeptide. Therefore, "complex" as used herein includes molecules prepared by linking a plurality of types of polypeptides, polynucleotides, lipids, saccharides, small molecules, or other molecules.

As used herein, "detection" or "quantification" of polynucleotide expression can be attained, for example, by using an appropriate method including mRNA measurement and an immunological measuring method, which includes binding or interaction with a marker detection agent. This can be measured in the present invention with the amount of PCR product. Examples of molecular biological measuring methods include Northern blot, dot blot, PCR, and the like. Examples of immunological measuring methods include, as a method, ELISA using a microtiter plate, RIA, fluorescent antibody method, luminescence immunoassay (LIA), immunoprecipitation (IP), single radical immuno-diffusion (SRID), turbidimetric immunoassay (TIA), Western blot, immunohistological staining method, and the like. Further, examples of quantification methods include ELISA, RIA, and the like. Detection or quantitation can also be performed using a genetic analysis method using an array (e.g., DNA array or protein array). A DNA array is extensively reviewed in (Saibo Kogaku Bessatsu "DNA maikuroarei to saishin PCR method" [Cell engineering, separate volume, "DNA Microarray and Advanced PCR method"], edited by Shujunsha Co., Ltd.). A protein array is described in detail in Nat Genet. 2002 Dec; 32 Suppl: 526-32. Examples of methods for analyzing gene expression include, but are not limited to, RT-PCR, RACE, SSCP, immunoprecipitation, two-hybrid system, in vitro translation, and the like in addition to the aforementioned methods. Such additional analysis methods are described, for example, in Genomu Bunseki Jikkenho/Nakamura Yusuke Labo/Manuaru [Genome Analysis Experimental Method, Nakamura Yusuke Lab. Manual], edited by Yusuke Nakamura, Yodosha Co., Ltd. (2002) and the like. The entire descriptions therein are incorporated herein by reference.

As used herein, "means" refers to anything which can be a tool for attaining a certain objective (e.g., detection, diagnosis, or therapy). As used herein, "means for selective recognition (detection)" especially refers to means which can recognize (detect) a certain subject differently from others.

As used herein, a "(nucleic acid) primer" refers to a substance required for initiation of a reaction of a polymer compound to be synthesized in a polymer synthesizing enzymatic reaction. In a reaction of synthesizing a nucleic acid molecule, a nucleic acid molecule (e.g., DNA, RNA, or the like) complementary to a part of a sequence of a polymer compound to be synthesized can be used. As used herein, a primer can be used as marker detection means.

Examples of a nucleic acid molecule which is generally used as a primer include nucleic acid molecules having a nucleic acid sequence with a length of at least 8 consecutive nucleotides, which is complementary to a nucleic acid sequence of a polynucleotide of interest (e.g., microRNA). Such a nucleic acid sequence can be a nucleic acid sequence with a length of preferably at least 9 consecutive nucleotides, more preferably at least 10 consecutive nucleotides, still more preferably at least 11 consecutive nucleotides, at least 12 consecutive nucleotides, at least 13 consecutive nucleotides, at least 14 consecutive nucleotides, at least 15 consecutive nucleotides, at least 16 consecutive nucleotides, at least

17 consecutive nucleotides, at least 18 consecutive nucleotides, at least 19 consecutive nucleotides, at least 20 consecutive nucleotides, at least 25 consecutive nucleotides, at least 30 consecutive nucleotides, at least 40 consecutive nucleotides, or at least 50 consecutive nucleotides. A nucleic acid sequence used as a probe includes nucleic acid sequences which are at least 70% homologous, more preferably at least 80% homologous, still more preferably at least 90% homologous, or at least 95% homologous to the aforementioned sequences. A sequence suitable as a primer can vary depending on the nature of a sequence which is intended to be synthesized (amplified), but those skilled in the art can appropriately design a primer depending on the intended sequence. Design of such a primer is well known in the art. Designing may be performed manually or by using a computer program (e.g., LASERGENE, PrimerSelect, or DNAStar).

As used herein, "probe" refers to a substance that is usable as means for search, used in a biological experiment such as in vitro and/or in vivo screening or the like. Examples thereof include, but are not limited to, a nucleic acid molecule comprising a specific base sequence, a peptide comprising a specific amino acid sequence, a specific antibody, a fragment thereof, and the like. As used herein, the probe can be used as means for marker detection.

As used herein, "mass spectrometry" or "MS" is used in the meaning that is commonly used in the art. This refers to an analytical approach for identifying a compound by its mass, referring to a technology for producing gaseous ions (ionization) from particles such as atoms, molecules, or clusters by some type of method, allowing the ions to move in a vacuum, and using electromagnetic force or the like or difference in the time of flight or the like to separate/detect the ions in accordance with the mass to charge ratio. MS refers to a method of filtering, detecting, and measuring ions based on mass to charge ratio, i.e., "m/z". With the recent dramatic improvement in the detection sensitivity and mass resolution, the scope of application thereof has further broadened such that utility is found in many fields. Typically, a method exemplified in Clark J et al., Nat Methods. 2011 March; 8(3): 267-272.doi:10.1038/nmeth.1564. can be used. The MS technology generally includes: (1) ionizing a compound to form a charged compound; and (2) detecting the molecular weight of the charged compound to calculate the mass to charge ratio. A compound can be ionized and detected by suitable means. A "mass spectrometer" generally comprises an ionization apparatus, a mass spectrometer, and an ion detector. Generally, one or more molecules of interest is ionized. The ion is then introduced into a mass spectrometer, where the ion follows a path in space that is dependent on mass ("m") and charge ("z") due to the combination of magnetic field and electric field. See, for example, Jurgen H, "Mass Spectrometry", Maruzen Publishing (2014) for an outline of a mass spectrometer. Examples of mass spectrometers include magnetic field, electric field, quadrupole, time-of-flight mass spectrometers, and the like. Examples of ion detection in quantification include selective ion monitoring for selectively detecting only ions of interest, selective response monitoring (SRM) for selecting one of the ion types purified at the first mass spectrometry unit as a precursor ion and detecting a product ion generated by cleaving the precursor ion in the second mass spectrometry unit, and the like. With SRM, selectivity increases, and noise decrease, thus improving the signal/noise ratio.

As used herein, the term "resolution" or "resolution (FWHM)" (also known in the art as "m/Δm50%") refers to the observed mass to charge ratio divided by the width of mass peak at 50% of the maximum height (full width at half maximum, "FWHM"). The qualitative and quantitative determination can improved with higher resolution.

As used herein, "label" refers to an entity (e.g., substance, energy, electromagnetic wave, or the like) for distinguishing a molecule or substance of interest from others. Examples of such a labeling method include RI (radioisotope) method, stable isotope labeling, fluorescence method, biotin method, optical approaches utilizing Raman scattering, chemiluminescent method, and the like. When a plurality of markers of the invention or agents or means for capturing the same are labeled by a fluorescence method, labeling is performed with fluorescent substances having different fluorescent emission maximum wavelengths. When a plurality of markers of the invention or agents or means for capturing the same are labeled by an optical approach utilizing Raman scattering, labeling uses substances with different Raman scattering from each other. In the present invention, such a labeled can be utilized to alter a subject of interest so that the subject is detectable by detection means that is used. Such an alteration is known in the art. Those skilled in the art can practice such a method as appropriate in accordance with the label and subject of interest.

As used herein, "diagnosis" refers to identifying various parameters associated with a condition (e.g., disease, disorder, or the like) in a subject or the like to determine the current or future state of such a condition. The condition in the body can be investigated by using the method, apparatus, or system of the invention. Such information can be used to select and determine various parameters of a formulation or method for the treatment or prevention to be administered, or condition in a subject, or the like. As used herein, "diagnosis" when narrowly defined refers to diagnosis of the current state, but when broadly defined includes "early diagnosis", "predictive diagnosis", "prediagnosis", and the like. Since the diagnostic method of the invention in principle can utilize what comes out from a body and can be conducted away from a medical practitioner such as a physician, the present invention is industrially useful. In order to clarify that the method can be conducted away from a medical practitioner such as a physician, the term as used herein may be particularly called "assisting" "predictive diagnosis, prediagnosis, or diagnosis". The technology of the invention can be applied to such a diagnostic technology.

As used herein, "therapy" refers to the prevention of exacerbation, preferably maintaining of the current condition, more preferably alleviation, and still more preferably disappearance of a condition (e.g., disease or disorder) in case of such a condition, including being capable of exerting a prophylactic effect or an effect of improving a condition of a patient or one or more symptoms accompanying the condition. Preliminary diagnosis with suitable therapy is referred to as "companion therapy" and a diagnostic agent therefor may be referred to as "companion diagnostic agent". If a modification of RNA can be identified using the technology of the invention, the modification can be associated with a specific disease condition, so that can be useful in such companion therapy of companion diagnosis.

The term "prognosis" as used herein refers to prediction of the possibility of death due to a disease or disorder such as cancer or progression thereof. A prognostic agent is a variable related to the natural course of a disease or disorder, which affects the rate of recurrence of an outcome of a patient who has developed the disease or disorder. Examples of clinical indicators associated with exacerbation in prognosis include any cell indicator used in the present invention. A prognostic agent is often used to classify patients into subgroups with different pathological conditions. If a modification of RNA can be identified using the technology of the invention, the modification can be associated with a specific disease condition, so that this can be useful as a technology for providing a prognostic agent.

As used herein, "detector" broadly refers to any instrument that can detect or test a subject of interest. As used herein, "diagnostic drug" broadly refers to all agents capable of diagnosing a condition of interest (e.g., cancer, species classification, senescence, or the like).

As used herein, "kit" refers to a unit generally providing portions to be provided (e.g., test drug, diagnostic drug, therapeutic drug, reagent, label, descriptions, and the like) into two or more separate sections. This form of a kit is preferred when a composition that should not be provided in a mixed state and is preferably mixed immediately before use for safety or other reasons is intended to be provided. Such a kit advantageously comprises an instruction or descriptions describing how the provided portions (e.g., test drug, diagnostic drug, therapeutic drug, reagent, label, and the like) are used or handled. When the kit is used herein as a reagent kit, the kit generally comprises instructions describing how to use a test drug, diagnostic drug, therapeutic drug, reagent, label, and the like. When combined with a testing instrument, diagnostic instrument, or the like, a "kit" can be provided as a "system".

As used herein, "instruction" is a document with an explanation of the method of using the present invention for a physician or other users. The instruction has a description of the detection method of the invention, method of use of a diagnostic agent, or instruction to administer a drug or the like. The instruction is prepared in accordance with a format specified by the regulatory agency of the country in which the present invention is practiced (e.g., the Ministry of Health, Labour and Welfare in Japan, Food and Drug Administration (FDA) in the U.S., or the like), with an explicit description showing approval by the regulatory agency. An instruction is a so-called package insert and is typically provided in, but not limited to, paper media. An instruction may also be provided in a form such as electronic media (e.g., web sites provided on the Internet or emails).

As used herein, "program" is used in the meaning that is commonly used in the art. A program describes the processing to be performed by a computer in order, and is legally considered a "product". All computers operate in accordance with a program. Programs are expressed as data in modern computers and stored in a recording medium or a storage device.

As used herein, "recording medium" is a medium for storing a program for executing the present invention. A recording medium can be anything, as long as a program can be recorded. For example, a recording medium can be, but is not limited to, a ROM or HDD or a magnetic disk that can be stored internally, or an external storage device such as flash memory such as a USB memory.

As used herein, "system" refers to a configuration that executes the method of program of the invention. A system fundamentally means a system or organization for executing an objective, wherein a plurality of elements are systematically configured to affect one another. In the field of computers, system refers to the entire configuration such as the hardware, software, OS, and network.

### (Summary of the method of the invention)

### (RNA modification)

The present invention provides a method of analyzing a condition of a subject based on modification information on an RNA. Modification (e.g., methylation) information on an RNA (e.g., microRNA) is closely associated with a condition (e.g., acquired condition such as a disease or drug resistance) of various subjects (mammals such as humans and microorganisms such as E. coli). It was surprising that a condition of a subject can be analyzed with a high level of precision by analyzing modification information on an RNA, especially a microRNA. In particular, the ability to distinguish a condition that was difficult to diagnose such as early stage pancreatic cancer shows that the present invention is medically and industrially very significant. In one embodiment, modification information comprises modification information on a microRNA. In one embodiment, modification information comprises methylation information. In one embodiment, modification information comprises methylation information on a microRNA.

### (Directly linked beads concentration analysis)

In one aspect, the present invention provides a method of analyzing a modification on an RNA by using a mass spectrometer, the method comprising:
(A) purifying an RNA of interest by using beads of nucleic acids complementary to the RNA of interest linked by a covalent bond; and
(B) ionizing the purified RNA by MALDI and measuring ions with a mass spectrometer.

It was found that a capture nucleic acid and beads, when directly bound, can be washed under a harsher condition compared to a capture nucleic acid and beads that are indirect bound by a biotin-streptavidin bond, thereby the intensity of MALDI-MS measurement is improved significantly.

The advantage of this approach is in targeting RNAs, which were not contemplated in the past. In the past, the internal sequence and the location of a modified base could not be studied, but the present invention enabled improvement by optimizing the setting of in source decay. The internal sequence and the location of a modified base can be observed by concomitant use of alkaline hydrolysis using ammonium, which is also a feature of the present invention. The ability to improve the efficiency by combining a technology of directly binding a capture nucleic acid with beads with the method of J. Engberg et al., (J. Engberg et al., Eur. J. Biochem, 41, 321-328 (1974)) in the new protocol of "purifying an miRNA of a specific sequence by adding a denaturant to a starting material (homogenate or cell lysate, serum, or the like) and performing direct hybridization" is an important aspect in one embodiment.

### (Exosome concentration analysis)

In one aspect, the present invention provides a method of analyzing modification on an RNA using a mass spectrometer, the method comprising:
(A-1) purifying an exosome by cell fractionation;
(A-2) purifying an exosome by using an anti-CD63 antibody;
(B) purifying an RNA of interest by using a nucleic acid that is complementary to an RNA of interest; and
(C) ionizing the purified RNA by ionization (e.g., MALDI) and measuring ions with a mass spectrometer.

It was found that, when purifying a nucleic acid of interest with a complementary nucleic acid, the intensity of mass spectrometry (e.g., MALDI-MS) measurement is improved significantly by performing the step of purifying an exosome in advance.

Although not wishing to be bound by any theory, it was found that purification is improved, or the intensity of mass spectrometry (e.g., MALDI-MS) measurement is improved significantly by combining step (A) and step (B), or step (A) and step (C) in the invention. When (A-1) and (A-2) were both performed, the MALDI-MS signal intensity was about 2.5-fold, which is an effect that was unexpected in the past.

The specific procedure for practicing the method of the invention is described hereinafter.

### (Sample)

While the method of the invention can be practiced by using any sample comprising an RNA, a sample that is readily obtained clinically is preferred. In one embodiment, a sample is derived from a subject. The Examples of the subject include, but are not limited to, mammals (e.g., human, chimpanzee, monkey, mouse, rat, rabbit, dog, horse, pig, cat, and the like), microorganisms (e.g., pathogen, microorganism used for fermentation, microbe such as E. coli, parasite, fungus, virus, and the like), edible organisms (avian, fish, reptile, fungus, plant, and the like), organisms raised as pets, and bioindicator organisms. In one embodiment, a sample is derived from a subject who has, or has the potential to have, a specific condition. In one embodiment, examples of a specific condition include, but are not limited to, disease, age, sex, race, familial lineage, medical history, treatment history, status of smoking, status of drinking, occupation, information on living environment, and the like. In one embodiment, a sample is an organ, tissue, cell (e.g., circulating tumor cell (CTC) or the like), blood (e.g., plasma, serum, or the like), epidermis of the mucous membrane (e.g., in the oral cavity, nasal cavity, ear cavity, vagina, or the like), epidermis of the skin, biological secretion (e.g., saliva, nasal mucus, sweat, tear, urine, bile, or the like), stool, epidermal microorganism or a portion thereof obtained from a subject. In one embodiment, a sample is a cultured cell (e.g., organoid based on a cell obtained from a subject, specific cell strain, or the like). In one embodiment, a sample is food or a portion thereof, or a microorganism on food.

### (RNA purification method)

In one embodiment, an RNA may or may not be purified in advance to analyze an RNA modification. As used herein, a "purified" substance or a biological agent (e.g., RNA such as a genetic marker, protein, or the like) refers to a substance or biological agent with at least a part of an agent naturally accompanying it removed. Therefore, the purity of a biological agent in a purified biological agent is higher than the normal state of the biological agent (e.g., concentrated). As used herein, the term "purified" means that preferably at least 75% by weight, more preferably at least 85% by weight, still more preferably at least 95% by weight, and most preferably at least 98% by weight of the same type of biological agents are present. A substance used in the present invention is preferably a "purified" substance. As used herein, "isolated" refers to a substance with at least one of any agent that is present in a naturally-occurring state removed. For example, retrieval of a specific microRNA sequence from a complete RNA sequence can be considered isolation. In one embodiment, an RNA can be purified from another component without distinction of all types of RNAs. In one embodiment, an RNA can be purified from another component by using poly A. In one embodiment, all microRNAs can be purified from another component. In one embodiment, an RNA having a sequence of interest (one or more types) can be purified from another component. In one embodiment, RNAs having a plurality of types of sequences of interest can be purified separately for each sequence. In one embodiment, an RNA having a modification (one or more types) can be purified from another component. In one embodiment, an RNA having a methylation modification can be purified from another component. In one embodiment, an RNA having a sequence of interest (one or more types) and a modification (one or more types) can be purified from another component.

In one embodiment, an RNA of interest comprises at least a portion of a sequence selected from the group consisting of SEQ ID NOs: 1 to 5:
CAAAGUGCUUACAGUGCAGGUAG (SEQ ID NO: 1)
UAGCUUAUCAGACUGAUGUUGA (SEQ ID NO: 2)
CGUCUUACCCAGCAGUGUUUGG (SEQ ID NO: 3)
UAAUACUGCCGGGUAAUGAUGGA (SEQ ID NO: 4)
UGAGGUAGUAGGUUGUAUAGUU (SEQ ID NO: 5)

Any known approach can be used for purification of RNAs. In one embodiment, a total RNA can be purified using an RNA specific molecule. In one embodiment, an RNA of interest can be purified by effecting a DNA degradation enzyme and then purifying a nucleic acid molecule. A plurality of type of RNAs can be purified separately or in parallel or in a mixed state. In one embodiment, 1, 2, 3, 3, 4, 5, 10, 15, 20, 25, 30, 35, 40, 45, 50, 75, 100, 150, 200, 300, 400, 500, 750, 1000, 1500, 2000, 2500, or 3000 type of RNAs can be purified in parallel (e.g., by using a sequence specific RNA capturing molecule that is bound to a carrier). In one embodiment, an RNA with a sequence of interest can be purified using a nucleic acid molecule (e.g., DNA and RNA) that is at least partially complementary to a sequence of interest, wherein the complementary nucleic acid molecule can comprise any portion for purification. Examples of any portion for purification include, but are not limited to, carriers such as beads (can be magnetic as needed), one of the pair molecules that bind to each other such as biotin and streptavidin, a portion that allows pair molecules binding to each other to bind (e.g., alkyne moiety in click chemistry), antibody recognition moiety, and the like. In one embodiment, an RNA of interest can be purified by using a specific binding molecule (e.g., antibody). In one embodiment, an RNA of interest can be purified using a binding molecule (e.g., antibody) that is specific to an RNA modification (e.g., methylation). In one embodiment, an RNA of interest can be purified using a binding molecule (e.g., antibody) that is specific to a specific type of RNA (e.g., microRNA). In one embodiment, an RNA of interest can be purified using a binding molecule (e.g., antibody) that is specific to a specific sequence. In one embodiment, an RNA of interest can be purified using a binding molecule (e.g., antibody) that is specific to a specific modification and a specific sequence.

In one embodiment, an RNA is purified using a DNA comprising at least a portion of a sequence selected from the group consisting of SEQ ID NOs: 6 to 10:
CTACCTGCACTGTAAGCACTTTG (SEQ ID NO: 6)
TCAACATCAGTCTGATAAGCTA (SEQ ID NO: 7)
CCAAACACTGCTGGGTAAGACG (SEQ ID NO: 8)
TCCATCATTACCCGGCAGTATTA (SEQ ID NO: 9)
AACTATACAACCTACTACCTCA (SEQ ID NO: 10)

In one embodiment, a modification in a modified RNA (optionally DNA) is an artificially introduced modification. Examples of artificially introduced modifications include, but are not limited to, modifications introduced by chemical synthesis. This also includes modifications imparted by an agent binding to an RNA in an organism (including viruses) when the organism is treated with the agent. For example, treatment of an organism with an agent (e.g., anticancer agent) that chemically interacts directly with a nucleic acid in the organism can result in a modified RNA (optionally DNA) introduced with an agent derived portion. The method of the invention can readily identified a nucleic acid that is highly likely to be introduced with such an artificial modification (type, location, or the like). A nucleic acid that is highly likely to be introduced with such an artificial modification can be useful as an indicator, biomarker, or the like for research and development of agents. Information on modifications artificially introduced in a nucleic acid (RNA or DNA) can be used in combination with RNA modification information.

In one embodiment, detection of a nucleic acid with an artificially introduced modification can use mass spectrometry, radioactive isotopic labeling, or the like, or a molecule (e.g., antibody (BrdU antibody or the like), streptavidin for a modified portion introduced with a biotin moiety, or the like) that specifically binds to this chemical structure can be designed based on the chemical structure of the modified portion, so that a detection method (e.g., post-purification sequencing, fluorescence detection, or the like) utilizing such a specific binding molecule can be used.

In one embodiment, an RNA of interest can be purified by purifying an organelle (e.g., exosome). In one embodiment, an organelle (e.g., exosome) can be purified by centrifugation. In one embodiment, an RNA of interest can be purified by using a molecule (antibody) binding to a molecule in an organelle (e.g., purify an exosome using an anti-CD63 antibody).

An RNA of interest can be purified in a single or multiple stages. For example, purification of an RNA with a sequence of interest can purify an RNA with a sequence of interest directly from a sample, or purify all RNAs from the sample and then purify an RNA with the sequence of interest.

In one embodiment, when purifying a plurality of types of RNAs of interest, at least two of the RNAs of interest can be purified in a mixed state, or each RNA of interest can be purified separately. For example, when RNAs with a plurality of sequences of interest are purified separately, a sample can be divided into a plurality of samples, and nucleic acids of interest with sequences that are different from one another can be purified from each divided sample, or a nucleic acid of interest can be purified by applying a sample to a carrier having nucleic acid molecules complementary to each sequence of interest placed at a plurality of spaced locations.

### (Detection and identification of modification)

A modification on an RNA can be detected and identified using any known approach. Examples of such methods for detecting and identifying a modification on an RNA include fluorescence detection using a modification specific antibody, sequencing of RNA that has been immunoprecipitated by a modification and/or RNA specific protein (antibody or the like), mass spectrometry of purified RNA, sequencing of an RNA subjected to chemical treatment such as bisulfite sequencing (combined with PCR as needed), nanopore sequencing (e.g., of Oxford Nanopore Technologies), and tunneling current sequence (Scientific Reports 2, doi: 10.1038/srep00501 (2012)).

In one embodiment, a modification on an RNA can be detected and identified in parallel with identification of sequence information of the RNA. In the present invention, any RNA modification information can be identified. In one embodiment, at least one of the type of modification on an RNA of interest (including the type of modifications by a functional group of the same type introduced at different locations on a single nucleotide), the amount and ratio of a modified RNA on interest, the amount and ratio of a modification on an RNA of interest, and the location of a modification on an RNA of interest, is identified, optionally with the amount of the RNA. In one embodiment, a modification condition on an RNA of interest can comprise the reliability of the modification condition (e.g., probability of true positive). In one embodiment, methylation on an RNA is identified. In one embodiment, methylation on a nucleoside is identified. In one embodiment, methylation on a nucleobase of an RNA is identified. In one embodiment, methylation on a nucleoside is identified. In one embodiment, m⁶A of an RNA is identified. In one embodiment, a modification condition of an RNA (e.g., modification location, reliability of a modification condition, or the like) is identified based on at least one of information on a recognition motif of an enzyme adding a modification, information on a recognition motif of an enzyme removing a modification, and information on a recognition motif of a protein binding to a modification.

In one embodiment, a modification including a modification selected from the group consisting of:
Methylation of 13^{th} A of SEQ ID NO: 1 CAAAGUGCUUACAGUGCAGGUAG (SEQ ID NO: 15)
Methylation of 9^{th} C of SEQ ID NO: 2 UAGCUUAUCAGACUGAUGUUGA (SEQ ID NO: 16)
Methylation of 13^{th} C of SEQ ID NO: 3 CGUCUUACCCAGCAGUGUUUGG (SEQ ID NO: 17)
Methylation of 9^{th} C of SEQ ID NO: 4 UAAUACUGCCGGGUAAUGAUGGA (SEQ ID NO: 18)
Methylation of 19^{th} A of SEQ ID NO: 5 UGAGGUAGUAGGUUGUAUAGUU (SEQ ID NO: 19)
is identified.

In one embodiment, a modification on an RNA can be detected and identified by radiation released from a radioactive atom contained in a moiety constituting the modification (e.g., methyl moiety). In one embodiment, a modification on an RNA can be identified by detection of a bond of a molecule (modification specific antibody) specifically binding to the modification (e.g., detection of fluorescence) or detection of a reactant generated by reacting with a molecule that reacts specifically to the modification (e.g., detection of reactant, i.e., light, detection of a biotin derivative generated by reaction with streptavidin, or the like).

In one embodiment, a modification on an RNA can be identified in a method of sequencing a nucleic acid such as bisulfite sequencing or sequencing of an RNA that has been concentrated with a modification specific antibody (RIP sequencing). Any suitable sequencing method can be used in accordance with the present invention. A next generation sequencing (NGS) technology is preferred. As used herein, the term "next generation sequencing" or "NGS" refers to all new high-throughput sequencing technologies, which divide the entirety of various nucleic acid into small pieces to randomly read nucleic acid templates in parallel along the entire nucleic acid, in comparison to "conventional" sequencing methods known as Sanger chemistry. The NGS technologies (also known as massive parallel sequencing technologies) can deliver nucleic acid sequence information of the full genome, exome, transcriptome (all transcribed sequences of the genome) or methylome (all methylated sequences of the genome) in a very short period of time, such as 1 to 2 weeks or less, preferably 1 to 7 days or less, or most preferably less than 24 hours, which enables a single cell sequencing approach in principle. Any NGS platform that is commercially available or mentioned in a reference can be used for practicing the present invention. In one embodiment, a modification on an RNA can be identified by sequencing a nucleic acid amplified by PCR.

In one embodiment, an RNA is identified as a modified RNA based on being purified by a modification specific binding molecule (e.g., antibody such as an anti-m6A antibody). Data obtained by sequencing can be processed by any analytical method and converted to RNA modification information. Examples of such an analytical method include, but are not limited to, MetPeak (see Cui et al., Bioinformatics (2016) 32 (12): i378-i385).

In one embodiment, a modification on an RNA can be identified with a mass spectrometer. Examples of mass spectrometers that can be used in the present invention include magnetic field, electric field, quadrupole, time-of-flight (TOF) mass spectrometers, and the like. In one embodiment, a single stranded RNA can be measured, or an RNA forming a double strand with a DNA or RNA can be measured with a mass spectrometer.

Mass spectrometry can be combined with any ionization method. Examples of ionization method that can be used in the present invention include, but are not limited to, electron ionization (EI), chemical ionization (CI), fast atom bombardment (FAB), matrix-assisted laser desorption/ionization (MALDI), and electrospray ionization (ESI). ESI can be combined with liquid chromatography, supercritical chromatography, or the like. A plurality of types of RNAs can be measured while being separated by chromatography. Examples of columns that can be used in chromatography include hydrophilic interaction chromatography (HILIC) columns, reverse phase (RP) chromatography columns, and the like.

For MALDI, a sample is premixed with a substance (coating agent) that is ready ionized with a laser beam as a matrix, and is placed at a spot (anchor position) on a target plate. Irradiation thereof with a laser beam results in ionization. Examples of coating agent that can be used in the present invention include, but are not limited to, 3-HPA (3-hydroxypicolinic acid), DHC (diammonium hydrogen citrate), CHCA (α-cyano-4-hydroxycinnamic acid), and the like. In one embodiment, an RNA placed on a spot (anchor position) on a target plate can comprise an RNA with a plurality of sequences, but is preferably a group of RNAs sharing the same sequence. It can become more difficult to check the sequence and/or modification location as the types of sequences of RNA that are present at a single anchor position increase.

In one embodiment, a modification condition of an RNA (e.g., presence/absence of a modification, modification location, number of modifications, reliability of a modification, or the like) can be identified based on a measurement value for an unfragmented ion (parental ion) and/or fragmented ion (daughter ion). In one embodiment, a modification condition (e.g., amount of modification or the like) of an RNA can be identified by comparison with a control molecule (e.g., stable isotope labeled nucleic acid, unmodified nucleic acid, the other nucleic acid of a pair forming a complementary double strand, or the like). In one embodiment, a modification condition of an RNA can be identified based on reference information (e.g., measurement result of a standard sample, known modification information, or the like). Mass spectrometry data can be converted into an RNA modification condition by processing with any software. Examples of such software include, but are not limited to, DNA methylation analysis system MassARRAY® EpiTYPER (Sequenom).

### (Pretreatment)

In one embodiment, an RNA of interest can be physically, chemically, or biologically pretreated prior to measurement. Pretreatment can attain an effect of, for example, improvement in the sensitivity, accuracy, and/or precision in the measurement of an RNA of interest, distinction of different types of modifications, improvement in quantification in inter-sample comparison, and improvement of separation in a measuring apparatus. Examples of such pretreatment include, but are not limited to, dimethylsulfate treatment, halogen compound treatment, alkaline hydrolysis treatment, stable isotope label introducing treatment, and treatment with a detection improving agent (e.g., compound comprising a portion with excellent absorption of laser in MALDI, such as fluorescent dyes). In one embodiment, pretreatment can be performed on a substrate (beads or the like) carrying an RNA. In one embodiment, an agent used for pretreatment can be designed to introduce a group into an amine moiety on a base of an RNA, a hydroxyl group or a phosphoric acid group at a terminus.

Dimethyl sulfate treatment can selectively methylate a nitrogen atom at position 1 of a purine ring of adenine of an RNA to impart a mass of +14 Da, but this reaction does not progress when the nitrogen atom at position 1 of a purine ring is already methylated, so that 1-mA and N6-mA can be distinguished. Methyl trifluoromethanesulfonate can be similarly used. For example, halogenated acetaldehyde can be used for halogen compound treatment. This changes the mass number by crosslinking between an amino group at position 4 and a nitrogen atom at position 3 of cytosine of an RNA to form a new 5-membered ring, but this reaction does not progress when a nitrogen atom at position 3 of cytosine is methylated. Examples of halogenated acetaldehyde include bromoacetaldehyde and chloroacetaldehyde. Since the boiling point of these compounds is about 60°C, removal by vacuum drying is possible without degradation of an RNA. Alkaline hydrolysis fragments an RNA by, for example, treating the RNA with ammonium.

### (Analysis)

Various conditions can be analyzed using the obtained RNA modification information. In one embodiment, a medical condition or a biological condition of a subject is analyzed using obtained RNA modification information. Examples of the medical condition or biological condition of a subject include, but are not limited to, a disease, senescence, immunological condition (e.g., intestinal tract immunity, systemic immunity, and the like), cell differentiation condition, responsiveness to an agent or treatment, and a condition of a microorganism (e.g., enterobacteria, epidermal bacteria, or the like) of a subject. Examples of diseases that can be analyzed by the present invention include, but are not limited to, a cranial nerve disease, pollution disease, pediatric surgery disease, fungal disease, specific disease, infectious disease, cancer (malignant tumor), gastrointestinal disease (including inflammatory bowel disease), neurodegenerative disease, allergic disease, parasitic disease, infectious disease of an animal, urinary tract tumor, various syndromes, respiratory disease, mammary gland tumor, personality disorder, skin disease, sexually transmitted disease, dental disease, psychiatric disease, renal urinary disease, ophthalmic disease, food poisoning, intermediate host for Gymnosporangium, hepatitis, cardiovascular disease, rare disease, connective tissue disease, symptom, zoonosis, paraphilia, immune disease (including intestinal tract immunity), congenital disease, developmental disorder, skin rash, congenital heart disease, regional disease name, phobia, viral infection, male reproductive system disease, animal disease, fish disease, proliferative disease, polyp, periodontal disease, mammary gland disease, genetic disease, hematological disease, endocrine metabolic disease, gynecological disease, disease causing fever and rash, soft tissue tumors, plant disease, and the like. Examples of diseases that can be particularly suitably analyzed by the present invention include, but are not limited to, cancer, inflammatory bowel disease, Alzheimer's or angiopathic dementia, borderline mental illness, dilated cardiomyopathy, hypertrophic cardiomyopathy, heart failure (including nonobvious mild heart failure), heart disease (e.g., including those that are fatal, inducing sudden death due to arrhythmia), and the like. These diseases can affect the modification condition of an RNA via specific metabolism of a cell. Examples of a condition of a microorganism of a subject include, but are not limited to, a condition that can be a public health incident such as resistance to heating, disinfectant, or the like (e.g., sporulation of hepatitis E virus living on food that is not completely cooked or the like), a modification condition (methylation or the like) of a nucleic acid of a virus (e.g., hepatitis RNA virus, papilloma DNA virus) that has infiltrated a host, and the like.

The present invention is significant from the medical viewpoint in that cancers such as pancreatic cancer (e.g., early stage pancreatic cancer), liver cancer, gallbladder cancer, cholangiocarcinoma, gastric cancer, large intestinal cancer (rectal cancer, colon cancer), bladder cancer, kidney cancer, breast cancer, lung cancer, brain tumor, and skin cancer can be targeted. The present invention can also analyzed the degree (stage) of progression of cancer (e.g., pancreatic cancer, liver cancer, gallbladder cancer, cholangiocarcinoma, gastric cancer, colon cancer, bladder cancer, kidney cancer, breast cancer, lung cancer, brain tumor, or skin cancer). In one embodiment, a condition of cancer (e.g., pancreatic cancer, liver cancer, gallbladder cancer, cholangiocarcinoma, gastric cancer, colon cancer, bladder cancer, kidney cancer, breast cancer, lung cancer, brain tumor, or skin cancer) can be analyzed based on methylation of at least one of miR-21, miR-17, let-17a, and miR-200c. In one embodiment, a condition of cancer (e.g., pancreatic cancer, liver cancer, gallbladder cancer, cholangiocarcinoma, gastric cancer, colon cancer, bladder cancer, kidney cancer, breast cancer, lung cancer, brain tumor, or skin cancer) can be analyzed based on methylation of at least one of miR-21, miR-17, let-17a, and miR-200c. A condition of cancer (e.g., pancreatic cancer) can be analyzed based on methylation of at least one of let-7, miR-21, miR-100, miR-222, miR-92a, miR-10a, miR-99b, miR-30d, miR-26a, miR-320a, miR-148a, miR-125a, miR-423, miR-182, miR-7641, miR-378a, miR-1307, miR-221, miR-183, miR-25, miR-24, miR-30a, miR-128, miR-941, miR-1246, miR-92b, miR-122, miR-5100, miR-106b, miR-181a, miR-27b, miR-29a, miR-224, miR-191, miR-146b, miR-27a, miR-3182, miR-532, miR-3184, miR-30c, miR-181b, miR-744, miR-7706, miR-148b, miR-629, miR-103b, miR-103a, miR-98, miR-23a, miR-425, miR-192, miR-22, miR-3615, miR-5701, miR-155, miR-149, miR-7704, miR-1180, miR-1275, miR-769, miR-1273g, miR-484, and miR-17.

The present invention also can analyze responsiveness to an agent (e.g., anticancer agent, molecularly targeted drug, antibody drug, a biological formulation (e.g., nucleic acid or protein), an antibiotic, or the like) or treatment of a target organism. For example, drug resistance or the like can also be analyzed. The invention can also be applied to, for example, analysis of responsiveness of an anticancer agent, selection of a suitable therapeutic agent, or antibiotic resistance or the like. The analysis of the invention can also be used in analysis of following up or prognosis of surgery, radiation treatment or the like such as heavy particle beam (for example, Carbon/HIMAC) or X-ray treatment. It is understood that various drugs such as Lonsurf (TAS 102), gemcitabine, CDDP, 5-FU, cetuximab, a nucleic acid drug, and a histone demethylase inhibitor can be analyzed using the present invention, which can be utilized as basic information for a therapeutic strategy. If the agent is for example an anticancer agent, the present invention achieves establishment of a therapeutic strategy by testing the responsiveness as to whether a subject is resistant to the anticancer agent. Therefore, an agent for treating a subject and/or additional treatment of the subject can be selected based on responsiveness to treatment such as the agent by using the present invention. When the responsiveness for a plurality of agents is studied, an agent for treating the condition can be indicated from among the plurality of agents when using the present invention. Analysis can be performed based on comparison of modification information (e.g., methylation) of an RNA of the invention in the subject before and after administration of an agent or the treatment.

In one embodiment of the invention, a subject of analysis of a biological condition or a medical condition can be analyzed by further taking into consideration at least one piece of information selected from the group consisting of age, sex, race, familial information, medical history, treatment history, status of smoking, status of drinking, occupational information, information on living environment, disease marker information, nucleic acid information (including nucleic acid information on bacteria in the subject), metabolite information, protein information (expression level information or structural information), enterobacterial information, epidermal bacterial information, and a combination thereof. Examples of nucleic acid information that can be utilized in the present invention include genomic information, epigenomic information, transcriptome expression level information, RIP sequencing information, microRNA expression level information, and a combination thereof. RIP sequencing information that can be utilized individually can include RIP sequencing information on an agent resistant pump P-glycoprotein, RIP sequencing information on a stool, RIP sequencing information on E. coli in a stool, or the like.

The present invention can analyze the condition of the subject based further on modification information on the RNA in an agent or treatment resistant strain, or a combination of the resistant strain and a cell strain from which the resistant strain is derived. Examples of such an agent or treatment include, but are not limited to, Lonsurf (TAS 102), gemcitabine, CDDP, 5-FU, cetuximab, a nucleic acid drug, a histone demethylase inhibitor, and a treatment using a heavy particle beam (e.g., Carbon/HIMAC) or an X-ray.

The types of RNA subjected to analysis by the preset invention can be increased or decreased in accordance with the objective of the analysis. For example, modification information on at least 5 types, at least 10 types, at least 20 types, at least 30 types, at least 50 types, at least 100 types, at least 200 types, at least 300 types, at least 500 types, at least 1000 types, at least 1500 types, or at least 2000 types of RNAs can be analyzed. Alternatively, when a microRNA is targeted, all available microRNAs can be targeted. The types of RNAs are not particularly limited, but a single type or a plurality of types of mRNAs, tRNAs, rRNAs, IncRNAs, miRNAs, or the like can be combined and used. In one embodiment, a plurality of pieces of modification information on RNAs comprising the same sequence can be analyzed. In another embodiment, a condition of a subject can be analyzed based further on structural information of an RNA.

One embodiment can comprise analyzing the condition of the subject based further on modification information on an RNA in an organism with a knockdown of at least one of a methylase (e.g., Mettl3, Mettl14, or Wtap), a demethylase (e.g., FTO or AlkBH5), and methylation recognizing enzyme (e.g., family molecule with a YTH domain such as YTHDF1, YTHDF2, or YTHDF3) and/or recognition motif information on at least one of a methylase (e.g., Mettl3, Mettl14, or Wtap), a demethylase (e.g., FTO or AlkBH5), and methylation recognizing enzyme (e.g., family molecule with a YTH domain such as YTHDF1, YTHDF2, or YTHDF3).

One embodiment can perform calculating a probability of a condition based on a plurality of pieces of modification information. Any statistical approach can be performed as the step of calculating, such as primary component analysis.

In one embodiment, the efficacy of an anticancer agent with accumulated clinical evidence (e.g., Lonsurf (TAS 102), gemcitabine, CDDP, 5-FU, cetuximab, a nucleic acid drug, or a histone demethylase inhibitor) on tumor tissue can be studied to establish a therapeutic strategy.

In one embodiment, a new mechanism of action of various agents can be elucidated to develop a middle molecule compound that can be applied in a further therapeutic strategy. For example, the compound can be utilized in drafting a strategy to overcome advanced refractory cancer.

In one embodiment, analysis of a microRNA with the approach of the invention can further elucidate the mechanism of action. Specifically, a microRNA specific to an agent such as an anticancer agent can be analyzed using the method of the invention, and a companion diagnostic drug can be designed using the same.

In one embodiment, companion diagnosis using an miRNA in peripheral blood obtained by minimally invasive liquid biopsy can be performed. For example, clinical information using an agent (e.g., Lonsurf (TAS 102), gemcitabine, CDDP, 5-FU, cetuximab, a nucleic acid drug, or a histone demethylase inhibitor) was collated with data for miRNAs in peripheral blood, and collated with mechanism analysis in a cell resistant to the agent, and miRNAs transcribed from chromatin in response to exposure to the agent and miRNAs secreted as exosomes in peripheral blood were able to be identified as 60 panels. The present invention provides a technology for identifying and analyzing such a panel microRNA.

In one embodiment, the present invention can perform an analysis related to a cancer stem cell or Cancer Initiating Cell (CIC).

In one embodiment, the analysis of the invention can also be applied when a modified RNA is itself a target molecule of a drug. Specifically, a novel agent can be screened by detecting whether an RNA is modified or unmodified using the analysis technology of the invention. In particular, it was not known to apply a modification (e.g., methylation) of an RNA such as a microRNA in such screening for a novel agent in the past. The present invention can provide an agent with a new mechanism of action.

In another embodiment, the analysis of the invention can also be applied when a modified RNA is itself a component molecule of a drug. For example, a novel agent can be screened by analyzing whether a target modified RNA or an external agent such as an enzyme responsible for the modification can be utilized as an agent by detecting whether an RNA is modified or unmodified using the analysis technology of the invention.

In addition to RNA modifications, the present invention can also analyze other information on a nucleic acid besides RNA modifications such as a combination of information on base substitutions and/or modifications of a nucleic acid (DNA, RNA, or the like). Multi-omic analysis can be combined with a technology of multi-oic analysis of omics other than RNA modification (epitranscriptome) in Sijia Huang et al., Front Genet. 2017; 8:84, Yehudit Hasin et al., Genome Biol. 2017; 18:83 or the like.

Other information on nucleic acids can be analyzed by, for example, mass spectrometry or the like. For example, RIP-seq can be applied to RNAs, DIP-seq can be applied to DNAs, and FDIP-seq can be performed with BrdU or the like for FDNAs to perform analysis.

In one embodiment, the analysis technology of the invention can elucidate a new mechanism based on clinical evidence.

In still another embodiment, the present invention can study a drug development target. For example, a drug of a small or middle molecule compound can be developed, which targets the interaction between a complex of a plurality of molecules and a target. The analysis technology for RNA mod of the invention can be utilized when screening a library or screening a phenotype using an organoid or an individual animal.

In one embodiment, the present invention can be utilized in drug development that can handle tumor diversity. In addition to RNA mod of the invention (e.g., methylation information of a microRNA), single molecule measurement of a modified DNA incorporating ChIP-seq or FTD, single cell analysis (C1) of lymphocytes or CAF (Cancer Associated Fibroblasts) of the stroma of tumor tissue, or the like can be combined and applied. When an agent is administered to a patient, the overall effect can be understood including responses of not only cancer cells, but also the host side such as tumor stroma. If an inhibitor can be classified by utilizing information of RNA mod, an innovative drug that can differentiate the cancer cell side or stroma side can be developed.

In one embodiment, for a certain agent, the present invention can be applied to (1) expand diseases to which the agent is effective to others, (2) demonstrate the superiority to other existing agents and move to 2^{nd} line therapy or earlier, (3) elucidate a new mechanism of action and investigate a possibility leading to a therapeutic drug, or the like.

In one embodiment, for example, expression information of an miRNA inside a serum exosome as a liquid biopsy of a patient such as a cancer patient can be prepared to analyze expression information of an miRNA inside a serum exosome of a patient after therapy of the subject of analysis or modification information of the invention. For this reason, for colon cancer, expression information of an miRNA inside a serum exosome of an advanced colon cancer patient or modification information can be analyzed using, for example, a database for a total of 1000 cases (The Cancer Genome Atlas-Cancer Genome; TCCA).

Expression information of an miRNA inside a serum exosome of a colon cancer patient after therapy or modification information can also be analyzed.

In one embodiment, the present invention can provide a next generation RNA biomarker based on RNA modification information based on the results of analysis. This can be clinically applied. For example, the present invention can find the tissue homeostasis by microRNA modomics and perform clinical applications using the same.

In analysis using information on an RNA of the invention, it can be important that a target is a transcription factor, i.e., is an inducing agent that is a key to regulating (positively in many cases) expression of a target gene. In such a case, the number can be narrowed down by carefully selecting an independent transcription factor. For example, particularly noteworthy is that it was found "c-myc" having action as a cancer gene can be let go early in cancer diagnosis if the method of the invention is used. It is understood that limited independence of a transcription factor is lost in the presence of a cancer gene, and various actions are manifested in a cell context dependent manner, so that the minimum number cannot be found clearly. It was found that in such a case, "c-myc" would be noise since c-myc acts on many sideway actions.

In a preferred embodiment of the invention, a miRNA (microR) is used. Such a case is characterized in having many-to-many relationship. Specifically, one of the important points is that a single microR acts on many, and shares a common target between microRNAs as different molecules. It is not surprising that, given that there is an important set inducing a certain event, this is not a single molecule in such a regulatory system with "many-to-many relationship". Rather, this being a limited set, and being expressable with weightings that can express the hierarchy within the set are features of analysis provided by the present invention.

In one embodiment, cancer diagnosis with RNA modomics for microRNAs is envisioned. This is not limited thereto. Additionally, agent resistance (not only anticancer agent, but also molecularly targeted drug, antibody drug, nucleic acid, and other biological formulations, and more broadly a microorganism derived antibiotic or the like), classification of a population of species, inflammatory bowel disease, E. coli, food classification (production region, age, taste, quality, expiration date, sense of taste) and the like are also envisioned. RNA modomics can be used for selecting koji yeast.

In one embodiment, it is known for example that other agents such as 5-FU and CDDP have significantly different IC50 distributions, where 5-FU is very effective when effective, but almost completely ineffective when ineffective. This can be found with the epitranscriptome. For example, for microRNAs, it is known that classification lines can be drawn more precisely by largely differentiating with primary component analysis (PCA) from studying the epitranscriptome rather than studying at the expression (see the Examples).

RNA methylation is known to be associated with Circadian rhythm (Sanchez et al., Nature. 2010 Nov 4; 468 (7320): 112-6, Jean-Michel et al., Cell Vol. 155, Issue 4, pp. 793-806 7 November 2013, and the like). In one embodiment, RNA modification information can be used to analyze sleep activity related to time difference (jet lag, etc.) For example, determination of the possibility of an impact of time difference on sleep activity of a subject, personnel and medical management associated therewith, management of a pilot or flight attendant, stratification of whether dosing of melatonin is recommended, or the like can be performed based on such analysis. In one embodiment, RNA modification information can be used to analyze jet lag during space flight.

In one embodiment, RNA modification information can be used to analyze whether sleep of a subject is sufficient. Although latent sleep deprivation is an issue, the subject is not self-aware in many cases. In this regard, RNA modification information can be used for the correction thereof. In one embodiment, this is matched with sleep habit therapy. In one embodiment, RNA modification information can be used to manage the health of long distance bus drivers. In one embodiment, RNA modification information can be used for welfare management. In one embodiment, RNA modification information can be used to manage the health of night shift workers (steel manufacturing plant, nuclear power plant, hospital workers, medical practitioners, security guards, building management company employee, etc.)

In one embodiment, the age of a subject can be analyzed using RNA modification information of a blood sample (without using base sequence information of a nucleic acid as needed) for use in crime investigation.

In one embodiment, RNA modification information can be used to analyze the presence/absence of doping.

### (Biomarker screening)

In one embodiment, a new biomarker can be searched using obtained RNA modification information. In one embodiment, RNA modification information obtained in a subject in a certain condition can be compared to RNA modification information obtained in a subject who is not in such a condition, and an RNA or group of RNAs observed to have a difference (e.g., statistically significant difference) in an RNA modification condition (e.g., amount, modification location, or the like) can be used as a biomarker for predicting the condition.

In one embodiment, RNA modification information obtained in a subject administered with a drug and/or treatment can be compared to RNA modification information obtained in a subject who is not administered with such a drug and/or treatment, and an RNA or group of RNAs observed to have a difference (e.g., statistically significant difference) in an RNA modification condition (e.g., amount, modification location, or the like) can be used as a biomarker for predicting the responsiveness and/or resistance to the drug and/or treatment.

### (Resistant strain)

In one embodiment, RNA modification information obtained in a resistant strain with resistance to a drug and/or treatment can be compared to RNA modification information obtained in a wild-type strain from which the resistant strain originated, and an RNA or group of RNAs observed to have a difference (e.g., statistically significant difference) in an RNA modification condition (e.g., amount, modification location, or the like) can be used as a biomarker for predicting the responsiveness and/or resistance to the drug and/or treatment.

Such a resistant strain can be prepared, for example, by maintenance culture of a wild-type strain in the presence of a drug and/or treatment. In one aspect, the present invention provides a method of preparing such a resistant strain. In one aspect, a strain resistant to a drug and/or treatment can be evaluated as to whether the strain is a resistant strain based on IC₅₀ with respect to the drug and/or treatment. In one aspect, the present invention provides a resistant strain with resistance to each of trifluridine (FTD), 5-fluorouracil (5-FU), gemcitabine, cisplatin, Carbon/HIMAC (heavy particle beam), and X ray.

### (Drug screening)

In one embodiment, a new drug can be evaluated using obtained RNA modification information. In one embodiment, RNA modification information obtained in a subject treated with a certain drug can be compared to RNA modification information obtained in a subject treated with another drug for classification of drugs based on an RNA changed by treatment with each drug.

### (Species classification)

In one embodiment, organism species can be classified by using obtained RNA modification information. In one embodiment, a microorganism (e.g., E. coli) can be classified by using obtained RNA modification information. In one embodiment, a microorganism (e.g., E. coli) can be classified by using obtained RNA modification information. In one embodiment, a microorganism (e.g., E. coli) can be classified by using modification information on an RNA encoding an agent resistant pump P-glycoprotein. In one embodiment, a subject (e.g., mammal such as a human, food, or the like) from which a microorganism (e.g., E. coli) was obtained can be analyzed based on a result of classifying the microorganism.

### (Food)

In one embodiment, quality of food can be analyzed using obtained RNA modification information. Examples of quality of food include, but are not limited to, production region, age, time since processing, freshness, denaturation after processing, quality of taste, status of active oxygen, microorganism contamination (E. coli, Salmonella, Clostridium botulinum, virus, parasite, and the like), fermentation status (including condition of microorganisms associated with fermentation), chemical factors (e.g., pesticides, additives, and the like), physical factors (e.g., foreign objects, radiation, and the like), status of fatty acid, degree of maturation, and the like. In one embodiment, quality of food can be analyzed using RNA modification information obtained for controlling quality of food by a public institution such as a governing body. In one embodiment, quality of food can be analyzed by using RNA modification information obtained to provide an indicator for a consumer to determine the quality of a product (objectively express quality which was expressed by taste or odor).

Unlike DNAs, RNAs, and proteins, RNA modifications (e.g., methylation) provide a new development, when viewed from a different viewpoint, by using the present invention. For example, DNAs and RNAs lose information on contiguous base sequences with degradation (become short and fragmented). Methylation is expressed as a methylation ratio as an indicator expressing the quality thereof, as long as there is a target site. Thus, this is unique in that "how the original factors diminish and remain during chronological changes" can be monitored. Proteins are not only in the middle thereof, but a target is not determined in the present case, such that proteins have limitations as a tracking tool or a tracer. Therefore, modifications attain a particularly significant effect unlike DNAs, RNAs, and proteins.

### (Utilization of additional information)

In one embodiment, a condition of a subject can be analyzed by using RNA modification information obtained from a subject as well as other information, such as RNA modification information obtained from a subject at another time (e.g., before and after treatment), information related to the subject, information on a motif of a protein associated with a modification, information related to RNA modification obtained from another subject, information related to a complex of a substance binding to an RNA (protein, lipid, or the like) and the RNA (optionally, an additional condition associated with an RNA modification condition), and the like.

Examples of information related to a subject that can be additionally used include the subject's age, sex, race, familial information, medical history, treatment history, status of smoking, status of drinking, occupational information, information on living environment, disease marker information, nucleic acid information (including nucleic acid information of bacteria in the subject), metabolite information, protein information, enterobacterial information, epidermal bacterial information, and the like. Examples of nucleic acid information include genomic information, genomic modification information, transcriptome information (including information on the expression level and sequence), RIP sequencing information, and microRNA information (including information on the expression level and sequence). Examples of RIP sequencing information that can be used individually include RIP sequencing information on an agent resistant pump P-glycoprotein, RIP sequencing information on a stool, RIP sequencing information on E. coli in a stool, and the like.

Examples of motif information of a protein associated with a modification that can be additionally used include information on a recognition motif of an enzyme adding a modification, information on recognition motif of an enzyme removing a modification, and information on a recognition motif of a protein binding to a modification. Specific examples thereof include motif information on methylase (e.g., Mettl3, Mettl14, and Wtap), demethylase (e.g., FTO and AlkBH5), and methylation recognizing enzyme (e.g., family molecule with a YTH domain such as YTHDF1, YTHDF2, or YTHDF3).

Examples of information related to RNA modifications obtained from another subject that can be additionally used include, but are not limited to, RNA modification information in a subject in a certain condition, RNA modification information in an organism genetically engineered for expression of a protein associated with a modification, RNA modification information in a resistant strain having resistance to a drug and/or treatment, RNA modification information in a subject administered with a drug and/or treatment, and information related to a complex of a substance binding to an RNA (protein, lipid, or the like) and the RNA (optionally a condition associated with an RNA modification condition).

In the present invention, the condition of the subject can be analyzed based further on modification information on the RNA in an agent or treatment resistant strain or a combination of the resistant strain and a cell strain from which the resistant strain is derived. Examples of such an agent or treatment include, but are not limited to, Lonsurf (TAS 102), gemcitabine, CDDP, 5-FU, cetuximab, a nucleic acid drug, a histone demethylase inhibitor, and a treatment using a heavy particle beam (e.g., Carbon/HIMAC) or an X-ray.

### (Subject condition analysis method)

In one embodiment, the present invention provides a method of analyzing a condition of a subject, comprising: obtaining modification (e.g., methylation) information on at least one type of RNA (e.g., microRNA) in a subject; and analyzing a condition of the subject based on the modification information. Modification information can be obtained by measuring a sample derived from a subject. In one embodiment, analysis is onsite analysis for taking measurement in a short period of time (e.g., 1 day or less, 10 hours or less, 5 hours or less, 2 hours or less, 1 hour or less, 30 minutes or less, 15 minutes or less, or the like) after obtaining a sample. In one embodiment, a result of onsite analysis is outputted in a short period of time after obtaining a sample (e.g., 1 day or less, 10 hours or less, 5 hours or less, 2 hours or less, 1 hour or less, 30 minutes or less, 15 minutes or less, or the like). In one embodiment, after a sample is obtained, the sample is delivered to a location of a measurement instrument and/or analyzer, where analysis is performed. A sample can be obtained by the subjects themselves. In one embodiment, an obtained sample is frozen and delivered. A result of analysis can be sent to the sender, or made available through accessing an Internet site.

If the method of the invention is practiced for example in a medical institution such as a hospital, a sample (e.g., blood, extracted organ, stool, or the like) is obtained from a subject (e.g., a patient, a subject at risk of a disease or the like), and the sample is treated to purify an RNA (e.g., microRNA) of interest to identify modification information of the RNA of interest. A condition (e.g., possibility of development or recurrence or cancer, possibility of acquiring resistance to a specific drug therapy, or the like) of a subject can be analyzed based on RNA modification information identified in this manner. RNA modification information, once obtained, can be used in analysis of a condition of another subject, used in analysis of a condition at another time in the same subject, or accumulated in a database.

If, for example, the method of the invention is practiced in a research institution such as a pharmaceutical company, a sample obtained from a subject (e.g., tissue or organ of an experimental animal, clinical sample, cultured cell, or the like) is treated to purify an RNA (e.g., microRNA) of interest to identify modification information of the RNA of interest. RNA modification information identified in this manner can be accumulated while being associated with a condition of the same subject found by another analysis (e.g., condition of cancer, condition of having acquired drug resistance, condition of a drug attaining a therapeutic effect, or the like). A drug that can be suitably applied to a condition of a subject (patient, a subject at risk of a disease, or the like) can be determined based on RNA modification information obtained in this manner.

### (Program)

In one aspect, the present invention provides a program for implementing a method of analyzing a condition of a subject based on RNA modification information on a computer. A method implemented by a program comprises: (a) comparing modification information on at least one type of RNA in a subject with reference modification information of the RNA; and (b) determining the condition of the subject based on an output result of the comparing step. In one embodiment, reference modification information comprises modification information on the RNA in a subject that is different from the subject. In one embodiment, reference modification information comprises modification information on the RNA in the subject obtained at another time from the modification information.

In one aspect, the present invention provides a recording medium for storing a program for implementing a method of analyzing a condition of a subject based on modification information of an RNA on a computer. The method executed by the program stored in the recording medium comprises: (a) comparing modification information on at least one type of RNA in a subject with reference modification information of the RNA; and (b) determining the condition of the subject based on an output result of the comparing step. In one embodiment, reference modification information comprises modification information on the RNA in a subject that is different from the subject. In one embodiment, reference modification information comprises modification information on the RNA in the subject obtained at another time from the modification information.

### (System)

In one aspect, the present invention provides a system for analyzing a condition of a subject based on RNA modification information. The system comprises: (a) a measurement unit for measuring an RNA; (b) calculation unit for calculating a modification condition on an RNA based on a result of the measurement; and (c) an analysis unit for analyzing a condition of the subject based on the modification condition. In one embodiment, the system further comprises a sample treatment unit for treating a sample to purify an RNA of interest.

A measurement unit can have any configuration, as long as the unit has a function and arrangement for providing RNA modification information. The unit can be provided as the same or different structure as the calculation unit or analysis unit. In one embodiment, the measurement unit is a mass spectrometer (e.g., MALDI-MS). In one enablement, a measurement unit is a sequencer.

A calculation unit identifies a modification condition (e.g., modification location, amount of modification, or the like) on an RNA based on measurement data.

An analysis unit analyzes a condition of a subject based on obtained RNA modification information. In one embodiment, analysis can be performed by referencing the additional information described above.

The configuration of the system of the invention is described while referring to the functional block diagram in Figure **34****.** While this diagram shows a case materializing the invention in a single system, it is understood that a case materializing the invention with a plurality of systems is also encompassed within the scope of the invention. A method materialized with this system can be described as a program. Such a program can be recorded on a recording medium and materialized as a method.

The system **1000** of the invention is constituted by connecting a RAM **1003,** a ROM, SSD, or HDD or a magnetic disk, an external storage device **1005** such as flash memory such as a USB memory, and an input/output interface (I/F) **1025** to a CPU **1001** built into a computer system via a system bus **1020.** An input device **1009** such as a keyboard or a mouse, an output device **1007** such as a display, and a communication device **1011** such as a modem are each connected to the input/output I/F **1025.** The external storage device **1005** comprises an information database storing section **1030** and a program storing section **1040,** which are both constant storage areas secured within the external storage apparatus **1005.**

In such a hardware configuration, various instructions (commands) are inputted via the input device **1009** or commands are received via the communication I/F, communication device **1011,** or the like to call up, deploy, and execute a software program installed on the storage device **1005** on the RAM **1003** by the CPU **1001** to achieve the function of the invention in cooperation with an OS (operating system). Of course, the present invention can be implemented with a mechanism other than such a cooperating setup.

In the implementation of the present invention, RNA modification data, when obtained by measuring (e.g., mass spectrometry and/or sequencing) an RNA sample or information equivalent thereto (e.g., data obtained by simulation) can be inputted via the input device **1009,** inputted via the communication I/F, communication device **1011,** or the like, or stored in the database storing section **1030.** The step of obtaining RNA modification data by measuring (e.g., mass spectrometry and/or sequencing) the RNA sample and analyzing the RNA modification data can be executed with a program stored in the program storing section **1040,** or a software program installed in the external storage device **1005** by inputting various instructions (commands) via the input device **1009** or by receiving commands via the communication I/F, communication device **1011,** or the like. As such software for performing analysis, software shown in the Examples can be used, but software is not limited thereto. Any software known in the art can be used. Analyzed data can be outputted through the output device **1007** or stored in the external storage device **1005** such as the information database storing section **1030.**

The data or calculation result or information obtained via the communication device **1011** or the like is written and updated immediately in the database storing section **1030.** Information attributed to samples subjected to accumulation can be managed with an ID defined in each master table by managing information such as each of the sequences in each input sequence set and each RNA information ID of a reference database in each master table.

The above calculation result can be associated with various information such as other nucleic acid information obtained from the same sample or known information such as biological information and stored in the database storing section **1030.** Such association can be performed directly to data available through a network (Internet, Intranet, or the like) or as a link to the network.

A computer program stored in the program storing section **1040** is a constituent of a computer as the above processing system, e.g., a system for performing data provision, modification condition analysis, comparison with reference data, classification, clustering, or other processes. Each of these functions is an independent computer program, a module thereof, or a routine, which is executed by the CPU **1001** to use a computer as each system or device.

### (Reagent)

In one embodiment, the present invention provides a composition for purifying an RNA whose modification is associated with a condition to determine a condition of a subject based on RNA modification information, comprising means for capturing at least one type of RNA in the subject. In one embodiment, the capturing means comprises a nucleic acid that is at least partially complementary to an RNA of interest. In one embodiment, capturing means comprises means for capturing a modified RNA (e.g., modification specific antibody or the like). In one embodiment, capturing means comprises a molecule specific to a modified RNA of interest. In one embodiment, capturing means comprises a portion for purification (e.g., a carrier that can be magnetic or one side of a pair that can bind to each other (e.g., biotin and streptavidin)). In one embodiment, capturing means comprises a linker linked to a portion for purification.

### (Plate, chip)

In one embodiment, the present invention provides a plate or chip for determining a condition of a subject based on modification information of at least one type of RNA, wherein means for capturing the RNA is placed on a surface of the plate or chip. In one embodiment, the plate or chip is for MALDI measurement. In one embodiment, the plate or chip has a plurality of spots, and means for capturing RNAs having sequences that are different from one another are placed in each spot. In one embodiment, the size of each spot can be, for example, a diameter of 10 µm to 100 µm. In one embodiment, 1, 2, 3, 3, 4, 5, 10, 15, 20, 25, 30, 35, 40, 45, 50, 75, 100, 150, 200, 300, 400, 500, 750, 1000, 1500, 2000, 2500, or 3000 spots can be formed on a single plate or chip. In one embodiment, 10⁷ or more capturing means can be placed at each spot. In one embodiment, capturing means is a nucleic acid that is at least partially complementary to an RNA of interest. Examples of the substrate of a plate or chip include, but are not limited to, conductivity imparted glass and plastic (polyethylene), and the like.

If different sports are close to each other, when a laser is irradiated to a spot to ionize an RNA contained in the spot by MALDI or the like, a laser can be irradiated onto another spot around the spot of interest. This can result in interference by a signal originating from a nearby spot in fragmented measurement by mass spectrometry and can affect the measurement value at a spot of interest. For this reason, in one embodiment, spots on a plate or chip can be placed to reduce or minimize the effect of interference between signals. In one embodiment, the effect of interference between signals is evaluated based on the overlap and/or difference between values of m/z resulting from fragmentation (can be based on a result of actual measurement or theory) of each RNA placed at each spot. At this time, an increase/decrease in the mass number due to the presence of a modification may or may not be taken into consideration in the value of m/z resulting from fragmentation of each RNA. In one embodiment, spots on a plate or chip are placed based on a mathematical or statistical methodology such as the Monte Carlo method.

### (Kit)

In one embodiment, the present invention provides a kit for determining a condition of a subject based on RNA modification information, comprising at least one of a composition for purifying an RNA of interest, a plate or chip on which means for capturing the RNA of interest is placed and a device for obtaining a sample for the subject, and descriptions for using the kit. In one embodiment, a kit comprises means for purifying an RNA from a sample.

In one embodiment, a kit is for treating a sample to be compatible with MALDI measurement. In one embodiment, a kit further comprises a coating agent (e.g., including 3-hydroxypicolinic acid) for use in MALDI measurement.

In one embodiment, a kit is for obtaining a sample from a subject. In one embodiment, a kit comprising a device for obtaining a sample from a subject comprises descriptions describing where a sample is to be sent. In one embodiment, a kit comprises means for cryopreserving a harvested sample. In one embodiment, a kit comprises a device for obtaining from a subject blood, epidermis of the mucous membrane (e.g., in the oral cavity, nasal cavity, ear cavity, vagina, or the like), epidermis of the skin, biological secretion (e.g., saliva, nasal mucus, sweat, tear, urine, bile, or the like), stool, or epidermal microorganism.

### (General technology)

The molecular biological approaches, biochemical approaches, and microbiological approaches used herein are well known or conventional in the art, which are described for example in Current Protocols in Molecular Biology (http://onlinelibrary.wiley.com/book/10.1002/0471142727) and Molecular Cloning: A Laboratory Manual (Fourth Edition) (http://www.molecularcloning.com). The relevant portions (can be the entire document) thereof are incorporated herein by reference.

As used herein, "or" is used when "at least one" of the elements listed in the sentence can be used. When explicitly described herein as "within a range" of "two values", the two values themselves are included in the range.

Reference literatures such as scientific literatures, patents, and patent applications cited herein are incorporated herein by reference to the same extent that the entirety of each document is specifically described.

As described above, the present invention has been described while showing preferred embodiments to facilitate understanding. While the present invention is described hereinafter based on Examples, the above descriptions and the following Examples are not provided to limit the present invention, but for the sole purpose of exemplification. Thus, the scope of the present invention is not limited to the embodiments and Examples specifically described herein and is limited only by the scope of claims.

### [Examples]

The Examples are described hereinafter.

For reagents, the specific products described in the Examples were used. However, the reagents such as an equivalent product from another manufacturer (Sigma-Aldrich, Wako Pure Chemical, Nacalai Tesque, R & D Systems, USCN Life Science INC, or the like) may be alternatively used.

### (Abbreviations)

In addition to the abbreviations described herein, the following abbreviations are also used.

### 3-HPA (3-hydroxypicolinic acid)

### DHC (diammonium hydrogen citrate)

In the following Examples, methylation of an RNA was detected by using the following methylation on a microRNA as an example (in the table, underlines indicate methylation). Those skilled in the art understand that the same analysis can be performed for induction of other RNAs.

**[Table 3]**

| | | |
|---|---|---|
| miR-17-5p | 5' -CAAAGUGCUUACAGUGCAGGUAG -3' | **(13^{th} mA form 5')** |
| mIR-21-5p | 5' -UAGCUUAUCAGACUGAUGUUGA-3' | **(9^{th} mC from 5')** |
| miR-200c-5p | 5' - CGUCUUACCCAGCAGUGUUUGG -3' | **(13^{th} mC from 5')** |
| miR-200c-3p | 5' - UAAUACUGCCGGGUAAUGAUGGA -3' | **(9^{th} mC from 5')** |
| miR-let7a-5p | 5' - UGAGGUAGUAGGUUGUAUAGUU -3' | **(19^{th} mA from 5')** |

### *Common protocol

An example of the standard protocol used herein is shown below.

### (RNA extraction)

TRIzol (Invitrogen) was used according to the descriptions when extracting total RNA from a sample.

### (Purification of an RNA of interest)

For purification of a specific RNA, an oligo DNA that is complementary to each RNA was used.

Oligo DNAs that are complementary to each human miRNA in the following table were designed herein.

**[Table 4]**

| Sequence of target RNA | | |
|---|---|---|
| **Name** | **Sequence** | **Molecular weight (Dalton)** |
| **Capture** 17-5p | CAAAGUGCUUACAGUGCAGGUAG **(SEQ ID NO: 1)** | 7631.49 |
| **Capture** 21-5p | UAGCUUAUCAGACUGAUGUUGA **(SEQ ID NO: 2)** | 7225.19 |
| **Capture** 200c-5p | CGUCUUACCCAGCAGUGUUUGG **(SEQ ID NO: 3)** | 7192.14 |
| **Capture** 200c-3p | UAAUACUGCCGGGUAAUGAUGGA **(SEQ ID NO: 4)** | 7632.48 |
| **Capture** let7a-5p | UGAGGUAGUAGGUUGUAUAGUU **(SEQ ID NO: 5)** | 7322.24 |

| | | |
|---|---|---|
| *The left side is the 5' end, and the right side is the 3' end. | | |

DNAs complementary to these target RNAs (capture oligo DNAs) were synthesized.

**[Table 5]**

| Sequence of oligo DNA capturing a target RNA | |
|---|---|
| **Name** | **Sequence** |
| **Capture** 17-5p | CTACCTGCACTGTAAGCACTTTG **(SEQ ID NO: 6)** |
| **Capture** 21-5p | TCAACATCAGTCTGATAAGCTA **(SEQ ID NO: 7)** |
| **Capture** 200c-5p | CCAAACACTGCTGGGTAAGACG **(SEQ ID NO: 8)** |
| **Capture** 200c-3p | TCCATCATTACCCGGCAGTATTA **(SEQ ID NO: 9)** |
| **Capture** let7a-5p | AACTATACAACCTACTACCTCA **(SEQ ID NO: 10)** |

It was confirmed that the possibility of these capture oligo DNAs hybridizing with an RNA other than the RNA of interest is low by collating with a known sequence database.

### (Directly binding beads)

Directly binding beads were prepared as follows. A 6-aminohecyl group was introduced into a phosphoric acid moiety at the 5' end of the capture oligo DNAs described above. Each modified capture oligo DNA was linked to a magnetic bead (Dynabeads M270 Amine, Thermo Fisher Scientific, Tokyo) with an amino group covalently bound to a surface by a divalent amino crosslinker (BS3, bis(sulfosuccinimidyl)suberate).

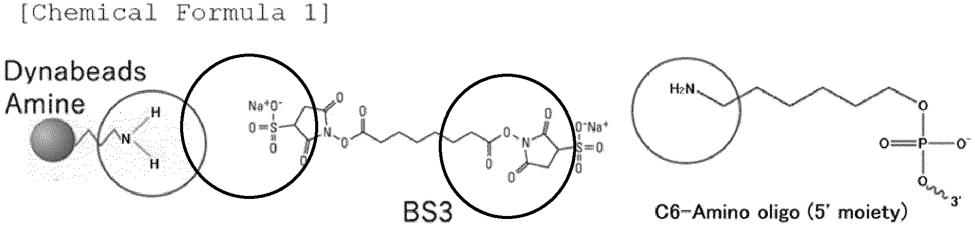

### (Streptavidin binding beads)

Another type of beads was also prepared. Biotin was introduced into a phosphoric acid moiety at the 5' end of the capture oligo DNAs described above. Magnetic beads (Dynabeads M270 Streptavidin, Thermo Fisher Scientific, Tokyo) with streptavidin covalently bound to the surface were mixed with the biotinylated capture oligo DNAs described above to generate an avidin-biotin bond and immobilize the capture oligo DNA on the magnetic beads.

A protocol for purification with magnetic beads after hybridizing a biotinylated capture oligo DNA with an RNA of interest was also used.

### (Purification protocol for directly binding beads)

This protocol is a modified method of J. Engberg et al., Eur. J. Biochem, 41, 321-328 (1974).

When a plurality of types of capture oligo DNAs were used, a sample was divided, and one type of capture oligo DNA was used for each divided sample.

Specifically, the protocol is as follows.
1. One of the capture oligo DNA binding beads described above and 10 mL of 6x SSPE (0.9M NaCl, 60 mM NaH2PO4, 7.5 mM EDTA, pH 7.4) were added to the sample.
2. 5 mL of denaturation solution D (4M guanidine thiocyanate, 25 mM citric acid (pH 7.0), 0.5% sarcosine, and 0.1 M 2-mercaptoethanol) was added (the final concentration of guanidine thiocyanate was 1.25 M).
3. After heating for 10 minutes at 60°C, the mixture was swirled for 45 minutes at room temperature.
4. The supernatant was removed using a magnet stand, and the beads were washed 4 times with 1 mL of BW Buffer (10mM Tris-HCl, pH 7.5, 1 mM EDTA, 2.0 M NaCl) and twice with 1 mL of Volatile Rinse Buffer (10 mM ammonium acetate, pH 7.0).
5. After complete removal of the washing solution, 100 µL of RNase free SDW was added to the beads. The mixture was heated for 3 minutes at 70°C and then rapidly cooled on ice to collect the supernatant.
6. When the concentration was low, the supernatant was lyophilized.

### (Purification protocol for streptavidin binding beads)

When a plurality of types of capture oligo DNAs were used, a sample was divided, and one type of capture oligo DNA was used for each divided sample.

1. Saline and the biotinylated capture oligo DNA described above were added to the purified RNA for phosphate buffer with a final concentration of 10 mM (pH 7.0) and 50 mM KCl.
2. The mixture was placed in a PCR apparatus, denatured for 1 minute at 90°C, gradually cooled to 45°C and annealed.
3. Avidin magnetic beads (Dynabeads M-280 Streptavidin) were added.
4. The unadsorbed fraction (supernatant) on the magnet stand was discarded.
5. The beads were washed three times on the magnet stand using 10 mM phosphate buffer (pH 7.0) and 50 mM KCl.
6. The beads were washed three times on the magnet stand using 10 mM ammonium acetate (pH 7.0), which was then eluted using RNase free water.
7. When the concentration was low, the supernatant was lyophilized.

### (Dimethylsulfate treatment)

A reaction was performed for dimethyl sulfate treatment in accordance with the following procedure. Purified RNAs were each individually dissolved in 10 mM phosphate buffer (pH 7.5) to reach 100 µM. An ethanol solution of 15% dimethyl sulfate prepared before use was added at a final concentration of 0.5% for 1 minute of treatment at 25°C. β mercaptoethanol was added to arrive at a final concentration of 2%, and the mixture was sufficiently stirred to stop the reaction.

### (Chloroacetaldehyde treatment)

A reaction was performed for chloroacetaldehyde treatment in accordance with the following procedure. Purified RNAs were each individually dissolved in 10 mM potassium phosphate (pH 5) to reach 100 µM. Bromoacetaldehyde or chloroacetaldehyde was added so that the final concentration would be 1% and treated for 2 hours at 37°C. Excessive bromoacetaldehyde or chloroacetaldehyde was removed by diethyl ether extraction, and then the aqueous layer was dried to obtain a residue.

### (Mass spectrometry measurement of purified RNA)

An RNA sample was purified using Zip Tip C18 (Millipore) as needed.

3-HPA (3-hydroxypicolinic acid) was added to a 1:1 solution of acetonitrile:aqueous 0.1% TFA solution so that the concentration would be 10 mg/mL. 1 µL of a mixture prepared by mixing this solution and an aqueous 10 mg/mL DHC (diammonium hydrogen citrate) solution at 1:1 was applied to a target plate (Target Plate MTP Anchor Chip 384 (600 micrometer), Bruker Dalotnics) as a matrix (coating agent) for MALDI and dried. 1 µL of purified aqueous RNA solution was laminated and dried at the same location. After confirming complete drying, mass spectrometry was performed with a MALDI mass spectrometer (ultrafleXtreme-TOF/TOF mass spectrometer, Bruker Dalotnics).

The setting of the MALDI apparatus was as follows.

### positive-mode (positive ion detection mode)

### reflector-mode (reflector mode)

### Laser Power Max (maximum possible output setting)

### (Analysis of measurement results from a mass spectrometer)

The measurement results obtained through MALDI were analyzed as follows.

A list of expected masses was created based on sequence information for microRNAs obtained from miRBase (Release 21) (http://www.mirbase.org) and compared to the mass spectrogram obtained by measurement to manually identify a sequence and modification.

### (RIP sequencing measurement)

RIP sequencing was performed in accordance with the following protocol.

This is a protocol for when three 15 cm dishes of subconfluent cells are used as one sample. 500 µg to 700 µg of RNA can be recovered from three 15 cm dishes using 2 ml of Trizol (Invitrogen) for each 15 cm dish. RNA can also be recovered using 2 ml of Trizol (Invitrogen) for 1 ml of serum.

Reagents and the like that were used

**[Table 6]**

| Product name | Manufacturer | Catalog number | Remarks |
|---|---|---|---|
| Oligotex™ -dT30<Super> mRNA Purification Kit | Takara Bio | 9086 | 1 kit is for 10 samples |
| anti-m6A rabbit polyclonal antibody | Synaptic Systems | 202 003 | Dissolved with 100 µL of RNAse free water and stocked at 0.5 mg/ml (1 product is for 10 samples) |
| Dynabeads® Protein G for Immunoprecipitation | Thermo Fisher scientific | 100040 | (1 product is for 10 samples) |
| N6-Methyladenosine 5-monophosphate sodium salt 10mg | Sigma-aldrich | M2780-10MG | 10 mg is dissolved with 1305 µL of RNAse free water as 20 mM stock (1 product is for 20 samples) |
| Ribonucleoside vanadyl complexes (RVC) | Sigma-aldrich | R3380-5ML | Undiluted solution is used |
| RNasin® Plus RNase Inhibitor 2500u | Promega | N2611 | Undiluted solution is used |
| IGEPAL® CA-630 50ml | Sigma-aldrich | 18896-50ml | |
| Glycogen solution (20mg/ml) | Nacalai Tesque | 17110-11 | Used at 200-fold |
| Zinc chloride 25 mg | Wako | 268-01902 | 1363 mg is dissolved with 10 mL of RNAse free water as 1M stock |

### (A) Purification of ployA RNA

ployA was purified in accordance with the protocol of an Oligotex® (Takara Bio) kit.
*Extraction was performed three times with 30 µL of 75°C DW, and a total of 90 µL was recovered.
*1 to 3% of polyA RNA is obtained from total RNA.
*5 µg of polyA RNA is required for each sample.

### (B) RNA fragmentation

**[Table 7]**

| 10X Fragmentation Buffer | | |
|---|---|---|
| Tris-HCl (1M) (pH 7. 4) | 10 *µ*L | (Final concentration Tris-Hcl 100mM) |
| ZnCl₂ (1M **stock**) | 10 µL | (Final concentration Zncl₂ 100mM) |
| RNAse-free water | 80 *µ*L | |
| **Total** | 100 *µ*L | |

1. RNA was adjusted to 750 ng/18 µL.
2. 750 ng/18 µL of RNA + 2 µL of 10x Fragmentation Buffer for a total of 20 µL were dispensed into each well of an 8-strip tubes.
3. The mixture was incubated for 2 minutes at 90°C.
4. 2 µL of EDTA (0.5 M) was quickly added to 20 µL of the reaction solution, and the reaction was stopped.
5. A reaction solution was collected for each sample and precipitated with ethanol.
   Example: 200 µL of RNA
   + 20 µL (1/10 of the amount of RNA) of 3M sodium acetate (pH 5.2)
   + 3.6 µL (200-fold) of glycogen (20 mg/ml stock)
   + 500 µL of 100% ethanol (2.5-fold of RNA)
6. The product was incubated for 1 hour at -80°C.
7. Centrifugation (12000 G, 30 minutes, 4°C)

The supernatant was removed, and 1 ml of 75% ethanol was added.

Centrifugation (12000 G, 15 minutes, 4°C)

The supernatant was removed, and the remainder was dissolved with 200 µL of RNAse free water. 8. 10 µL of each sample was preserved at -80°C as INPUT.

### (C) Formation of RNA antibody complex

**[Table 8]**

| 5X IP buffer | | |
|---|---|---|
| Tris-HCl (1M) (pH7.4) | 0.5 mL | (Final concentration 50 mM Tris-HCl) |
| Nacl (5M) | 1.5 ml | (Final concentration 750 mM NaCl) |
| 10% Igepal CA-630 | 0.5 ml | (Final concentration 0.5% (vol/vol) |
| RNAse free water | 7.5 ml | |
| Total | 10ml | |
| Fragmented RNA (from step (B)) | | 190 *µ*L |
| 5X IP Buffer | | 50 *µ*L |
| RNAse inhibitor | | 1 *µ*L |
| RVC | | 2.5 *µ*L |
| m6A antibody (0.5mg/ml) | | 10 *µ*L |
| Total | | 250 *µ*L (per sample) |

The above were mixed and incubated for 2 hours at 4°C while being rotated.

### (D) Immunoprecipitation with magnetic beads

1. 50 µL of Dynabeads Protein G (Thermo Fisher Scientific) was used for each sample and washed twice with 1 ml of 1x IP Buffer.
2. 1 ml of 1x IP Buffer + 10 µL of RVC + 1 µL of RNAse inhibitor + 50 µL BSA (10 mg/ml) were added to beads separated with magnetism. The beads were incubated for 2 hours at 4°C while being rotated (suppression of nonspecific binding of beads).
3. The beads were washed twice with 1 ml of 1x IP Buffer + 10 µL of RVC + 1 µL of RNAse inhibitor.
4. This was separated for each sample, and the reaction solution of step (C) was added to the beads separated by magnetism, and the mixture was incubated for 2 hours (or overnight) at 4°C while being rotated.

### (E) Elution

1. The beads in step (D) were washed three times with a washing buffer (10 ml of 1x IP Buffer + 10 µL of RVC + 5 µL of RNAse inhibitor).

**[Table 9]**

| Elution buffer | | |
|---|---|---|
| m6A salt ^{(20 mM stock)} | 33.3 *µ*L | (Final concentration 6.7mM) |
| 5X IP buffer | 20 *µ*L | |
| RNAse inhibitor | 1 *µ*L | |
| RNAse free water | 50 *µ*L | |
| Total | 100 µL | |

2. 100 µL of Elution buffer was added for each sample, and the solution was incubated for 2 hours at 4°C while being vortexed every 15 minutes.
3. The beads were magnetically separated, and the supernatant was recovered.
4. 100 µL of (1 ml of 1x IP Buffer + 10 µL of RVC + 1 µL of RNAse inhibitor) were further added to the beads. After tapping and magnetic separation of the beads, the supernatant was recovered.
5. Steps 5 to 7 described above were repeated to recover the supernatant. Supernatant from two runs was combined.
6. 400 µL of supernatant + 40 µL of sodium acetate + 1000 µL of 100% ethanol were mixed, which was precipitated with ethanol (carrier such as glycogen was not added).
7. The precipitate was incubated overnight at -80°C.
8. Centrifugation (12000 G, 30 minutes, 4°C)
The supernatant was removed, and 1 ml of 75% ethanol was added.
Centrifugation (12000 G, 15 minutes, 4°C)
9. Pellets were dissolved with 15 µL of RNAse free water. The pellets were subjected to sequencing with INPUT of 8 in step (B).

Sequencing was performed using Hi-seq (2000 Ilumina).

The protocol for analyzing data obtained by RIP sequencing is shown below (Linux/R).

### (From fastq file to peak detection)

SRA Toolkit, bowtie, samtools, and macs were installed, and annotation files for hg19 were downloaded (see DRY Kaiseki Kyohon [DRY analysis tutorial] (Shujunsha) or the like).

Quality check was performed on the data, and data for IP and INPUT were summarized. For example, this is the following if there are immunoprecipitation samples (IP1, IP2, and IP3: 3 replicates) and corresponding INPUT (INPUT1, INPUT2, and INPUT3).
[Numeral 1]
fastqc --nogroup *.fastq
IP1.fastq IP2.fastq IP3.fastq> IP.fastq
INPUT1.fastq INPUT2.fastq INPUT3.fastq > Input.fastq

A fastq file was mapped with bowtie, a sam file was created, and a bam file was created from the sam file.
[Numeral 2]
bowtie -m 1 -p 4 --sam ∼/chip/hg19-q IP.fastq IP.sam
bowtie -m 1 -p 4 --sam ∼/chip/hg19-q Input.fastq Input.sam
samtools view -S -b IP.sam >IP.bam
samtools view -S -b Input.sam >Input.bam

The data was sorted in the order of chromosomes and indexed, and peaks were detected with macs.
[Numeral 3]
samtools sort IP.bam IP_sorted
samtools index IP_sorted.bam
samtools sort IP.bam Input_sorted
samtools index Input_sorted.bam
macs14 -t IP_sorted.bam -c Input_sorted.bam --name=IP_normalized -f BAM -g hs -S
--wig >macs.out

### (Analysis of peak with R)

The following packages were used.
(Guitar)
(MeTPeak)
(openxlsx)
(ChIPpeakAnno)
(BSgenome.Hsapiens.UCSC.hg19)
(TxDb.Hsapiens.UCSC.hg19.knownGene)
(rGADEM)
(ChIPseeker)

### (1. metagene plot from output Excel file of macs)

Only the transcriptome information was retrieved from hg19, and stored in gc_txdb. The Excel file was saved after deleting the top portion that could not be read and converting xls to xlsx.
[Numeral 4]
library(Guitar)
txdb <- makeTxDbFromUCSC(genome="hg19")
gc_txdb <- makeGuitarCoordsFromTxDb(txdb, noBins=100)
library(openxlsx)
IP<-read.xlsx("m6A_IP_peaks.xlsx")

Those with a change in factor of 4 or greater, and FDR of 5% or less were extracted as peaks, and the files were changed to Granges files.
[Numeral 5]
IP.sig<-IP[IP[,9]<5 & IP[,8]>4,]
IP.sig.gr <- GRanges(seqnames=Rle(IP.sig$chr), ranges = IRanges(IP.sig$start, end=IP.sig$end), strand = Rle(strand(c(rep("*", length(IP.sig$chr))))), Cone = IPsig$tags)
IP.gr <- GRanges(seqnames=Rle(IP$chr), ranges = IRanges(IP$start, end=IP$end), strand = Rle(strand(c(rep("*", length(IP$chr))))), Cone = IP$tags)

The Granges files were connected to make a list, and each element of the list was named.
[Numeral 6]
GRs <- list(IP.gr,IP.sig.gr)
names(GRs) <- c("IP","IP.sig")
GuitarPlot(GRs, GuitarCoordsFromTxDb = gc_txdb)

### (2. Motif search)

50 bases before and after the summit for a total of 100 bases were extracted and subjected to a motif search. 1000 were selected from those with lowest FDR.
[Numeral 7]
library("ChIPpeakAnno")
library("BSgenome. Hsapiens. UCSC. hg19")
IP<-read.x)sx("m6A_IP_peaks.xlsx") summit<-IP$start+IP$summit-1
tmp<-cbind(IP$chr,summit-50,summit+49,IP[,c(6:9)])
tmp<-head(tmp[order(tmp[,7]),],1000)

The file was changed to a Granges file.
[Numeral 8]
IP.summit.gr <- GRanges(seqnames=Rle(tmp[,1]), ranges = IRanges(start=tmp[,2], end=tmp[,3]), strand = Rle(strand(c(rep("*', length(tmp[,1]))))), Cone = tmp$tags)

The sequence was obtained with a ChIPpeakAnno package.
[Numeral 9]
IP.peaksWithSeqs <- getAllPeakSequence(IP.summit.gr, upstream = 0, downstream = 0, genome = Hsapiens)
write2FASTA(IP.peaksWithSeqs, file = "iP.fa")
library("rGADEM")
seqs <- readDNAStringSet("IP.fa", "fasta")
motif <- GADEM(seqs, verbose = 1, genome = Hsapiens) plot(motif)

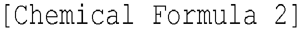

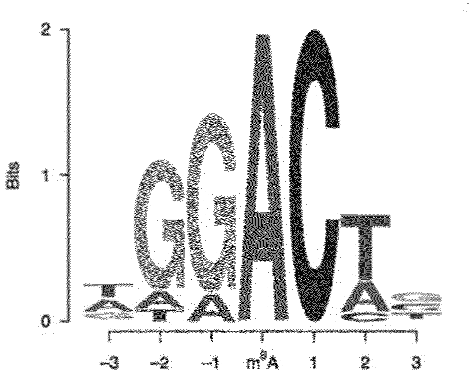

### (3. Obtain an annotation of a gene with a peak)

[Numeral 10]
require(TxDb.Hsapiens.UCSC.hg19.knownGene)
txdb <- TxDb.Hsapiens.UCSC.hg19.knownGene
library(ChIPseeker)
peak<-IP.summit.gr
AP<-annotatePeak(peak, tssRegion = c(-3000, 3000), TxDb = txdb,
level = "transcript", assignGenomicAnnotation = TRUE,
genomicAnnotationPriority = c("Exon","5UTR", "3UTR", "Intron","Promoter", "Downstream", "Intergenic"),
annoDb = NULL,
addFlankGeneInfo = FALSE,
flankDistance = 5000,
sameStrand = FALSE,
ignoreOverlap = FALSE,
ignoreUpstream = FALSE,
ignoreDownstream = FALSE, overlap = "all",
verbose = F)

### (4. Analysis with MetPeak (analysis package specializing in RIP sequencing))

### (See Cui et al., Bioinformatics (2016) 32 (12): i378-i385)

[Numeral 11]
library(MeTPeak)
IP_BAM<-c("IP1_sorted.bam","IP2_sorted.bam")
INPUT_BAM<-c("INPUT1_sorted.bam"."INPUT2_sorted.bam")
metpeak(GENOME="hg19",IP_BAM = IP_BAM,INPUT_BAM = INPUT_BAM, EXPERIMENT_NAME="metpeak")

### (Example 1) Analysis on methylation of microRNAs

This Example performed an analysis on methylation of microRNAs. The specifics thereof are the following.

A microRNA 200-c-5p (human sequence, SEQ ID NO: 11) synthesized to comprise methylated adenine and methylated cytosine was dissolved in ultrapure RNase Free water. The concentration was determined by measurement of absorbance and adjusted to 1 pmol/µL. Mass spectrometry was performed with a MALDI mass spectrometer in accordance with the protocol described above by using 1 µL of aqueous microRNA solution. To find the internal sequence, RNA was decomposed (5' → 3') by ammonium treatment. Measurement was performed using in source decay (ISD) on observed precursor ions (Figure 1).

The same number of moles of a synthetic oligo DNA (complementary strand of human 369-3p, SEQ ID NO: 12) having a sequence that is complementary to microRNA 369-3p and an antisense synthetic DNA thereof (SEQ ID NO: 13) were mixed and heated, and then gradually cooled to anneal the DNAs to form a DNA double strand, and the concentration was adjusted to 1 pmol/µL. Each of them was also independently prepared in the same manner. In the same manner described above, mass spectrometry was performed, precursor ions of the strand of each DNA were observed, the overall weight was determined, and ISD was performed. It was confirmed that each strand can also be identified and sequenced in a double stranded state in the same manner as for each DNA strand independently (Figure **2**).

The same analysis was performed on a doubled strand formed from synthetic microRNA 369-3p (human, SEQ ID NO: 14). It was confirmed that strands can be sequenced from an RNA double strand (Figure **3**).

The following experiment was conducted to confirm that an RNA in the body can also be sequenced by mass spectrometry. A small molecule RNA fraction was obtained from HEK293 cultured cells by using TRIzol (Invitrogen). The obtained RNA fraction was dissolved in ultrapure RNase Free water. After determining the concentration by measurement of absorbance, the concentration was adjusted to 100 pmol/µL. When mass spectrometry was performed and precursor ions were observed to identify a parental ion with a mass number corresponding to miRNA 369-3p (human), and ISD was applied to the precursor ions to study the internal sequence in the same manner, this was confirmed to be miRNA 369-3p (Figure **4**). In this manner, a specific RNA can be observed without purifying a specific sequence.

It was confirmed in this manner that an RNA modification can be analyzed by combining a suitable mass spectrometry method and RNA purification.

### (Example 2) Analysis of cancer utilizing RNA modification

This Example demonstrated that cancer can be detected or diagnosed by analyzing the effect on an RNA modification and using the RNA modification.

### (Example 2-1: RNA modification analysis in a cell strain)

Human pancreatic cancer cell strains BxPC-3, Panc 10.5, PSN-1, and Capan-2 were obtained from the American Type Culture Collection (Manassas, VA, USA). When methylated miRNA analysis was performed with RIP-Seq using an anti-m6A antibody for these four strains, 63 types shown in the following table were found as methylated miRNAs that were shared by the four pancreatic cancer cell strains.

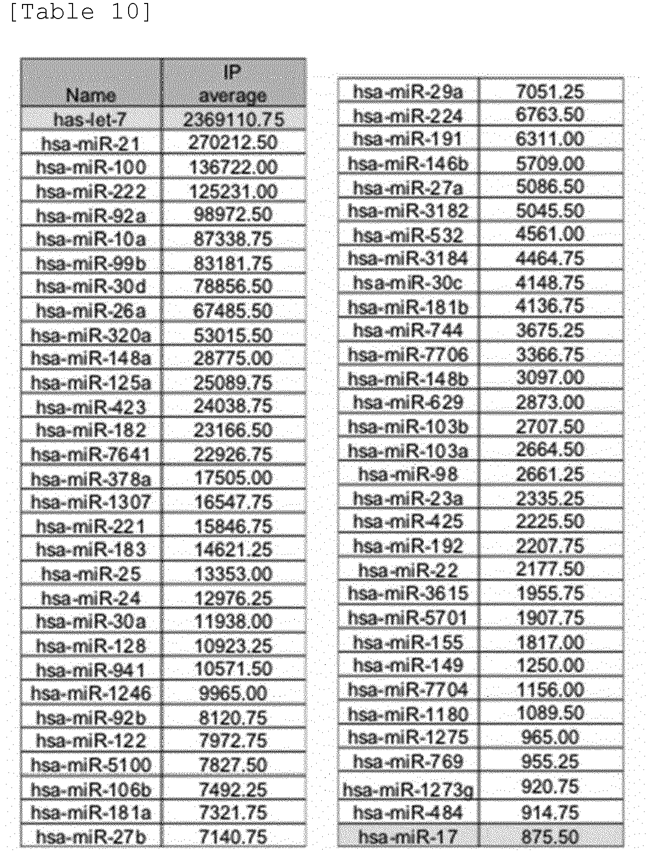

These methylations of miRNAs can be useful in determining cancer (e.g., pancreatic cancer).

### (Example 2-2: Colon cancer)

Tissue specimens of colon cancer (three patients) of only stage 2 to 4 primary lesions were harvested during surgery from human patients who provided an informed consent in advance. At the same time, tissues specimens were harvested from a region that was 5 cm or more apart from a malignant tumor region as healthy tissue.

The capture 17-5p, capture 21-5p, capture 200c-3p, capture 200c-5p, and capture let7a-5p described above were prepared as streptavidin binding beads. Total RNA was produced with Trizol from the tissue specimen samples described above. A target miRNA was then purified using the beads and measured with an ultrafleXtreme-TOF/TOF mass spectrometer (Bruker Dalotnics).

As a result, unmethylated RNAs as well as RNAs with methylation at a location indicated in the table were observed in these miRNAs.

**[Table 11]**

| miRNA name | Sequence | Discovered methylation location |
|---|---|---|
| miR-17-5p | 5'-CAAAGUGCUUACAGUGCAGGUAG -3' | 13^{th} mA from 5' (SEQ ID NO: 15) |
| miR-21-5p | 5'-UAGCUUAUCAGACUGAUGUUGA-3' | 9^{th} mC from 5' (SEQ ID NO: 16) |
| miR-200c-5p | 5'- CGUCUUACCCAGCAGUGUUUGG -3' | 13^{th} mC from 5' (SEQ ID NO: 17) |
| miR-200c-3p | 5'-UAAUACUGCCGGGUAAUGAUGGA -3' | 9^{th} mC from 5' (SEQ ID NO: 18) |
| miR-let7a-5p | 5'-UGAGGUAGUAGGUUGUAUAGUU -3' | 19^{th} mA from 5' (SEQ ID NO: 19) |

MS signals that are the basis of methylation of each of them were compared between normal tissue and tumor tissue (Figures **5** to **7**). It was found as a result thereof that there is a difference in the methylation condition of RNAs between normal tissue and tumor tissue. The following table shows the result of calculating the ratio (%) of methylated RNAs in each RNA of a target sequence.

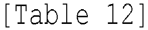

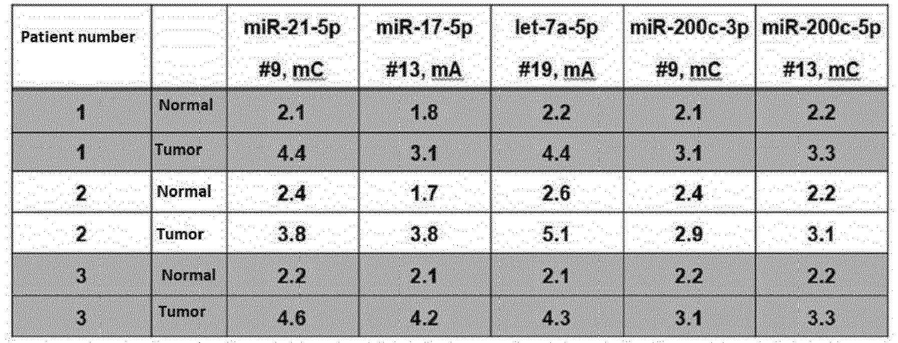

RNA expression levels were analyzed by qRT-PCR. qRT-PCR was performed as follows. TaqMan microRNA reverse transcription kit and TaqMan microRNA assays (Applied Biosystem) were used in accordance with the product protocols. THUNDERBIRD SYBR® qPCR Mix (Toyobo) was used as a PCR master mix. LightCycler® system (Roche) was used as the apparatus for qRT-PCR.

The expression levels of each of the miRNAs described above were compared between normal tissue and tumor tissue by qRT-PCR. The results of the aforementioned methylation ratio (MS) and expression level were compared for each miRNA (Figure **8**).

As a result, a difference in the expression levels of these miRNAs was hardly observed between normal tissue and tumor tissue, whereas the difference in methylation ratio (MS) was significant. This shows that the methylation ratio (MS) of an RNA can be a more useful marker than the expression level (RT-PCR).

To check the internal sequence, an RNA was degraded (5' → 3') by ammonium treatment and mass spectrometry was performed for miR-200c-5p (Figure **9**). As shown in Figure **9**, it was confirmed that mass spectrometry can accurately determine a modification at a specific location of a specific RNA.

A sample was obtained from a human colon cancer patient. The methylation ratio on 4 types of miRNAs was studied. These patients were diagnosed with colon cancer and underwent primary tumor resection surgery and then found to have metastasis within 1 to 2 years thereafter. Metastasis was found in the liver or lymph node. Before is a serum sample obtained from a patient before primary tumor resection. After is a serum sample obtained when metastasis was found within 1 to 2 years after the primary resection.

The methylation levels were analyzed by mass spectrometry according to the common protocol for these Before and After samples and samples of serum derived from inflammatory bowel disease (IBD) and Crohn's disease (Crohn) patients (Figure **10**).

When a cancer cell was not present in the body (After, IBD, and Crohn), methylation was lower compared to cases where cancer cells were present (Before). A high level of methylation of these miRNAs can be an indicator of cancer (e.g., colon cancer).

### (Example 2-3: Pancreatic cancer)

### Detection of methylation in the body

Methylated miRNAs were identified by RIP-Seq using an anti-m6A antibody according to the common protocol in pancreatic cancer tissue. Let-7a and miR-17 among the miRNAs having methylation detected were further analyzed.

In a pancreatic cancer tissue sample, an miRNA of interest was concentrated with capture beads for Let-7a and miR-17 described above, and methylation was detected by MALDI-TOF-MS/MS (Figure **11**). As a result, the location of methylation was determined. Quantification of the amount of methylation was also possible.

Next, the methylation levels in pancreatic cancer tissue or pancreatic cancer patient serum were compared to the methylation levels in various control samples (normal tissue, normal subject, subject after removing cancer by surgery) for let-17a, miR-17, miR-21, and miR-200c. A difference in miRNA expression levels was not detected in quantitative reverse transcription PCR. A miRNA of interest was concentrated with capture beads for each miRNA, and methylation was detected by MALDI-TOF-MS/MS (Figures **12** to 15). The methylation level and methylation location of let-17a, miR-17, miR-21, and miR-200c were able to be detected in pancreatic cancer tissue.

The methylation levels of these miRNAs in a pancreatic cancer sample were elevated compared to each control sample of normal tissue, normal subject, and subject after removing cancer by surgery (Figures **16** and **17**).

A high level of methylation of these miRNAs can be an indicator of cancer (e.g., pancreatic cancer).

### (Example 2-3A: Early stage pancreatic cancer)

This Example studied the RNA modification condition using a serum sample harvested from 17 human pancreatic cancer patients. The pancreatic cancer was early stage pancreatic cancer diagnosed as stage I to II (four patients as stage IA, 5 patients as stage IIA, and 7 patients as stage IIB). A serum sample of a healthy individual was used as a normal sample.

Directly binding beads of capture 17-5p, capture 21-5p, capture 200c-3p, capture 200c-5p, and capture let7a-5p described above were used. Total RNA was purified with Trizol from the aforementioned serum sample. A target miRNA was then purified using the beads and measured with an ultrafleXtreme-TOF/TOF mass spectrometer (Bruker Dalotnics).

While methylation of miR-17-5p was detected in all pancreatic cancer serum samples, such methylation was not detected or the degree of methylation was low in a normal sample.

It was demonstrated that the degree of methylation of miR-17 can be a more accurate indicator in the detection of pancreatic cancer compared to the determination results using existing pancreatic cancer markers CA19-9 and CEA.

80% or more mRNAs have modification of m6A, and a large volume of information on RNA modifications is listed in Modomics (http://modomics.genesilico.pl/). In addition, according to highly comprehensive RIP sequencing developed by the inventors, it is likely that at least half, or if the frequency is not considered, all adenines that bind to a methylase are methylated/demethylated. The inventors studied the methylation condition for 50 types of microRNAs considered important in digestive organ cancer to reveal that 4 types of microRNAs are important at the early stage of digestive organ cancer including pancreatic cancer. It is understood that RNA modification information similarly reflects the condition of other diseases.

In this manner, this Example revealed that there is a significant difference in early stage pancreatic cancer patients compared to healthy individuals.

### (Example 2-5: Other cancer samples)

This Example analyzed other cancer samples.
Methylation was analyzed in the scope of RNAs of Examples 2-3 for various cancer samples using the same approach as Examples 2-2. Tissue specimens of gastric cancer (four patients) were harvested during surgery from human patients who provided an informed consent in advance. At the same time, tissues specimens were harvested from a region that was 5 cm or more apart from a malignant tumor region as healthy tissue.

The capture 17-5p, capture 21-5p, capture 200c-3p, capture 200c-5p, and capture let7a-5p described above were prepared as streptavidin binding beads. Total RNA was produced with Trizol from the samples described above. A target miRNA was then purified using beads and measured with an ultrafleXtreme-TOF/TOF mass spectrometer (Bruker Dalotnics).

The results in gastric cancer are shown (Figure 18). In the same manner as Example 2-2, it is demonstrated that the methylation ratio (MS) of a microRNA can also be a useful marker for diagnosis of gastric cancer.

The results in colorectal cancer patients are shown (Figure **19**). The following are each of the patients. Characterization of colorectal cancer patients

**[Table 13]**

| Sample number | Location | Tumor depth | Lymph node metastasis | Distant metastasis | Pathological stage* |
|---|---|---|---|---|---|
| No.1 | Rectum | T2 | NO | - | I |
| No.2 | Rectum | T2 | NO | - | I |
| No.3 | Colon | T2 | NO | - | I |
| No.4 | Rectum | T2 | NO | - | I |
| No.5 | Colon | T1 | NO | - | I |
| No.6 | Colon | T2 | NO | - | I |
| No.7 | Rectum | T3 | N3 | Liver metastasis | IV |
| No.8 | Colon | T3 | N2 | Liver metastasis | IV |
| No.9 | Rectum | T3 | N1 | Liver metastasis | IV |
| No.10 | Rectum | T3 | NA | Liver metastasis | IV |
| No.11 | Colon | T4a | NA | Liver metastasis | IV |
| No.12 | Rectum | NA | NA | Liver metastasis | IV |

| | | | | | |
|---|---|---|---|---|---|
| *Tumor-node-metastasis (TNM) was categorized according to the 7^{th} edition of TNM staging of the Union for International Cancer Control (https://www.nccn.org/professionals/physician_gls/f_guideli nes.asp). | | | | | |

The methylation of miRNAs was compared between normal colorectal tissue and colorectal cancer tissue in a colorectal cancer patient. It was found that methylation is elevated in early stage colon cancer (Stage I) and advanced colon cancer (Stage IV) compared to a normal site.

In this manner, the degree of progression of cancer (e.g., colon cancer) can be predicted by observing a modification of an RNA.

The same experiment was conducted for liver cancer and gallbladder cancer from human patients who provided an informed consent in advance. The same result was able to be obtained when conducted after obtaining a sufficient number for statistical analysis.

### (Example 2-7)

### Analysis of CTC (circulating tumor cells)

Methylation of all microRNAs was analyzed for CTC samples by using the same approach as Example 2-3.

### (Example 3) Analysis of resistant strain using RNA modification

This Example demonstrated that a resistant strain can be analyzed using an RNA modification.

### (Example 3-1: Preparation of resistant strain (Chm^{R} cell))

This Example prepared a resistant strain (Chm^{R} cell).

The cancer cell strain DLD-1 obtained from a cell bank such as ATCC or RIKEN was subjected to maintenance culture for 6 months or more in the presence of trifluridine (FTD) (Aldrich-Sigma) (about 10 mg/mL). The maintenance culture was subcultured about twice a week to maintain 60 to 80% confluence at 37°C in a serum 10% added DMEM medium on a plastic dish. Measurement of the IC₅₀ for trifluridine for the cultured cells resulted in IC₅₀ = 300 µmol/L, confirming that a trifluridine resistant strain was established.

Likewise, 5-fluorouracil (5-FU), gemcitabine, cisplatin, nucleic acid drug, histone demethylase inhibitor, cetuximab (antibody drug), Carbon/HIMAC (heavy particle beam), and X ray resistant strains were prepared from cancer cell strains HCT116, RKO, and the like obtained from a cell back such as ATCC or RIKEN. The drugs described above were purchased from Aldrich-Sigma and used at about 10 mg/mL. Resistant strains were established by culturing for 6 months or longer. Each drug resulted in IC₅₀ = 300 µmol/L, confirming that each resistant strain was established. As the X-ray source, linear Gammacell® 40 Exactor (Best Theratronics Ltd.) was used for treatment at a radiation dose of about 0 to 10 Gy. As the Carbon/HIMAC beam source, National Institute of Radiological Sciences' HIMAC was used for treatment at a radiation dose of about 0 to 10 Gy (Katsutoshi Sato et al., Cancer Sci. 2017 Oct; 108(10): 2004-2010 and Katsutoshi Sato et al., Sci Rep. 2018; 8: 1458).

### (Example 3-2: Analysis of RNA modification of resistant strain)

This Example analyzed the RNA modification of a resistant strain.

For an original DLD-1 cell strain and each resistant strain (5-FU resistant strain and FTD resistant strain), RNA methylation analysis in accordance with the protocol for RIP sequencing described above and microarray or mRNA expression analysis similar to the qRT-PCR of Example 2-2 was performed.

As a result of measurement, methylation was observed on a total of 1024 types of microRNAs. Representative examples are shown in the following table.

**[Table 14]**

| | 5-FU parental strain | 5-FU resistant strain | FTD parental strain | FTD resistant strain | Rate of change, 5-FU resistance (fold) | Rate of change, FTD resistance (fold) |
|---|---|---|---|---|---|---|
| **miR-378a** | 21309 | 10525 | 13746 | 12466 | 2.44 | 0.48 |
| miR-378d | 144 | 30 | 80 | 63 | 1.54 | 0.36 |
| miR-378e | 11 | 2 | 3 | 6 | 1.64 | 0.86 |
| miR-378f | 3 | 1 | 2 | 1 | 0.67 | **(0.50)** |
| miR-378h | 1 | 0 | 0 | 0 | 0.00 | - |
| miR-378i | 13 | 1 | 6 | 3 | 0.50 | 0.33 |
| | | | | | | |
| miR-492 | 0 | 0 | 0 | 0 | - | - |
| **miR-3690** | 0 | 0 | 1 | 0 | - | 0.00 |
| miR-4754 | 3 | 4 | 4 | 0 | (1.33) | 0.00 |
| miR-6861 | 1 | 0 | 1 | 0 | 0.00 | 0.00 |
| | | | | | | |
| miR-3122 | 0 | 2 | 0 | 0 | ∞ | - |
| **miR-3131** | 9 | 3 | 1 | 10 | **(0.33)** | **(10.00)** |
| miR-6847 | 7 | 1 | 7 | 3 | 0.07 | **(0.43)** |
| **miR-6887** | 0 | 0 | 0 | 0 | - | - |

Methylation of miRNAs in the following table is particularly noteworthy.

**[Table 15]**

| | | |
|---|---|---|
| miR378a-3p | ACUGGACUUGGAGUCAGAAGGC | (SEQ ID NO: 20) |
| miR378d | ACUGGACUUGGAGUCAGAAA | (SEQ ID NO: 21) |
| miR378e | ACUGGACUUGGAGUCAGGA | (SEQ ID NO: 22) |
| miR378f | ACUGGACUUGGAGCCAGAAG | (SEQ ID NO: 23) |
| miR378h | ACUGGACUUGGUGUCAGAUGG | (SEQ ID NO: 24) |
| miR378i | ACUGGACUAGGAGUCAGAAGG | (SEQ ID NO: 25) |
| miR492 | AGGACCUGCGGGACAAGAUUCUU | (SEQ ID NO: 26) |
| miR3690 | ACCUGGACCCAGCGUAGACAAAG | (SEQ ID NO: 27) |
| miR4754 | AUGCGGACCUGGGUUAGCGGAGU | (SEQ ID NO: 28) |
| miR6861-5p | ACUGGGUAGGUGGGGCUCCAGG | (SEQ ID NO: 29) |
| miR3122 | GUUGGGACAAGAGGACGGUCUU | (SEQ ID NO: 30) |
| miR3131 | UCGAGGACUGGUGGAAGGGCCUU | (SEQ ID NO: 31) |
| miR6847-3p | GGCUCAUGUGUCUGUCCUCUUC | (SEQ ID NO: 32) |
| miR6887-3p | UCCCCUCCACUUUCCUCCUAG | (SEQ ID NO: 33) |
| miR-6887-5p | UGGGGGGACAGAUGGAGAGGACA | (SEQ ID NO: 34) |

For individual microRNAs, methylation of for example miR-378a decreased in a trifluridine resistant strain, but increased in a 5-FU resistant strain. Such modification information on an RNA in a resistant strain can be used, for example, to predict whether the same drug can be continuously used without imparting resistance when the drug is administered to a patient (for example, predicting the possibility of resistance to 5-FU when methylation of miR-378a increases after administration of 5-FU).

RIP sequencing and RNA expression information based primary component analysis (two dimensional projection using a component resulting in maximum dispersion) was performed. The results are represented as mean ± standard deviation (SD) based on three independent experiments. The statistical significance of the results was evaluated by pairwise T-TEST using R version 3.4.3 (2017-11-30) on Microsoft Excel® software (Microsoft Campus, Redmond, WA, USA). P value < 0.05 was considered statistically significant. As a result, wild-type strain and each resistant strain was clearly distinguished. It was also suggested that an RNA modification condition represents a characteristic of a resistant strain more closely than an RNA expression condition (Figure **20**).

Next, based on methylated sites and peripheral sequences thereof, motif analysis was performed to study the correlation with methylation associated enzymes (following table). The results suggest that some microRNAs correlate strongly with Mettl3, Mettl14, and YTHDF1. Based on results of such motif analysis, the correlation between RNA modification and other biological agents can be revealed to perform network analysis. This can also be utilized in deductive analysis of a condition of an organism.

**[Table 16]**

| **• miR comprising YTHDF1 motif** | | | |
|---|---|---|---|
| **miR-378a** | miR-378e | | miR-378i |
| miR-378b | miR-378f | | |
| miR-378d | miR-378h | | |

| **• miR comprising METTL3 motif** | | | |
|---|---|---|---|
| miR-492 | | miR-4754 | |
| miR-611 | | miR-6861 | |
| **miR-3690(candidate for CHOL)** | | | |

| **• miR comprising METTL14 motif** | | | |
|---|---|---|---|
| miR-3122 | | miR-6847 | |
| **miR-3131** | | **miR-6837(candidate for PAAD)** | |

The result of the analysis suggests that methylation on an miRNA shown below can be suitably used especially as a biomarker for evaluating FTD resistance.

**[Table 17]**

| miRNA | Ensemble ID | Parental strain_ Expression | Parental strain_ Methylation | Resistant strain_ Expression | Resistant strain_ Methylation | Change in expression | change in methylation |
|---|---|---|---|---|---|---|---|
| miR-3937 | ENSG00000263730 | 0 | 2 | 33 | 182 | ∞ | 5.52 |
| miR-4488 | ENSG00000266006 | 0 | 3 | 9 | 175 | ∞ | 19.44 |
| miR-3662 | ENSG00000283409 | 0 | 1 | 26 | 53 | ∞ | 2.04 |
| miR-3141 | ENSG00000264760 | 2 | 0 | 2 | 19 | 1 | ∞ |
| niR-6734-5p | ENSG00000283836 | 3 | 1 | 3 | 23 | 1 | 23 |
| miR-1262 | ENSG00000221203 | 15 | 50 | 20 | 272 | 1.33 | 4.08 |

### (Example 4) Analysis of effect of treatment by RNA modification

This Example analyzed the effect of treatment such as surgery, therapy, or prevention on an RNA modification, and analyzed the effect of treatment such as surgery, therapy, or prevention by analyzing an RNA modification.

(Example 4-1: Analysis of the effect of surgery using an RNA modification)

This Example studied the effect of surgery on an RNA modification.

Total RNA was purified in accordance with the protocol for Trizol (Invitrogen) from the original DLD-1 cell strain and each resistant strain (5-FU resistant strain and FTD resistant strain), and then RNA methylation analysis was performed according to the protocol for RIP sequencing described above. Ribo-Zero rRNA Removal Kit (Illumina) was used for the removal of rNRAs.

miRNA expression information was obtained from The Cancer Genome Atlas (TCGA) (https://cancergenome.nih.gov/), and those with expression levels decreasing to 1/2 or less post-surgery were selected as follows.

**[Table 18]**

| | | |
|---|---|---|
| miR-16-1-3p | miR-34b-5p | miR369-5p |
| miR-431-5p | miR-494-3p | miR-519-d-5p |
| miR-3181 | miR-4435 | miR-4467 |
| miR-5581-5p | miR-5587-5p | |

For the miRNAs that decrease post-surgery, the relationship with miRNA methylation information observed to change by drug resistance is shown in the following table.

**[Table 19]**

| | 5-FU parental strain | 5-FU resistant strain | FTD parental strain | FTD resistant strain | Rate of change 5-FU (fold) | Rate of change, FTD (fold) |
|---|---|---|---|---|---|---|
| **miR-4435** | **28** | **14** | **46** | **100** | **2.38** | **1.41** |
| **miR-4467** | **6** | **6** | **1** | **12** | **(1.00)** | **8.00** |

For miRNAs that decrease post-surgery, it was observed that the methylation condition thereof can change significantly with drug resistance.

When it was studied whether a common methylation motif RRAC is found on these miRNAs that decrease post-surgery, the possibility of methylation was found at the following sites.

**[Table 20]**

| | | |
|---|---|---|
| miR-16-1-3p | CCAGUAUUAACUGUGCUGCUGA | **(SEQ ID NO: 35)** |
| miR-34b-5p | UAGGCAGUGUCAUUAGCUGAUUG | **(SEQ ID NO: 36)** |
| miR369-5p | AGAUCGACCGUGUUAUAUUCGC | **(SEQ ID NO: 37)** |
| miR-431-5p | UGUCUUGCAGGCCGUCAUGCAG | **(SEQ ID NO: 38)** |
| miR-494-3p | UGAAACAUACACGGGAAACCUC | **(SEQ ID NO: 39)** |
| miR-519-d-5p | CCUCCAAAGGGAAGCGCUUUCUGUU | **(SEQ ID NO: 40)** |
| miR-3181 | AUCGGGCCCUCGGCGCCGG | **(SEQ ID NO: 41)** |
| miR-4435 | AUGGCCAGAGCUCACACAGAGG | **(SEQ ID NO: 42)** |
| miR-4467 | UGGCGGCGGUAGUUAUGGGCUU | **(SEQ ID NO: 43)** |
| miR-5581-5p | AGCCUUCCAGGAGAAAUGGAGA | **(SEQ ID NO: 44)** |
| miR-5587-5p | AUGGUCACCUCCGGGACU | **(SEQ ID NO: 45)** |

| | | |
|---|---|---|
| *The table shows, as an underline, adenine in a sequence that is a single base mismatch from an RRAC motif and the motif. | | |

The expression data for miRNAs in pancreatic adenocarcinoma (PAAD, n = 4, Figure **21**), rectal adenocarcinoma (READ, n = 3, Figure **22**), cholangiocarcinoma (CHOL, n = 9, Figure **23**), and colon adenocarcinoma (COAD, n = 8, Figure **24**) was obtained from The Cancer Genome Atlas (TCGA) (https://cancergenome.nih.gov/), and the expression of miRNAs decreasing post-surgery among them was compared between normal and cancerous sites (same patient).

In this manner, the relationship of cancer, surgery, and drug resistance was able to be analyzed based on RNA modification information. Such analysis is expected to be able to determine the resection range of surgery, post-surgery drug therapy, intensity of radiation therapy (regimen), and post-hospitalization follow up from the viewpoint of a risk of recurrence, based on RNA modification information. It is expected that a patient requiring treatment can be selected based on RNA modification information.

The above results suggest that especially miR-3131, miR-3690, miR-6887-5p, miR-6887-3p, miR-378a-3p, miR-4435, and miR-4467 can be suitably used as a methylated miRNA.

### (Example 4-2: Analysis of other treatments)

This Example analyzes the effect of other treatments on an RNA modification and analyzes whether other treatments can be analyzed in the same manner. For example, temperature, hypoxia (e.g., 1%, +19%: nitrogen substitution), malnutrition (glucose, glutamine, or amino acid deficiency), temperature (range of 32 to 42°C), and the like can be analyzed.

It is possible to examine how an RNA modification changes before and after treatment with 5-FU, gemcitabine, cisplatin, nucleic acid drug described above, histone demethylase inhibitor described above, cetuximab (antibody drug), Carbon/HIMAC (heavy particle beam), and X ray.

### (Example 5: Effect due to RNA modification)

### (Example 5-1: Effect of RNA methylation on RNA-protein interaction)

The effect of RNA methylation on RNA-protein interaction was studied.

### Molecular dynamics simulation

To predict binding of methylated and unmethylated miRNAs (miR-200c, let-17a, and miR-17) to a human AGO2 protein, the X-ray structure of an AGO2/RNA complex was used as a guide (PDB ID: 4OLB²⁵ and 4W5N²⁶). First, an RNA binding base was replaced with a corresponding base in each miRNA. Molecular dynamics simulation was performed on each miRNA complex structure under the condition of 1 atmospheric pressure and about 37°C. After thermodynamic sampling, energy was minimized to predict structure docking. All calculations were performed using the Amber 12 program (http://ambermd.org/). The results are shown in Figures **25** to **27****.**

In Figure **25****,** methylated cytosine at position 9 was near an RNA recognition base in miR-200c. While there was no significant difference in the first 6 nucleotides of methylated and unmethylated miR-200c in an AGO complex, a change in the binding interaction between AGO and miRNA was observed in the vicinity of a methylated site. This resulted in decreased space around the methylated site. A methyl group of cytosine at position 9 likely obstructed a hydrogen bond of AGO with Ser220 due to steric hindrance, thus inducing a shift in the location of guanine at position 8. It is understood that this was induced by an interaction of AGO with Arg761.

In Figures **26** and **27****,** methylated adenine was located away from an RNA binding site in miR-17 and let-7a. However, adenine methylation induced a significant structural change to the entire complex, including the periphery of an RNA recognition site. This can affect the target RNA recognition efficiency. These findings show that the ability to suppress target mRNA translation of an miRNA can be diminished by an m6A modification.

### (Example 5-2: Effect of RNA methylation on organisms)

The actual effect of RNA methylation on organisms was studied.

### Synthetic oligonucleotide transfection

Synthesis of methylated and unmethylated double stranded RNA oligonucleotides (miR-200c, let-7a) was commissioned to GeneDesign (Osaka, Japan). The sequences of these synthetic RNAs were studied by MALDI-TOF-MS/MS. The miRNA sequences were obtained from miRBASE (release 21; http://www.mirbase.org/). Methylated or unmethylated synthetic miRNA was transfected into DICER exon 5 disrupted colon cancer cell strain HCT116 (HCT116^{EX5}) (provided by Bert Vogelstein (Johns Hopkins University, Baltimore, MS, USA)) with very low expression levels of endogenous miRNAs using Lipofectamine 3000 (Invitrogen) according to the manufacturer's protocol.

### Expression data analysis

A gene wherein an mRNA reported in Tarbase14 (http://diana.imis.athena-innovation.gr/DianaTools/index.php?r=tarbase/index) binds to miR-200c or let-7a was used a target gene. The expression levels of a target gene observed by a microarray were compared for miR-200c (Figure **28**). For let-7a, reads per kilobase of exons per a million reads were similarly analyzed while estimating from the sequence (Figure **29**).

It was found from gene expression profiling that unmodified miR-200c and m5C modified miR-200c exhibit a potent gene suppression effect, but the gene expression suppression effect of m6A modified miR-200c is weak, and m6A modified let-7a does not reduce target mRNA expression compared to unmethylated let-7a.

In this manner, a modification condition of an RNA was confirmed to affect the condition of an organism. This demonstrated that a modification condition of an RNA reflects the condition of an organism, and the condition of the organism is predicted by studying the RNA modification condition.

### (Example 6: Gene knockdown analysis using RNA modification)

This Example analyzed whether gene knockdown analysis can be performed using an RNA modification.

### (Example 6-1) Effect of Mettl3 knockdown on RNA modification

This Example analyzed whether gene knockdown analysis can be performed using an RNA modification. Human pancreatic adenocarcinoma cell strain MIA PaCa-2 cells were treated by a standard method using shRNA or siRNA to prepare a Mettl3 knockdown cell strain (see Kosuke Taketo et al., Int J Oncol. 2018 Feb; 52(2): 621-629). RIP sequencing was subsequently performed.

### (Example 6-2) Effect of overexpression of Mettl3

A mouse Mettl3 gene was incorporated into a CAG expression vector. This was infused into a fertilized egg of BL6 mice and transplanted into the uterus of a foster parent to prepare Mettl3 gene overexpressing mice. A vector was prepared by connecting a viral protein SV downstream of a mouse pancreatic enzyme gene. This was infused into a fertilized egg of BL6 mice and transplanted into the uterus of a foster parent to prepare EL1-SV40 mice with inactivated P53 and RB of the pancreas. A Mettl3 gene overexpressing mouse and an EL1-SV40 mouse prepared in this manner were combined with a common method to prepare a double transgenic mouse. It was then confirmed by PCR and the like that a gene of interest is incorporated into the double transgenic mouse. Mice were raised under an SPF environment in a normal animal experiment facility.

Tumors naturally develop in these mice. Survival was observed for the double transgenic mice (10 mice) and EL1-SV40 mice (10 mice) (Figure **30**). Pictures (Figure **31**) show tumor extracted from each mouse at 20 week old (left: EL1-SV40 mouse, right: double transgenic mouse). Tumor growth was faster and the survival rate was lower in the double transgenic mice.

Mettl3 is an RNA methylase. These results revealed that 1) an RNA modification (epitranscriptome) serves an important role in the development/progression of cancer, and 2) a change in an RNA modification has a greater effect on cancer than a change in a gene (P53, RB) that were considered to have a significant contribution to cancer in the past. As shown in the above Examples, an RNA modification is not limited to only a modification of an mRNA, but an miRNA is also modified by the same enzyme. Thus, the epitranscriptome in cells at a deep portion of the body can be monitored by monitoring an miRNA (e.g., in the blood). For this reason, this result not only supports the importance of an RNA modification as a biomarker, but also shows the importance of a microRNA as a biomarker for a liquid biopsy.

### (Example 7) Relationship between disease and RNA modification

This Example analyzes RNA modifications in samples of cancer, dementia, heart failure, inflammatory bowel disease, senescence, and intestinal tract immunity. It is shown as a result thereof that RNA modification information is useful in predicting these condition.

For these analyses, the following can be performed:
(1) a mouse that is an Alzheimer's dementia model by an oxidative phosphorylation reaction in the brain by genetic engineering of the key enzyme for metabolism, protein kinase M (PKM)
(2) a mouse generated by interaction with immunity as a result of senescence or inflammation in digestive organs including the digestive tract in a model using manipulation of a cancer suppressing gene of a digestive organ is used to study methylation of a ribosomal RNA or the like from microflora in a stool,
(3) study on a stool sample of a mouse with an altered gene associated with cancer metabolism or the like.

### (Example 8) Agent screening by RNA modification

This Example demonstrates that agent screening can be performed using an RNA modification.

A cell strain is treated with any compound library. An RNA modification of these cells is analyzed. If these compounds are classified based on RNA modification information, some of the compounds form a cluster and can be analyzed. This can be applied to screening of a compound library by referring to the concept for stains resistant to each agent in the above Examples. For example, the descriptions in Example 4 can be referenced as appropriate. In addition, a biopsy can be used for determination of the degree of malignancy, and diagnosis of cancer from cytodiagnosis, determination of the degree of malignancy, diagnosis or therapy of cancer with unknown primary lesion, or search for a novel target agent can be performed.

### (Example 9) E. coli RNA modification analysis

This Example demonstrates whether E. coli can be classified using RNA modification analysis.

For example, an RNA modification is analyzed for a certain E. coli strain. RIP sequencing was performed. The genetic information of agent resistant pump P-glycoprotein can also be concurrently used.

It is found that microorganism species can be very readily classified by utilizing RNA modification information.

Further, an RNA modification can be analyzed for a stool of a mouse.

As a result, the identity of the presented E. coli species can be analyzed, and can suggest the relationship between the condition of the mouse and the condition of E. coli.

An RNA modification can also be analyzed for information on microorganisms such as E. coli contained in food.

As a result thereof, microorganism species (e.g., E. coli species) in food can be analyzed and suggest the relationship between the quality of food and condition of E. coli.

### (Example 10) RNA modification analysis for food

This Example shows an example of analyzing food using RNA modification information.

For example, it is possible to demonstrate that quality (e.g., days from the manufacturing date) of food (e.g., processed meat) can be classified based on RNA modification information.

### (Example 11-1) Analysis of various modifications on an RNA

This Example demonstrates an analysis using other RNA modifications.

This Example demonstrates that types of modifications of RNA-mod span a wide range. Mass spectrometry data can be reanalyzed to analyze modifications other than methylation on a microRNA. Various modifications on an RNA can be analyzed using such information.

### (Example 11-2) Analysis of modification on various RNAs

Modification information of an RNA other than microRNAs can be linked to data associated with some type of condition. For example, mass spectrometry has a large number of peaks, each one of which can be found manually or by machine learning. Bruker's Maldi specification can be referenced for them.

### (Example 12) Analysis combining RNA modification information with other data

This Example demonstrates an analysis combining RNA modification information with other data.

For example, transomics (RNA; e.g., Pagliarini DJ Cell Metab. 2016 Jul 12; 24(1): 13-4. doi: 10.1016/j.cmet.2016.06.018.), methylome (methylation binding protein; e.g., Shabalin AA., Bioinformatics. 2018 Feb 12. doi: 10.1093/bioinformatics/bty069.), transcriptome (RNAseq; e.g., Jeong H, Front Neurosci. 2018 Feb 2; 12:31. doi: 10.3389/fnins.2018.00031. eCollection 2018), epitranscritome of the invention (low density or high density), metabolome (mass spectrometry; e.g., Gupta R et al., Proteomics. 2018 Feb 19. doi: 10.1002/pmic.201700366), or the like can be referenced. These articles are merely exemplification. Other appropriate information sources can also be used and applied.

It is understood that the RNA modification information of the invention can be combined with other information to further improve the precision of analysis as a result thereof or in such a manner.

For example, if "information on miRNAs that decrease post-surgery" in Example 4-1 is referred to in combination with information other than that on microRNA, the usefulness of such information other than that on microRNA increases by combining with "information on miRNAs that decrease post-surgery".

For example, when analyzed using a dataset (GDS4103) of Gene Expression Omnibus (GEO), it can be understood that an increase in the RNA expression levels of RNA methylases METTL3 and METTL14 is observed in pancreatic ductal adenocarcinoma (PDAC) patients. For example, an analysis associating methylase with detected methylated RNA can be performed in combination with such information.

### (Example 13) High density array design

This Example demonstrates analysis using an example of a design for a methylated RNA chip.

In MALDI, laser irradiation results in rapid heating of matrix molecules and gasification/ionization of a plurality of RNAs with a partially fractured phosphate bond. On a methylated RNA chip, multiple different RNA capture nucleic acids are placed on a single plate for efficiency. When laser is irradiated onto a well where a capture nucleic acid of interest is placed, the laser is also irradiated onto a surround well, so that an RNA that is different from an RNA of interest is detected in some case. For this reason, it is desirable to optimize the placement of capture nucleic acids on a plate so that the mass peak originating from an RNA captured in a surrounding well is distinguished from a mass peak observed from a well of interest. The placement of a capture nucleic acid is determined according to the following procedure.
1. Calculate "theoretical mass of an observed partial RNA" for each RNA
2. Place a capture probe randomly on a plate using the Monte Carlo method
3. Calculate the difference in theoretical masses of observed partially RNAs between adjacent wells
4. (After the second or more repeats)
   If the difference in the theoretical masses is greater than the previous difference: adopt the current placement If less: reject the current placement
5. Return to 2 and repeat the Monte Carlo sampling.

The placement of a capture nucleic acid obtained after repeating the above procedure for a certain number of times or more is considered the optimal placement expected to have the minimum measuring error (the difference in masses of partial RNAs of captured RNAs between adjacent wells is the greatest).

Among the combinations of capture nucleic acids that can capture a plurality of types of RNAs, examples of combinations understood to have no issue when adjacent are shown below.

**[Table 21]**

| (Capture nucleic acid probe number 911, types of captured RNA = 4) Consensus sequence 1: CGUAA | |
|---|---|
| **Captured RNA** | **Molecule weight** |
| GUUCUCCCAACGUAAGCCCAGC, | 7157.19 |
| UAGCAGCACGUAAAUAUUGGCG. | 7286.30 |
| GGGACCCAGGGAGAGACGUAAG. | 7442.46 |
| | 7451.32 |

| (Capture nucleic acid probe number 27323, types of captured RNA = 5) Consensus sequence 2: GGGAGGUGUG | |
|---|---|
| **Captured RNA** | **Molecule weight** |
| GGGGAGGUGUGCAGGGCUGG. | 6834.02 |
| GGGAGGUGUGAUCUCACACUCG. | 7294.27 |
| CUGGGAGGUGUGAUAUCGUGGU. | 7352.28 |
| CUGGGAGGUGUGAUAUUGUGGU. | 7353.27 |
| UGGGGAGGUGUGGAGUCAGCAU. | 7414.36 |

| (Capture nucleic acid probe number 27896, types of captured RNA = 3) Consensus sequence 3: CUCCCUGCCC | |
|---|---|
| **Captured RNA** | **Molecule weight** |
| UCCCCUUOCUCCCUGCCCAG, | 6371.64 |
| CCUCCCUGCCOSCCUCUCUGCAG, | 7367.24 |
| AGCCGCUCUUCUCCCUGCCCACA, | 7375.27 |

| (Capture nucleic acid probe number 29699, types of captured RNA =4) Consensus sequence 4: GACUUGGAGUCA | |
|---|---|
| **Captured RNA** | **Molecule weight** |
| ACUGGACUUGGAGUCAGGA, | 6361.74 |
| ACUGGACUUGGAGUCAGAAA, | 6674.95 |
| ACUGGACUUGGAGUCAGAAGGC, | 7341.34 |
| ACUGGACUUGGAGUCAGAAGAGUGG, | 8361.96 |

| (Capture nucleic acid probe number 30593, types of captured RNA = 4) Consensus sequence 5: CCCUUUAGAGUGU | |
|---|---|
| **Captured RNA** | **Molecule weight** |
| AACGCACUUCCCUUUAGAGUGU, | 7160. 16 |
| AUCGUGCAUCCCUUUAGAGUGU, | 7177.15 |
| AAAAUGGUUCCCUUUAGAGUGU, | 7225.21 |
| ACAAAGUGCUUCCCUUUAGAGUGU, | 7835.57 |

### (Example 14) Optimization of purification by oligo DNA (Figure 32)

This Example shows that RNA modification analysis efficiency is improved by optimizing purification by oligo DNA.

Detection of modified RNA using the directly binding beads described above was compared with that using the streptavidin binding beads described above.

Total RNA was purified with TRIzol (Invitrogen) from cultured HeLa cells and human skin fibroblasts. Capture oligo DNA was prepared as the directly binding beads and streptavidin binding beads described above. A target miRNA was purified according to the protocol for each described above from purified total RNA, and measured with an ultrafleXtreme-TOF/TOF mass spectrometer (Bruker Dalotnics). For the capture oligo DNA, the capture 17-5p, capture 21-5p, capture 200c, and capture let7a-5p described above were used.

As a result thereof, signals with intensities in the following table were obtained.

**[Table 22]**

| miRNAs | | HeLa cells | Human skin fibroblasts |
|---|---|---|---|
| 17 | Streptavidin binding beads | 17544 | 13142 |
| | Directly binding beads | 35252 | 30332 |
| | Improvement in signal intensity (%) | 201 | 231 |
| 21 | Streptavidin binding beads | 15432 | 9646 |
| | Directly binding beads | 29541 | 23546 |
| | Improvement in signal intensity (%) | 191 | 244 |
| 200c | Streptavidin binding beads | 13535 | 10574 |
| | Directly binding beads | 25432 | 25035 |
| | Improvement in signal intensity (%) | 188 | 237 |
| 7a | Streptavidin binding beads | 15413 | 15413 |
| | Directly binding beads | 30142 | 24352 |
| | Improvement in signal intensity (%) | 196 | 158 |

An MS signal intensity of RNA that is about 2-fold compared to that for streptavidin binding beads was obtained for directly binding beads.

Since MALDI ionizes a sample on a target plate with laser, the presence of an impurity that ionizes on a target plate besides a subject of measurement and matrix can result in laser energy being wasted on ionization of the impurity and reduced signal intensity. For this reason, suppression of impurities can be effective.

Since directly binding beads have a more stable bond with an oligo DNA than streptavidin binding beads, the beads can be washed and eluted at a higher temperature after hybridization. This suggests that high sensitization is achieved by suppressing impurities that nonspecifically bind to capture nucleic acids and magnetic beads.

### (Example 15) Exosome concentration

This Example shows that the precision of analysis of an RNA modification is improved by concentrating exosomes (Figure **33**).

A change in detection intensity of a modified RNA due to concentration of exosomes was studied.

Serum/plasma (purchased from Dojindo Laboratories) were used as samples.

The capture 17-5p, capture 21-5p, capture 200c, and capture let7a-5p described above were prepared as streptavidin binding beads. A target miRNA was purified from samples from each of the following treatment 1 and treatment 2 using these beads and then measured with an ultrafleXtreme-TOF/TOF mass spectrometer (Bruker Dalotnics).

### *Treatment 1 (no immunoprecipitation (IP) treatment)

After extracting/purifying an miRNA with TRIzol, each miRNA was purified by hybridization.

### *Treatment 2 (with IP treatment with an anti-CD63 antibody)

Each miRNA was purified by hybridization with respect to an immunoprecipitate obtained by adding an anti-CD63 antibody (Santa Cruz Biotechnology) to an exosome fraction lightly purified with ExoQuick (System Biosciences).

As a result, the signals with intensities in the following table were obtained.

**[Table 23]**

| miRNAs | | purcased | 54 male |
|---|---|---|---|
| 17 | No IP + RNA capture | 15235 | 13135 |
| | CD63 IP + RNA capture | 38243 | 30143 |
| | Improvement in signal intensity (%) | 251 | 229 |
| 21 | No IP + RNA capture | 11435 | 9743 |
| | CD63 IP + RNA capture | 29242 | 23324 |
| | Improvement in signal intensity (%) | 256 | 239 |
| 200c | No IP + RNA capture | 12439 | 10475 |
| | CD63 IP + RNA capture | 31314 | 24931 |
| | Improvement in signal intensity (%) | 252 | 238 |
| 7a | No IP + RNA capture | 13153 | 10642 |
| | CD63 IP + RNA capture | 33943 | 24753 |
| | Improvement in signal intensity (%) | 258 | 233 |

The MS signal intensity of a target miRNA obtained from the same starting material (serum/plasma) increased to about 2.5 fold by purification with immunoprecipitation using a CD63 antibody.

Since MALDI ionizes a sample on a target plate with laser, the presence of an impurity that ionizes on a target plate besides a subject of measurement and matrix can result in laser energy being wasted on ionization of the impurity and reduced signal intensity. For this reason, suppression of impurities can be effective.

The above results suggest that impurities in the starting material can be removed by sorting/concentrating only exosomes with an antibody.

Since a more intense signal can be obtained with this method, it is expected that methylation ratios can be measured more certainly relative to prior methods.

### (Example 16) Effect of freezing on RNA modification

This Example analyzed the effect of freezing a sample on the precision of analysis of an RNA modification.

Commercially available serum and human serum from blood collection were used to study the stability of a modified RNA with respect to temperature.

The RNA of interest was purified using the let7a specific capture oligo DNA described above.

The preparation of synthetic 200c was commissioned to GeneDesign.

Serum was separated from blood obtained by blood collection by using a serum separation tube.

Commercially available serum and serum from blood collection were treated under each condition in the table. let7a was then purified with capture oligo DNA binding beads and measured with an ultrafleXtreme-TOF/TOF mass spectrometer (Bruker Dalotnics).

**[Table 24]**

| Commercially aveilable serum | let7a | | |
|---|---|---|---|
| | non-methylated | methylated | % |
| Immediately after melting | 10431 | 94 | 0.90 |
| 24 hours at 4 °C after melting | 5023 | 31 | 0.62 |
| 24 hours at 25°C after melting | 4131 | ND | - |
| | | | |

| Serum | let7a | | |
|---|---|---|---|
| | non-methylated | methylated | % |
| Immediately after serum separation | 20413 | 153 | 0.75 |
| 24 hours at 4 °C after separation | 15426 | 31 | 0.20 |
| 24 hours at 25°C after separation | 5031 | ND | - |
| | | | |

| Incubation after separation | let7a | | |
|---|---|---|---|
| | non-methylated | methylated | % |
| Immediately after serum separation | 20015 | 157 | 0.78 |
| Separation after 24 hours at 4 °C after collecting blood | 9430 | 51 | 0.54 |
| Separation after 24 hours at 25°C after collecting blood | 4931 | ND | - |

Synthetic 200c was treated under each condition in the table and measured with an ultrafleXtreme-TOF/TOF mass spectrometer (Bruker Dalotnics).

**[Table 25]**

| Milli-Q/autoclaved | Synthetic 200c |
|---|---|
| | non-methylated |
| Immediately after preparation of solution | 29413 |
| 24 hours at 4 °C | 26413 |
| 24 hours at 25°C after melting | 20525 |
| | |

| **RNaseFree Water** (Commercially aveilable) | Synthetic 200c |
|---|---|
| | **non-methylated** |
| Immediately after preparation of solution | 28953 |
| 24 hours at 4 °C | 27014 |
| 24 hours at 25°C after melting | 20842 |
| | |

| Tap water | Synthetic 200c |
|---|---|
| | **non-methylated** |
| Immediately after preparation of solution | 18493 |
| 24 hours at 4 °C | 3641 |
| 24 hours at 25°C after melting | - |

After blood collection and after serum separation, the effect of incubation at 4°C or 25°C was significant. A synthetic product was stable in 4°C pure water.

EDTA or heparin was added to blood collected with a syringe. The blood was centrifuged for 5 minutes at 3000 rpm to obtain the supernatant.

Commercially available serum and serum from blood collection were treated under each condition in the table. let7a was then purified with capture oligo DNA binding beads and measured with an ultrafleXtreme-TOF/TOF mass spectrometer (Bruker Dalotnics).

**[Table 26]**

| Commercially aveilable plasma (citric acid) | let7a | | |
|---|---|---|---|
| | non-methylated | methylated | % |
| Immediately after melting | 9406 | 84 | 0.89 |
| 24 hours at 4 °C after melting | 3015 | ND | - |
| 24 hours at 25°C after melting | ND | ND | - |
| | | | |

| Fresh plasma (EDTA) | let7a | | |
|---|---|---|---|
| | non-methylated | methylated | % |
| Immediately after cetrifugation of collected blood | 19531 | 178 | 0.91 |
| 24 hours at 4 °C after separation | 19042 | 174 | 0.91 |
| 24 hours at 25°C after separation | 16423 | 121 | 0.74 |
| | | | |

| Fresh plasma (heparin) | let7a | | |
|---|---|---|---|
| | non-methylated | methylated | % |
| Immediately after cetrifugation of collected blood | 16841 | 131 | 0.78 |
| 24 hours at 4 °C after separation | 15741 | 103 | 0.65 |
| 24 hours at 25°C after separation | 11351 | 89 | 0.78 |

Synthetic 200c was treated under each condition in the table and measured with an ultrafleXtreme-TOF/TOF mass spectrometer (Bruker Dalotnics).

**[Table 27]**

| Freeze thaw | Synthetic 200c |
|---|---|
| | non-methylated |
| Immediately after preparation | 28413 |
| -80/4°C, 1 time | 28501 |
| -80/4°C, 5 times | 28015 |

It was observed that the quality of the RNA of interest decreased further by addition of heparin. It was observed that a synthetic product hardly degraded after repeating freeze thawing about 5 times.

### (Notes)

As described above, the present invention is exemplified by the use of its preferred embodiments. However, it is understood that the scope of the present invention should be interpreted solely based on the claims. It is also understood that any patent, any patent application, and any references cited herein should be incorporated herein by reference in the same manner as the contents are specifically described herein.

### [Industrial Applicability]

The present invention has the potential to be used in analysis in almost any field that is associate with organisms. The application in medical field in particular is limitless.

### [Sequence Listing Free Text]

The name of the sequence herein, and the specific sequence thereof are shown below.
miR-17-5p CAAAGUGCUUACAGUGCAGGUAG (SEQ ID NO: 1)
miR-21-5p UAGCUUAUCAGACUGAUGUUGA (SEQ ID NO: 2)
miR-200c-5p CGUCUUACCCAGCAGUGUUUGG (SEQ ID NO: 3)
miR-200c-3p UAAUACUGCCGGGUAAUGAUGGA (SEQ ID NO: 4)
miR-let7a-5p UGAGGUAGUAGGUUGUAUAGUU (SEQ ID NO: 5)
Capture 17-5p CTACCTGCACTGTAAGCACTTTG (SEQ ID NO: 6)
Capture 21-5p TCAACATCAGTCTGATAAGCTA (SEQ ID NO: 7)
Capture 200c-5p CCAAACACTGCTGGGTAAGACG (SEQ ID NO: 8)
Capture 200c-3p TCCATCATTACCCGGCAGTATTA (SEQ ID NO: 9)
Capture let7a-5p AACTATACAACCTACTACCTCA (SEQ ID NO: 10)
Synthetic miR-200c-5p CGUCUUACCCAGCAGUGUUUGG (#7, mA; #13, mC) (SEQ ID NO: 11)
Synthetic miR-369-3p AAUAAUACAUGGUUGAUCUUU (SEQ ID NO: 12)
Complementary DNA 369-3p AATAATACATGGTTGATCTTT (SEQ ID NO: 13)
Antisense complementary DNA 369-3p AAAGATCAACCATGTATTATT (SEQ ID NO: 14)
miR-21-5p UAGCUUAUCAGACUGAUGUUGA (#9, mC) (SEQ ID NO: 15)
miR-17-5p CAAAGUGCUUACAGUGCAGGUAG (#13, mA) (SEQ ID NO: 16)
let-7a-5p UGAGGUAGUAGGUUGUAUAGUU (#19, mA) (SEQ ID NO: 17)
miR-200c-3p UAAUACUGCCGGGUAAUGAUGGA (#9, mC) (SEQ ID NO: 18)
miR-200c-5p CGUCUUACCCAGCAGUGUUUGG (#13, mC) (SEQ ID NO: 19)
miR378a-3p ACUGGACUUGGAGUCAGAAGGC (SEQ ID NO: 20)
miR378d ACUGGACUUGGAGUCAGAAA (SEQ ID NO: 21)
miR378e ACUGGACUUGGAGUCAGGA (SEQ ID NO: 22)
miR378f ACUGGACUUGGAGCCAGAAG (SEQ ID NO: 23)
miR378h ACUGGACUUGGUGUCAGAUGG (SEQ ID NO: 24)
miR378i ACUGGACUAGGAGUCAGAAGG (SEQ ID NO: 25)
miR492 AGGACCUGCGGGACAAGAUUCUU (SEQ ID NO: 26)
miR3690 ACCUGGACCCAGCGUAGACAAAG (SEQ ID NO: 27)
miR4754 AUGCGGACCUGGGUUAGCGGAGU (SEQ ID NO: 28)
miR6861-5p ACUGGGUAGGUGGGGCUCCAGG (SEQ ID NO: 29)
miR3122 GUUGGGACAAGAGGACGGUCUU (SEQ ID NO: 30)
miR3131 UCGAGGACUGGUGGAAGGGCCUU (SEQ ID NO: 31)
miR6847-3p GGCUCAUGUGUCUGUCCUCUUC (SEQ ID NO: 32)
miR6887-3p UCCCCUCCACUUUCCUCCUAG (SEQ ID NO: 33)
miR-6887-5p UGGGGGGACAGAUGGAGAGGACA (SEQ ID NO: 34)
miR-16-1-3p CCAGUAUUAACUGUGCUGCUGA (SEQ ID NO: 35)
miR-34b-5p UAGGCAGUGUCAUUAGCUGAUUG (SEQ ID NO: 36)
miR369-5p AGAUCGACCGUGUUAUAUUCGC (SEQ ID NO: 37)
miR-431-5p UGUCUUGCAGGCCGUCAUGCAG (SEQ ID NO: 38)
miR-494-3p UGAAACAUACACGGGAAACCUC (SEQ ID NO: 39)
miR-519-d-5p CCUCCAAAGGGAAGCGCUUUCUGUU (SEQ ID NO: 40)
miR-3181 AUCGGGCCCUCGGCGCCGG (SEQ ID NO: 41)
miR-4435 AUGGCCAGAGCUCACACAGAGG (SEQ ID NO: 42)
miR-4467 UGGCGGCGGUAGUUAUGGGCUU (SEQ ID NO: 43)
miR-5581-5p AGCCUUCCAGGAGAAAUGGAGA (SEQ ID NO: 44)
miR-5587-5p AUGGUCACCUCCGGGACU (SEQ ID NO: 45)
miR-629-3p GUUCUCCCAACGUAAGCCCAGC (SEQ ID NO: 46)
miR-16-5p UAGCAGCACGUAAAUAUUGGCG (SEQ ID NO: 47)
miR-711 GGGACCCAGGGAGAGACGUAAG (SEQ ID NO: 48)
miR-3977 GUGCUUCAUCGUAAUUAACCUUA (SEQ ID NO: 49)
Consensus sequence 2 GGGAGGUGUG (SEQ ID NO: 50)
miR-6799-5p GGGGAGGUGUGCAGGGCUGG (SEQ ID NO: 51)
miR-3689d GGGAGGUGUGAUCUCACACUCG (SEQ ID NO: 52)
miR-3689a-3p CUGGGAGGUGUGAUAUCGUGGU (SEQ ID NO: 53)
miR-3689c CUGGGAGGUGUGAUAUUGUGGU (SEQ ID NO: 54)
miR-6825-5p UGGGGAGGUGUGGAGUCAGCAU (SEQ ID NO: 55)
Consensus sequence 3 CUCCCUGCCC (SEQ ID NO: 56)
miR-6756-3p UCCCCUUCCUCCCUGCCCAG (SEQ ID NO: 57)
miR-7113-3p CCUCCCUGCCCGCCUCUCUGCAG (SEQ ID NO: 58)
miR-6743-3p AGCCGCUCUUCUCCCUGCCCACA (SEQ ID NO: 59)
Consensus sequence 4 GACUUGGAGUCA (SEQ ID NO: 60)
miR-378c ACUGGACUUGGAGUCAGAAGAGUGG (SEQ ID NO: 61)
Consensus sequence 5 CCCUUUAGAGUGU (SEQ ID NO: 62)
miR-521 AACGCACUUCCCUUUAGAGUGU (SEQ ID NO: 63)
miR-517a-3p AUCGUGCAUCCCUUUAGAGUGU (SEQ ID NO: 64)
miR-522-3p AAAAUGGUUCCCUUUAGAGUGU (SEQ ID NO: 65)
miR-520g-3p ACAAAGUGCUUCCCUUUAGAGUGU (SEQ ID NO: 66)

## Claims

1. A method of analyzing a condition of a subject, comprising:
obtaining modification information on at least one type of RNA in a subject; and
analyzing a condition of the subject based on the modification information.

2. The method of claim 1, wherein the RNA comprises a microRNA.

3. The method of claim 1 or 2, wherein the modification comprises methylation of a microRNA.

4. The method of any one of claims 1 to 3, wherein the modification is m⁶A.

5. The method of any one of claims 1 to 4, wherein the modification information comprises at least one of modified location information and information on an amount of modified RNA.

6. The method of any one of claims 1 to 5, wherein the condition comprises cancer.

7. The method of claim 6, wherein the cancer comprises at least one of pancreatic cancer (e.g., early stage pancreatic cancer), liver cancer, gallbladder cancer, bile duct cancer, gastric cancer, and colon cancer.

8. The method of any one of claims 1 to 7, wherein the condition comprises responsiveness as to whether the subject is resistant to the anticancer agent.

9. The method of claim 8, wherein an agent for treating the subject and/or a treatment for the subject is indicated based on the responsiveness.

10. A system for determining a condition of a subject based on RNA modification information, comprising:
a measurement unit for measuring a modification condition of an RNA;
a calculation unit for calculating a modification condition on an RNA based on a result of the measurement;
and
an analysis/determination unit for analyzing/determining the condition of the subject based on the modification condition.

11. The system of claim 10, wherein the RNA comprises a microRNA.

12. The system of claim 10 or 11, wherein the modification comprises methylation of a microRNA.

13. The system of any one of claims 10 to 12, wherein the modification information comprises at least one of modified location information and information on an amount of modified RNA.

14. The system of any one of claims 10 to 13, wherein the condition comprises cancer.

15. The system of any one of claims 10 to 14, wherein the condition comprises responsiveness as to whether the subject is resistant to the anticancer agent.
